# EUROPEAN PATENT SPECIFICATION

(11) **EP 3 053 911 B1**
(45) Date of publication and mention of the grant of the patent: **22.01.2020**
(21) Application number: 14847067.7
(22) Date of filing: 30.09.2014
(51) Int. Cl.: C07D 211/62, A61K 31/4468, A61K 31/4525, A61K 31/495, A61K 31/496, A61P 3/04, A61P 3/06, A61P 3/10, A61P 9/12, A61P 43/00, C07D 215/12, C07D 295/16

(54) **ADIPONECTIN RECEPTOR-ACTIVATING COMPOUND**
ADIPONECTINREZEPTOR-AKTIVIERENDE VERBINDUNG
COMPOSÉ ACTIVATEUR DU RÉCEPTEUR DE L'ADIPONECTINE

(30) Priority: 30.09.2013 US 201361884638 P
(43) Date of publication of application: 10.08.2016
(73) Proprietor: The University of Tokyo, Tokyo 113-8654 (JP); Riken, Wako-shi, Saitama 351-0198 (JP)
(72) Inventor: KADOWAKI, Takashi, Tokyo 113-8654 (JP); YAMAUCHI, Toshimasa, Tokyo 113-8654 (JP); IWABU, Miki, Tokyo 113-8654 (JP); IWABU, Masato, Tokyo 113-8654 (JP); YOKOYAMA, Shigeyuki, Wako-shi Saitama 351-0198 (JP); HONMA, Teruki, Wako-shi Saitama 351-0198 (JP)
(74) Representative: Blodig, Wolfgang
(86) International application number: PCT/JP2014/076185
(87) International publication number: WO 2015/046595

(56) References cited:
- EP-A1- 0 120 558
- WO-A1-00/76513
- WO-A1-01/14333
- WO-A1-03/024929
- WO-A1-2005/061442
- WO-A1-2005/090330
- WO-A1-2007/087585
- WO-A1-2008/157844
- WO-A1-2010/075356
- WO-A1-2010/119992
- WO-A1-2012/016217
- WO-A2-2007/098086
- WO-A2-2008/016811
- JP-A- 2002 322 163
- MIKI OKADA-IWABU ET AL: "A small-molecule AdipoR agonist for type 2 diabetes and short life in obesity", NATURE, vol. 503, no. 7477, 30 October 2013 (2013-10-30), pages 493-499, XP055343097, United Kingdom ISSN: 0028-0836, DOI: 10.1038/nature12656
- ZHANG LISHA ET AL: "A High-Throughput Screen for Chemical Inhibitors of Exocytic Transport in Yeast", CHEMBIOCHEM - A EUROPEAN JOURNAL OF CHEMICAL BIOLOGY, WILEY VCH, WEINHEIM, DE, vol. 11, no. 9, 1 June 2010 (2010-06-01), pages 1291-1301, XP009148787, ISSN: 1439-4227, DOI: 10.1002/CBIC.200900681
- DATABASE REGISTRY [Online] CHEMICAL ABSTRACTS SERVICE, COLUMBUS, OHIO, US; 18 July 2013 (2013-07-18), Chemical Library - Supplier: Enamine: XP002767086, Database accession no. 1445210-76-3
- DATABASE REGISTRY [Online] CHEMICAL ABSTRACTS SERVICE, COLUMBUS, OHIO, US; 17 July 2013 (2013-07-17), Chemical Library - Supplier: Enamine: XP002767087, Database accession no. 1445150-93-5
- DATABASE REGISTRY [Online] CHEMICAL ABSTRACTS SERVICE, COLUMBUS, OHIO, US; 16 July 2013 (2013-07-16), Chemical Library - Supplier: Enamine: XP002767088, Database accession no. 1444382-77-7
- DATABASE REGISTRY [Online] CHEMICAL ABSTRACTS SERVICE, COLUMBUS, OHIO, US; 11 April 2013 (2013-04-11), Chemical Library - Supplier: Enamine: XP002767089, Database accession no. 1428091-77-3
- DATABASE REGISTRY [Online] CHEMICAL ABSTRACTS SERVICE, COLUMBUS, OHIO, US; 2 August 2012 (2012-08-02), Chemical Library - Supplier: Ukrorgsyntez Ltd.: XP002767090, Database accession no. 1385372-49-5
- DATABASE REGISTRY [Online] CHEMICAL ABSTRACTS SERVICE, COLUMBUS, OHIO, US; 29 May 2011 (2011-05-29), Chemical Library - Supplier: FCH Group: XP002767091, Database accession no. 1302257-50-6
- DATABASE REGISTRY [Online] CHEMICAL ABSTRACTS SERVICE, COLUMBUS, OHIO, US; 5 May 2011 (2011-05-05), Chemical Library - Supplier: FCH Group: XP002767092, Database accession no. 1290278-99-7
- DATABASE REGISTRY [Online] CHEMICAL ABSTRACTS SERVICE, COLUMBUS, OHIO, US; 24 October 2008 (2008-10-24), Chemical Library - Supplier: ChemBridge Corporation: XP002767093, Database accession no. 1065601-12-8
- DATABASE REGISTRY [Online] CHEMICAL ABSTRACTS SERVICE, COLUMBUS, OHIO, US; 17 September 2008 (2008-09-17), XP002767094, Database accession no. 1049717-36-3
- DATABASE REGISTRY [Online] CHEMICAL ABSTRACTS SERVICE, COLUMBUS, OHIO, US; 9 October 2007 (2007-10-09), Chemical Library - Supplier: Enamine: XP002767095, Database accession no. 949745-75-9
- DATABASE REGISTRY [Online] CHEMICAL ABSTRACTS SERVICE, COLUMBUS, OHIO, US; 21 August 2007 (2007-08-21), Chemical Library - Supplier: Scientific Exchange, Inc.: XP002767096, Database accession no. 945162-05-0
- DATABASE REGISTRY [Online] CHEMICAL ABSTRACTS SERVICE, COLUMBUS, OHIO, US; 2 March 2007 (2007-03-02), Chemical Library - Supplier: Aurora Fine Chemicals: XP002767097, Database accession no. 924416-43-3
- DATABASE REGISTRY [Online] CHEMICAL ABSTRACTS SERVICE, COLUMBUS, OHIO, US; 28 June 2004 (2004-06-28), Chemical Library - Supplier: ALDRICH Chemical Company, Inc.: XP002767098, Database accession no. 717871-36-8
- DATABASE REGISTRY [Online] CHEMICAL ABSTRACTS SERVICE, COLUMBUS, OHIO, US; 29 May 2003 (2003-05-29), XP002767099, Database accession no. 521975-30-4
- DATABASE REGISTRY [Online] CHEMICAL ABSTRACTS SERVICE, COLUMBUS, OHIO, US; 3 December 2002 (2002-12-03), XP002767100, Database accession no. 474970-07-5
- DATABASE REGISTRY [Online] CHEMICAL ABSTRACTS SERVICE, COLUMBUS, OHIO, US; 12 June 2002 (2002-06-12), Chemical Library - Supplier: ChemBridge Corporation: XP002767101, Database accession no. 428846-37-1
- DATABASE REGISTRY [Online] CHEMICAL ABSTRACTS SERVICE, COLUMBUS, OHIO, US; 24 September 2001 (2001-09-24), Chemical Library - Supplier: ChemBridge Corporation: XP002767102, Database accession no. 358363-57-2
- DATABASE REGISTRY [Online] 13 October 2008 'BENZAMIDE, 3-CHLORO-N-CYCLOPENTYL-4-[[1-[[5-(METHOXYME THYL)-2- FURANYL]METHYL]-4-PIPERIDINYL]OXY]- (CA INDEX NAME)', XP055332820 Retrieved from STN Database accession no. 1060428-05-8
- DATABASE REGISTRY [Online] 14 October 2008 'BENZAMIDE, 3-CHLORO-4-[[1-(2,2-DIMETHYLPROPYL)-4-PIPER IDINYL]OXY]-N-(3- ETHOXYPROPYL)- (CA INDEX NAME)', XP055353888 Retrieved from STN Database accession no. 1061037-69-1
- DATABASE REGISTRY [Online] 02 November 2008 'BENZAMIDE, 3-CHLORO-4-[[1-[(2-FLUORO-5-METHOXYPHENYL)M ETHYL]-4- PIPERIDINYL]OXY]-N-(2-METHOXYETHYL)- (CA INDEX NAME)', XP055353893 Retrieved from STN Database accession no. 1069855-00-0
- DATABASE REGISTRY [Online] 13 October 2008 'BENZAMIDE, 4-[[1-(3-FURANYLMETHYL)-4-PIPERIDINYL]OXY]- N-(4- PYRIDINYLMETHYL)- (CA INDEX NAME)', XP055353895 Retrieved from STN Database accession no. 1060384-26-0
- DATABASE REGISTRY [Online] 13 October 2008 'BENZAMIDE, 3-CHLORO-4-[[1-[(2-FLUORO-4-METHOXYPHENYL)M ETHYL]-4- PIPERIDINYL]OXY]-N-(2-METHOXYETHYL)- (CA INDEX NAME)', XP055353898 Retrieved from STN Database accession no. 1060425-92-4
- DATABASE REGISTRY [Online] 24 October 2008 'BENZAMIDE, 3-CHLORO-N-(2-METHOXYETHYL)-4-[[1-(1-METHYL -2-PHENOXYETHYL)-4- PIPERIDINYL]OXY]- (CA INDEX NAME)', XP055353900 Retrieved from STN Database accession no. 1065552-59-1
- DATABASE REGISTRY [Online] 13 October 2008 'BENZAMIDE, 3-CHLORO-N-(2-METHOXYETHYL)-4-[[1-[(5-METHY L-2-THIENYL)METHYL]- 4-PIPERIDINYL]OXY]- (CA INDEX NAME)', XP055353905 Retrieved from STN Database accession no. 1060378-36-0
- DATABASE REGISTRY [Online] 14 October 2008 'BENZAMIDE, 4-[[1-[(2-METHYLPHENYL)METHYL]-4-PIPERIDINY L]OXY]-N-(2- PYRIDINYLMETHYL)- (CA INDEX NAME)', XP055353909 Retrieved from STN Database accession no. 1060938-35-3
- DATABASE REGISTRY [Online] 02 November 2008 'BENZAMIDE, 3-CHLORO-4-[[1-[(4-ETHOXYPHENYL)METHYL]-4-P IPERIDINYL]OXY]-N-(2- METHOXYETHYL)- (CA INDEX NAME)', XP055353912 Retrieved from STN Database accession no. 1069626-66-9
- DATABASE REGISTRY [Online] 24 October 2008 'BENZAMIDE, 3-CHLORO-4-[[1-(2,2-DIMETHYLPROPYL)-4-PIPER IDINYL]OXY]-N-[2-(4- PYRIDINYL)ETHYL]- (CA INDEX NAME)', XP055353914 Retrieved from STN Database accession no. 1065611-82-6
- DATABASE REGISTRY [Online] 16 April 2009 'BENZAMIDE, N-(1-METHYLETHYL)-N-[(1-METHYL-1H-PYRAZOL-4 -YL)METHYL]-4-[[1-(2- PYRIDINYLMETHYL)-4-PIPERIDINYL]OXY]- (CA INDEX NAME)', XP055353919 Retrieved from STN Database accession no. 1135318-71-6
- DATABASE REGISTRY [Online] 27 October 2008 'BENZAMIDE, 3-CHLORO-N-CYCLOPENTYL-4-[[1-[(3-HYDROXYPHE NYL)METHYL]-4- PIPERIDINYL]OXY]- (CA INDEX NAME)', XP055353921 Retrieved from STN Database accession no. 1066883-94-0
- DATABASE REGISTRY [Online] 21 March 2010 'BENZAMIDE, N-[2-(1R,2R,4R)-BICYCLO[2.2.1]HEPT-5-EN-2-Y LETHYL]-4-[[1-(2- PYRIDINYLMETHYL)-4-PIPERIDINYL]OXY]-, REL- (CA INDEX NAME)', XP055353924 Retrieved from STN Database accession no. 1212101-72-8
- DATABASE REGISTRY [Online] 27 October 2008 'BENZAMIDE, N-[2-(4-CHLOROPHENYL)ETHYL]-4-[[1-(2-PYRIDI NYLMETHYL)-4- PIPERIDINYL]OXY]- (CA INDEX NAME)', XP055353928 Retrieved from STN Database accession no. 1066995-66-1
- DATABASE REGISTRY [Online] 04 November 2008 'BENZAMIDE, 4-[[1-(2-BENZOFURANYLMETHYL)-4-PIPERIDINYL] OXY]-3-CHLORO-N-(2- METHOXYETHYL)- (CA INDEX NAME)', XP055353939 Retrieved from STN Database accession no. 1070688-89-9
- DATABASE REGISTRY [Online] 02 November 2008 'BENZAMIDE, 3-CHLORO-N-CYCLOPENTYL-4-[[1-(3-FURANYLMETH YL)-4- PIPERIDINYL]OXY]- (CA INDEX NAME)', XP055353942 Retrieved from STN Database accession no. 1069620-31-0
- DATABASE REGISTRY [Online] 02 November 2008 'BENZAMIDE, N-(2-FURANYLMETHYL)-N-2-PROPYN-1-YL-4-[[1-( 2-PYRIDINYLMETHYL)-4- PIPERIDINYL]OXY]- (CA INDEX NAME)', XP055353944 Retrieved from STN Database accession no. 1069716-06-8
- DATABASE REGISTRY [Online] 04 November 2008 'BENZAMIDE, 4-[[1-(2,1,3-BENZOXADIAZOL-4-YLMETHYL)-4-PI PERIDINYL]OXY]-3- CHLORO-N-(2-METHOXYETHYL)- (CA INDEX NAME)', XP055353949 Retrieved from STN Database accession no. 1070742-57-2
- DATABASE REGISTRY [Online] 02 November 2008 'BENZAMIDE, 3-CHLORO-N-CYCLOPENTYL-4-[[1-[(2-METHOXYPHE NYL)METHYL]-4- PIPERIDINYL]OXY]- (CA INDEX NAME)', XP055353957 Retrieved from STN Database accession no. 1069517-18-5
- DATABASE REGISTRY [Online] 02 November 2008 'BENZAMIDE, N-2-BUTYN-1-YL-N-(2-FURANYLMETHYL)-4-[[1-(2 -PYRIDINYLMETHYL)-4- PIPERIDINYL]OXY]- (CA INDEX NAME)', XP055353961 Retrieved from STN Database accession no. 1069611-11-5
- DATABASE REGISTRY [Online] 04 November 2008 'BENZAMIDE, 3-CHLORO-N-CYCLOPENTYL-4-[[1-[(6-METHYL-2-P YRIDINYL)METHYL]-4- PIPERIDINYL]OXY]- (CA INDEX NAME)', XP055353964 Retrieved from STN Database accession no. 1070722-55-2
- DATABASE REGISTRY [Online] 04 November 2008 'BENZAMIDE, 3-CHLORO-4-[[1-[(4-ETHYLPHENYL)METHYL]-4-PI PERIDINYL]OXY]-N-(2- METHOXYETHYL)- (CA INDEX NAME)', XP055353967 Retrieved from STN Database accession no. 1070474-65-5
- DATABASE REGISTRY [Online] 31 July 2002 'METHANONE, (3,4-DIMETHOXYPHENYL)[4-[[4-(TRIFLUOROMETHY L)PHENYL]METHYL]-1- PIPERAZINYL]- (CA INDEX NAME)', XP055353970 Retrieved from STN Database accession no. 441314-59-6
- DATABASE REGISTRY [Online] 07 June 2012 '1-PIPERAZINEPROPANAMIDE, N-CYCLOPROPYL-4-(3-METHOXYBENZOYL)- (CA INDEX NAME)', XP055353973 Retrieved from STN Database accession no. 1376314-21-4
- PESSOA-MAHANA H ET AL: "SYNTHESIS OF 4-ARYLPIPERAZINE DERIVATIVES OF MOCLOBEMIDE: POTENTIAL ANTIDEPRESSANTS WITH A DUAL MODE OF ACTION", SYNTHETIC COMMUNICAT, TAYLOR & FRANCIS INC, PHILADELPHIA, PA; US, vol. 34, no. 14, 1 January 2004 (2004-01-01), pages 2513-2521, XP009083364, ISSN: 0039-7911, DOI: 10.1081/SCC-200025581

### SEQUENCE LISTING

<110> THE UNIVERSITY OF TOKYO
   RIKEN
<120> ADIPONECTIN RECEPTOR-ACTIVE COMPOUNDS
<130> TU0010
<150> US 61/884,638
   <151> 2013-09-30
<160> 1
<170> PatentIn version 3.5
<210> 1
   <211> 23
   <212> DNA
   <213> Artificial Sequence
<220>
   <223> an artificially synthesized sense strand of siRNA
<220>
   <221> misc_RNA
   <222> (1)..(21)
   <223> RNA
<400> 1
   gagacuggca acaucuggac att 23

## Description

### Technical Field

The present invention relates to an activator of adiponectin receptor compound or a salt thereof.

### Background Art

The number of overweight individuals worldwide has grown dramatically, leading to a stepwise increase of obesity-related health problems associated with increased morbidity and mortality. Insulin resistance is a common feature of obesity and predisposes the affected individuals to a variety of pathologies, including type 2 diabetes and cardiovascular diseases.

Adiponectin is an antidiabetic and antiatherogenic adipokine. Plasma adiponectin levels are decreased in obesity, insulin resistance and type 2 diabetes (Non-Patent Document 1). Replenishment of adiponectin has been known to exhibit glucose lowering effect and ameliorate insulin resistance in mice (Non-Patent Documents 2-4). This insulin sensitizing effect of adiponectin appears to be mediated, at least in part, by an increase in fatty acid oxidation via activation of AMP-activated protein kinase (AMPK) (Non-Patent Documents 5-7) and also via peroxisome proliferator-activated receptor (PPAR) α (Non-Patent Documents 8-9) .

It is elucidated that Adiponectin exerts its action via adiponectin receptor (hereinafter, also referred to as "AdipoR"). Furthermore, it has been reported that AdipoR has two subtypes of AdipoR1 and AdipoR2; AdipoR1 activates the AMPK pathways and AdipoR2 activates the PPARα pathways (Non-Patent Document 10).

In skeletal muscle, AdipoR1 is predominantly expressed (Non-Patent Document 11) and activates AMPK and PPARγ coactivator (PGC)-1α as well as Ca²⁺ signaling pathways, which have also been shown to be activated by exercise. Exercise has been reported to have beneficial effects on obesity-related diseases such as type 2 diabetes, and could contribute to healthy longevity. Liver expresses AdipoR1 and AdipoR2, both of which have roles in the regulation of glucose and lipid metabolism, inflammation, and oxidative stress (Non-Patent Document 10).

Thus, AdipoR activating compound is efficacious for prevention, treatment or amelioration of diseases, against which activation of AdipoR is effective and which includes reduced insulin sensitivity, diabetic mellitus, obesity, metabolic syndrome, dyslipidemia, mitochondrial dysfunction, arteriosclerosis or the like by exhibiting adiponectin-like activities.

There have been some reports related to a compound activating AMPK pathways in cells (Patent Documents 1-5), but the development of more effective medicaments has been desired.

### Citation List

### Patent Document

Patent Document 1: JP Patent Publication (Kohyo) No. 2010-514788 A
Patent Document 2: JP Patent Publication (Kohyo) No. 2011-503210 A
Patent Document 3: JP Patent Publication (Kohyo) No. 2011-506480 A
Patent Document 4: JP Patent Publication (Kohyo) No. 2011-527665 A
Patent Document 5: JP Patent Publication (Kohyo) No. 2012-516349 A

### Non-Patent Document

Non-Patent Document 1: Hotta, K. , et al., Plasma concentrations of a novel, adipose-specific protein, adiponectin, in type 2diabetic patients. Arterioscler. Thromb. Vasc. Biol., 20, 1595-1599 (2000)
Non-Patent Document 2: Yamauchi, T., et al., The fat-derived hormone adiponectin reverses insulin resistance associated with both lipoatrophy and obesity. Nature Med., 7, 941-946 (2001)
Non-Patent Document 3: Berg, A.H., Combs, T.P., Du, X., Brownlee, M., & Scherer, P.E., The adipocyte secreted protein Acrp30 enhance shepatic insulin action. Nature Med., 7, 947-953 (2001)
Non-Patent Document 4: Fruebis, J., et al., Proteolytic cleavage product of 30-k Da adipocyte complement related protein increases fatty acid oxidation in muscle and causes weight loss in mice. Proc. Natl Acad. Sci. USA, 98, 2005-2010 (2001)
Non-Patent Document 5: Yamauchi, T., et al., Adiponectin stimulates glucose utilization and fatty-acid oxidation by activating AMP-activated protein kinase. Nature Med., 8, 1288-1295 (2002)
Non-Patent Document 6: Tomas, E., et al., Enhanced muscle fat oxidation and glucose transport by ACRP30 globular domain: acetyl-CoA carboxylase inhibition and AMP-activated protein kinase activation. Proc. Natl Acad. Sci. USA, 99, 16309-16313 (2002)
Non-Patent Document 7: Kahn, B.B., Alquier, T., Carling, D., & Hardie, D.G., AMP-activated protein kinase: ancient energy gauge provides clues to modern understanding of metabolism. Cell Metab., 1, 15-25 (2005)
Non-Patent Document 8: Kersten, S., Desvergne, B., & Wahli, W., Roles of PPARs in health and disease. Nature, 405, 421-424 (2000)
Non-Patent Document 9: Yamauchi, T., et al., Globular adiponectin protected ob/ob mice from diabetes and apoE deficient mice from atherosclerosis. J. Biol. Chem., 278, 2461-2468 (2003)
Non-Patent Document 10: Yamauchi, T., et al., Targeted disruption of AdipoR1 and AdipoR2 causes abrogation of adiponectin binding and metabolic actions. Nature Med., 13, 332-339 (2007)
Non-Patent Document 11: Iwabu, M., et al, Adiponectin and AdipoR1 regulated PGC-lalpha and mitochondria by Ca(2+) and AMPK/SIRT1. Nature, 464, 1313-1319 (2010)
Okada-Iwabu, M. et al, Nature, 503, 493-499 (2013) discloses small-molecule AdipoR agonists.
WO2012/016217 A1 discloses AMPK-activating heterocyclic compounds.

### Summary of Invention

### Technical Problem

The present invention provides an activator of AdipoR that activates both of AdipoR1 and AdipoR2 . The present invention also provides a medicament for use in preventing, or treating a symptom, disease, or disorder due to decreased production of adiponectin, decreased adiponectin levels in blood or decreased activation of AdipoR.

### Means for Solution

The present inventors have extensively studied above-mentioned problems, and as a result, have found that a compound of the formula (1b) has an excellent AdipoR activating activity.

That is, the present invention relates to the following 1) to 11) .
1) A compound of the formula (1b) or a salt thereof: 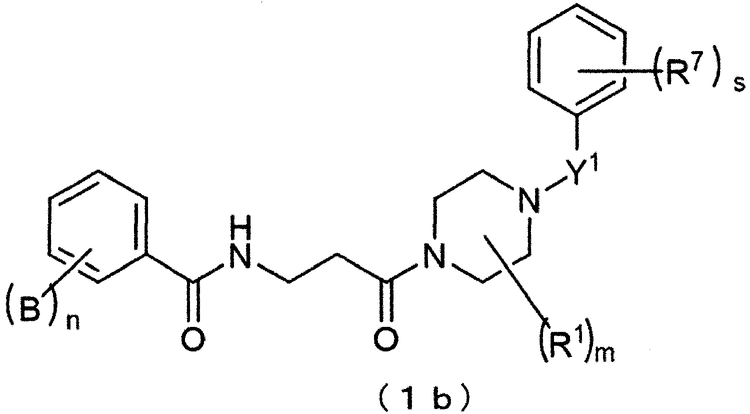 wherein
   Y¹ represents -(CHR²)ₐ- (wherein R² represents H or a C₁₋₃ alkyl group, and a represents 1, or R² represents H or a C₁₋₇ alkyl group, and a represents 2) or -CO-;
   R¹ represents a C₁₋₇ alkyl group, wherein a plurality of R¹s may be the same or different from each other;
   m represents an integer of 0 to 4
   B represents -CO-R⁴ or -O-R⁴ (wherein R⁴ represents a C₁₋₇ alkyl group, or a substituted or unsubstitutedphenyl group, a substituted or unsubstituted pyridyl group), -CONR⁵R⁶ (wherein R⁵ represents H, a C₃₋₇ cycloalkyl group, a C₁₋₄ alkoxy C₁₋₄ alkyl group, a norbornenyl C₁₋₄ alkyl group, or Ar²-C₁₋₄ alkyl group (in which Ar² is a substituted or unsubstituted aryl group or heteroaryl group), R⁶ represents H, a C₁₋₇ alkyl group, a C₂₋₄ alkenyl group or C₂₋₄ alkynyl group), a C₁₋₇ alkyl group, a C₃₋₇ cycloalkyl group, a halo C₁₋₇ alkyl group, a phenyl group, a halogen atom, -NO₂, or a group shown as follows: 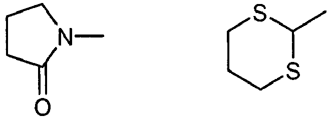
   n represents an integer of 0 to 3 (if n is 2 or more, Bs may be the same or different from each other);
   R⁷ represents a C₁₋₄ alkyl group, a halo C₁₋₄ alkyl group, a halogen atom, a C₁₋₄ alkoxy group, or a hydroxyl group; and
   s represents an integer of 0 to 3 (if it is 2 or more, then a plurality of R⁷ may be the same or different from each other).
2) The compound or salt thereof according to 1), wherein Y¹ represents -CH₂-, and wherein B is a C₁₋₇ alkyl group; a C₃₋₇ cycloalkyl group; a halogen atom or -NO₂.
3) A compound of the following formula or a salt thereof: 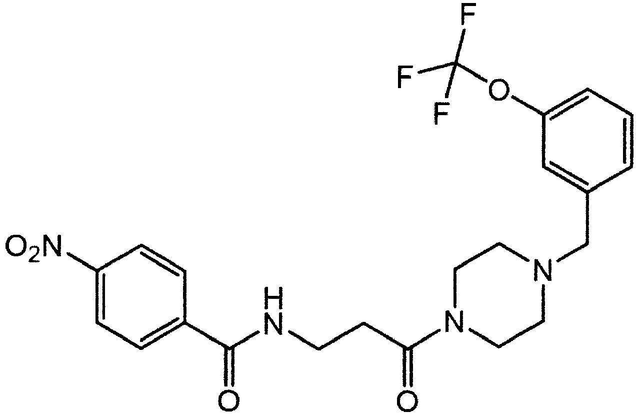
4) A medicament comprising the compound according to any one of 1) to 3) or a salt thereof, and a pharmaceutically acceptable carrier.
5) The medicament according to 4) for use in preventing, treating or ameliorating a symptom or disease due to decreased production of adiponectin or decreased activation of adiponectin receptor.
6) The medicament according to 5), wherein the symptom or disease due to decreased production of adiponectin or deceased activation of adiponectin receptor is type II diabetes, hypertension, lipid metabolism disorder, mitochondrial dysfunction, lifestyle-related disease, malignant tumor due to lifestyle-related disease or obesity.
7) An activator of adiponectin receptor comprising the compound or a salt thereof according to any one of 1) to 3) as an active ingredient.
8) The activator of adiponectin receptor according to 7), wherein the adiponectin receptor is AdipoR1 or AdipoR2.
9) The compound according to any one of 1) to 3) or a salt thereof for use in prevention, amelioration, or treatment of symptoms or diseases caused by reduced adiponectin production or adiponectin receptor activities.
10) The compound or a salt thereof for use according to 9), wherein the symptom or, disease caused by decreased production of adiponectin or decreased activation of adiponectin receptor is type II diabetes, hypertension, lipid metabolism disorder, mitochondrial dysfunction, lifestyle-related disease, malignant tumor due to lifestyle-related disease, or obesity.
11) The compound according to any one of 1) to 3) or a salt thereof for use in activation of adiponectin receptors.

### Advantageous Effects of Invention

The compound or a salt thereof according to the present invention has an activation effect for AdipoR. Accordingly, the compound or a salt thereof according to the present invention is useful as an agent for preventing, treating or ameliorating a symptom, disease, or disorder due to decreased activity of AdipoR (AdipoR1 or AdipoR2) such as hyperglycemia; glucose intolerance; decreased insulin sensitivity (such as insulin resistance); type II diabetes; hypertension; arteriosclerosis, atherosclerosis, lipid metabolism disorders such as hyperlipemia and fatty liver; mitochondrial dysfunction; obesity; metabolic syndrome and lifestyle-related disease; and malignant tumor due to lifestyle-related disease.

### Brief Description of Drawings

[Figure 1] Affinity for AdipoR1 and AdipoR2, and induction effect of phosphorylation of AMPK in skeletal muscle and liver. j, k; Surface plasmon resonance measuring AdipoRon binding to AdipoR1 and AdipoR2. 1, m; Phosphorylation and amount of AMPK in skeletal muscle (1) or in liver (m). All values are presented as mean ±s.e.m. 1, m, n=3 each; *P<0.05 and **P<0.01. NS, not significant.

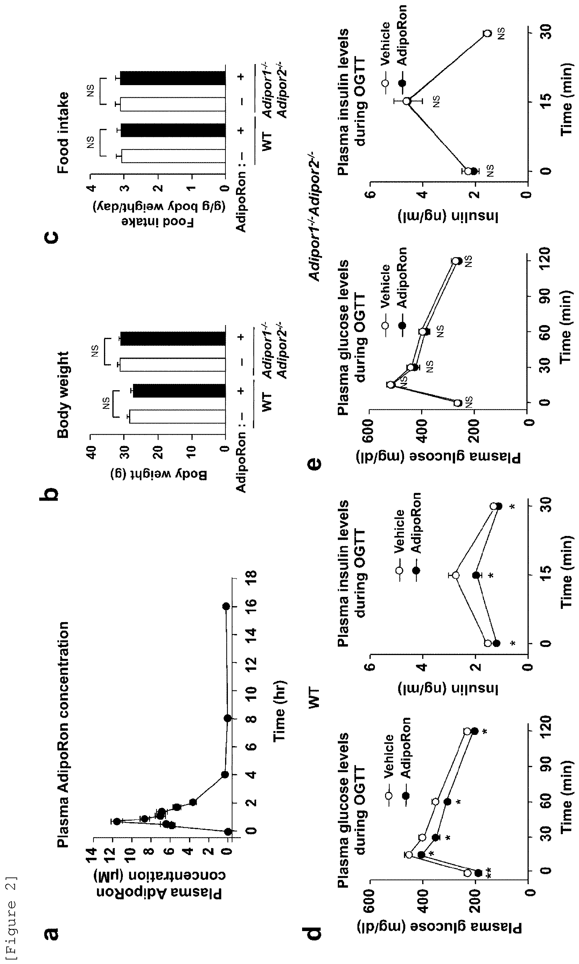

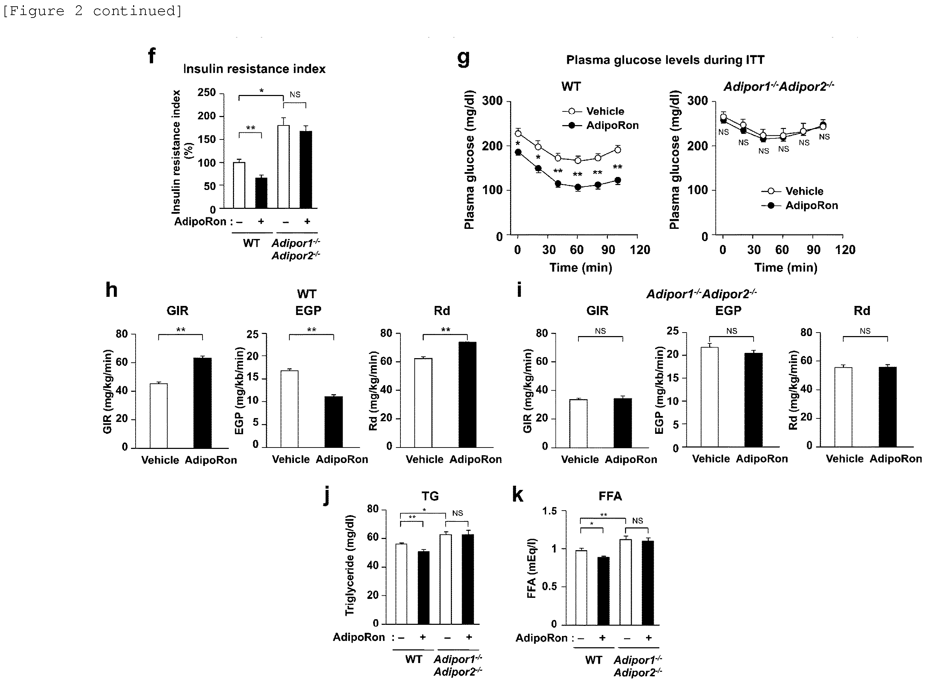

[Figure 2] Effect on glucose intolerance, insulin resistance and dyslipidemia. a to g; Plasma compound concentration (a), body weight (b), food intake (c), plasma glucose (d, e, g), plasma insulin (d , e) and insulin resistance (f) during oral glucose tolerance test (OGTT) (1.0 g glucose per kg body weight) (d, e) or during insulin tolerance test (ITT) (0.5U insulin per kg body weight) (g) in wild-type (WT) and Adipor1^{-/-} Adipor2^{-/-} double knockout mice orally administered with or without AdipoRon (50 mg per kg body weight). h, i, Glucose infusion rate (GIR), endogenous glucose production (EGP) and rates of glucose disposal (Rd) during hyperinsulinemic euglycemic clamp study in wild type and Adipor1^{-/-} Adipor2^{-/-} double-knockout mice, orally administered with or without AdipoRon (50 mg per kg body weight) . j, k, Plasma triglyceride (j) and free fatty acid (FFA) (k) in wild type and Adipor1^{-/-} Adipor2^{-/-} double-knockout mice, treated with or without AdipoRon (50 mg per kg body weight). All values are presented as mean ±s.e.m. a, n=12-32; b-g, j, k, n=10 each; h, i, n=5 each. *P<0.05 and **P<0.01 compared to control or as indicated. NS, not significant.

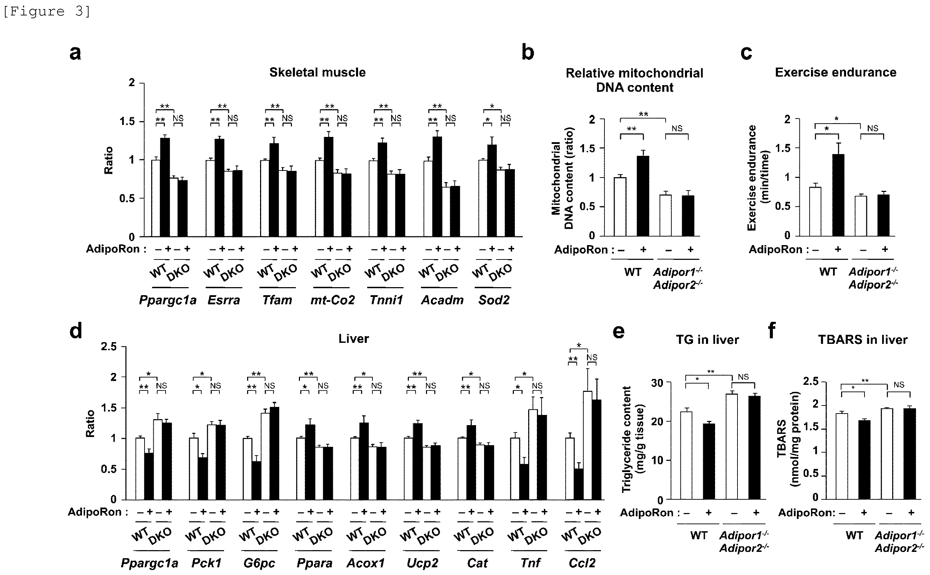

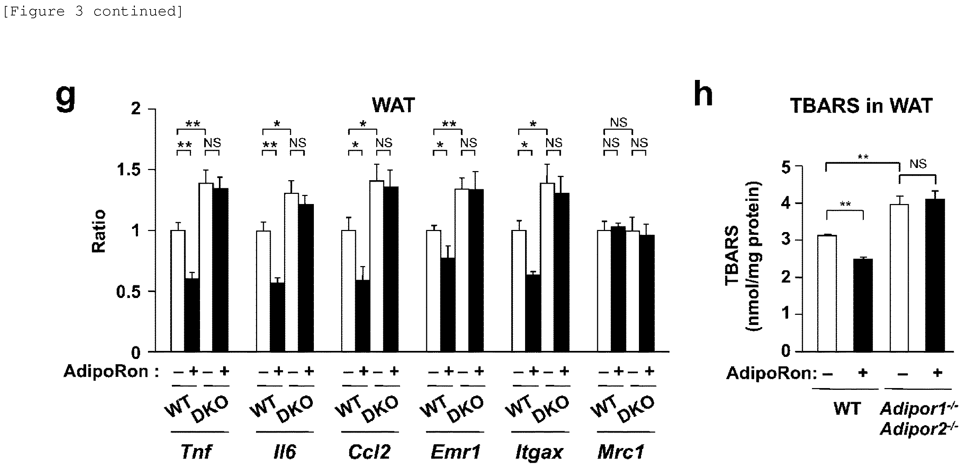

[Figure 3] Effect on mitochondrial biogenesis in muscle, tissue triglyceride content in liver, oxidative stress in liver and WAT, and inflammation. a-h; Ppargcla, Esrra, Tfam, mt-Co2, Tnni1, Acadm and Sod2 mRNA levels (a), mitochondrial content as assessed by mitochondrial DNA copy number in skeletal muscle (b), exercise endurance (c), Ppargcla, Pck1, G6pc, Ppara, Acox1, Ucp2, Cat, Tnf and Ccl2 mRNA levels (d), tissue triglyceride content (e), TBARS in liver (f), Tnf, Il6, Ccl2, Emr1, Itgax and Mrc1 mRNA levels (g) and TBARS in WAT (h). Results from wild-type (WT) and Adipor1^{-/-}Adipor2^{-/-}double-knockout mice (DKO) orally administered with or without AdipoRon (50 mg per kg body weight) . All values are presented as mean ± s.e.m. a, b, d-h, n=10 each; c, n=5 each; *P<0.05 and **P<0.01 compared to control or as indicated. NS, not significant.

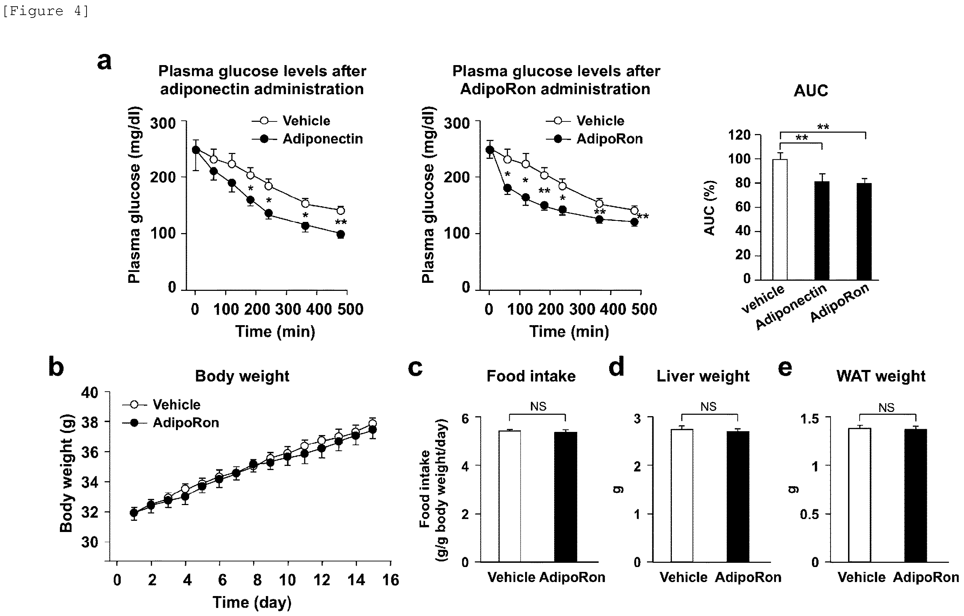

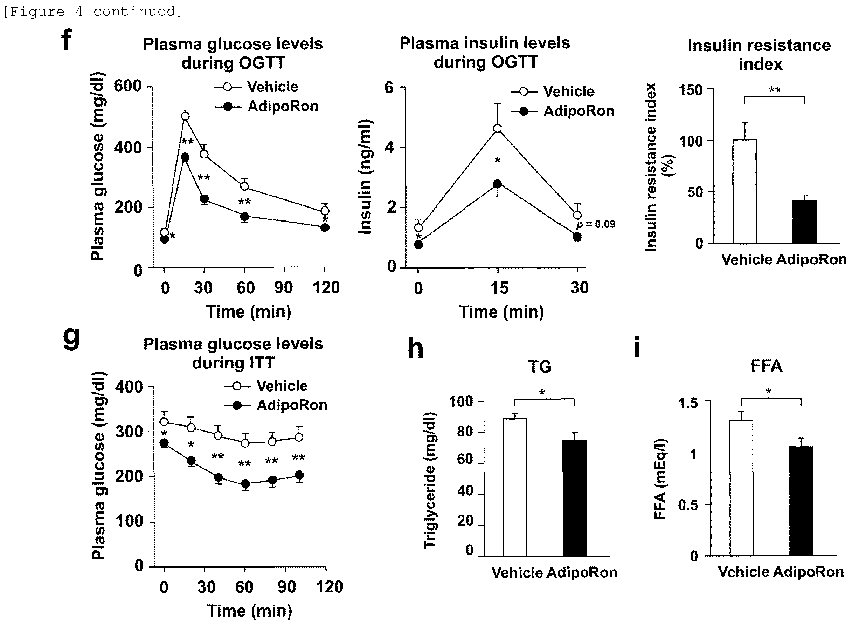

[Figure 4] Effect on insulin resistance, diabetes, and dyslipidemia in db/db mice. a; Plasma glucose level after intraperitoneal injection of adiponectin (30 *µ*g per 10 g body weight) (left) or after oral administration of AdipoRon (50 mg per kg body weight) (middle), area under curve (AUC) of left and middle panels is shown on the right. b-i, body weight (b), food intake (c), liver weight (d), WAT weight (e), plasma glucose (f left, g), plasma insulin (f middle) and insulin resistance index (f right) during oral glucose tolerance test (OGTT) (1.0 g glucose per kg body weight) (f) or during insulin tolerance test (ITT) (0.75 U insulin per kg body weight) (g), plasma triglyceride (h) and free fatty acid (FFA) (i) in db/db mice under normal chow, orally administered with or without AdipoRon (50 mg per kg body weight) . All values are presented as mean ± s.e.m. a, n = 6-7; b-i, n=10 each from 2-3 independent experiments, *P<0.05 and **P<0.01 compared to control or as indicated. NS, not significant.

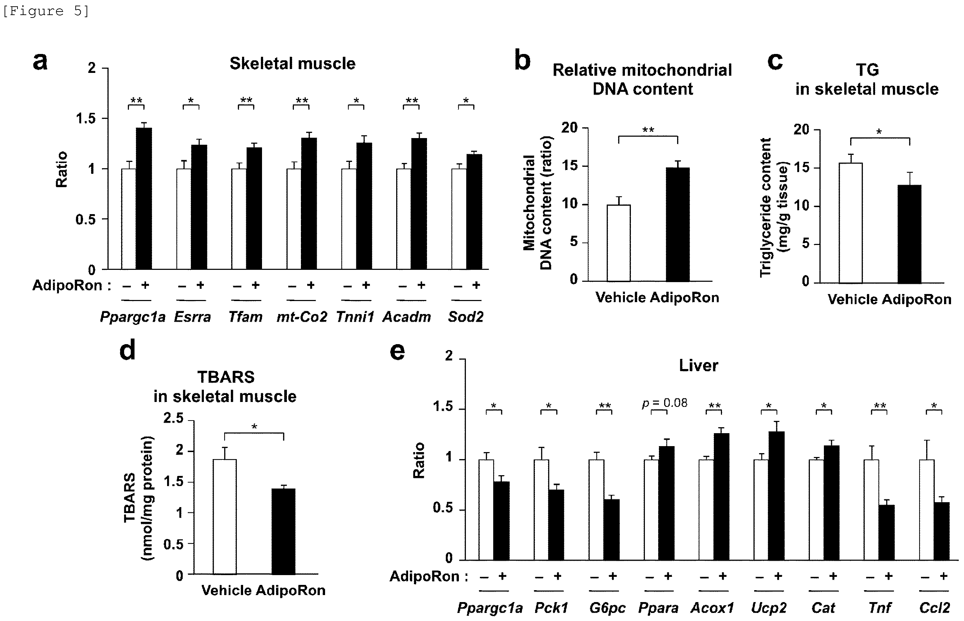

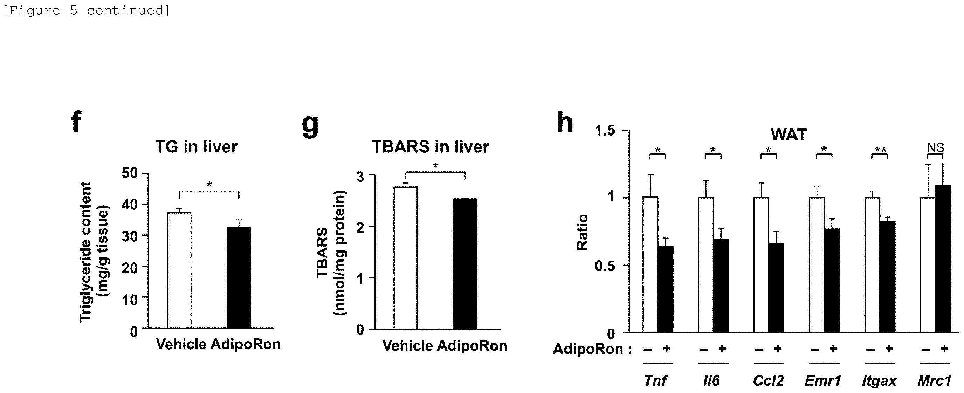

[Figure 5] Effect on mitochondrial biogenesis in the muscle, tissue triglyceride content in muscle and liver, oxidative stress, inflammation in liver and WAT in db/db mouse. a-h; Ppargcla, Esrra, Tfam, mt-Co2, Tnni1, Acadm and Sod2 mRNA levels (a), and mitochondrial content as assessed by mitochondrial DNA copy number (b), tissue triglyceride content (c), TBARS in skeletal muscle (d), Ppargcla, Pck1, G6pc, Ppara, Acoxl, Ucp2, Cat, Tnf and Ccl2 mRNA levels (e), tissue triglyceride content (f), and TBARS in liver (g), and Tnf, Il6, Ccl2, Emr1, Itgax and Mrcl mRNA levels in WAT (h), from db/db mice under normal chow, orally administered with or without AdipoRon (50 mg per kg body weight). All values are presented as mean ± s.e.m. n=10, *P<0.05 and **P<0.01 compared to control or as indicated. NS, not significant.
[Figure 6] Effect on lifespan of insulin sensitive, glucose tolerant and diabetic obese mice. a-c; Kaplan-Meier survival curves for the wild-type, Adipor1^{-/-}, Adipor2^{-/-}, and Adipor1^{-/-}Adipor2^{-/-} knockout mice on a normal chow (a) (n=50, 32, 29 and 35, respectively) or high-fat diet (b) (n=47, 33, 35 and 31, respectively), and for db/db mice orally administered with or without AdipoRon (30 mg per kg body weight) under normal chow or high-fat diet, n=20 each (c). P values were derived from log-rank calculations.
[Figure 7] Effects of Compound 74 (No.112254) on insulin resistance and glucose intolerance. Plasma glucose (d, e, g), plasma insulin (d, e) and insulin resistance (f).
[Figure 8] LCMS analysis chart of Compound 1
[Figure 9] LCMS analysis chart of Compound 2
[Figure 10] LCMS analysis chart of Compound 3
[Figure 11] LCMS analysis chart of Compound 4
[Figure 12] LCMS analysis chart of Compound 5
[Figure 13] LCMS analysis chart of Compound 6
[Figure 14] LCMS analysis chart of Compound 7
[Figure 15] LCMS analysis chart of Compound 8
[Figure 16] LCMS analysis chart of Compound 9
[Figure 17] LCMS analysis chart of Compound 10
[Figure 18] LCMS analysis chart of Compound 11
[Figure 19] LCMS analysis chart of Compound 12
[Figure 20] LCMS analysis chart of Compound 13
[Figure 21] LCMS analysis chart of Compound 14
[Figure 22] NMR analysis chart of Compound 14
[Figure 23] LCMS analysis chart of Compound 15
[Figure 24] LCMS analysis chart of Compound 16
[Figure 25] LCMS analysis chart of Compound 17
[Figure 26] LCMS Analysis chart of Compound 18
[Figure 27] NMR analysis chart of Compound 18
[Figure 28] LCMS analysis chart of Compound 19
[Figure 29] LCMS analysis chart of Compound 20
[Figure 30] LCMS analysis chart of Compound 21
[Figure 31] LCMS analysis chart of Compound 22
[Figure 32] LCMS analysis chart of Compound 23
[Figure 33] LCMS analysis chart of Compound 24
[Figure 34] LCMS analysis chart of Compound 25
[Figure 35] LCMS analysis chart of Compound 26
[Figure 36] NMR analysis chart of Compound 26
[Figure 37] LCMS analysis chart of Compound 27
[Figure 38] LCMS analysis chart of Compound 28
[Figure 39] LCMS analysis chart of Compound 29
[Figure 40] LCMS analysis chart of Compound 30
[Figure 41] LCMS analysis chart of Compound 31
[Figure 42] LCMS analysis chart of Compound 32
[Figure 43] LCMS analysis chart of Compound 33
[Figure 44] LCMS analysis chart of Compound 34
[Figure 45] LCMS analysis chart of Compound 35
[Figure 46] LCMS analysis chart of Compound 36
[Figure 47] LCMS analysis chart of Compound 37
[Figure 48] LCMS analysis chart of Compound 38
[Figure 49] LCMS analysis chart of Compound 39
[Figure 50] LCMS analysis chart of Compound 40
[Figure 51] LCMS analysis chart of Compound 41
[Figure 52] NMR analysis chart of Compound 41
[Figure 53] LCMS analysis chart of Compound 42
[Figure 54] LCMS analysis chart of Compound 43
[Figure 55] LCMS analysis chart of Compound 44
[Figure 56] LCMS analysis chart of Compound 45
[Figure 57] LCMS analysis chart of Compound 46
[Figure 58] LCMS analysis chart of Compound 47
[Figure 59] LCMS analysis chart of Compound 48
[Figure 60] LCMS analysis chart of Compound 49
[Figure 61] LCMS analysis chart of Compound 50
[Figure 62] LCMS analysis chart of Compound 51
[Figure 63] LCMS analysis chart of Compound 52
[Figure 64] LCMS analysis chart of Compound 53
[Figure 65] LCMS analysis chart of Compound 54
[Figure 66] LCMS analysis chart of Compound 55
[Figure 67] LCMS analysis chart of Compound 56
[Figure 68] LCMS analysis chart of Compound 57
[Figure 69] LCMS analysis chart of Compound 58
[Figure 70] LCMS analysis chart of Compound 59
[Figure 71] LCMS analysis chart of Compound 60
[Figure 72] LCMS analysis chart of Compound 61
[Figure 73] LCMS analysis chart of Compound 62
[Figure 74] LCMS analysis chart of Compound 63
[Figure 75] LCMS analysis chart of Compound 64
[Figure 76] LCMS analysis chart of Compound 65
[Figure 77] LCMS analysis chart of Compound 66
[Figure 78] LCMS analysis chart of Compound 67
[Figure 79] LCMS analysis chart of Compound 68
[Figure 80] LCMS analysis chart of Compound 69
[Figure 81] LCMS analysis chart of Compound 70
[Figure 82] LCMS analysis chart of Compound 71
[Figure 83] LCMS analysis chart of Compound 72

### Description of Embodiments

In the general formula (1b) of the present invention, examples of the "C₁₋₇ alkyl group" include a methyl group, an ethyl group, propyl group, an isopropyl group, a butyl group, an isobutyl group, a tert-butyl group, a pentyl group, a hexyl group, and a heptyl group. Among them, preferred is a C₁₋₄ alkyl group, with more preferred examples including a methyl group, an ethyl group, a propyl group, an isopropyl group, an n-butyl group, an s-butyl group, a t-butyl group, and an isobutyl group.

Examples of the "aryl group" include a C₆₋₁₄ aryl group such as a phenyl group, a naphthyl group, an indenyl group, and anthryl group, preferably a C₆₋₁₀ aryl group, more preferably a phenyl group.

Examples of the "heteroaryl group" include a 5 to 14-membered heteroaryl group such as a furyl group, a thienyl group, an oxazolyl group, a pyridyl group, a pyrimidinyl group, a pyrazolyl group, a benzofuranyl group, and a benzooxadiazolyl group, preferably a 5 or 6-membered heteroaryl group, more preferably a furyl group, a pyridyl group and a benzofuranyl group.

Examples of the aryl in the "aryloxy group" are the same as the above-mentioned aryl group. Preferred is a phenoxy group.

The "arylene group" may be a group obtained by removing one hydrogen atom bonded to the aromatic ring of the aryl group, more preferably a phenylene group, and a naphthylene group.

Examples of the substituents which may be substituted on the aryl group, the heteroaryl group and the arylene group include a C₁₋₄ alkyl group (for example, a methyl, an ethyl, a propyl, an n-butyl, an s-butyl , a t-butyl, an isobutyl group, and the like), a halo C₁₋₄ alkyl group (for example, a chloromethyl group, a dichloromethyl group, a fluoromethyl group, a difluoromethyl group, a trifluoromethyl group, a pentachloroethyl group, and the like), a halogen atom (for example, a fluorine atom, a chlorine atom, a bromine atom, an iodine atom, and the like), C₁₋₄ alkoxy (for example, a methoxy group, an ethoxy group, a propoxy group, an isopropoxy group, an n-butoxy group, an s-butoxy group, a t-butoxy group, an isobutoxy group, and the like), or a hydroxyl group.

Examples of the "C₃₋₇ cycloalkyl group" include a cyclopropyl group, a cyclobutyl group, a cyclopentyl group and a cyclohexyl group.

Examples of the "C₂₋₄ alkenyl group" include a vinyl group or a propenyl group.

Examples of the "C₂₋₄ alkynyl group" include an ethynyl group, a propynyl group, a butynyl, preferably a 2-propynyl group and a 2-butynyl group.

Examples of the "C₄₋₈ tertiary alkyl group" include a t-butyl group, a t-pentyl group, a t-hexyl group. Preferred is a t-butyl group.

Preferred examples of the heteroaryl group include a furyl group, a thienyl group, a pyridyl group, a benzofuranyl group, and a benzoxadiazolyl group. Preferred examples of the aryloxy group include a phenoxy group.

Examples of the substituents which may be substituted on the aryl group, heteroaryl group or aryloxy group include C₁₋₄ alkyl (for example, a methyl, an ethyl, a propyl, an n-butyl, an s-butyl, a t-butyl, an isobutyl group and the like), a halo C₁₋₄ alkyl group (for example, a trifluoromethyl group, a chloromethyl group, a dichloromethyl group, a fluoromethyl group, a difluoromethyl group and the like), a halogen atom (for example, a fluorine atom, a chlorine atom, a bromine atom and an iodine atom), C₁₋₄ alkoxy (for example, a methoxy group, an ethoxy group, a propoxy group, a butoxy group and the like), and a hydroxyl group. Examples of the substituted aryl group, substituted heteroaryl group or substituted aryloxy group include an aryl groups, heteroaryl group or aryloxy group substituted by one to three substituents mentioned above.

The group -(CHR²)ₐ- represented by Y¹, is preferably one having R² being a hydrogen atom or C₁₋₃ alkyl group (preferably a methyl group or an ethyl group), and a being 1, more preferably -CH₂- or -CH(CH₃)-.

As the C₁₋₇ alkyl group represented by R¹, C₁₋₄ alkyl group (for example, a methyl, an ethyl, propyl, an isopropyl, an n-butyl, an s-butyl, a t-butyl, an isobutyl and the like) is preferable, and a methyl group and an ethyl group are more preferable.

Also, m is preferably 0 or 1.

(B)ₙ- is a group, wherein B may be the same or different from each other if n is 2 or more. n is preferably 0, 1 or 2.

In -CO--R⁴ or -O-R⁴ represented by B, suitable examples of R⁴ include a C₁₋₄ alkyl group (suitably, a methyl group, an ethyl group, a propyl group or the like), a phenyl group, a phenyl group substituted with one to two halogen atoms and a pyridyl group.

Among them, -O-R⁴ is more preferred, and specific examples thereof include a C₁₋₄ alkoxy group (suitably, a methoxy group, an ethoxy group, a propoxy group or the like); a phenoxy group; a phenoxy group substituted with one to two halogen atoms (preferably, chlorine atom, fluorine atom or the like), nitro group or the like; and pyridyloxy group (2-pyridyloxy group, 3-pyridyloxy group, 4-pyridyloxy group).

In -CONR⁵R⁶ represented by B, R⁵ is preferably a hydrogen atom or a C₃₋₇ cycloalkyl group, R⁶ is preferably a hydrogen atom, a C₁₋₇ alkyl group or a C₂₋₄ alkenyl group, and both of R⁵ and R⁶ are preferably hydrogen atoms.

Further, in R⁵, the Ar² in Ar²-C₁₋₄ alkyl group is preferably a phenyl group, a furyl group, a pyrazolyl group, and a pyridyl group, and the C₁₋₄ alkyl is preferably a C₁₋₂ alkyl.

In R⁵, the C₁₋₄ alkoxy C₁₋₄ alkyl group is preferably a methoxyethyl group, ethoxyethyl group or ethoxypropyl group.

Preferred examples of the C₁₋₇ alkyl group represented by B include a methyl group, an ethyl group, a propyl group, an isopropyl group, an n-butyl group, an s-butyl group, a t-butyl group and an isobutyl group.

Examples of the halo C₁₋₇ alkyl group represented by B include a chloromethyl group, a dichloromethyl group, a fluoromethyl group, a difluoromethyl group and a trifluoromethyl group, and preferred is a trifluoromethyl group.

Suitable examples of the halogen atom represented by B include a fluorine atom, a chlorine atom, a bromine atom and an iodine atom.

The C₃₋₇ cycloalkyl group represented by B is preferably a cyclopropyl group, a cyclobutyl group, a cyclopentyl group and the like.

The B is preferably a C₁₋₇ alkyl group, a halogen atom, -NO₂, a phenyl group, a halo C₁₋₇ alkyl group, -CO-R⁴, -O-R⁴, and -CONR⁵R⁶.

In the formula (1b), R⁷ represents a C₁₋₄ alkyl group (such as a methyl group, an ethyl group, a propyl group, an n-butyl group, an s-butyl group, a t-butyl group and an isobutyl group), a halo C₁₋₄ alkyl group (such as a trifluoromethyl group, a chloromethyl group, a dichloromethyl group, a fluoromethyl group and a difluoromethyl group), a halogen atom (such as a fluorine atom, chlorine atom, a bromine atom and an iodine atom), a C₁₋₄ alkoxy group (such as a methoxy group, an ethoxy group, a propoxy group and a butoxy group) or a hydroxy group; and s represents an integer of 0 to 3 (if s is 2 or more, then a plurality of R⁷ may be the same or different from each other).

Specific examples of the suitable compounds represented by the formula (1b) according to the present invention are shown in the following Tables 1 to 9.

Compounds 1 to 48, 53, 70 to 73, No.103694, No.168198, No.189474, No.189640, No.191294, No.195831, No.209705, and No.274971, as well as the compounds shown in Tables 8 and 9 are Reference Compounds.

**[Table 1]**

| | | |
|---|---|---|
| Compound 1 | Compound 2 | Compound 3 |
| 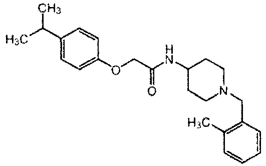 | 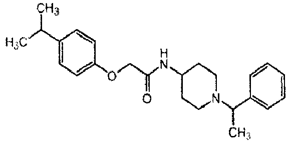 | 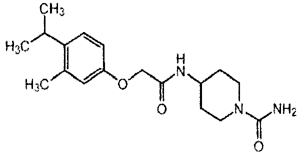 |
| Compound 4 | Compound 5 | Compound 6 |
| 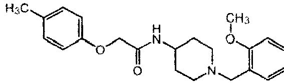 | 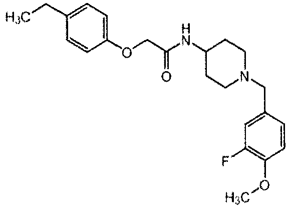 | 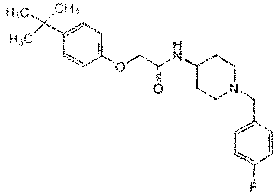 |
| Compound 7 | Compound 8 | Compound 9 |
| 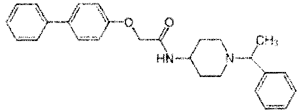 | 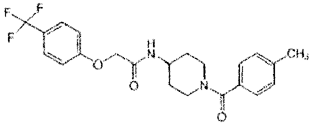 | 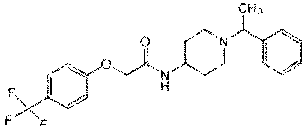 |
| Compound 10 | Compound 11 | Compound 12 |
| 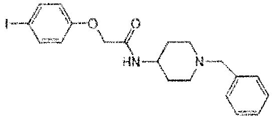 | 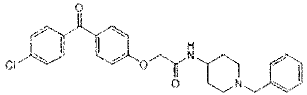 | 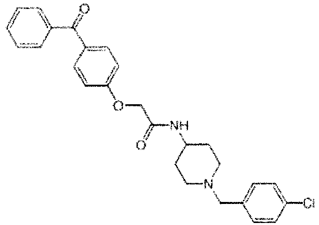 |

**[Table 2]**

| | | |
|---|---|---|
| Compound 13 | Compound 14 | Compound 15 |
| 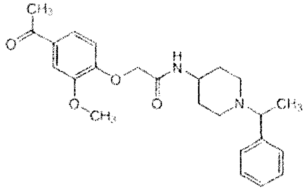 | 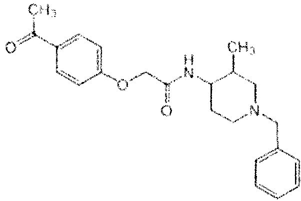 | 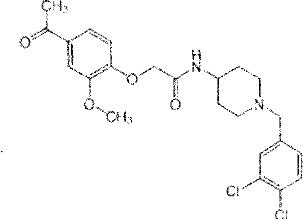 |
| Compound 16 | Compound 17 | Compound 18 |
| 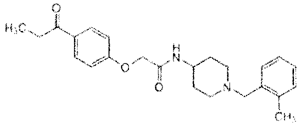 | 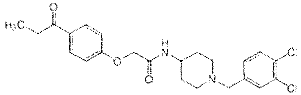 | 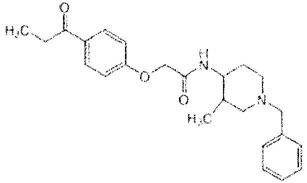 |
| Compound 19 | Compound 20 | Compound 21 |
| 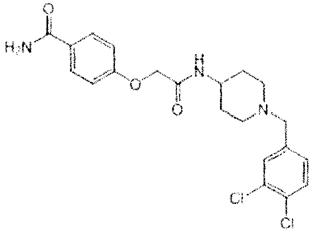 | 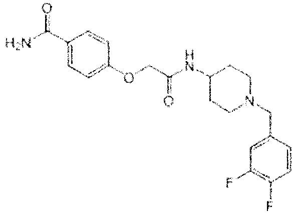 | 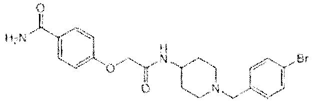 |
| Compound 22 | Compound 23 | Compound 24 |
| 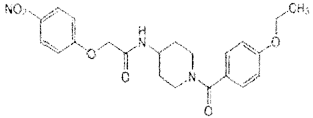 | 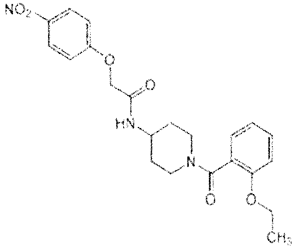 | 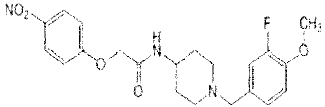 |

**[Table 3]**

| | | |
|---|---|---|
| Compound 25 | Compound 26 | Compound 27 |
| 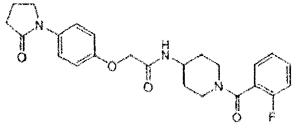 | 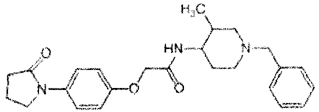 | 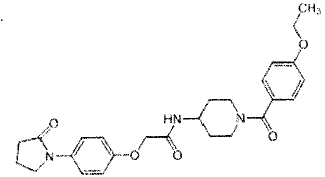 |
| Compound 28 | Compound 29 | Compound 30 |
| 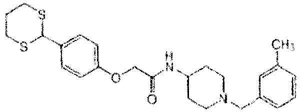 | 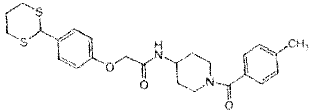 | 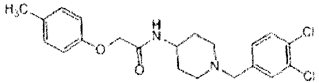 |
| Compound 31 | Compound 32 | Compound 33 |
| 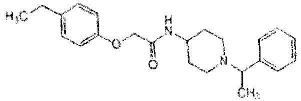 | 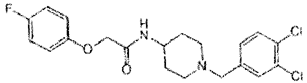 | 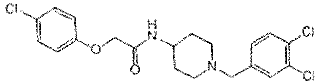 |
| Compound 34 | Compound 35 | Compound 36 |
| 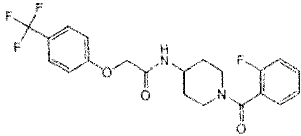 | 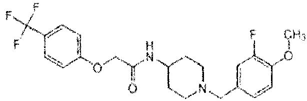 | 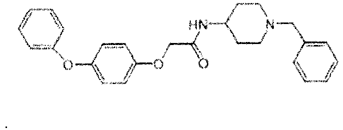 |

**[Table 4]**

| | | |
|---|---|---|
| Compound 37 | Compound 38 | Compound 39 |
| 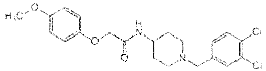 | 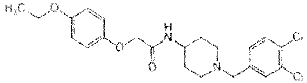 | 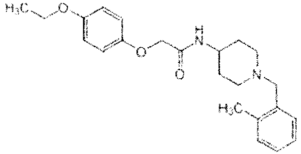 |
| Compound 40 | Compound 41 | Compound 42 |
| 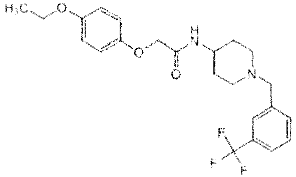 | 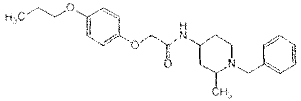 | 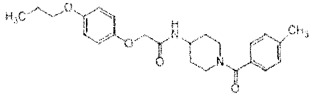 |
| Compound 43 | Compound 44 | Compound 45 |
| 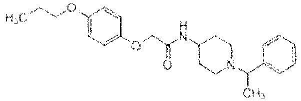 | 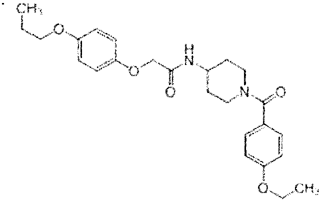 | 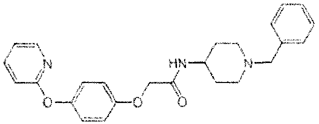 |
| Compound 46 | Compound 47 | Compound 48 |
| 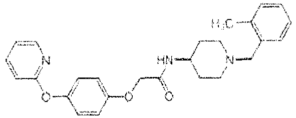 | 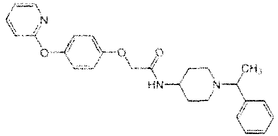 | 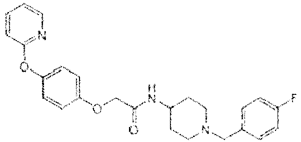 |

**[Table 5]**

| | | |
|---|---|---|
| Compound 49 | Compound 50 | Compound 51 |
| 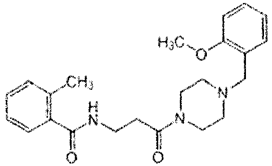 | 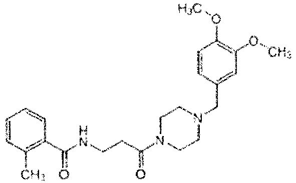 | 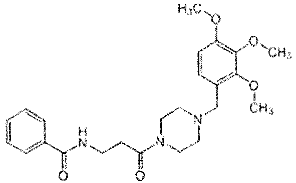 |
| Compound 52 | Compound 53 | Compound 54 |
| 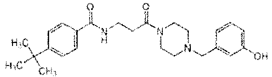 | 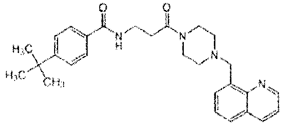 | 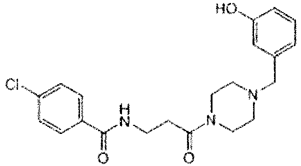 |
| Compound 55 | Compound 56 | Compound 57 |
| 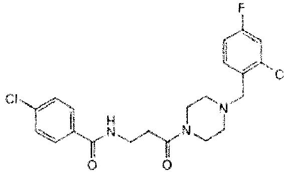 | 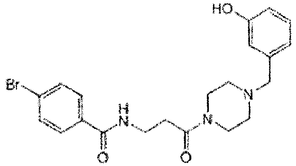 | 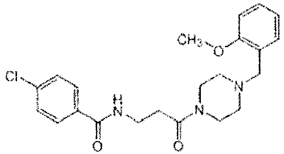 |
| Compound 58 | Compound 59 | Compound 60 |
| 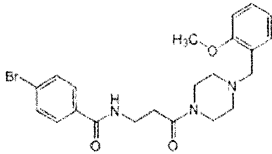 | 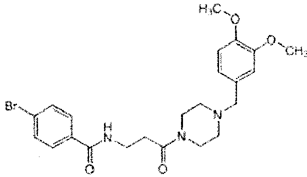 | 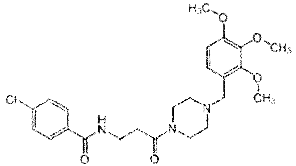 |

**[Table 6]**

| | | |
|---|---|---|
| Compound 61 | Compound 62 | Compound 63 |
| 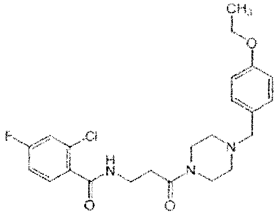 | 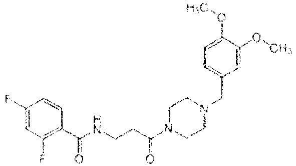 | 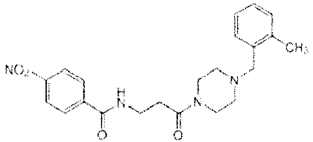 |
| Compound 64 | Compound 65 | Compound 66 |
| 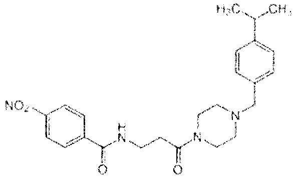 | 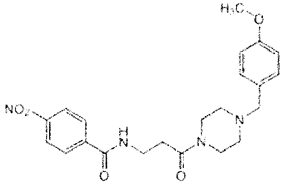 | 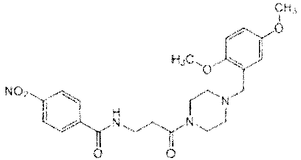 |
| Compound 67 | Compound 68 | Compound 69 |
| 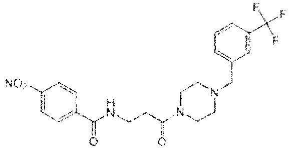 | 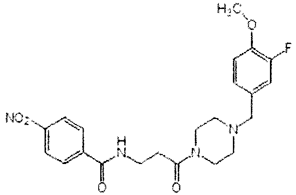 | 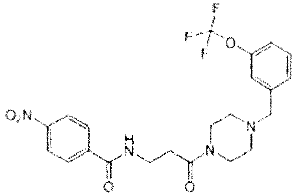 |
| Compound 70 | Compound 71 | Compound 72 |
| 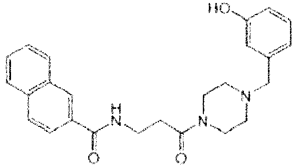 | 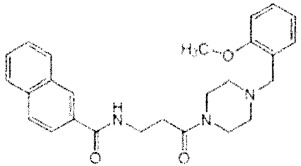 | 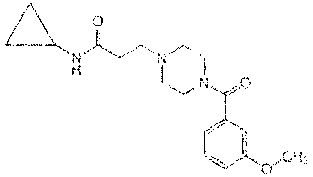 |

**[Table 7]**

| | | |
|---|---|---|
| Compound 73 | Compound 74 | |
| 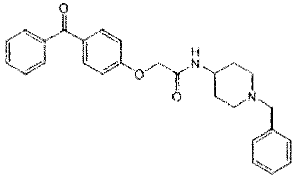 | 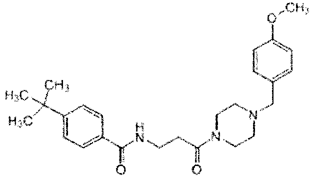 | |
| No.103694 | No.168198 | No.189474 |
| 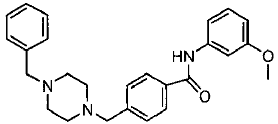 | 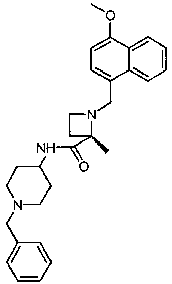 | 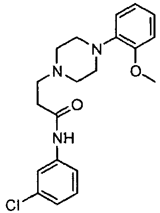 |
| No.189640 | No.191294 | No.183665 |
| 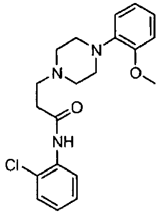 | 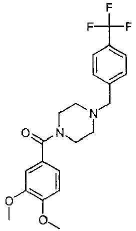 | 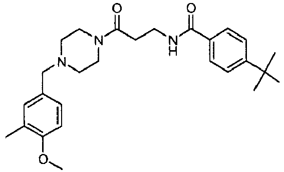 |
| No.195831 | No.209705 | No.274971 |
| 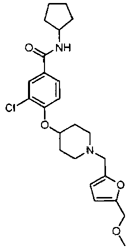 | 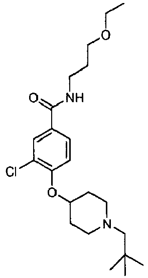 | 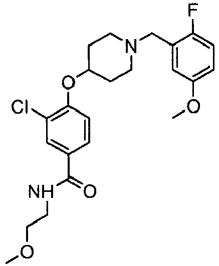 |

**[Table 8]**

| | | |
|---|---|---|
| No.196462 | No.197248 | No.211156 |
| 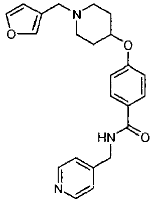 | 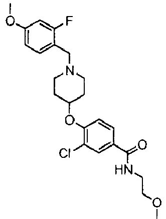 | 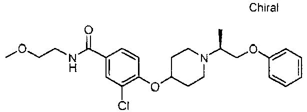 |
| No.197372 | No.198637 | No.214617 |
| 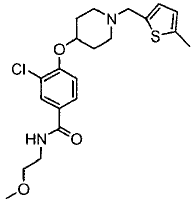 | 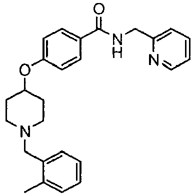 | 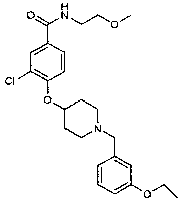 |
| No.200737 | No.206685 | No.251327 |
| 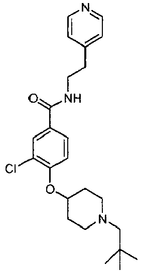 | 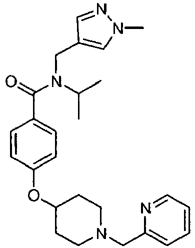 | 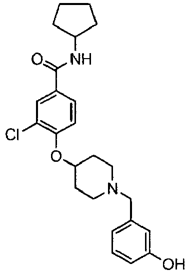 |
| No.260544 | No.264785 | No.473771 |
| 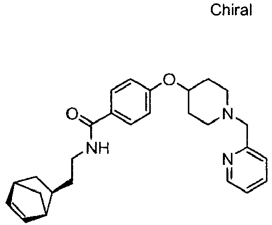 | 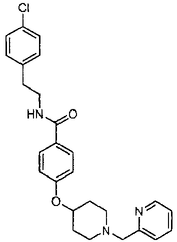 | 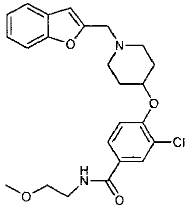 |

**[Table 9]**

| | | |
|---|---|---|
| No.268508 | No.268949 | No.484140 |
| 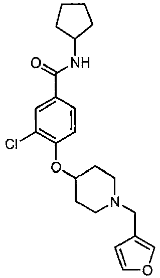 | 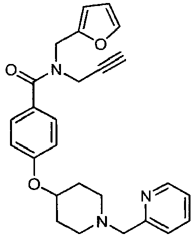 | 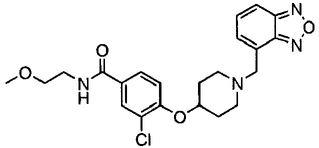 |
| No.272299 | No.272350 | No.492284 |
| 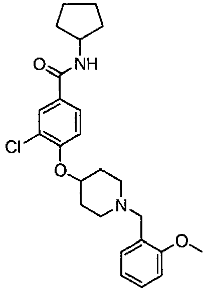 | 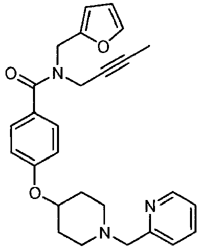 | 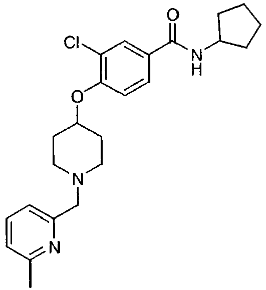 |
| No.466151 | No.550212 | |
| 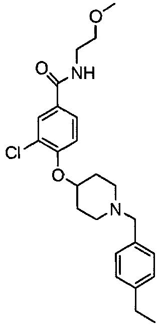 | 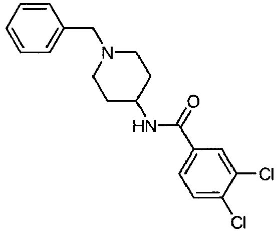 | |

The compounds represented by the formula (1b) according to the present invention include all of the stereoisomers such as geometric isomers such as cis-form and trans-form and optical isomers such as d-form and l-form, and may be a mixture containing the isomers at an arbitrary ratio.

The compounds represented by the general formula (1b) can form an acid addition salt and a base addition salt with an acid and a base, respectively, and examples of the acid addition salt include a salt with a mineral acid such as a hydrochloric acid and sulfuric acid; a salt with an organic carboxylic acid such as a formic acid, acetic acid, citric acid, trichloroacetic acid, trifluoroacetic acid, fumaric acid and maleic acid; and a salt with a sulfonic acid such as a methanesulfonic acid, benzenesulfonic acid, p-toluenesulfonic acid, mesitylenesulfonic acid and naphthalenesulfonic acid, and examples of the base addition salt include a salt with an alkali metal such as sodium and potassium; a salt with an alkaline earth metal such as calcium and magnesium; a salt with an ammonium salt, and a salt with a nitrogen-containing organic base such as, trimethylamine, triethylamine, tributylamine, pyridine, N,N-dimethylaniline, N-methyl-D(-)-glucamine, N-methylpiperidine, N-methylmorpholine, diethylamine, cyclohexylamine, procaine, dibenzylamine, N-benzyl-β-phenethylamine, 1-ephenamine and N,N'-dibenzylethylenediamine.

Further, the compounds represented by the formula (1b) or a salt thereof can be present not only in a non-solvated form, but also in a hydrate or solvate. Accordingly, all of the crystalline forms and hydrates or solvates of them are included in the present invention.

The compounds represented by the formula (1b) and a salt thereof according to the present invention can be manufactured by using the method illustrated in the following, for example.

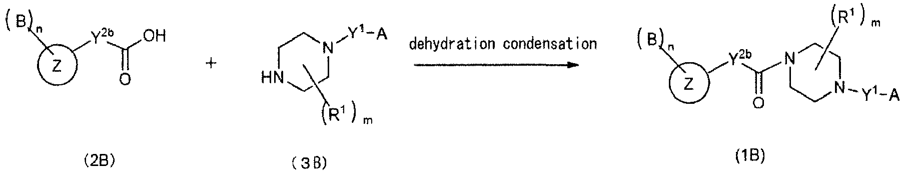

In the scheme, A is phenyl, substituted by (R⁷)ₛ, Z is phenyl, and Y¹, B, m and n represent the same ones as in the above, respectively;
and Y^{2b} represents *-CONH-(CH₂)_{b}- (wherein, b is 2 and * represent the same ones as in the above, respectively).

That is, the compound (1B) can be obtained by condensation reaction of the carboxylic acid of the compound (2B) with the amine of the compound (3B).

This reaction may be performed using the carboxylic acid (the compound (2B)) and the amine (the compound (3B)) with both of them being in an equivalent amount or with one of them being in an excess amount in the presence of a condensing agent in accordance with a conventional method.

Examples of the condensing agent which may be suitably used include N,N-dicyclohexylcarbodiimide (DCC), 1-ethyl-3-(3-dimethylaminopropyl)carbodiimide (EDC or WSC), O-benzotriazol-1-yl-N,N,N',N'-tetramethyluronium hexafluorophosphate (HBTU), carbonyldiimidazole (CDI), 4-(4,6-dimethoxy-1,3,5-triazin-2-yl)4-methylmorpholinium chloride (DMTMM) and 2-(7-azabenzotriazol-1-yl)-N,N,N',N'-tetramethyluronium hexafluorophosphate (HATU). These condensing agents are used in an equivalent amount or in an excess amount relative to the amount of the carboxylic acid.

As the solvent, a solvent which is not involved in the reaction such as N,N-dimethylformamide (DMF), dioxane, water, methanol, ethanol, tetrahydrofuran (THF), dichloromethane, dichloroethane, diethyl ether, chloroform, dimethoxyethane (DME), ethyl acetate, toluene, acetonitrile and dimethylsulfoxide (DMSO) or a mixture solvent thereof may be used, and it is preferred to select the solvent appropriately depending on the raw material, the type of the condensing agent or the like.

In addition, 1-hydroxybenzotriazole (HOBt), N-hydroxysuccinimide (HOSu) or the like may be added as an additive to promote the dehydration condensation and suppress the side reaction.

The reaction, which is typically performed under cooling to room temperature, may also be performed under heating depending on conditions for the condensing reaction.

Alternatively, the compound (1B) may be manufactured by using a method in which a carboxylic acid is derivatized into an active derivative and then condensed with an amine. In this case, the reaction is performed by using the carboxylic acid (the compound (2B)) and the amine (the compound (3B)) with both of them being in an equivalent amount or with one of them being in an excess amount. Examples of the active derivative of the carboxylic acid include active esters obtained by reacting with a phenolic compound such as p-nitrophenol, or an N-hydroxyamine compound such as 1-hydroxysuccinimide (HOSu), 1-hydroxybenzotriazole (HOBt) or 7-aza-1-hydroxybenzotriazole (HOAt); monoalkyl carbonates; mixed acid anhydrides obtained by reacting with an organic acid; phosphate mixed acid anhydrides obtained by reacting diphenylphosphoryl chloride and N-methylmorpholine; acid azides obtained by subjecting an ester to sequential reaction with hydrazine and an alkyl nitrite; acid halides such as acid chlorides or acid fluorides; and symmetric acid anhydrides.

The activating reagent in synthesizing an active derivative of the carboxylic acid is used in an equivalent amount or in an excess amount relative to the amount of the carboxylic acid (the compound (2B)). Even under reaction conditions other than those in this case, any reaction can be employed as long as an amide bond is formed by the reaction.

Note that the carboxylic acid (the compound (2B)) and the amine (the compound (3B)) which are used as the raw materials are each a known compound described in a publication and can be manufactured by using a known synthesis method, and in addition commercially available compounds may be used for them.

The compounds represented by the formula (1b) according to the present invention to be manufactured in this way may be isolated or purified as a free form or a salt thereof by using a conventional chemical process in the art such as extraction, precipitation, fractionation chromatography, fractional crystallization and recrystallization. A salt of the compound may be manufactured by subjecting the compound according to the present invention in a free state to a common salt-forming reaction. In the case that the compound according to the present invention has an asymmetric carbon, there exist optical isomers thereof. These optical isomers may be manufactured by using an approach in which the isomers is derivatized into a diastereomer salt with an optically-active acid or base and subjected to fractional crystallization, an approach in which the isomers is subjected to optical resolution with a conventional method such as column chromatography, an approach in which the isomers is synthesized using an optically-active raw material compound, or the like.

As illustrated in the following Test Examples, the compound represented by the formula (1b) or a salt thereof according to the present invention increases phosphorylation of AMPK in C2C12 cells expressing AdipoR1, and phosphorylation of AMPK is significantly reduced through suppression of AdipoR1 by specific siRNA (Test Example 1). That is, the compound or a salt thereof according to the present invention increases AMPK phosphorylation via AdipoR.

Further, the compound according to the present invention binds to both AdipoR1 and AdipoR2, and show very similar effects to adiponectin in muscle and liver, such as activation of AMPK and PPARα, pathways and ameliorated insulin resistance and glucose intolerance in mice fed a high-fat (HF) diet. Furthermore, the compound according to the present invention ameliorates diabetes in db/db mice, a rodent model of hereditary obesity, and prolongs the shortened lifespan of db/db mice on a HF diet (Test Examples 2, and 3).

Therefore, the compound or a salt thereof according to the present invention is useful as an agent for preventing, treating or ameliorating a symptom, disease, disorder or condition due to decreased production of adiponectin, decreased adiponectin levels in blood or decreased activity of AdipoR (AdipoR1 or AdipoR2), and can be used for preventing, treating or ameliorating the symptom, disease, disorder or condition. Specifically, the compound or a salt thereof according to the present invention is useful as a medicine for preventing or treating hyperglycemia; glucose intolerance; decreased insulin sensitivity (such as insulin resistance); type II diabetes; hypertension; arteriosclerosis, atherosclerosis, lipid metabolism disorders such as hyperlipidemia and fatty liver; mitochondrial dysfunction; obesity; metabolic syndrome and lifestyle-related disease; and malignant tumor due to lifestyle-related disease.

In the case that the compound represented by the formula (1b) or a salt thereof according to the present invention is used for a medicine (pharmaceutical composition), it may be formulated into a composition together with a pharmaceutically (medicinally) acceptable carrier for parenteral administration such as injection, rectal administration and transdermal administration, oral administration in a form of solid, semisolid or liquid, or the like.

Examples of the form of the composition according to the present invention for an injection include pharmaceutically acceptable sterile water, non-aqueous solution, suspension and emulsion. Examples of a suitable non-aqueous carrier, diluting agent, solvent or vehicle include propylene glycol, polyethylene glycol, vegetable oils such as olive oil and injectable organic esters such as ethyl oleate. Such a composition may contain auxiliary agents such as a preservative, wetting agent, emulsifier and dispersant. These compositions may be sterilized by for example, filtration with a bacteria-retaining filter, or by mixing therein a sterilizing agent in a form of a sterile solid composition which can be dissolved in sterilized water or a certain amount of other medium which can be used for a sterilized injection immediately before use.

Examples of the solid preparation for oral administration include a capsule, tablet, pill, troche, powder, granule and the like. In preparing the solid preparation, the compound according to the present invention is typically mixed together with at least one inert diluting agent such as sucrose, lactose and starch. This preparation may further include an additional substance other than the inert diluting agent, such as a lubricant (such as magnesium stearate) in a common preparation process. In the cases of a capsule, tablet and pill, a buffer may also be included. The tablet and pill may be further provided with an enteric coating.

Examples of the liquid preparation for oral administration include a pharmaceutically acceptable emulsion, solution, suspension, syrup and elixir which contain an inert diluting agent commonly used by those skilled in the art, for example, water. In addition to the inert diluting agents, the composition may be formulated with auxiliary agents such as a wetting agent, emulsifier, suspension, sweetener, seasoning agent and flavoring agent. In the case of a preparation for rectal administration, it preferably contains an excipient such as cacao butter and suppository wax in addition to the compound according to the present invention.

The dose of the compound represented by the formula (1b) or a salt thereof according to the present invention, which varies depending on the characteristics, route of administration, desired treatment period and other factors of the compound to be administered, is typically and preferably about 0.01 to 100 mg/kg per day in the case of intravenous administration, about 0.05 to 500 mg/kg per day in the case of intramuscular administration and about 0.1 to 1,000 mg/kg in the case of oral administration. Alternatively, this daily dose may be administered separately in 2 to 4 portions as desired.

Examples of the subject for administration include a human having any symptom, disease or disorder due to decreased production of adiponectin, decreased adiponectin levels in blood or decreased activity of AdipoR, and a human with a possibility of having any of them. Examples thereof include a patient suffering from hyperglycemia; glucose intolerance; decreased insulin sensitivity (such as insulin resistance); type II diabetes; hypertension; arteriosclerosis, atherosclerosis, lipid metabolism disorders such as hyperlipidemia and fatty liver; mitochondrial dysfunction; obesity; metabolic syndrome and lifestyle-related disease; or malignant tumor due to lifestyle-related disease.

Hereinafter, the present invention will be described more specifically by exemplifying Examples . Note that % means % by mass unless otherwise specified.

### Examples

### Preparation Example 1

### Synthesis of Compound 1

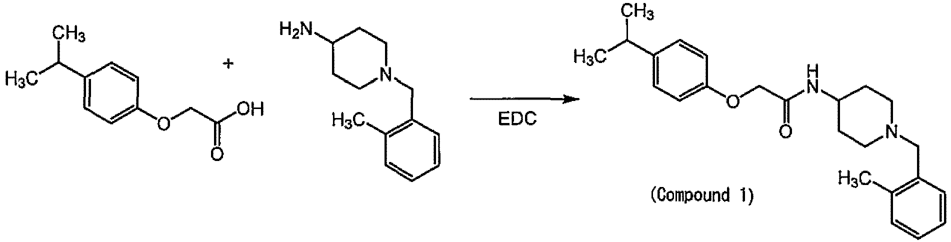

A vial was charged with a carboxylic acid (330 mg, 1.1 equiv.), a solvent (1 mL of a solution of 200 g N-oxybenzotriazole in 1 L of DMF), and an amine (316 mg, 1 equiv.) . To the stirred reaction mixture, EDC was added (290 mg, 1.21 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 425 mg (72%).

### Preparation Example 2

### Synthesis of Compound 2

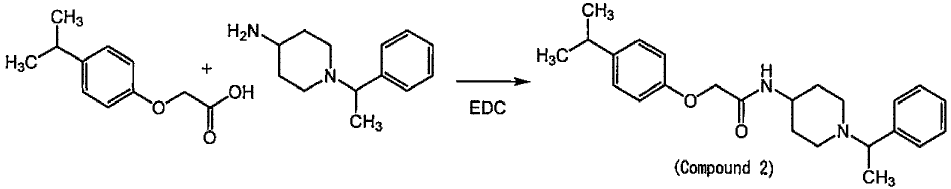

A vial was charged with a carboxylic acid (339 mg, 1.1 equiv.), a solvent (1 mL of a solution of 200 g N-oxybenzotriazole in 1 L of DMF), and an amine (324 mg, 1 equiv.) . To the stirred reaction mixture, EDC was added (298 mg, 1.21 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 590 mg (98%).

### Preparation Example 3

### Synthesis of Compound 3

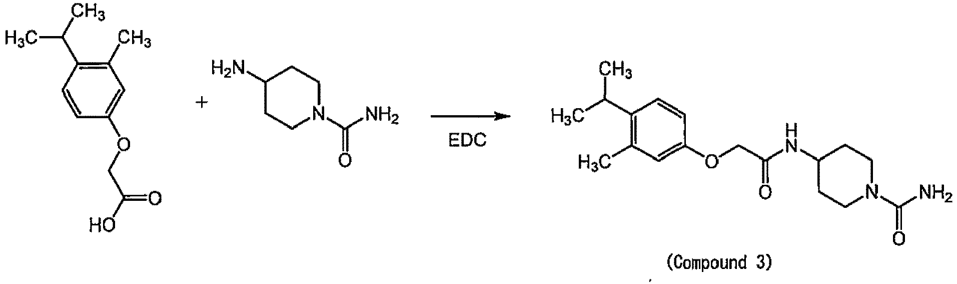

A vial was charged with a carboxylic acid (174 mg, 1.1 equiv.), a solvent (1 mL of a solution of 200 g N-oxybenzotriazole in 1 L of DMF), and an amine (109 mg, 1 equiv.) . To the stirred reaction mixture, EDC was added (143 mg, 1.21 equiv.) . In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively, 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 94 mg (37%).

### Preparation Example 4

### Synthesis of Compound 4

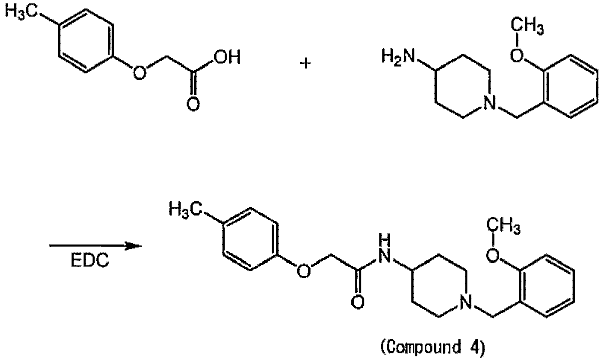

A vial was charged with a carboxylic acid (290 mg, 1.1 equiv.), a solvent (1 mL of a solution of 200g N-oxybenzotriazole in 1 L of DMF), and an amine (350 mg, 1 equiv.) . To the stirred reaction mixture, EDC was added (298 mg, 1.21 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 125 mg (21%).

### Preparation Example 5

### Synthesis of Compound 5

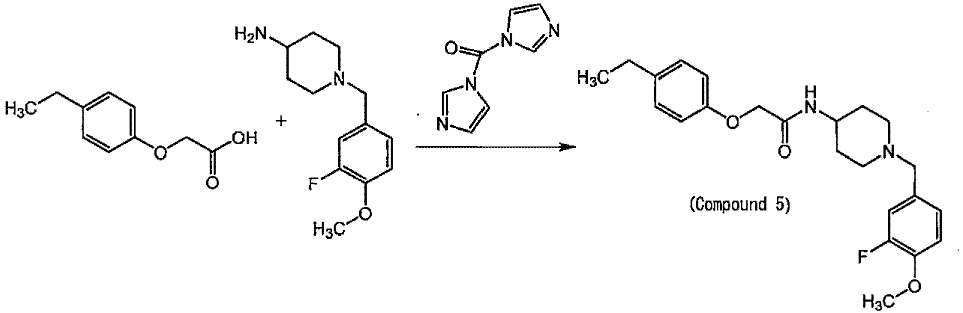

A vial was charged with a carboxylic acid (299 mg, 1.1 equiv.) and dry DMF (1 mL). To the stirred reaction mixture, N,N-carbodiimidazole (269 mg, 1.1 equiv.) was added. After 1 h of stirring, the vial was open and the reaction mixture was left for 2 h in a drying oven at 60°C. Then, an amine (359 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then, the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol =4:1). The yield was yield 450 mg (74%).

### Preparation Example 6

### Synthesis of Compound 6

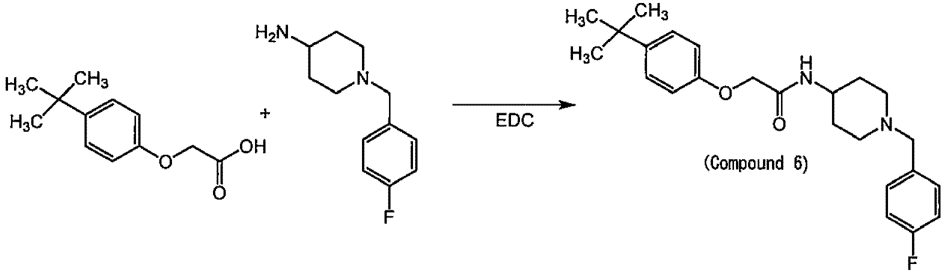

A vial was charged with a carboxylic acid (336 mg, 1.1 equiv.) and dry DMF (1 mL). To the stirred reaction mixture, N,N-carbodiimidazole (262 mg, 1.1 equiv.) was added. After 1 h of stirring, the vial was open and the reaction mixture was left for 2 h in a drying oven at 60°C. Then an amine (305 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol= 4:1). The yield was yield 253 mg (43%).

### Preparation Example 7

### Synthesis of Compound 7

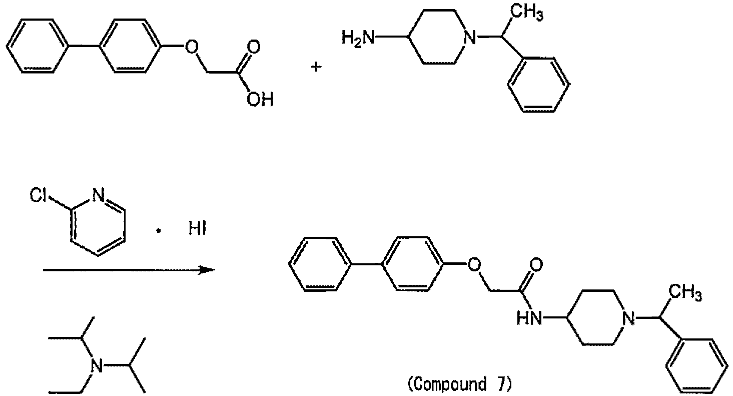

A vial was charged with a corresponding acid (331 mg, 1.0 equiv.), DIPEA (468 mg, 2.5 equiv.), and dry acetonitrile (1 mL) . To the stirred reaction mixture, an amine (296 mg, 1.0 equiv.) and 2-chloro-N-methylpyridinium iodide (444 mg, 1.2 equiv.) were added. The reaction vial was placed into a water bath and left at 100°C for 3 h. Then it was cooled to room temperature, and excess of water was added. Then the vial was sonicated. Nevertheless, an oily product was separated by extraction. The dried organic layers were concentrated, and crude residue was purified by column chromatography with CombiFlash on silica gel. The yield was 20 mg (3%).

### Preparation Example 8

### Synthesis of Compound 8

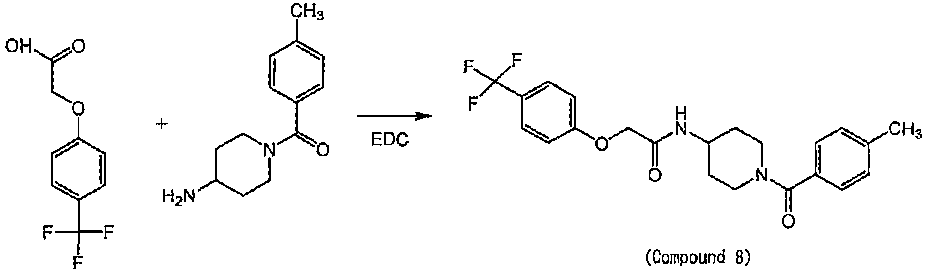

A vial was charged with a carboxylic acid (347 mg, 1.1 equiv.), a solvent (1 mL of a solution of 200 g N-oxybenzotriazole in 1 L of DMF), and an amine (313 mg, 1 equiv.) . To the stirred reaction mixture, EDC was added (269 mg, 1.21 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively, 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 399 mg (66%).

### Preparation Example 9

### Synthesis of Compound 9

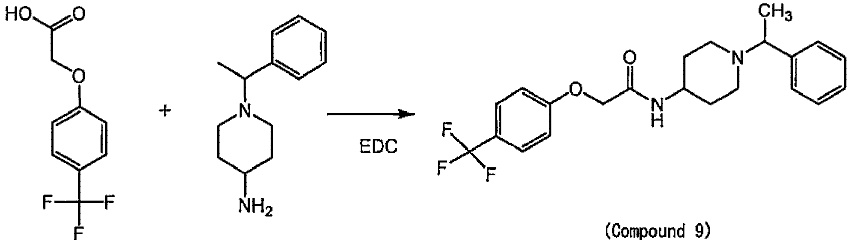

A vial was charged with a carboxylic acid (356 mg, 1.1 equiv.), a solvent (1 mL of a solution of 200 g N-oxybenzotriazole in 1 L of DMF), and an amine (300 mg, 1 equiv.) . To the stirred reaction mixture, EDC was added (276 mg, 1.21 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively, 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 250 mg (42%).

### Preparation Example 10

### Synthesis of Compound 10

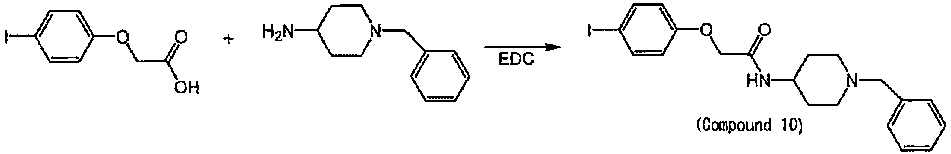

A vial was charged with a carboxylic acid (371 mg, 1 equiv.), a solvent (1 mL of a solution of 200 g N-oxybenzotriazole in 1 L of DMF), and an amine (254 mg, 1 equiv.) . To the stirred reaction mixture, EDC was added (228 mg, 1.1 equiv.). In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 506 mg (84%).

### Preparation Example 11

### Synthesis of Compound 11

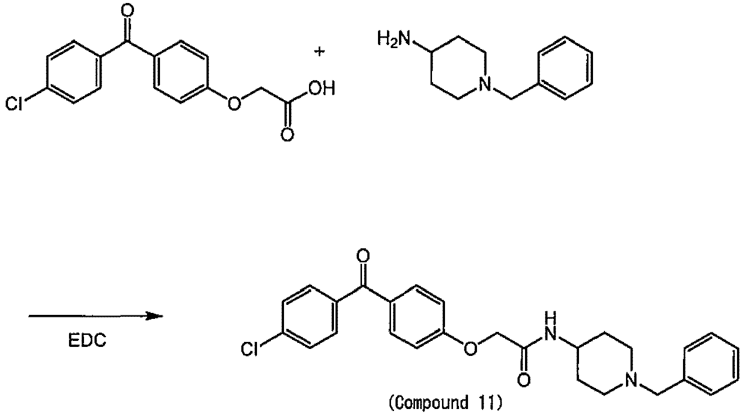

A vial was charged with a carboxylic acid (410 mg, 1.1 equiv.), a solvent (1 mL of a solution of 200 g N-oxybenzotriazole in 1 L of DMF), and an amine (244 mg, 1 equiv.) . To the stirred reaction mixture, EDC was added (241 mg, 1.21 equiv.). In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively, 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 49 mg (8%).

### Preparation Example 12

### Synthesis of Compound 12

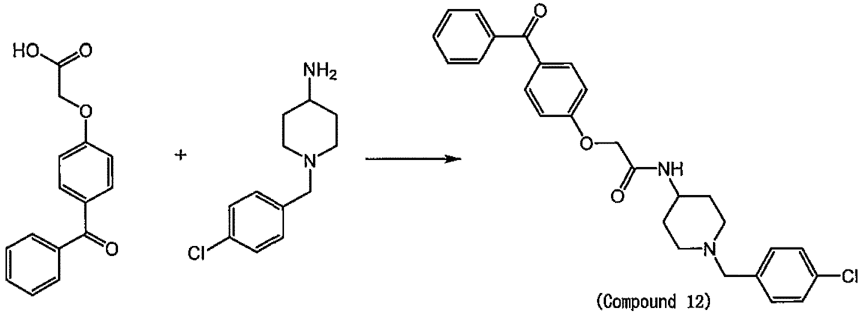

A vial was charged with a carboxylic acid (326 mg, 1 equiv.) and dry DMF (1 mL). To the stirred reaction mixture, N,N-carbodiimidazole (237 mg, 1.2 equiv.) was added. After 1 h of stirring the vial was open and the reaction mixture was left for 2 h in a drying oven at 60°C. Then an amine (286 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol=4:1). The yield was 63 mg (11%).

### Preparation Example 13

### Synthesis of Compound 13

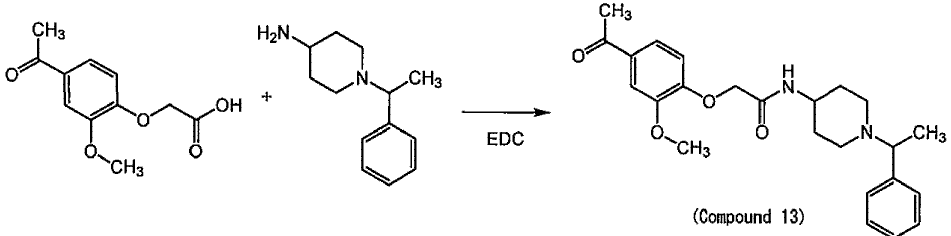

A vial was charged with a carboxylic acid (326 mg, 1 equiv.), a solvent (1 mL of a solution of 200 g N-oxybenzotriazole in 1 L of DMF), and an amine (297 mg, 1 equiv.) . To the stirred reaction mixture, EDC was added (248 mg, 1.1 equiv.). In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively, 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 419 mg (70%).

### Preparation Example 14

### Synthesis of Compound 14

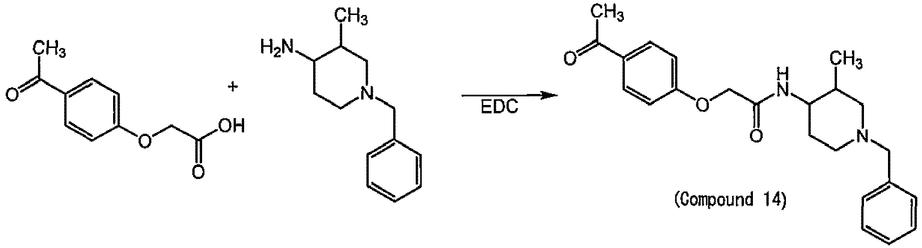

A vial was charged with a carboxylic acid (333 mg, 1.1 equiv.), a solvent (1 mL of a solution of 200 g N-oxybenzotriazole in 1 L of DMF), and an amine (319 mg, 1 equiv.). To the stirred reaction mixture, EDC was added (293 mg, 1.21 equiv.). In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively, 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform: 2-propanol=4:1). The yield was 517 mg (87%).

### Preparation Example 15

### Synthesis of Compound 15

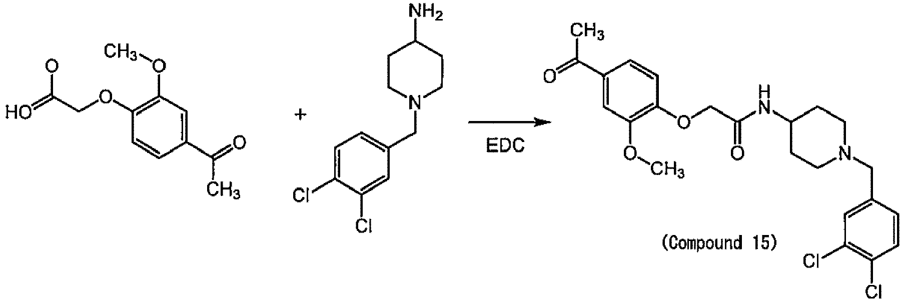

A vial was charged with a carboxylic acid (289 mg, 1 equiv.), a solvent (1 mL of a solution of 200 g N-oxybenzotriazole in 1 L of DMF), and an amine (334 mg, 1 equiv.). To the stirred reaction mixture EDC was added (220 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 525 mg (88%).

### Preparation Example 16

### Synthesis of Compound 16

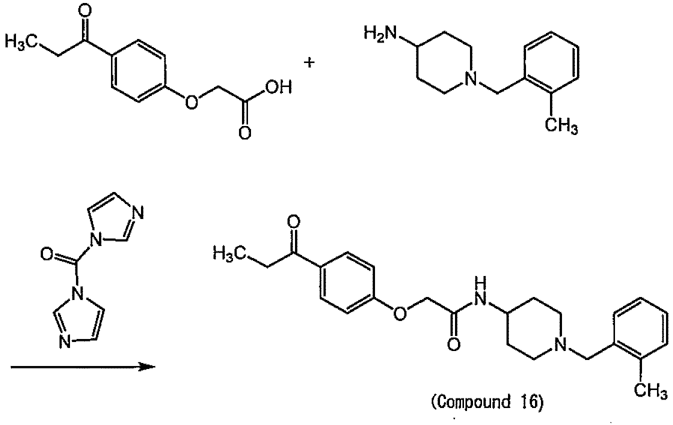

A vial was charged with a carboxylic acid (347 mg, 1.1 equiv.) and dry DMF (1 mL). To the stirred reaction mixture, N,N-carbodiimidazole (270 mg, 1.1 equiv.) was added. After 1 h of stirring, the vial was open and the reaction mixture was left for 2 h in a drying oven at 60°C. Then an amine (310 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol=4:1). The yield was yield 496 mg (83%).

### Preparation Example 17

### Synthesis of Compound 17

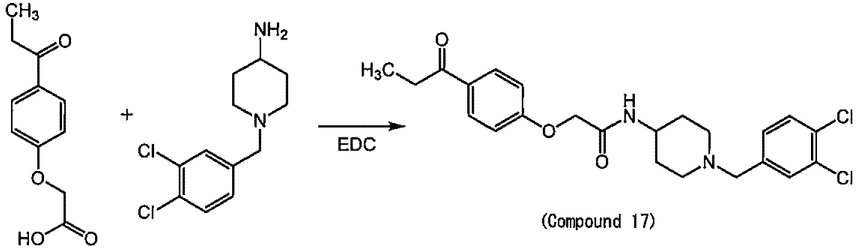

A vial was charged with a carboxylic acid (335 mg, 1.2 equiv.), a solvent (1 mL of a solution of 200 g N-oxybenzotriazole in 1 L of DMF), and an amine (348 mg, 1 equiv.). To the stirred reaction mixture, EDC was added (275 mg, 1. 32 equiv.). In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 496 mg (82%).

### Preparation Example 18

### Synthesis of Compound 18

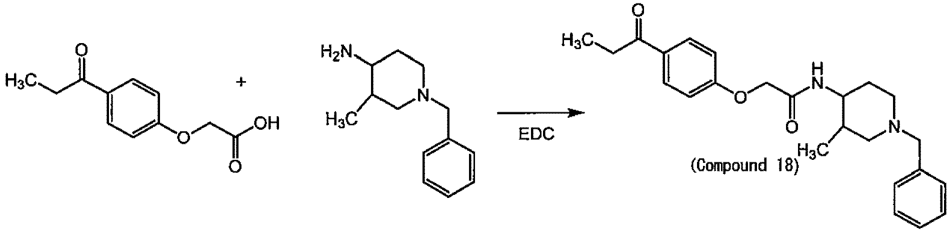

A vial was charged with a carboxylic acid (373 mg, 1.2 equiv.), a solvent (1 mL of a solution of 200 g N-oxybenzotriazole in 1 L of DMF), and an amine (305 mg, 1 equiv.). To the stirred reaction mixture, EDC was added (306 mg, 1.32 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 522 mg (89%).

### Preparation Example 19

### Synthesis of Compound 19

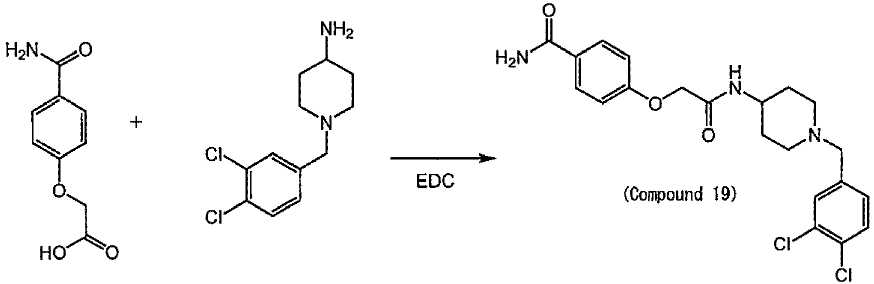

A vial was charged with a carboxylic acid (290 mg, 1.1 equiv.), a solvent (1 mL of a solution of 200 g N-oxybenzotriazole in 1 L of DMF), and an amine (350 mg, 1 equiv.). To the stirred reaction mixture, EDC was added (254 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 459 mg (78%).

### Preparation Example 20

### Synthesis of Compound 20

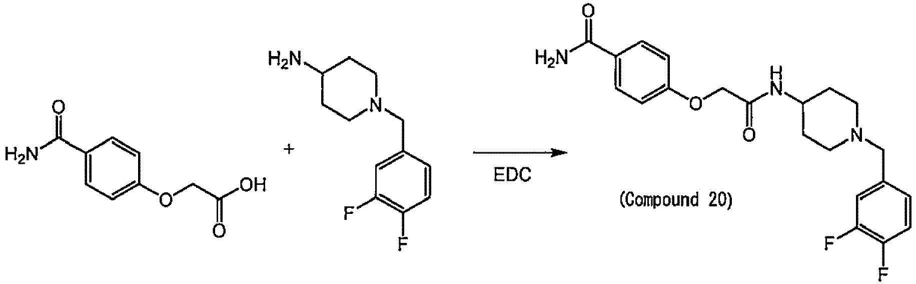

A vial was charged with a carboxylic acid (314 mg, 1.1 equiv.), a solvent (1 mL of a solution of 200 g N-oxybenzotriazole in 1 L of DMF), and an amine (331 mg, 1 equiv.). To the stirred reaction mixture, EDC was added (275 mg, 1.21 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 299 mg (51%).

### Preparation Example 21

### Synthesis of Compound 21

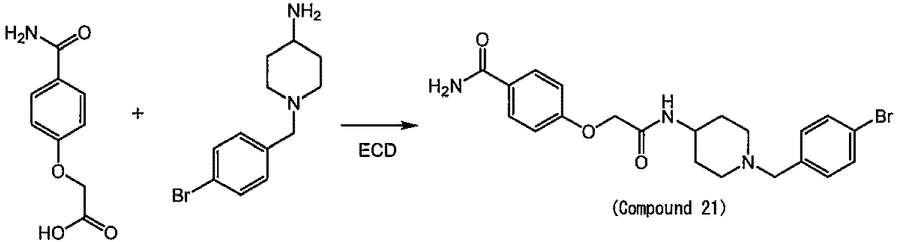

A vial was charged with a carboxylic acid (283 mg, 1.1 equiv.), a solvent (1 mL of a solution of 200 g N-oxybenzotriazole in 1 L of DMF), and an amine (355 mg, 1 equiv.). To the stirred reaction mixture, EDC was added (248 mg, 1.21 equiv.). In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 542 mg (92%).

### Preparation Example 22

### Synthesis of Compound 22

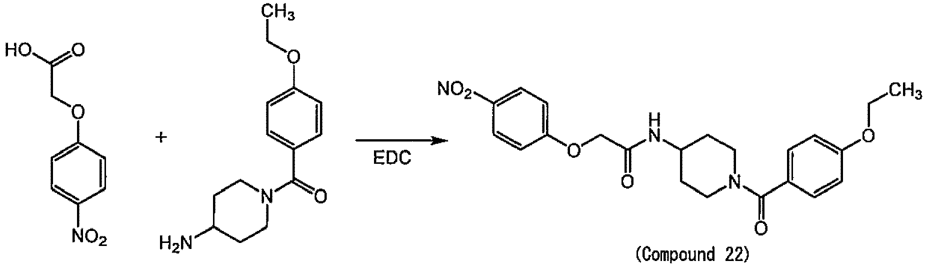

A vial was charged with a carboxylic acid (308 mg, 1. 1 equiv.), a solvent (1 mL of a solution of 200 g N-oxybenzotriazole in 1 L of DMF), and an amine (353 mg, 1 equiv.) . To the stirred reaction mixture, EDC was added (267 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 354 mg (58%).

### Preparation Example 23

### Synthesis of Compound 23

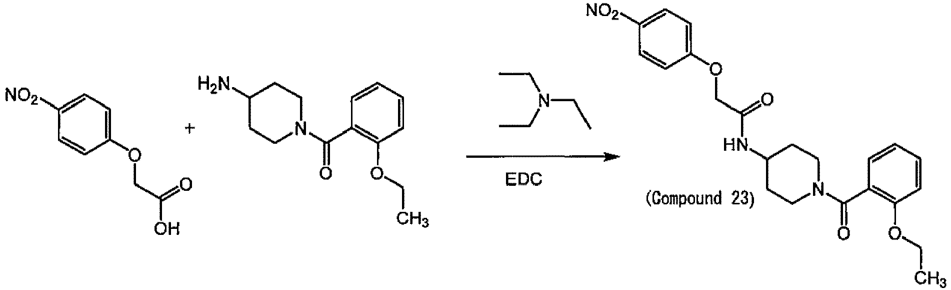

A vial was charged with a carboxylic acid (302 mg, 1.1 equiv.), a solvent (1 mL of a solution of 200 g N-oxybenzotriazole in 1 L of DMF), and an amine (397 mg, 1 equiv.). Triethylamine (169 mg, 1.2 equiv.) was added To the stirred reaction mixture, EDC was added (262 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 170 mg (29%).

### Preparation Example 24

### Synthesis of Compound 24

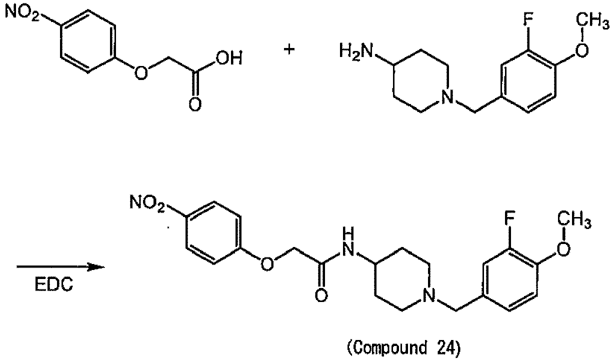

A vial was charged with a carboxylic acid (311 mg, 1.1 equiv.), a solvent (1 mL of a solution of 200 g N-oxybenzotriazole in 1 L of DMF), and an amine (342 mg, 1 equiv.). To the stirred reaction mixture, EDC was added (269 mg, 1.21 equiv.). In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 493 mg (82%).

### Preparation Example 25

### Synthesis of Compound 25

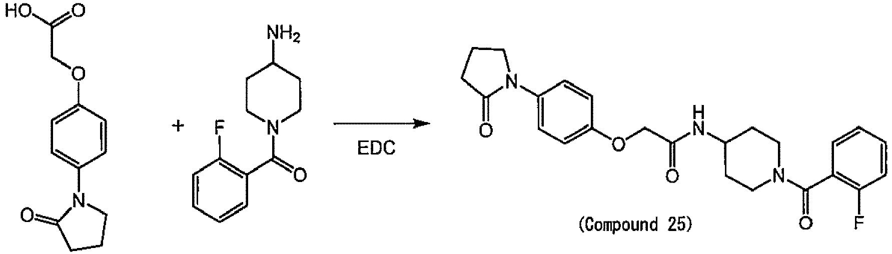

A vial was charged with a carboxylic acid (325 mg, 1 equiv.), a solvent (1 mL of a solution of 200 g N-oxybenzotriazole in 1 L of DMF), and an amine (307 mg, 1 equiv.) . To the stirred reaction mixture, EDC was added (236 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 526 mg (87%).

### Preparation Example 26

### Synthesis of Compound 26

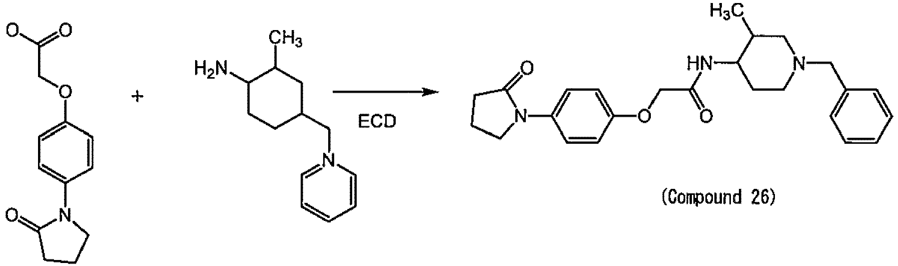

A vial was charged with a carboxylic acid (339 mg, 1 equiv.), a solvent (1 mL of a solution of 200 g N-oxybenzotriazole in 1 L of DMF), and an amine (294 mg, 1 equiv.). To the stirred reaction mixture, EDC was added (246 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 386 mg (64%).

### Preparation Example 27

### Synthesis of Compound 27

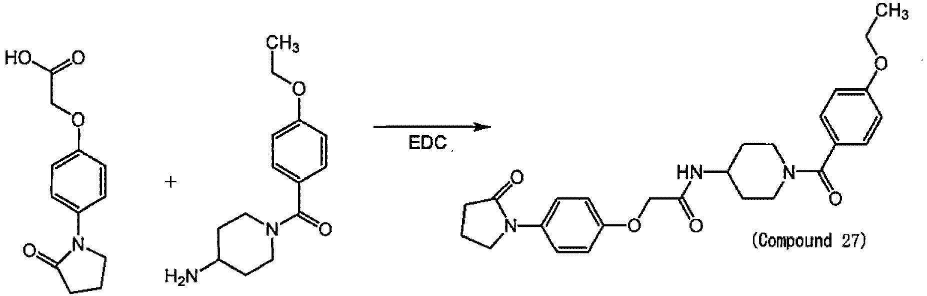

A vial was charged with a carboxylic acid (307 mg, 1 equiv.), a solvent (1 mL of a solution of 200 g N-oxybenzotriazole in 1 L of DMF), and an amine (324 mg, 1 equiv.) . To the stirred reaction mixture, EDC was added (223 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 252 mg (41%).

### Preparation Example 28

### Synthesis of Compound 28

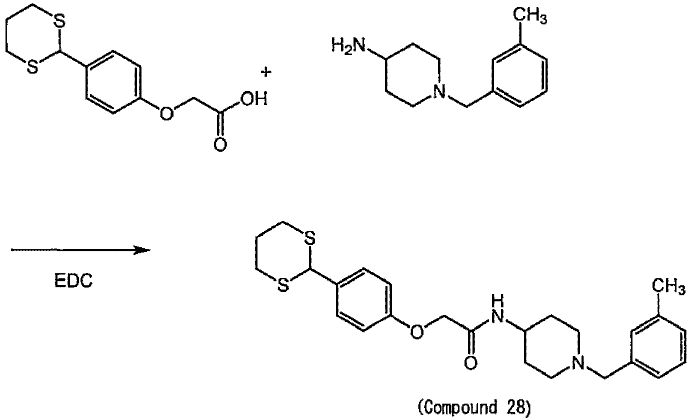

A vial was charged with a carboxylic acid (354 mg, 1 equiv.), a solvent (1 mL of a solution of 200 g N-oxybenzotriazole in 1 L of DMF), and an amine (268 mg, 1 equiv.) . To the stirred reaction mixture, EDC was added (224 mg, 1.1 equiv.) . In case the reaction mixture became highly viscous, some more DMF was added. In case the reaction mixture was a homogeneous solution, it was kept at room temperature for 72 hrs. Otherwise, the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 541 mg (90%).

### Preparation Example 29

### Sybdthesis of Compound 29

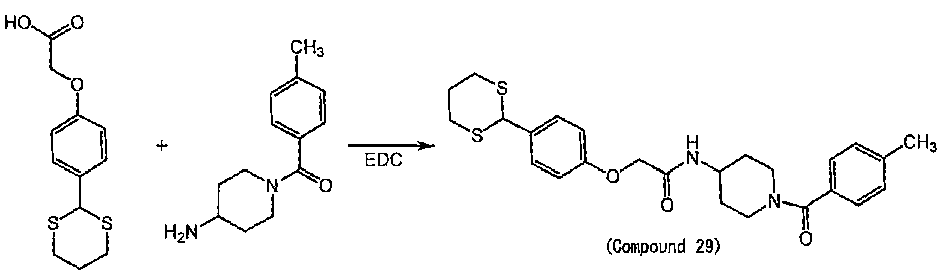

A vial was charged with a carboxylic acid (345 mg, 1 equiv.), a solvent (1 mL of a solution of 200 g N-oxybenzotriazole in 1 L of DMF), and an amine (279 mg, 1 equiv.) . To the stirred reaction mixture, EDC was added (218 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform: 2-propanol=4:1). The yield was 504 mg (84%).

### Preparation Example 30

### Synthesis of Compound 30

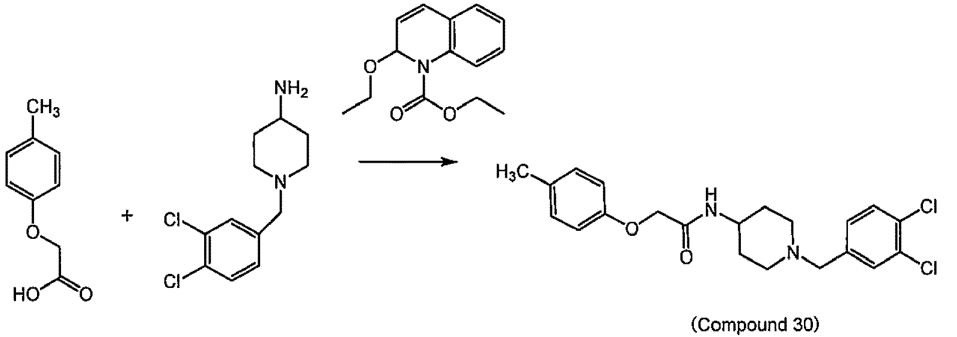

A vial was charged with a carboxylic acid (248 mg, 1.0 equiv.), 1 mL of solvent (3.4 g of imidazole in 200 mL of dry DMF), and an amine (387 mg, 1.0 equiv.) . To the stirred reaction mixture, quinoline (443mg, 1.2 equiv.) was added. The solution was kept at room temperature for 72 h. The reaction mixture was carefully diluted with 2% hydrochloric acid and then was left for 24 h. Then the reaction mixture was sonicated. The vial was left overnight, then the water layer was removed, and 2-propanol (1 mL) was added to cause the crystallization. Alternatively sodium carbonate was added to the aqueous solution in small portions to facilitate the amide crystallization. The gummy precipitate was filtered, washed with a sodium carbonate solution, and then washed with methanol. The crude product was purified by chromatography (silica gel, chloroform with 2-propanol as 4:1). The yield was 428 mg (70%).

### Preparation Example 31

### Synthesis of Compound 31

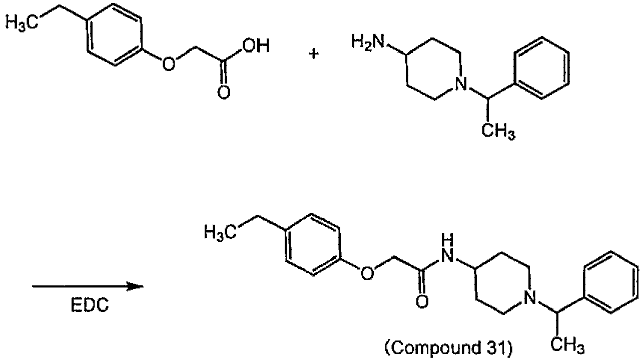

A vial was charged with a carboxylic acid (329 mg, 1.1 equiv.), a solvent (1 mL of a solution of 200 g N-oxybenzotriazole in 1 L of DMF), and an amine (339 mg, 1 equiv.). To the stirred reaction mixture, EDC was added (312 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 590 mg (97%).

### Preparation Example 32

### Synthesis of Compound 32

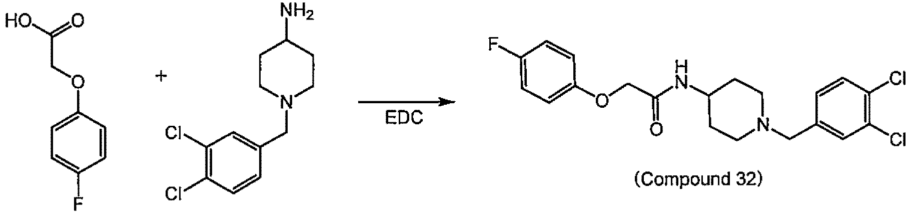

A vial was charged with a carboxylic acid (277 mg, 1.1 equiv.), a solvent (1 mL of a solution of 200 g N-oxybenzotriazole in 1 L of DMF), and an amine (384 mg, 1 equiv.). To the stirred reaction mixture, EDC was added (278 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 209 mg (34%).

### Preparation Example 33

### Synthesis of Compound 33

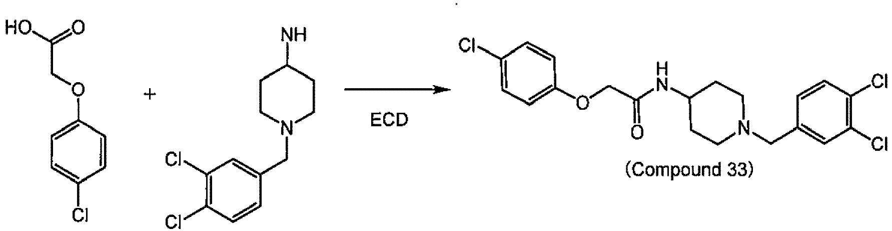

A vial was charged with a carboxylic acid (287 mg, 1.1 equiv.), a solvent (1 mL of a solution of 200 g N-oxybenzotriazole in 1 L of DMF), and an amine (362 mg, 1 equiv.). To the stirred reaction mixture, EDC was added (263 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 22 mg (4%).

### Preparation Example 34

### Synthesis of Compound 34

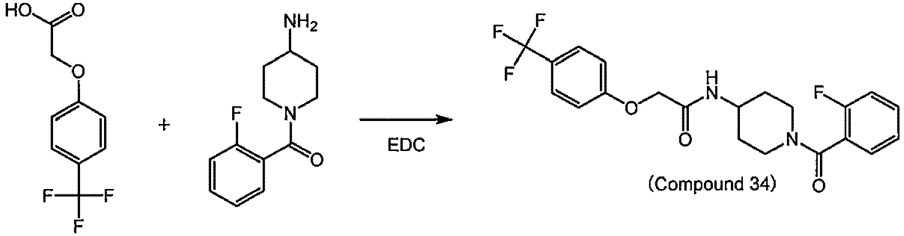

A vial was charged with a carboxylic acid (348 mg, 1.1 equiv.), a solvent (1 mL of a solution of 200 g N-oxybenzotriazole in 1 L of DMF), and an amine (319 mg, 1 equiv.). To the stirred reaction mixture, EDC was added (270 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 456 mg (75%).

### Preparation Example 35

### Synthesis of Compound 35

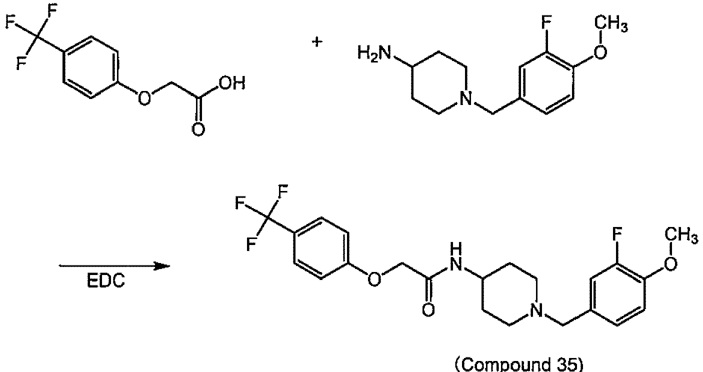

A vial was charged with a carboxylic acid (326 mg, 1.1 equiv.), a solvent (1 mL of a solution of 200 g N-oxybenzotriazole in 1 L of DMF), and an amine (321 mg, 1 equiv.). To the stirred reaction mixture, EDC was added (253 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 471 mg (79%).

### Preparation Example 36

### Synthesis of Compound 36

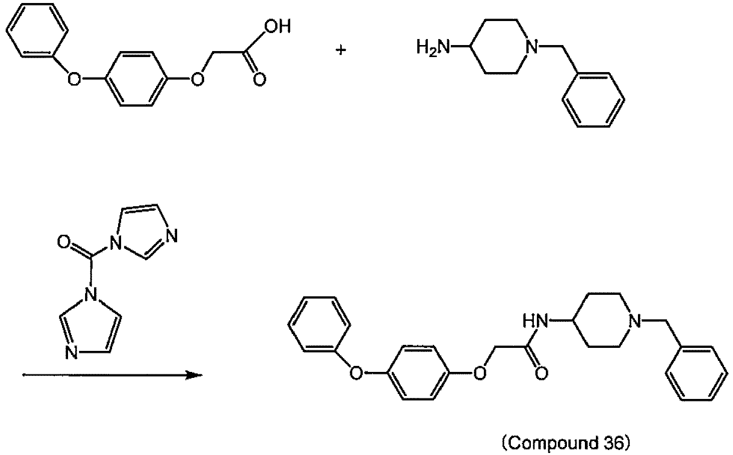

A vial was charged with a carboxylic acid (348 mg, 1 equiv.) and dry DMF (1 mL). To the stirred reaction mixture, N,N-carbodiimidazole (231 mg, 1 equiv.) was added. After 1 h of stirring, the vial was open and the reaction mixture was left for 2 h in a drying oven at 60°C. Then an amine (271 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol=4:1). The yield was yield 66 mg (11%).

### Preparation Example 37

### Synthesis of Compound 37

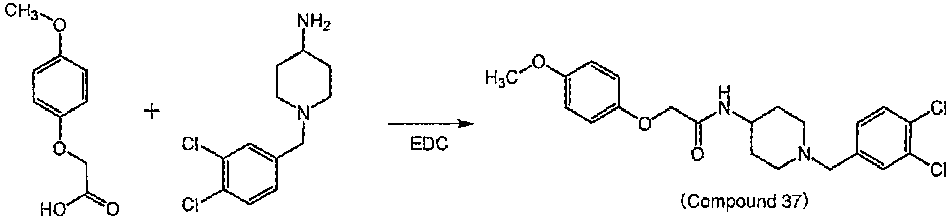

A vial was charged with a carboxylic acid (285 mg, 1.1 equiv.), a solvent (1 mL of a solution of 200 g N-oxybenzotriazole in 1 L of DMF), and an amine (369 mg, 1 equiv.). To the stirred reaction mixture, EDC was added (267 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% queous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 126 mg (21%).

### Preparation Example 38

### Synthesis of Compound 38

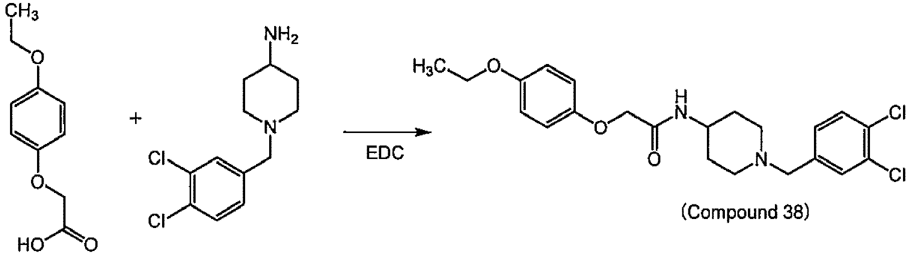

A vial was charged with a carboxylic acid (299 mg, 1.1 equiv.), a solvent (1 mL of a solution of 200 g N-oxybenzotriazole in 1 L of DMF), and an amine (359 mg, 1 equiv.). To the stirred reaction mixture, EDC was added (260 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 148 mg (24%).

### Preparation Example 39

### Synthesis of Compound 39

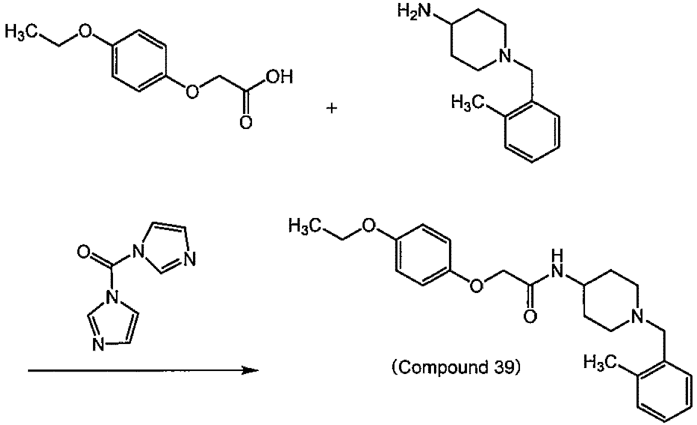

A vial was charged with a carboxylic acid (338 mg, 1.1 equiv.) and dry DMF (1 mL). To the stirred reaction mixture, N,N-carbodiimidazole (279 mg, 1.1 equiv.) was added. After 1 h of stirring, the vial was open and the reaction mixture was left for 2 h in a drying oven at 60°C. Then an amine (320 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol= 4:1). The yield was 17 mg (3%).

### Preparation Example 40

### Synthesis of Compound 40

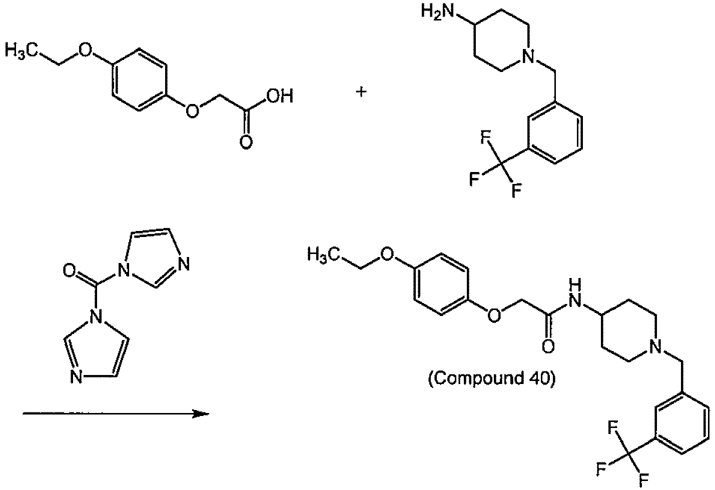

A vial was charged with a carboxylic acid (288 mg, 1.1 equiv.) and dry DMF (1 mL). To the stirred reaction mixture, N,N-carbodiimidazole (238 mg, 1.1 equiv.) was added. After 1 h of stirring, the vial was open and the reaction mixture was left for 2 h in a drying oven at 60°C. Then an amine (345 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol= 4:1). The yield was 131 mg (22%).

### Preparation Example 41

### Synthesis of Compound 41

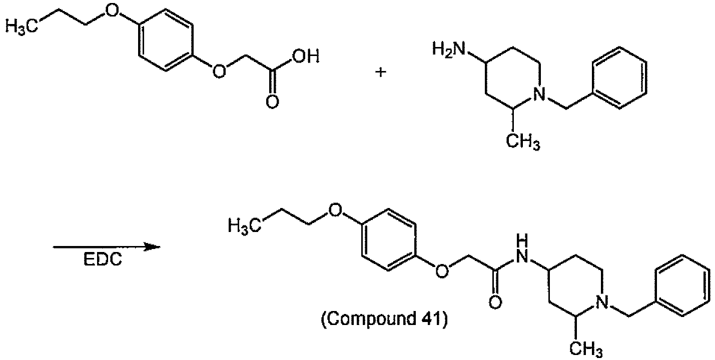

A vial was charged with a carboxylic acid (320 mg, 1 equiv.), a solvent (1 mL of a solution of 200 g N-oxybenzotriazole in 1 L of DMF), and an amine (311 mg, 1 equiv.). To the stirred reaction mixture, EDC was added (260 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 505 mg (84%).

### Preparation Example 42

### Synthesis of Compound 42

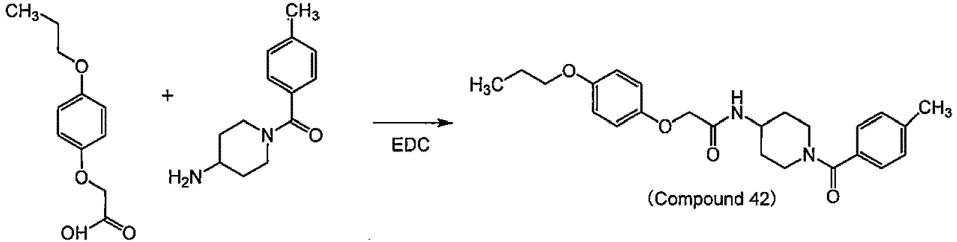

A vial was charged with a carboxylic acid (311 mg, 1 equiv.), a solvent (1 mL of a solution of 200 g N-oxybenzotriazole in 1 L of DMF), and an amine (323 mg, 1 equiv.). To the stirred reaction mixture, EDC was added (253 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 291 mg (48%).

### Preparation Example 43

### Synthesis of Compound 43

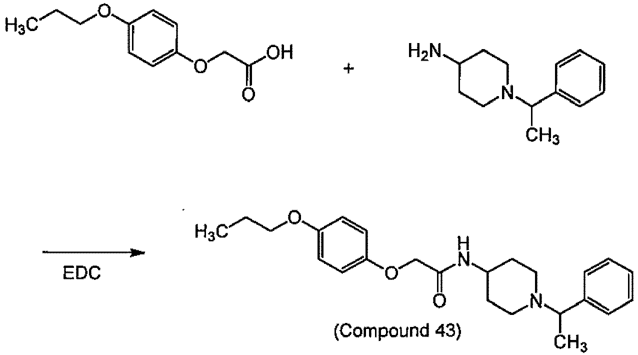

A vial was charged with a carboxylic acid (319 mg, 1 equiv.), a solvent (1 mL of a solution of 200 g N-oxybenzotriazole in 1 L of DMF), and an amine (310 mg, 1 equiv.). To the stirred reaction mixture, EDC was added (259 mg, 1. 1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 541 mg (90%).

### Preparation Example 44

### Synthesis of Compound 44

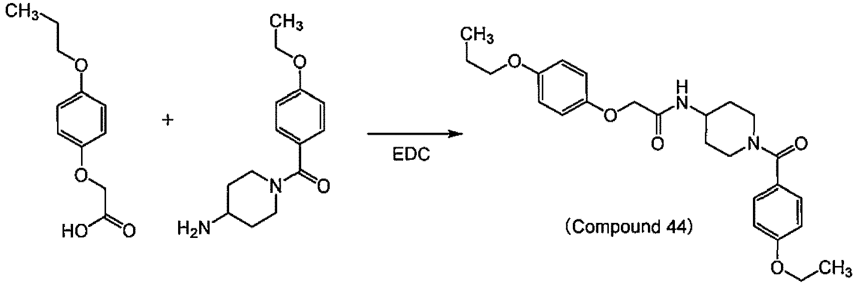

A vial was charged with a carboxylic acid (293 mg, 1 equiv.), a solvent (1 mL of a solution of 200 g N-oxybenzotriazole in 1 L of DMF), and an amine (346 mg, 1 equiv.). To the stirred reaction mixture, EDC was added (238 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 174 mg (28%).

### Preparation Example 45

### Synthesis of Compound 45

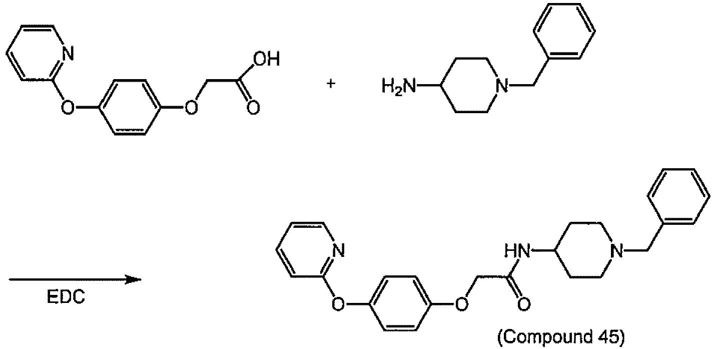

A vial was charged with a carboxylic acid (383 mg, 1.1 equiv.), a solvent (1 mL of a solution of 200 g N-oxybenzotriazole in 1 L of DMF), and an amine (270 mg, 1 equiv.). To the stirred reaction mixture, EDC was added (267 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 580 mg (98%).

### Preparation Example 46

### Synthesis of Compound 46

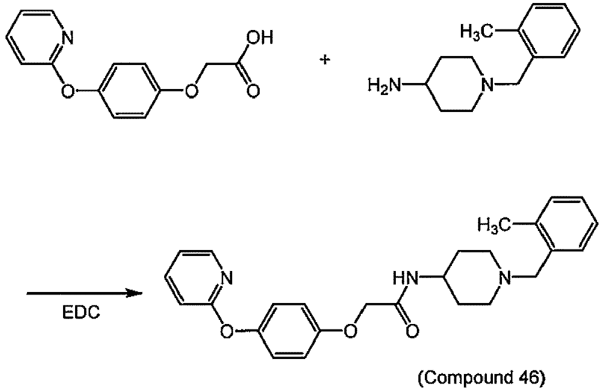

A vial was charged with a carboxylic acid (369 mg, 1.1 equiv.), a solvent (1 mL of a solution of 200 g N-oxybenzotriazole in 1 L of DMF), and an amine (279 mg, 1 equiv.). To the stirred reaction mixture, EDC was added (257 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 570 mg (97%).

### Preparation Example 47

### Synthesis of Compound 47

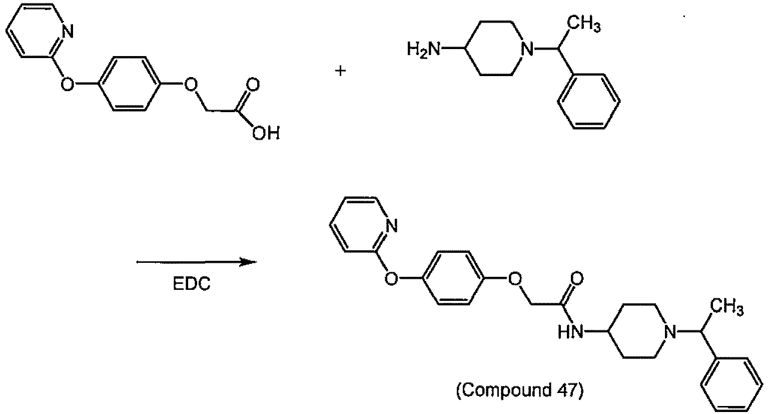

A vial was charged with a carboxylic acid (375 mg, 1.1 equiv.), a solvent (1 mL of a solution of 200 g N-oxybenzotriazole in 1 L of DMF), and an amine (284 mg, 1 equiv.). To the stirred reaction mixture, EDC was added (261 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 596 mg (99%).

### Preparation Example 48

### Synthesis of Compound 48

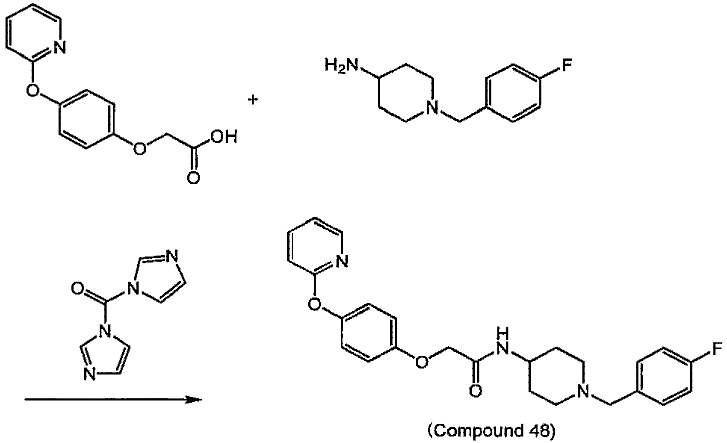

A vial was charged with a carboxylic acid (330 mg, 1 equiv.) and dry DMF (1 mL). To the stirred reaction mixture, N,N-carbodiimidazole (218 mg, 1 equiv.) was added. After 1 h of stirring, the vial was open and the reaction mixture was left for 2 h in a drying oven at 60°C. Then an amine (280 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol= 4:1). The yield was 453 mg (77%).

### Preparation Example 49

### Synthesis of Compound 49

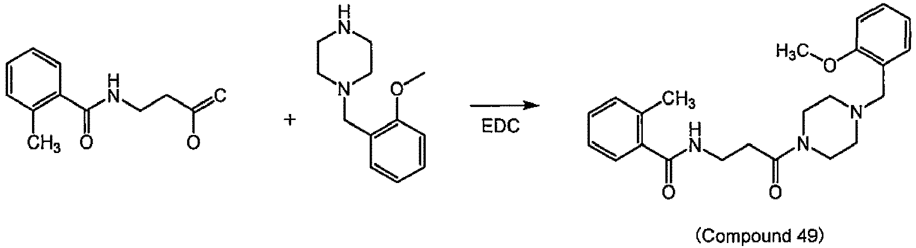

A vial was charged with a carboxylic acid (315 mg, 1 equiv.), a solvent (1 mL of a solution of 200 g N-oxybenzotriazole in 1 L of DMF), and an amine (314 mg, 1 equiv.). To the stirred reaction mixture, EDC was added (260 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 396 mg (66%).

### Preparation Example 50

### Synthesis of Compound 50

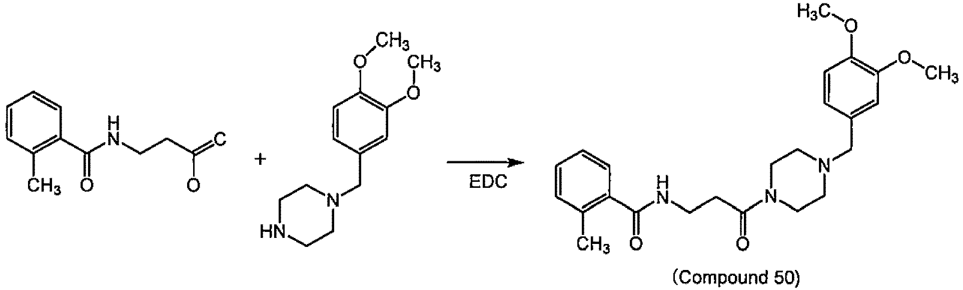

A vial was charged with a carboxylic acid (293 mg, 1 equiv.), a solvent (1 mL of a solution of 200 g N-oxybenzotriazole in 1 L of DMF), and an amine (334 mg, 1 equiv.). To the stirred reaction mixture, EDC was added (241 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 512 mg (85%).

### Preparation Example 51

### Synthesis of Compound 51

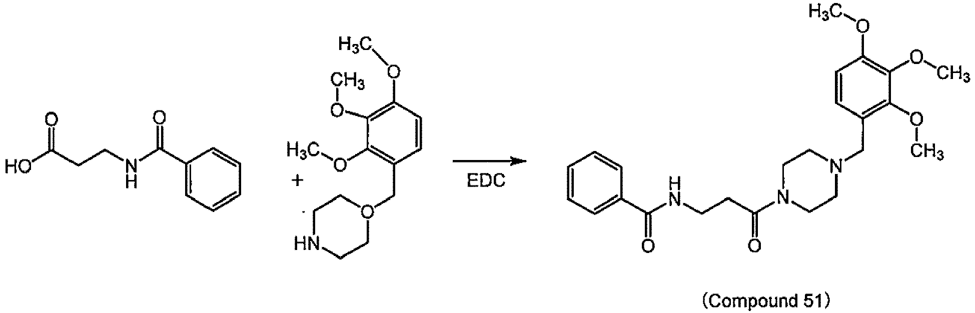

A vial was charged with a carboxylic acid (298 mg, 1.1 equiv.), a solvent (1 mL of a solution of 200 g N-oxybenzotriazole in 1 L of DMF), and an amine (373 mg, 1 equiv.) . To the stirred reaction mixture, EDC was added (263 mg, 1.21 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate water solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 444 mg (72%).

### Preparation Example 52

### Synthesis of Compound 52

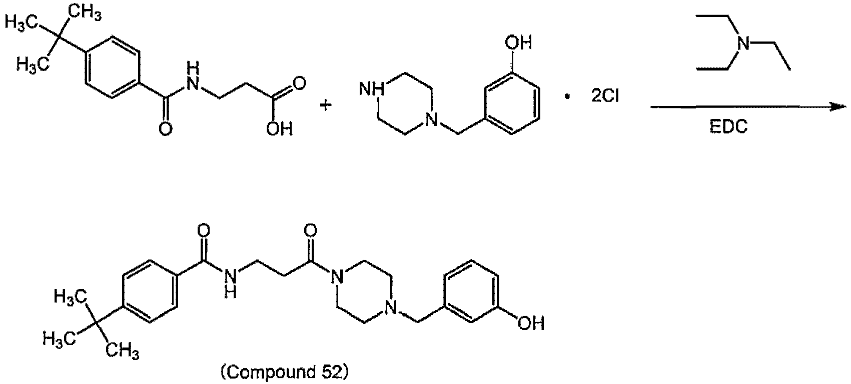

A vial was charged with a carboxylic acid (348 mg, 1 equiv.), a solvent (1 mL of a solution of 200 g N-oxybenzotriazole in 1 L of DMF), and an amine (370 mg, 1 equiv.). Triethylamine (339 mg, 2.4 equiv.) was added. To the stirred reaction mixture, EDC was added (238 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 291 mg (49%).

### Preparation Example 53

### Synthesis example of Compound 53

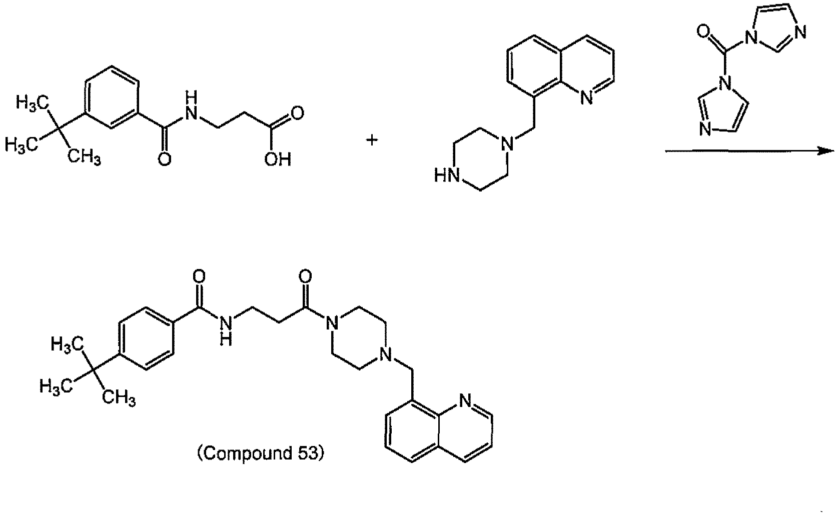

A vial was charged with a carboxylic acid (324 mg, 1 equiv.) and dry DMF (1 mL). To the stirred reaction mixture, N,N-carbodiimidazole (242 mg, 1.2 equiv.) was added. After 1 h of stirring, the vial was open and the reaction mixture was left for 2 h in a drying oven at 60°C. Then an amine (295 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol= 4:1). The yield was 239 mg (40%).

### Preparation Example 54

### Synthesis of Compound 54

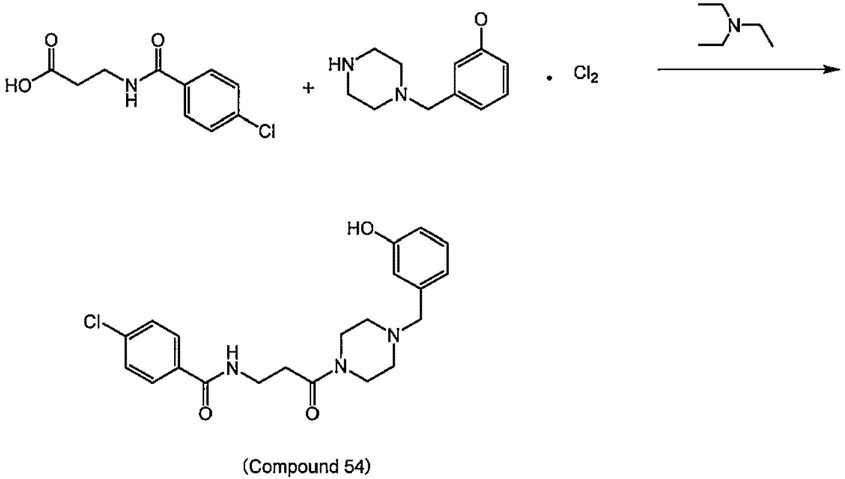

A vial was charged with a carboxylic acid (341 mg, 1 equiv.), a solvent (1 mL of a solution of 200 g N-oxybenzotriazole in 1 L of DMF), and an amine (397 mg, 1 equiv.). Triethylamine (364 mg, 2.4 equiv.) was added. To the stirred reaction mixture, EDC was added (256 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was passed to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 469 mg (78%).

### Preparation Example 55

### Synthesis of Compound 55

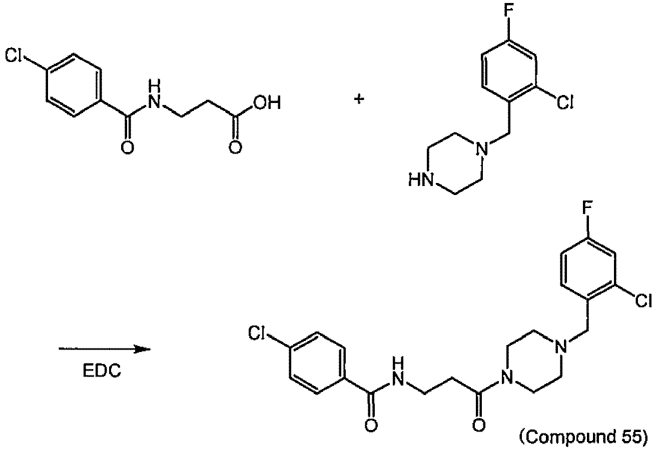

A vial was charged with a carboxylic acid (319 mg, 1 equiv.), a solvent (1 mL of a solution of 200 g N-oxybenzotriazole in 1 L of DMF), and an amine (320 mg, 1 equiv.). To the stirred reaction mixture, EDC was added (239 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 385 mg (63%).

### Preparation Example 56

### Synthesis of Compound 56

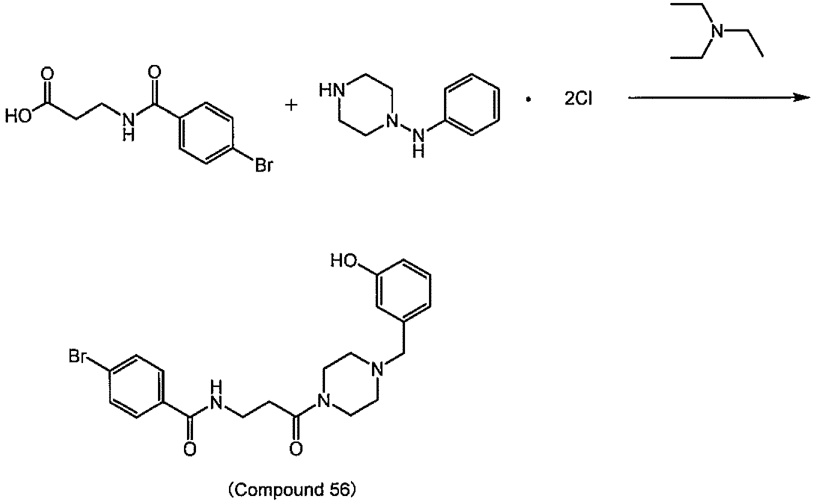

A vial was charged with a carboxylic acid (366 mg, 1 equiv.), a solvent (1 mL of a solution of 200 g N-oxybenzotriazole in 1 L of DMF), and an amine (357 mg, 1 equiv.). Triethylamine (327 mg, 2.4 equiv.) was added. To the stirred reaction mixture, EDC was added (230 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform: 2-propanol=4:1). The yield was 493 mg (82%).

### Preparation Example 57

### Synthesis of Compound 57

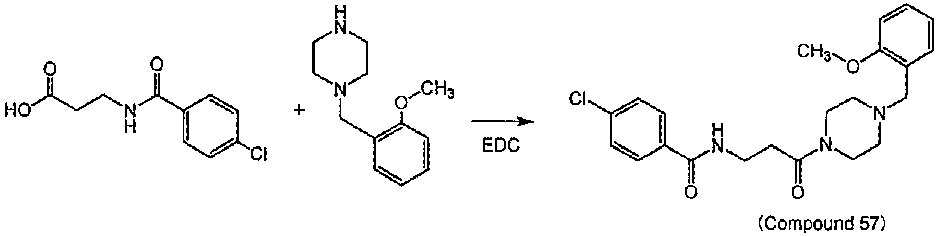

A vial was charged with a carboxylic acid (324 mg, 1 equiv.), a solvent (1 mL of a solution of 200 g N-oxybenzotriazole in 1 L of DMF), and an amine (294 mg, 1 equiv.) . To the stirred reaction mixture, EDC was added (243 mg, 1. 1 equiv.) . In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 493 mg (83%).

### Preparation Example 58

### Synthesis of Compound 58

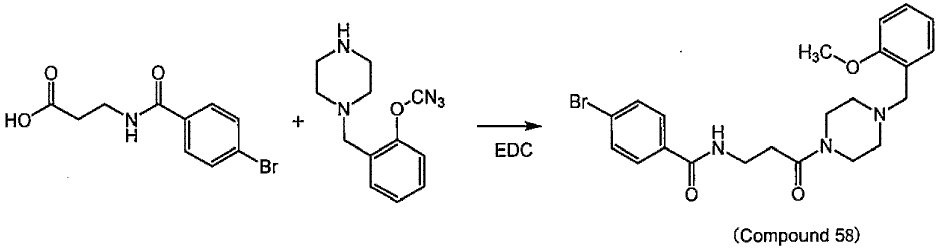

A vial was charged with a carboxylic acid (352 mg, 1 equiv.), a solvent (1 mL of a solution of 200 g N-oxybenzotriazole in 1 L of DMF), and an amine (267 mg, 1 equiv.). To the stirred reaction mixture, EDC was added (221 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was passed to the filtration. In case an oily product was formed , the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 555 mg (93%).

### Preparation Example 59

### Synthesis of Compound 59

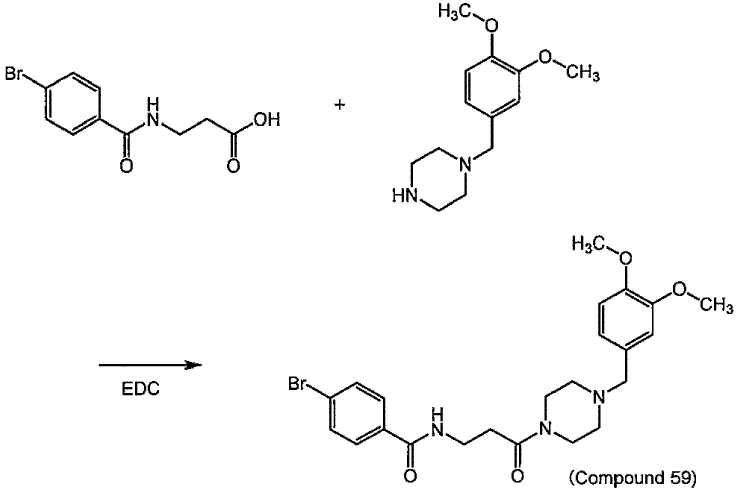

A vial was charged with a carboxylic acid (341 mg, 1 equiv.), a solvent (1 mL of a solution of 200 g N-oxybenzotriazole in 1 L of DMF), and an amine (296 mg, 1 equiv.). To the stirred reaction mixture, EDC was added (214 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 508 mg (83%).

### Preparation Example 60

### Synthesis of Compound 60

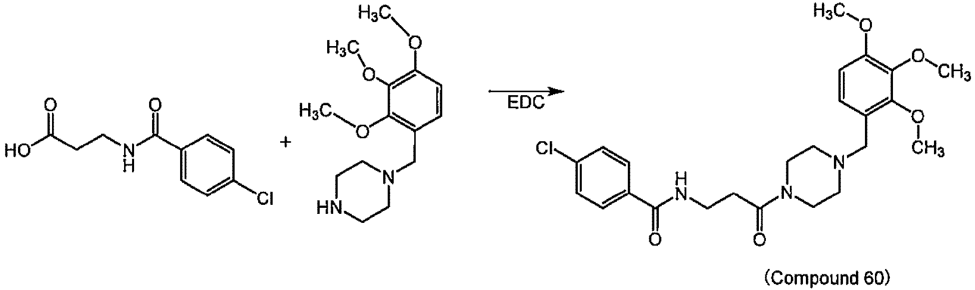

A vial was charged with a carboxylic acid (287 mg, 1 equiv.), a solvent (1 mL of a solution of 200 g N-oxybenzotriazole in 1 L of DMF), and an amine (336 mg, 1 equiv.). To the stirred reaction mixture, EDC was added (215 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 531 mg (88%).

### Preparation Example 61

### Synthesis of Compound 61

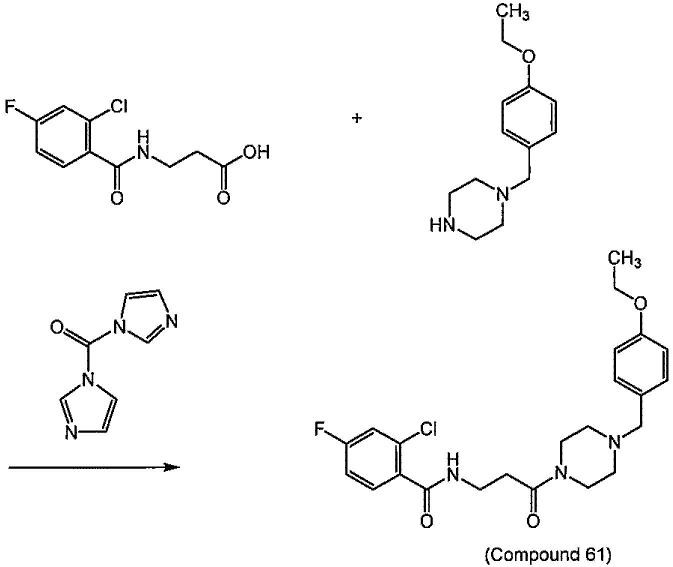

A vial was charged with a carboxylic acid (322 mg, 1 equiv.) and dry DMF (1 mL). To the stirred reaction mixture, N,N-carbodiimidazole (213 mg, 1 equiv.) was added. After 1 h of stirring, the vial was open and the reaction mixture was left for 2 h in a drying oven at 60°C. Then an amine (289 mg, 1 equiv.) was added, the vial was firmly closed, and the reaction mixture was stirred. The reaction vial was placed into a water bath and left at 100°C for 1h. The reaction mixture was cooled to room temperature and water was added until the vial was full. Then the vial was sonicated. In case a crystalline precipitate was formed, the vial was subjected to the filtration. In case an oily product was formed, the vial was left overnight, then the water layer was removed and 2-propanol (1 mL) was added to cause the crystallization. The precipitate was filtered, washed twice with a sodium carbonate solution, and then washed with a water/2-propanol (1:1) solution. The crude product was purified by chromatography (silica gel, chloroform:2-propanol=4:1). The yield was 469 mg (80%).

### Preparation Example 62

### Synthesis of Compound 62

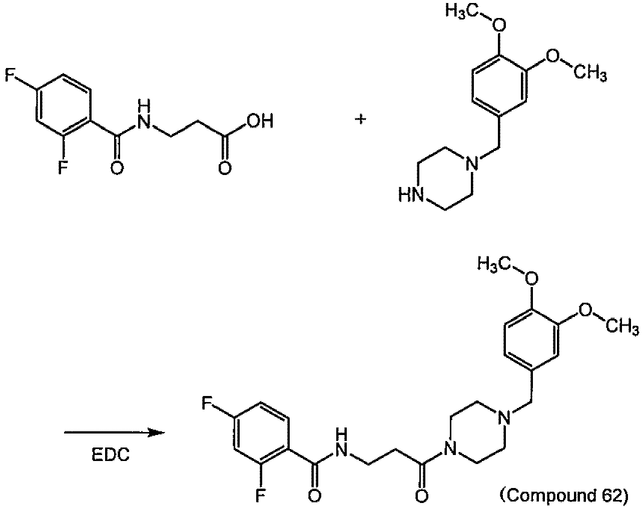

A vial was charged with a carboxylic acid (299 mg, 1 equiv.), a solvent (1 mL of a solution of 200 g N-oxybenzotriazole in 1 L of DMF), and an amine (308 mg, 1 equiv.). To the stirred reaction mixture, EDC was added (223 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 437 mg (75%).

### Preparation Example 63

### Synthesis of Compound 63

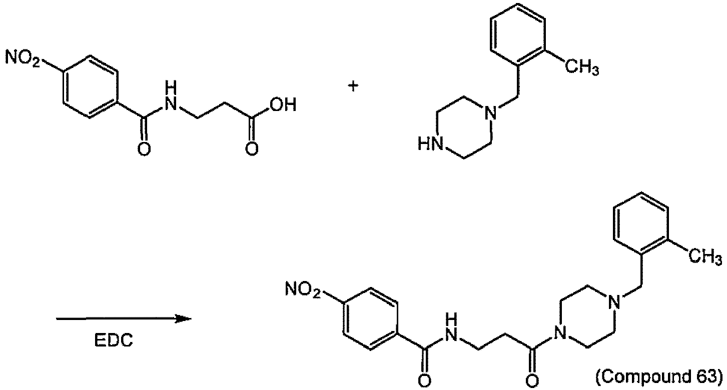

A vial was charged with a carboxylic acid (343 mg, 1 equiv.), a solvent (1 mL of a solution of 200 g N-oxybenzotriazole in 1 L of DMF), and an amine (274 mg, 1 equiv.). To the stirred reaction mixture, EDC was added (246 mg, 1.1 equiv.). In case the reaction mixture became highly viscous some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 571 mg (97%).

### Preparation Example 64

### Synthesis of Compound 64

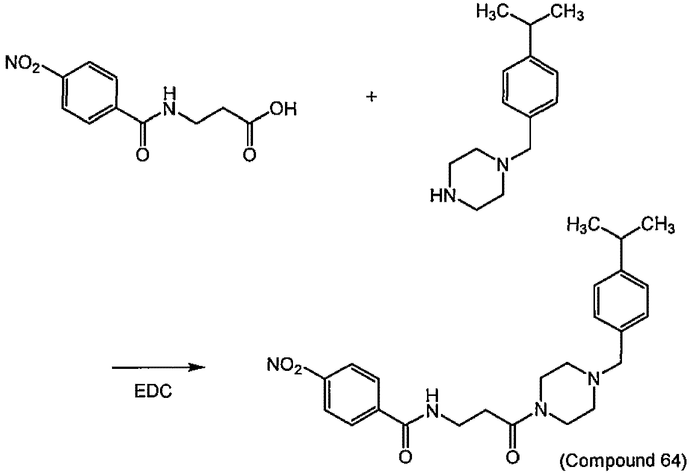

A vial was charged with a carboxylic acid (328 mg, 1 equiv.), a solvent (1 mL of a solution of 200 g N-oxybenzotriazole in 1 L of DMF), and an amine (301 mg, 1 equiv.). To the stirred reaction mixture, EDC was added (235 mg, 1.1 equiv.). In case the reaction mixture became highly viscous some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was passed to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 492 mg (81%).

### Preparation Example 65

### Synthesis of Compound 65

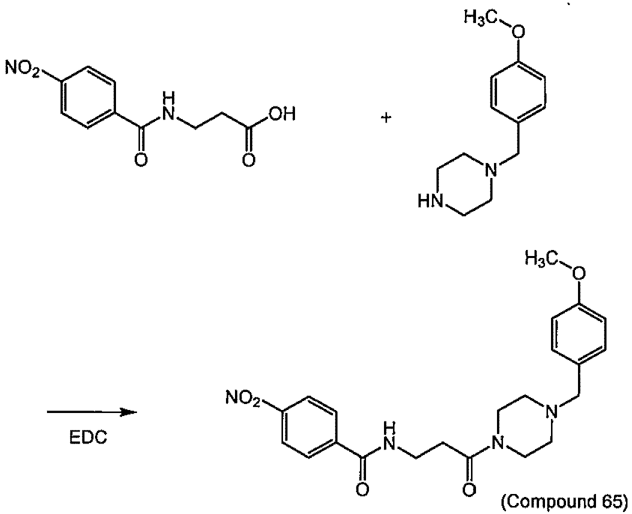

A vial was charged with a carboxylic acid (340 mg, 1 equiv.), a solvent (1 mL of a solution of 200g N-oxybenzotriazole in 1 L of DMF), and an amine (294 mg, 1 equiv.). To the stirred reaction mixture, EDC was added (244 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 467 mg (77%).

### Preparation Example 66

### Synthesis of Compound 66

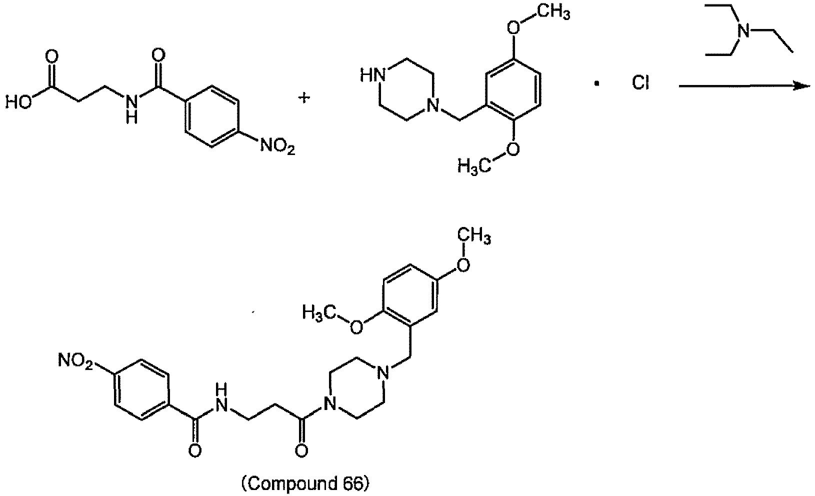

A vial was charged with a carboxylic acid (312 mg, 1 equiv.), a solvent (1 mL of a solution of 200 g N-oxybenzotriazole in 1 L of DMF), and an amine (357 mg, 1 equiv.). Triethylamine (159 mg, 1.2 equiv.) was added. To the stirred reaction mixture, EDC was added (224 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 379 mg (63%).

### Preparation Example 67

### Synthesis of Compound 67

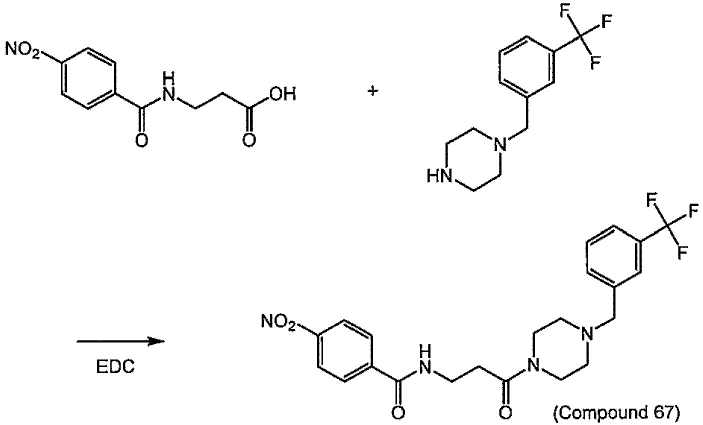

A vial was charged with a carboxylic acid (310 mg, 1 equiv.), a solvent (1 mL of a solution of 200 g N-oxybenzotriazole in 1 L of DMF), and an amine (318 mg, 1 equiv.). To the stirred reaction mixture, EDC was added (222 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 439 mg (73%).

### Preparation Example 68

### Synthesis of Compound 68

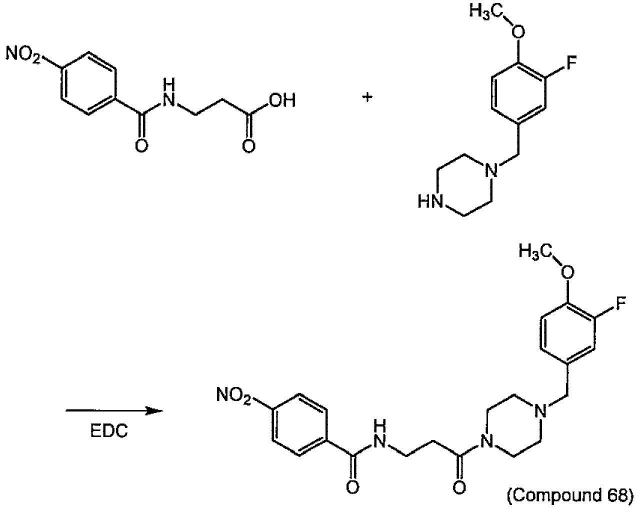

A vial was charged with a carboxylic acid (323 mg, 1 equiv.), a solvent (1 mL of a solution of 200 g N-oxybenzotriazole in 1 L of DMF), and an amine (304 mg, 1 equiv.). To the stirred reaction mixture, EDC was added (232 mg, 1.1 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 371 mg (62%).

### Preparation Example 69

### Synthesis of Compound 69

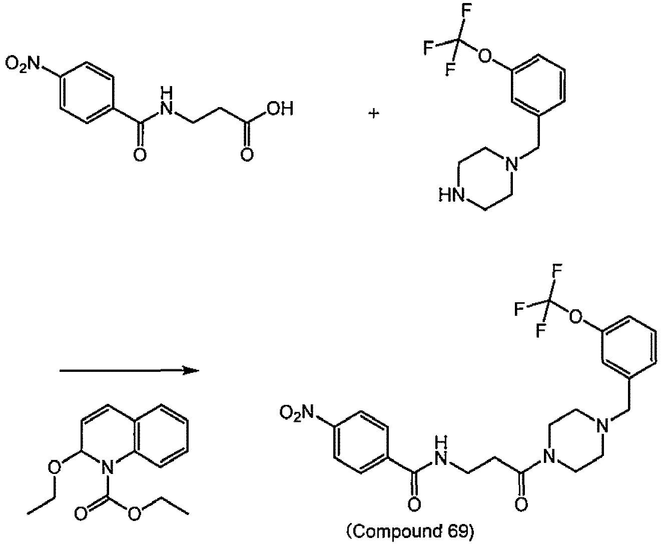

A vial is charged with a carboxylic acid (299 mg, 1), 1 mL of solvent (3.4 g of imidazole in 200 mL of dry DMF), and an amine (327 mg, 1 equiv.). To the stirred reaction mixture, ethyl 2-ethoxyquinoline-1(2H)-carboxylate (372 mg, 1.2 equiv.) was added. In case the reaction mixture was a homogeneous solution, it was kept at room temperature for 72 hrs. Otherwise the reaction mixture was sonicated at room temperature for 5 days. The reaction mixture was carefully diluted with 2% hydrochloric acid and then it was left for 24 hrs. Then the reaction mixture was sonicated. In case a crystalline precipitate was formed, the vial was subjected to the filtration. In case an oily product was formed, the vial was left overnight, then the water layer was removed, and 2-propanol (1 mL) was added to cause the crystallization. The precipitate was filtered, washed with a sodium carbonate solution and methanol. The crude product was purified by chromatography (silica gel, chloroform with 2-propanol=4:1). The yield was 223 mg (37%).

### Preparation Example 70

### Synthesis of Compound 70

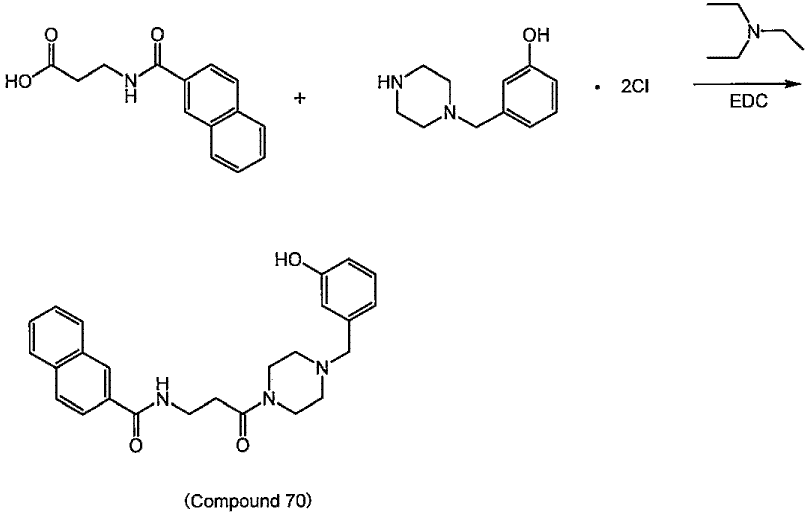

A vial was charged with a carboxylic acid (386 mg, 1.1 equiv.), a solvent (1 mL of a solution of 200 g N-oxybenzotriazole in 1 L of DMF), and an amine (383 mg, 1 equiv.). Triethylamine (350 mg, 2.4 equiv.) was added. To the stirred reaction mixture, EDC was added (271 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 587 mg (97%).

### Preparation Example 71

### Synthesis of Compound 71

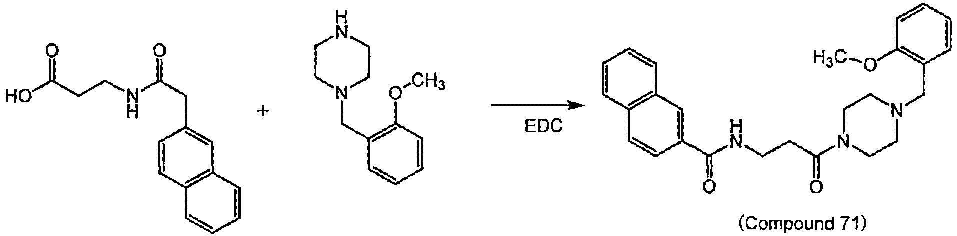

A vial was charged with a carboxylic acid (371 mg, 1.1 equiv.), a solvent (1 mL of a solution of 200 g N-oxybenzotriazole in 1 L of DMF), and an amine (286 mg, 1 equiv.). To the stirred reaction mixture, EDC was added (260 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was subjected to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary). The crude product was purified by chromatography (silica gel, chloroform:2-propanol=4:1). The yield was 412 mg (69%).

### Preparation Example 72

### Synthesis of Compound 72

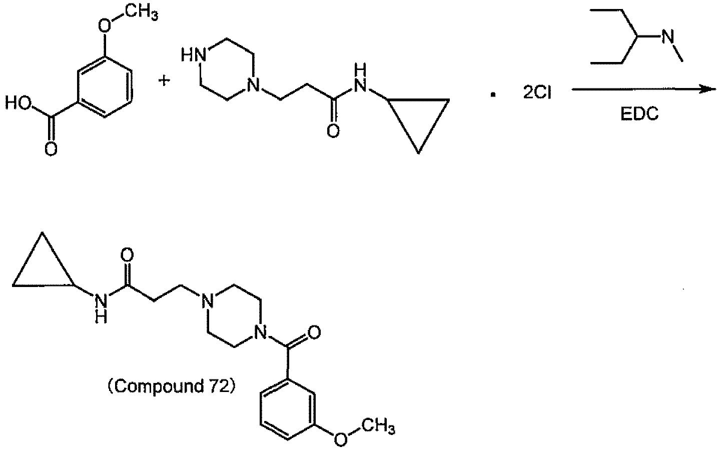

A vial was charged with a carboxylic acid (133 mg, 1.1 equiv.), a solvent (1 mL of a solution of 200 g N-oxybenzotriazole in 1 L of DMF), and an amine (215 mg, 1 equiv.). Triethylamine (193 mg, 2.4 equiv.) was added. To the stirred reaction mixture, EDC was added (149 mg, 1.21 equiv.). In case the reaction mixture became highly viscous, some more DMF was added. In case the reaction mixture was a homogeneous solution, it was kept at room temperature for 72 hrs. Otherwise the reaction mixture was sonicated at room temperature for 5 days. The reaction mixture was diluted with 1% aqueous sodium phosphate solution until the vial was full. Then the vial was sonicated. In case a crystalline precipitate was formed, the vial was passed to the filtration. In case an oily product was formed, the product was dissolved in methanol and precipitated by an addition of 4% hydrochloric acid. Alternatively 2-propanol (1 mL) was mixed with the crude product and the mixture was sonicated. Then the solution was diluted with 5% aqueous sodium hydrogen carbonate solution (the procedure repeated 2-3 times if necessary) . The crude product was purified by chromatography (silica gel, chloroform:2-propanol=4:1). The yield was 29 mg (11%).

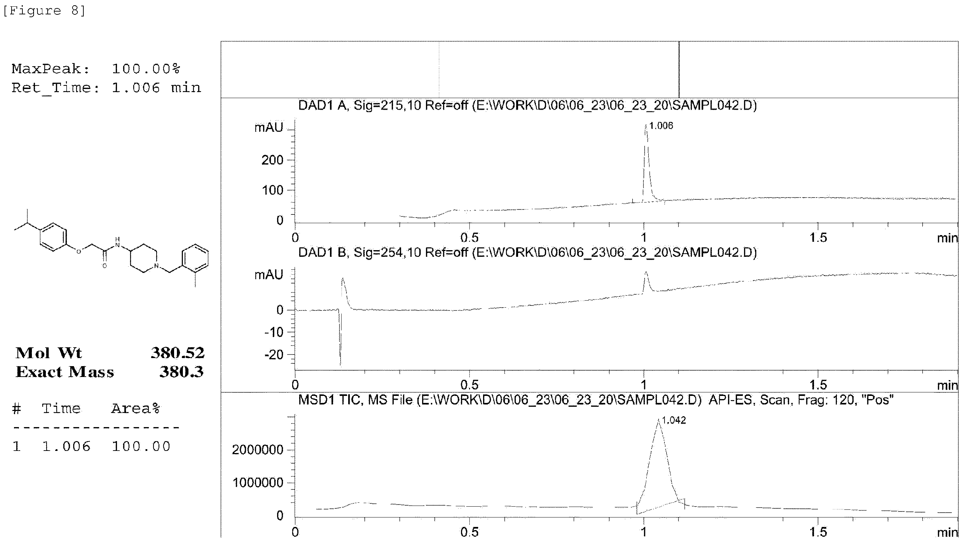

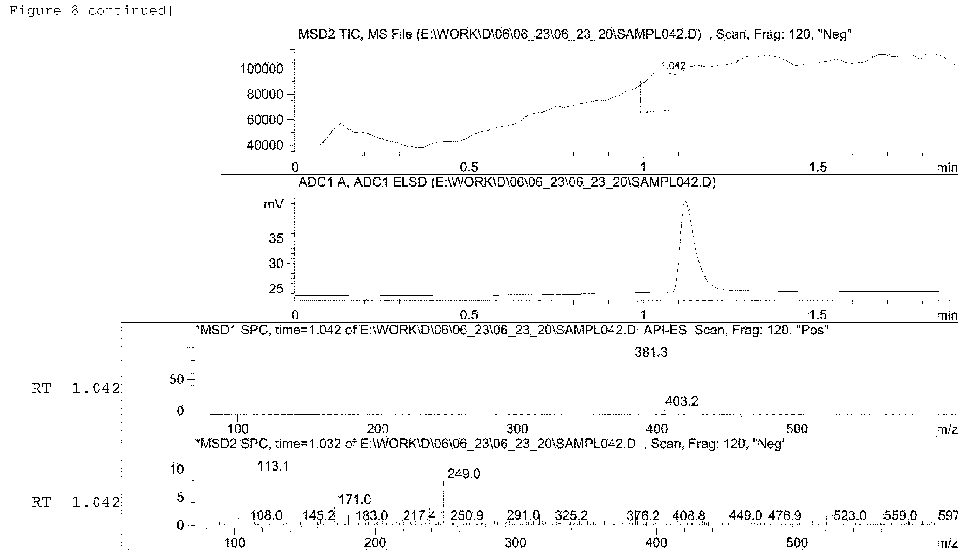

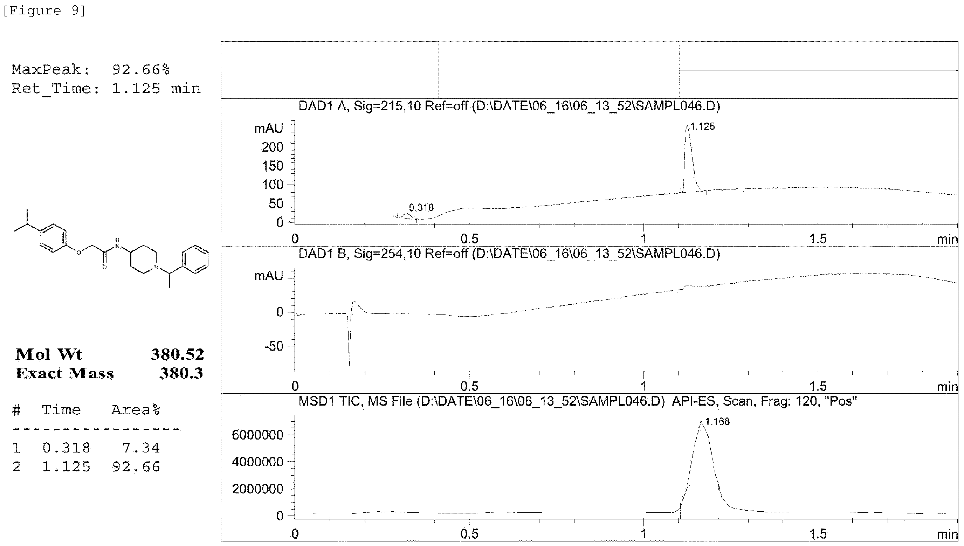

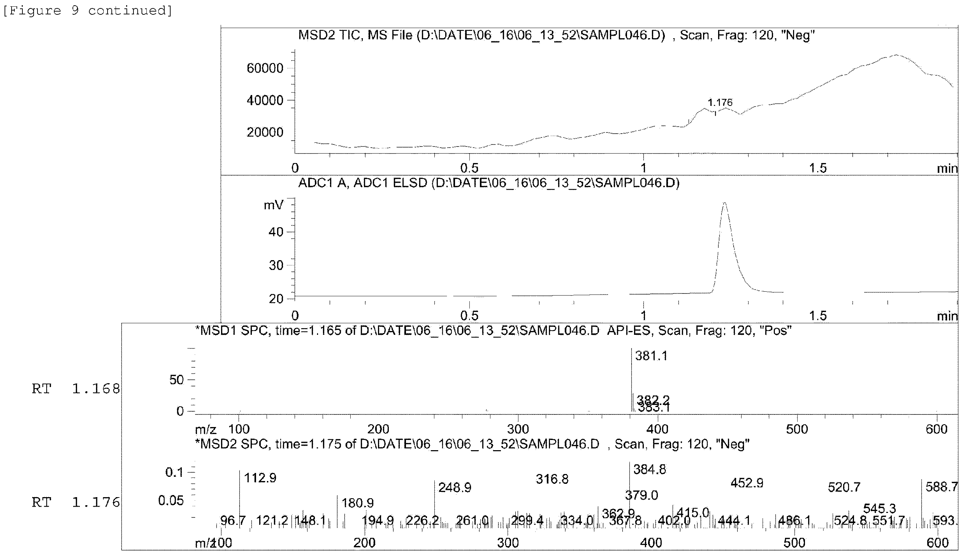

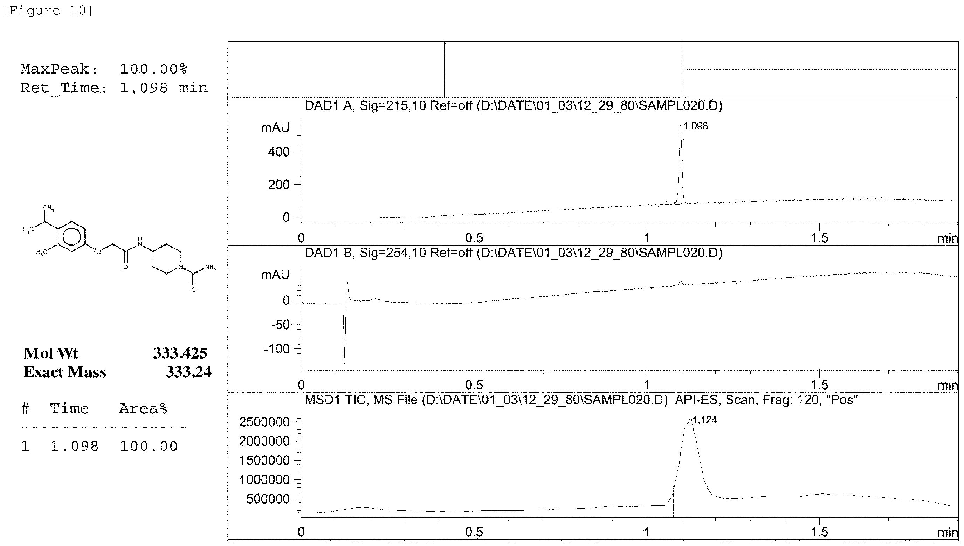

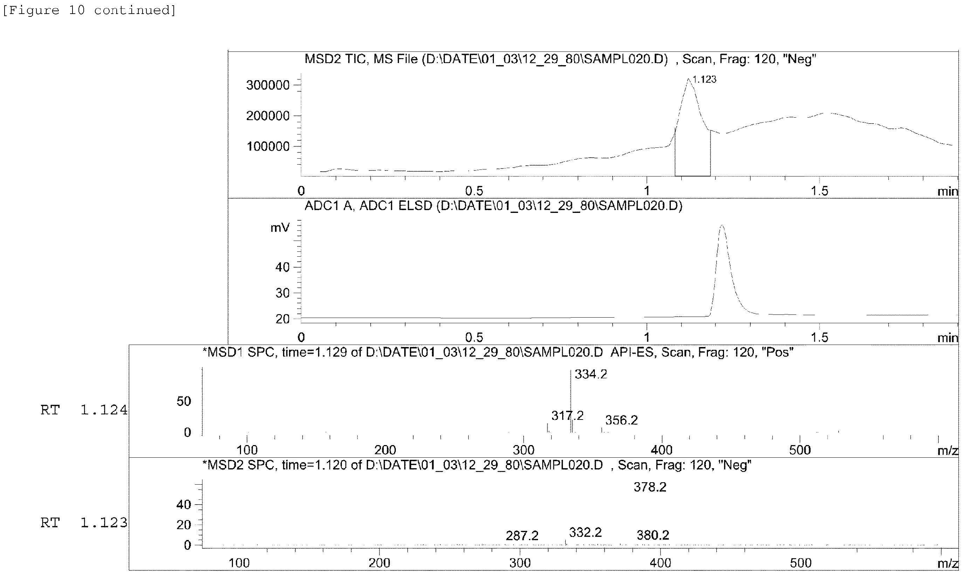

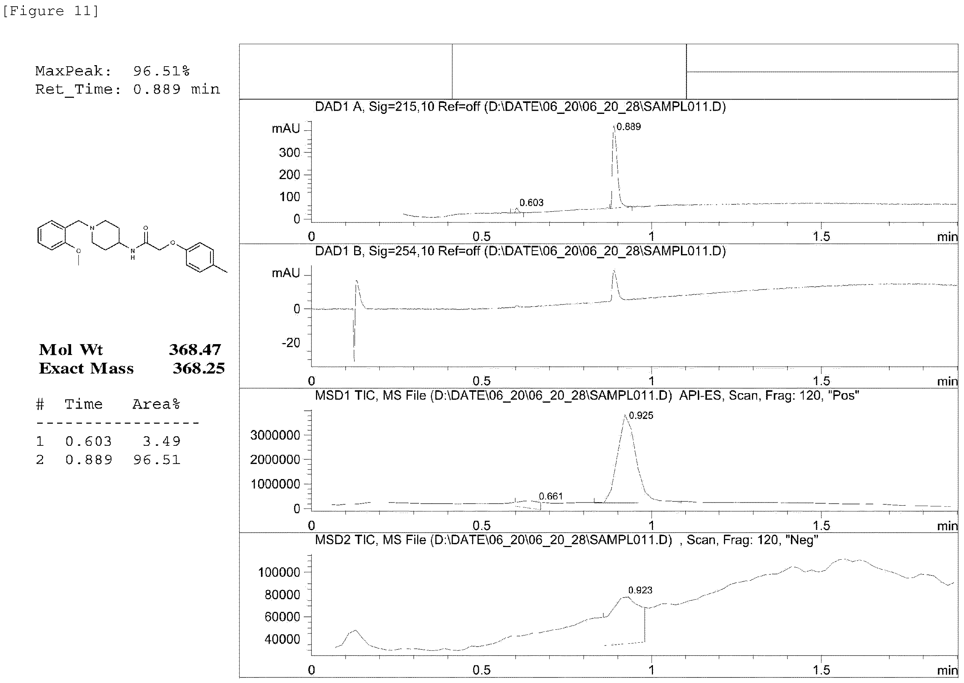

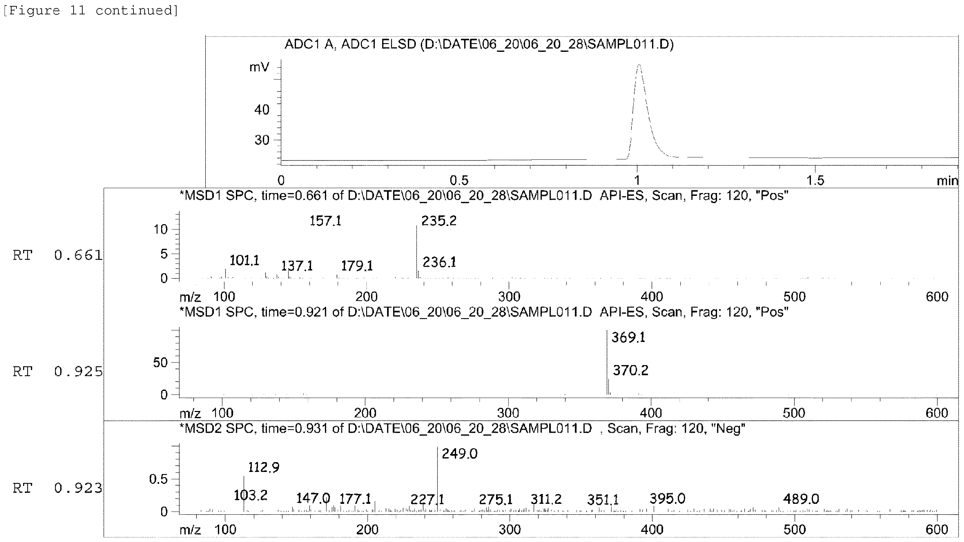

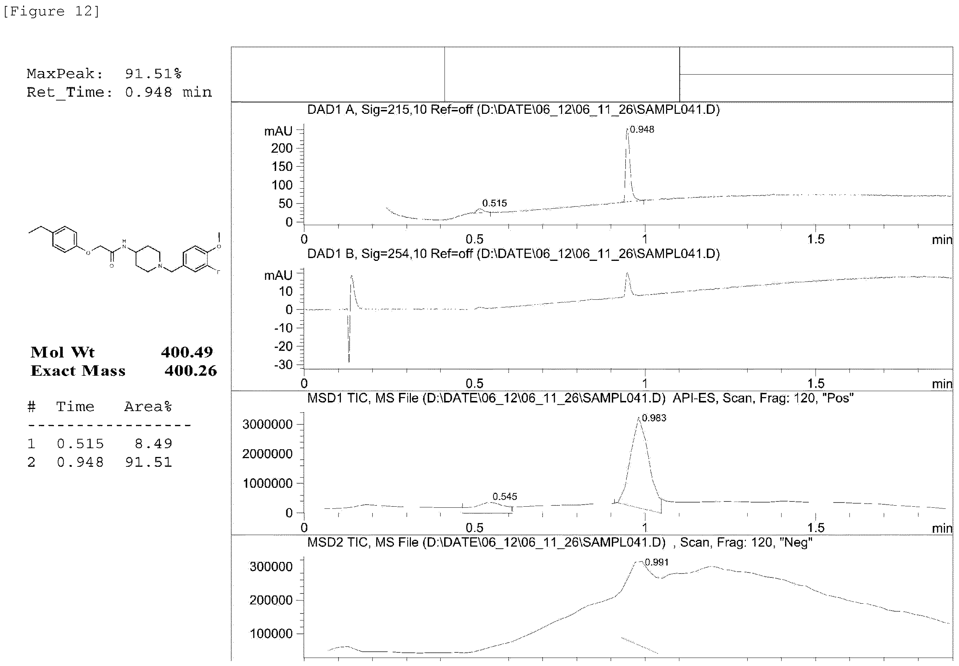

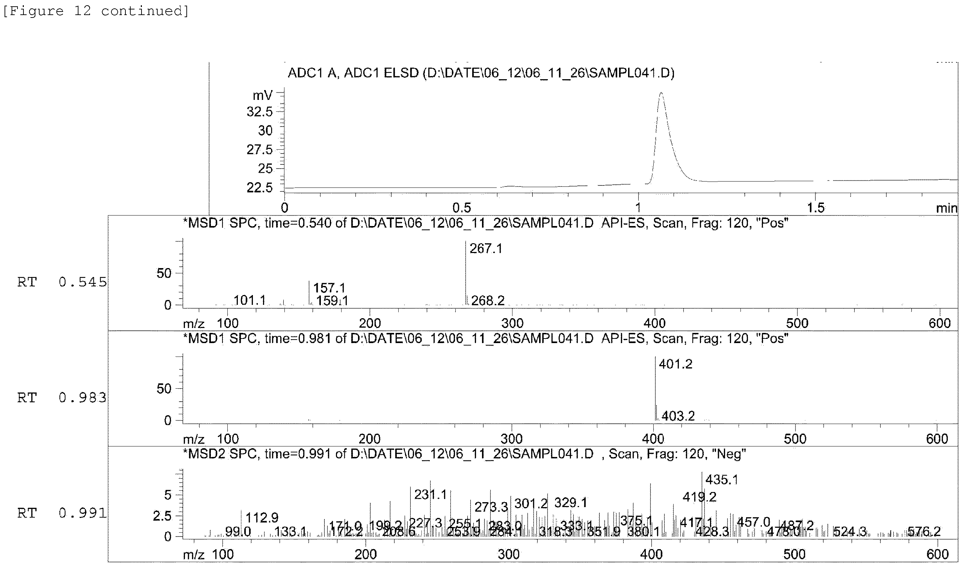

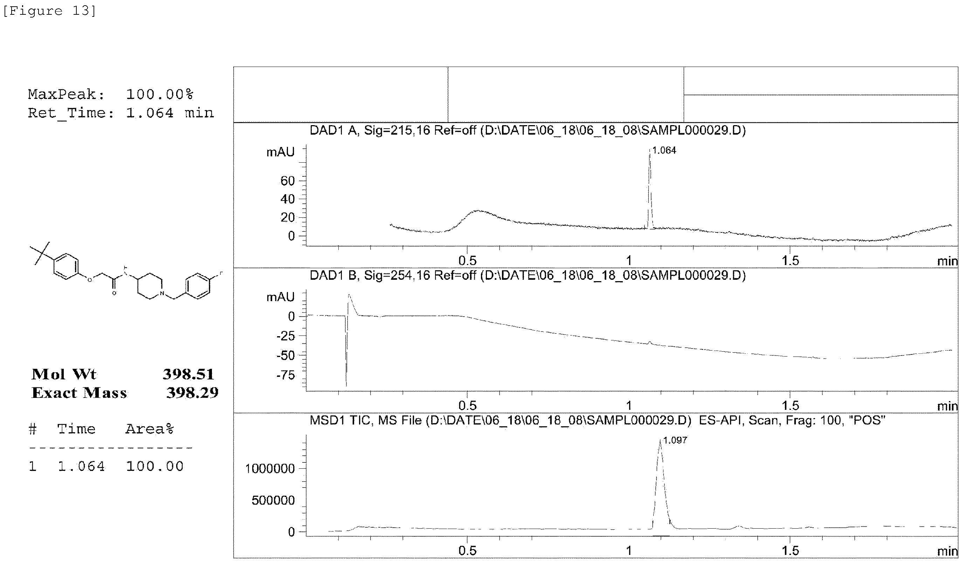

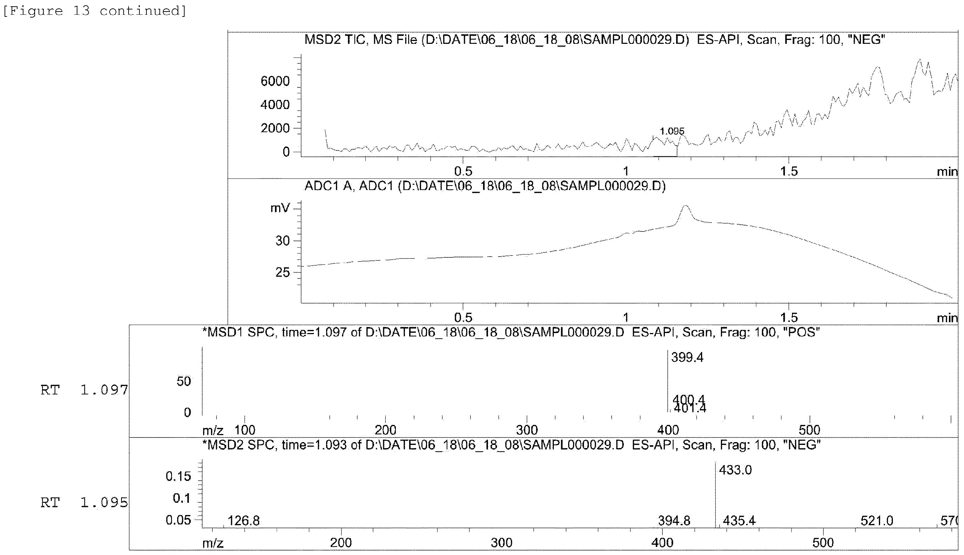

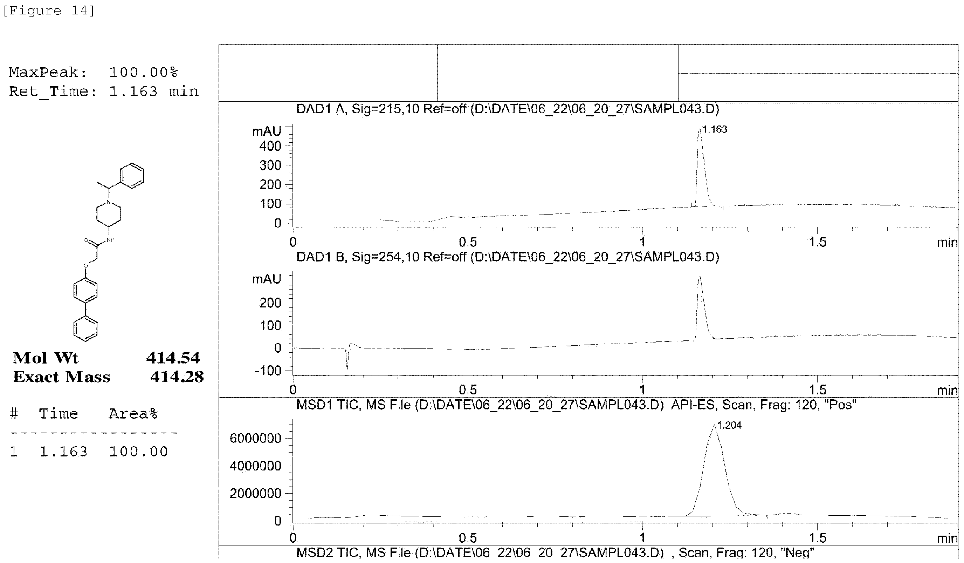

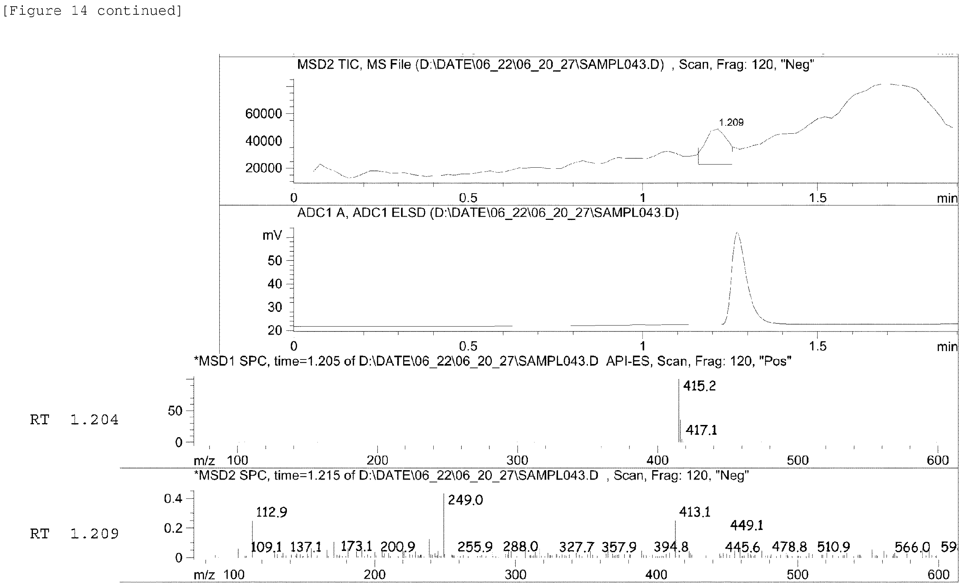

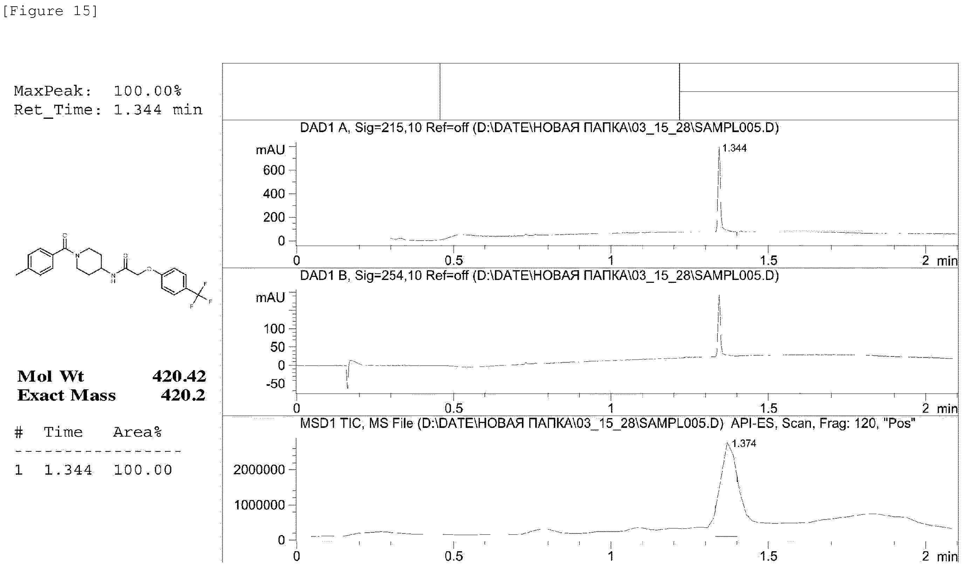

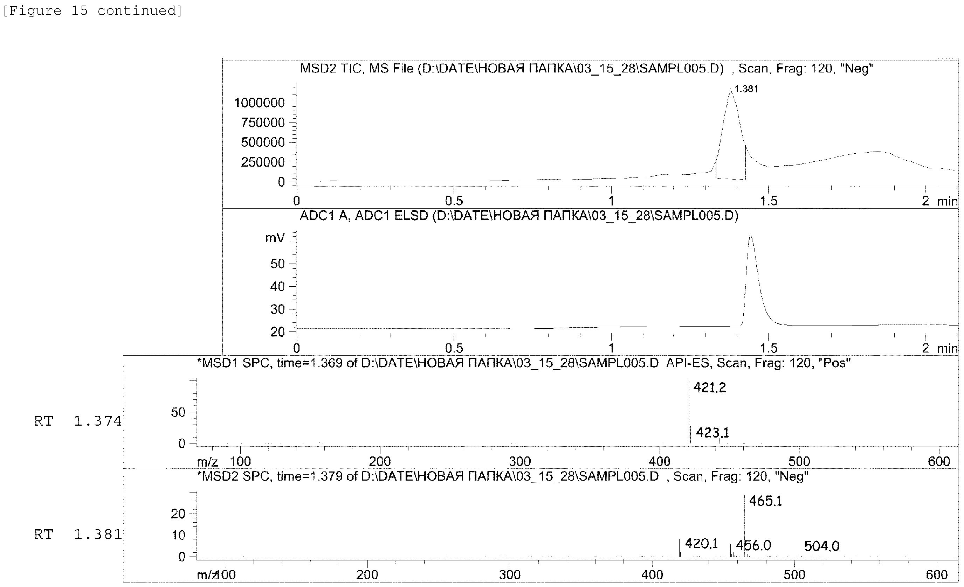

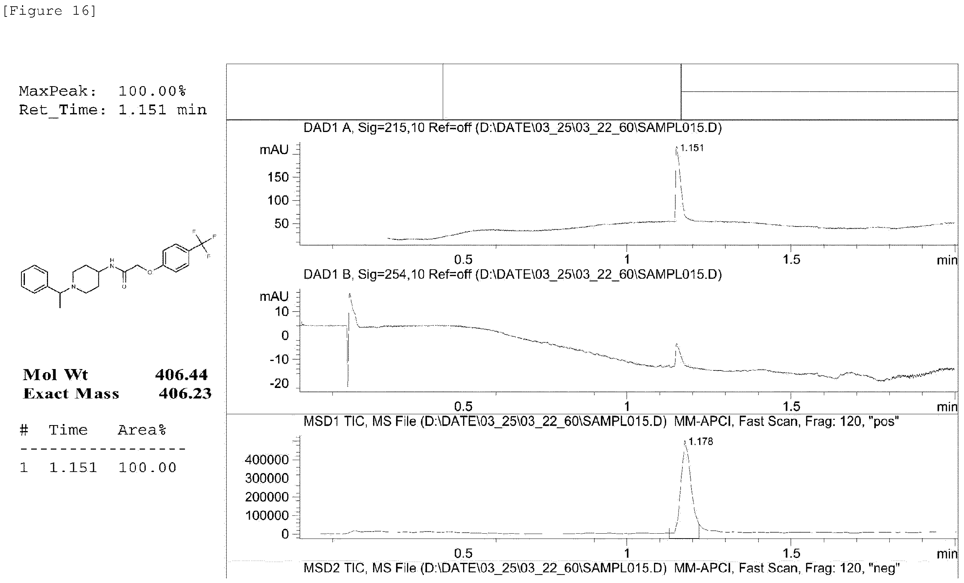

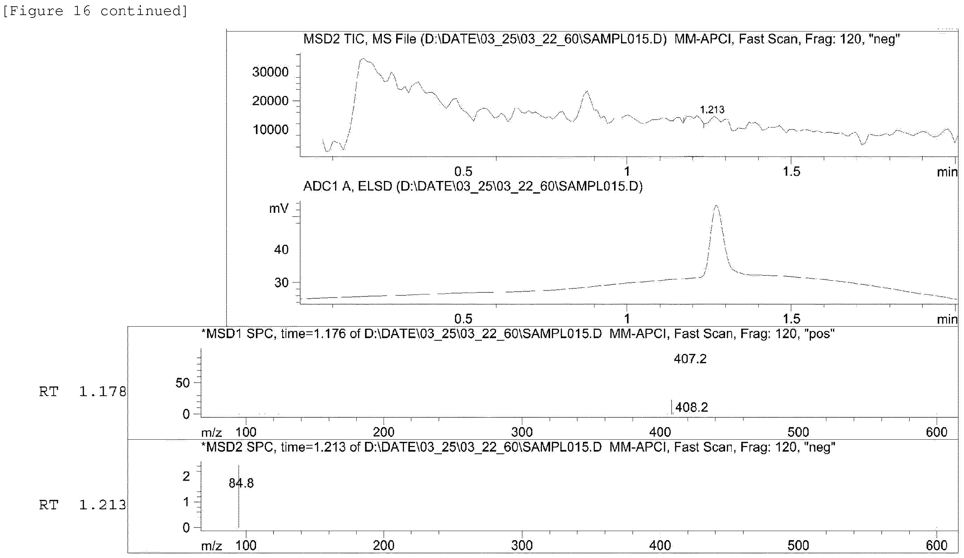

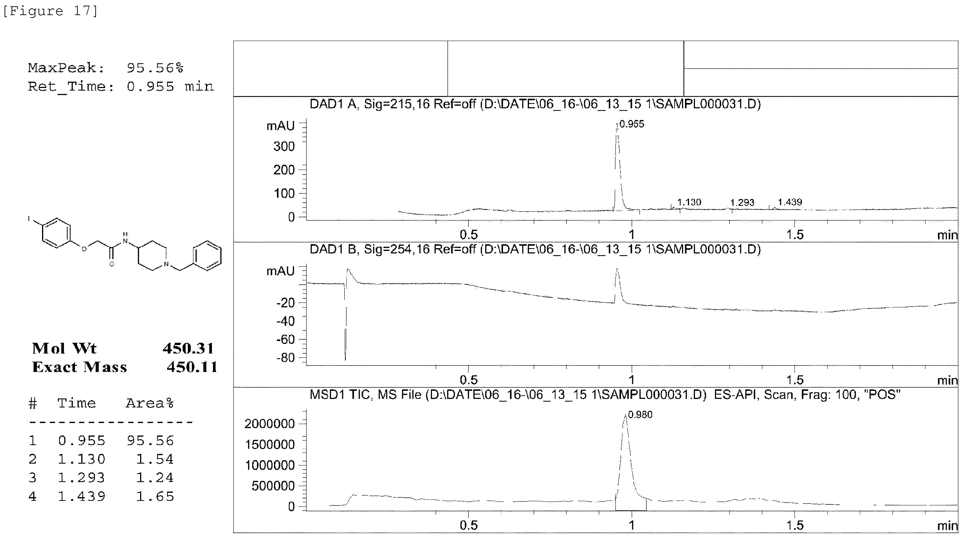

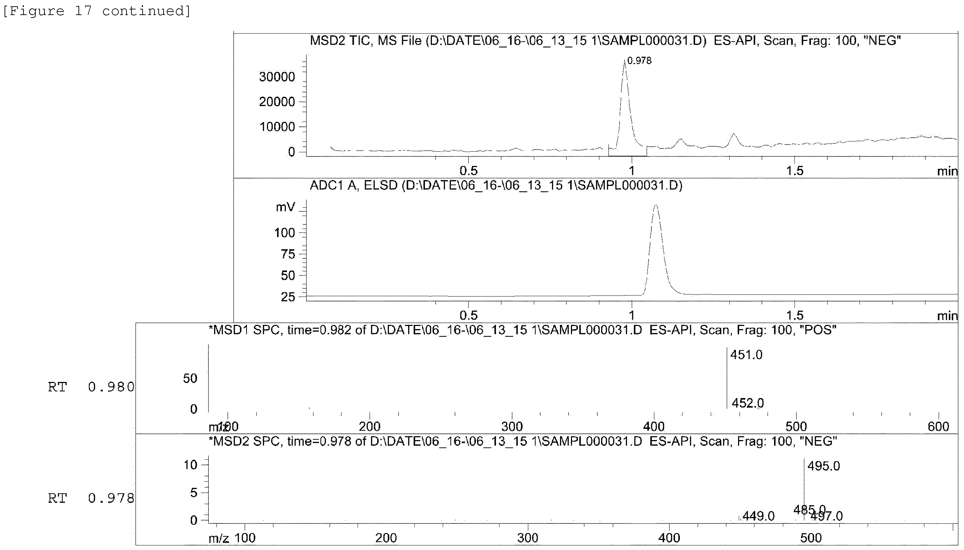

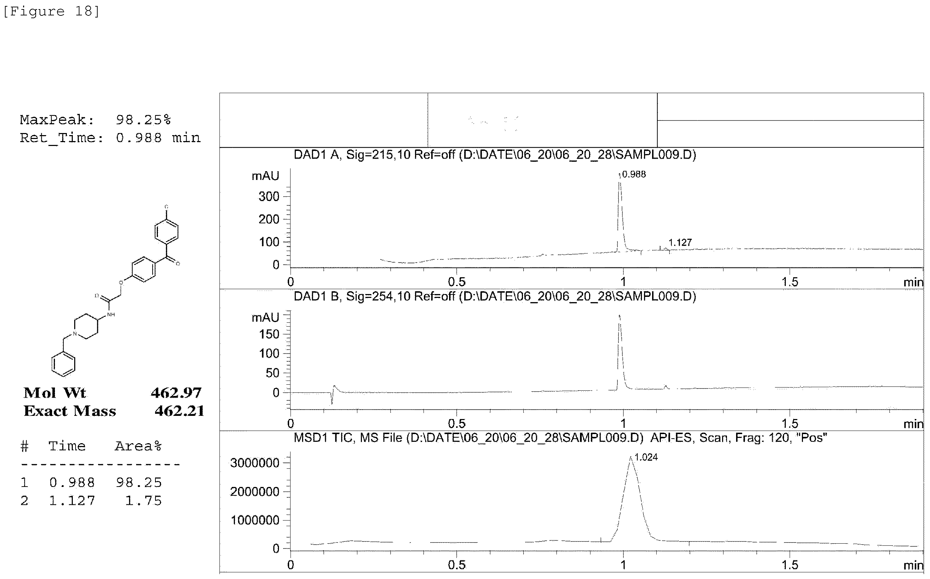

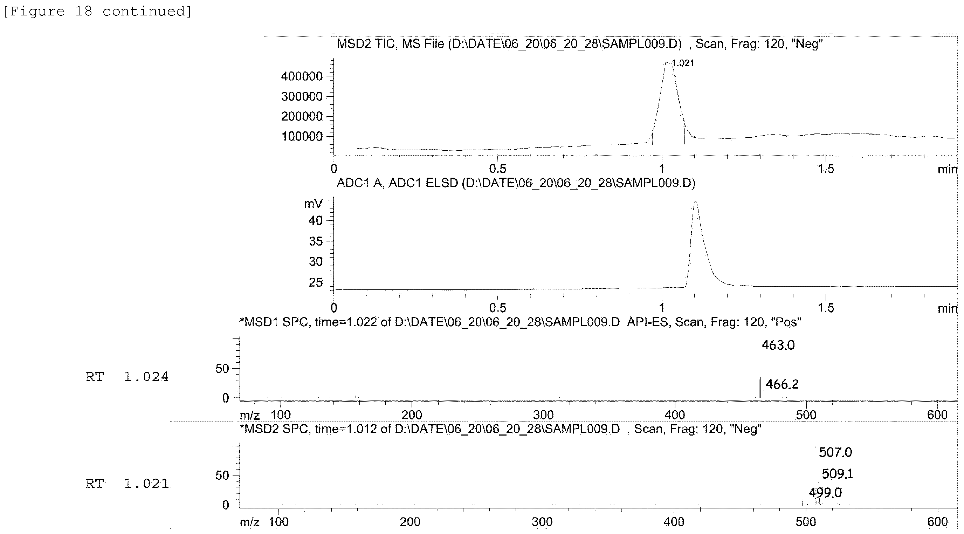

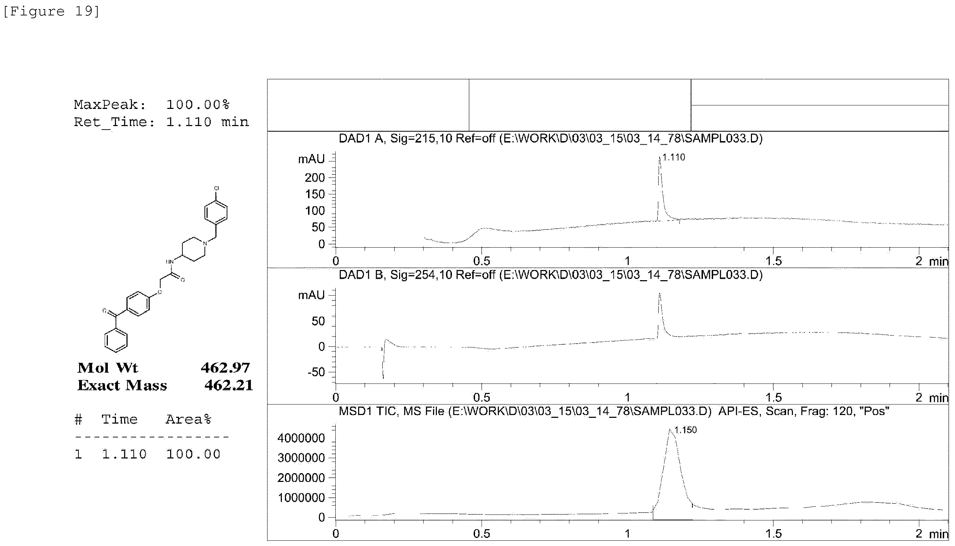

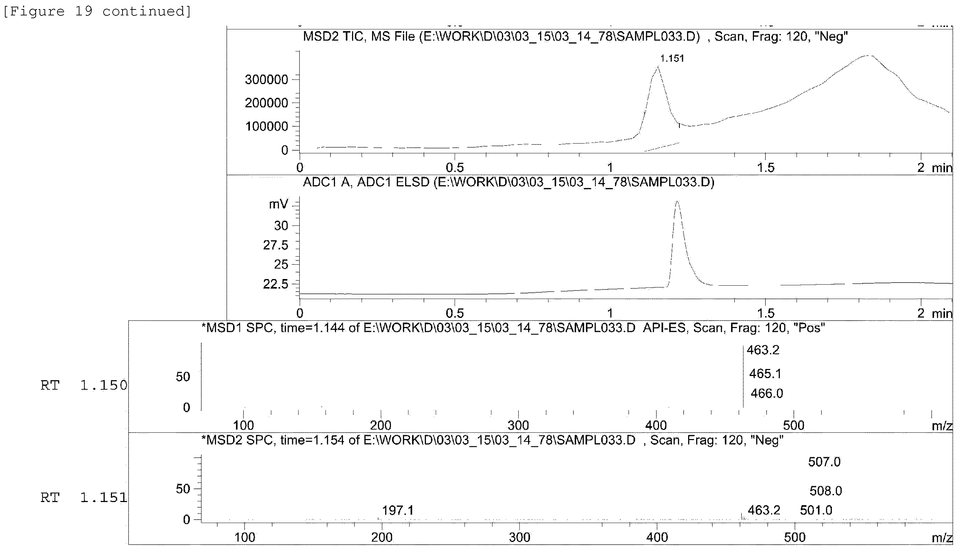

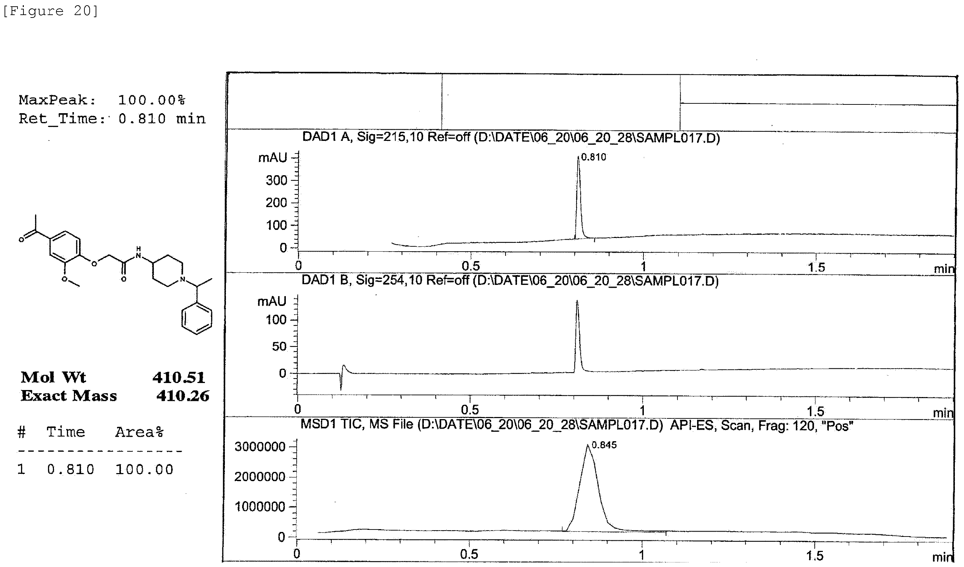

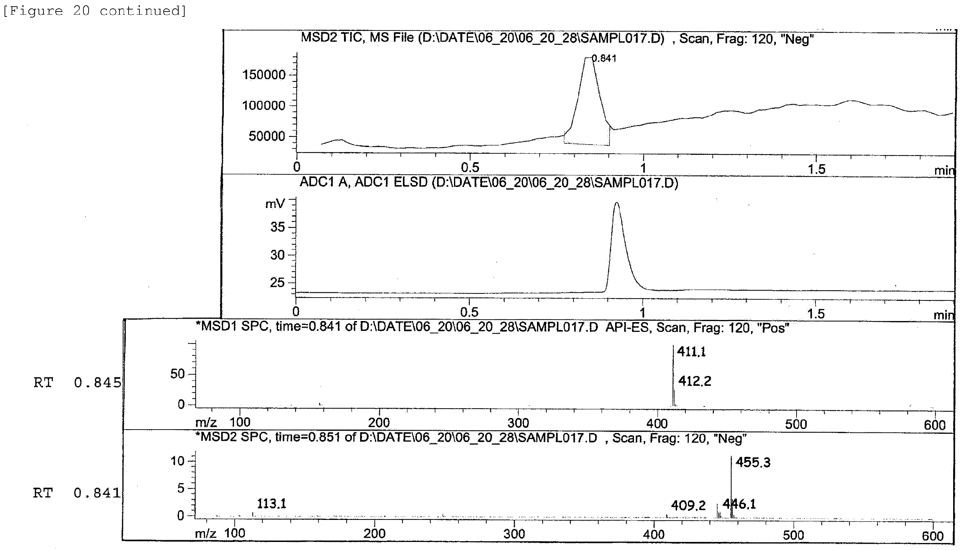

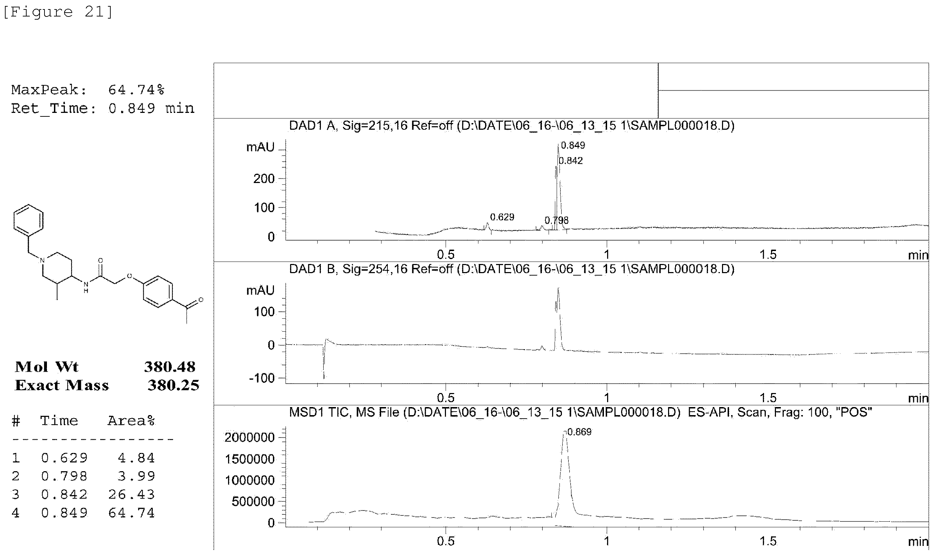

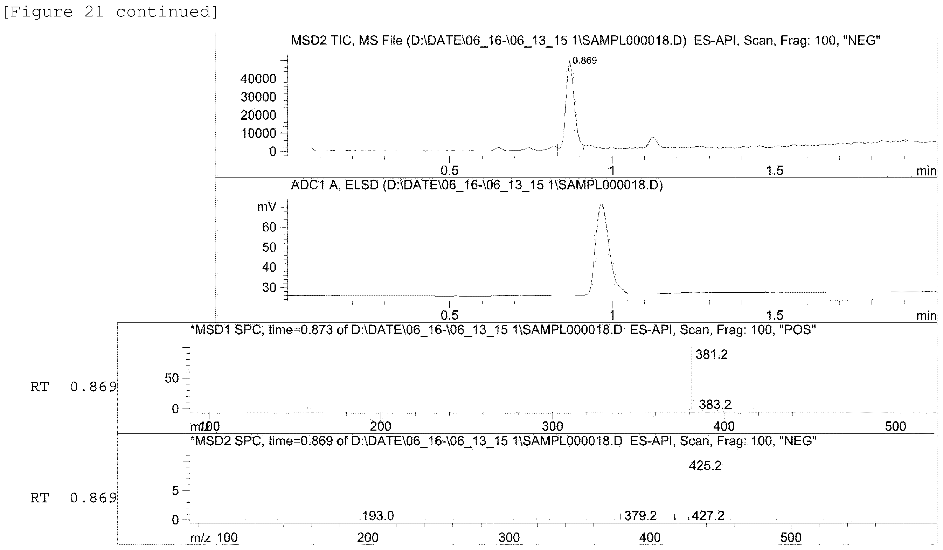

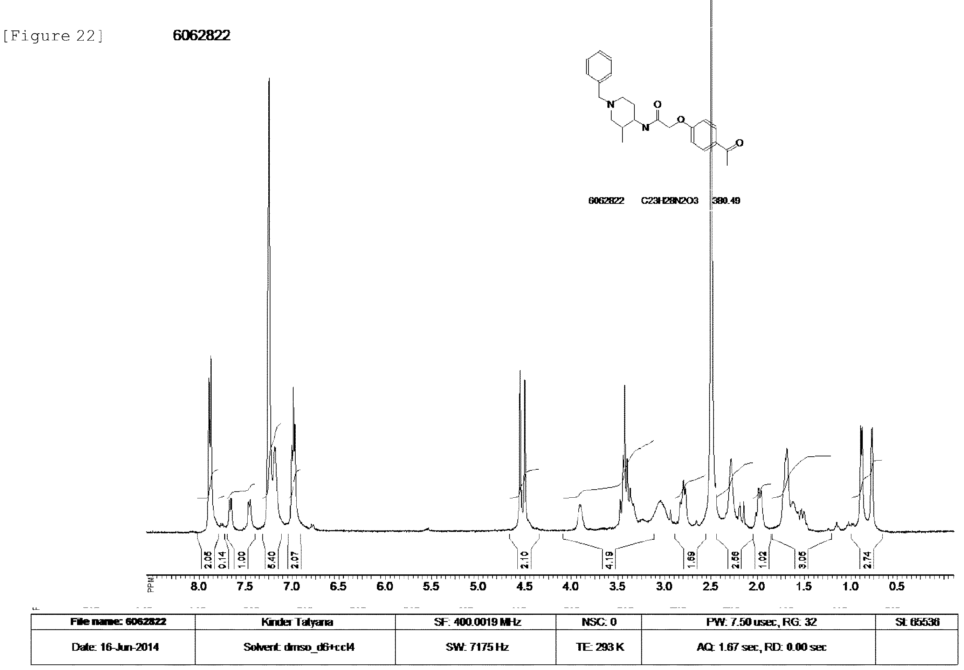

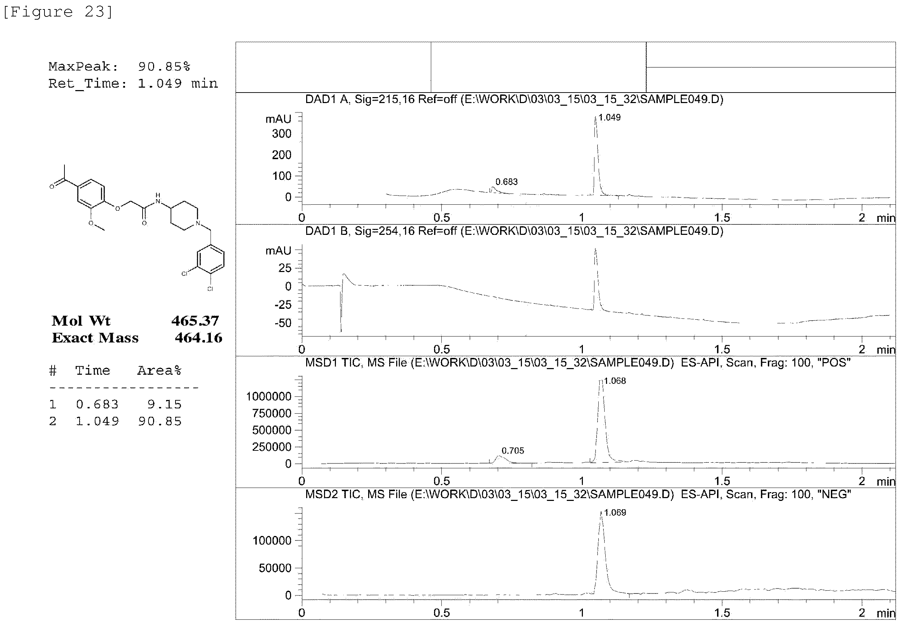

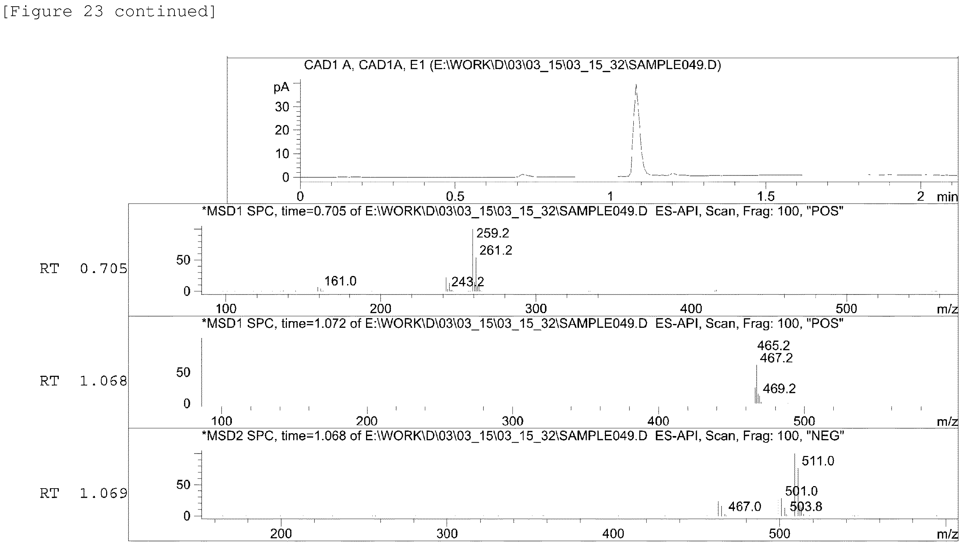

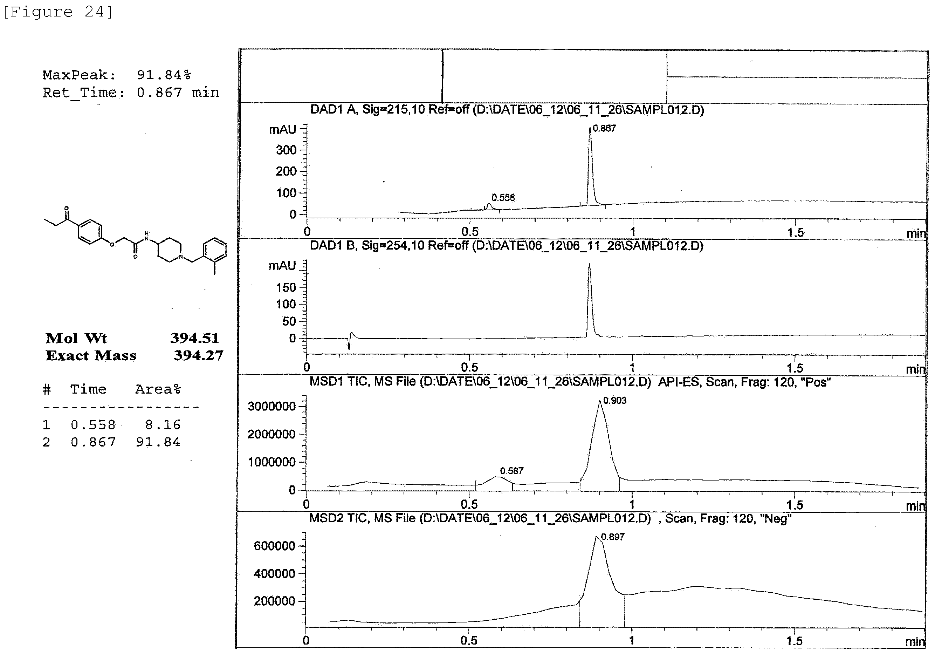

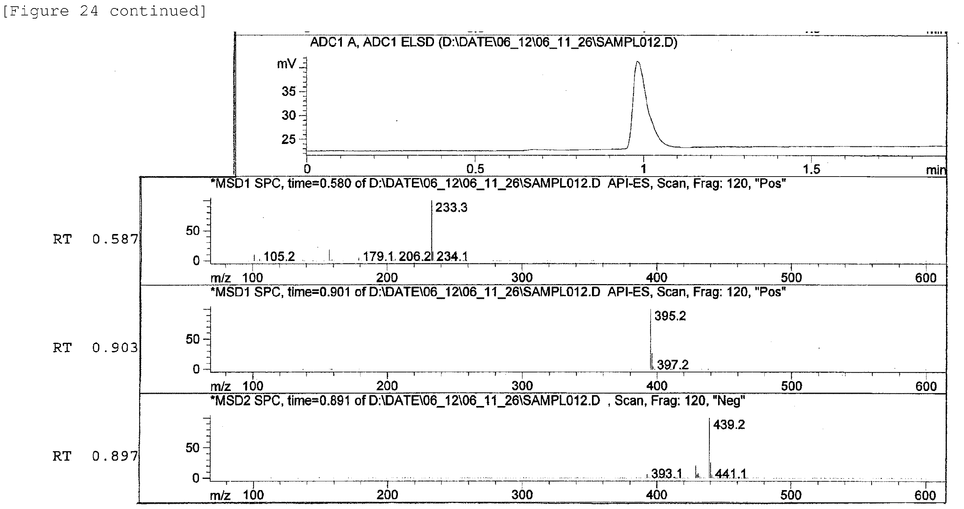

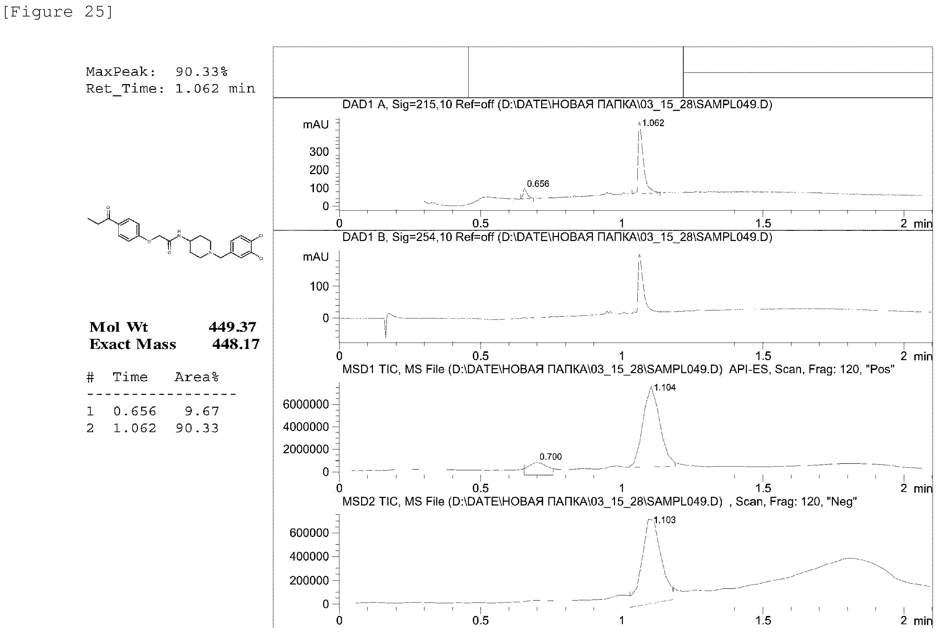

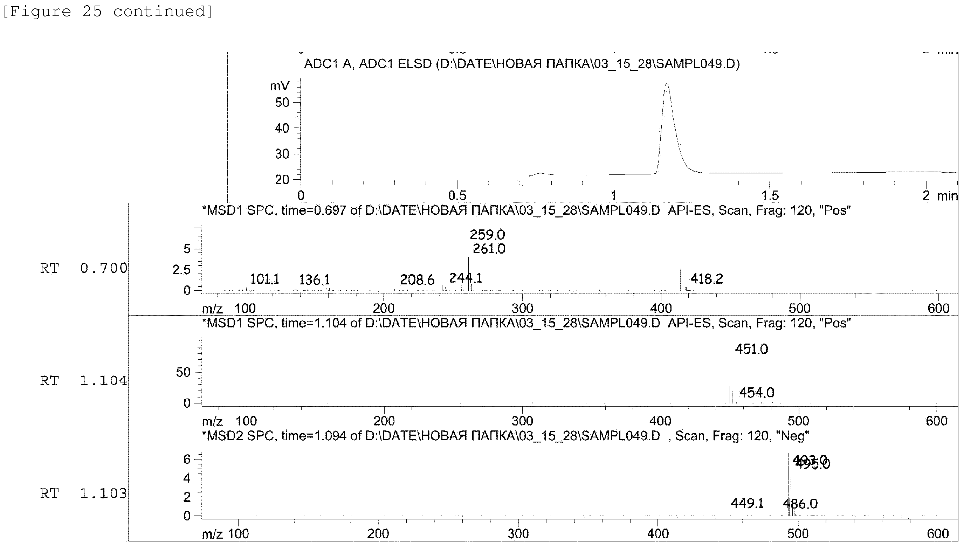

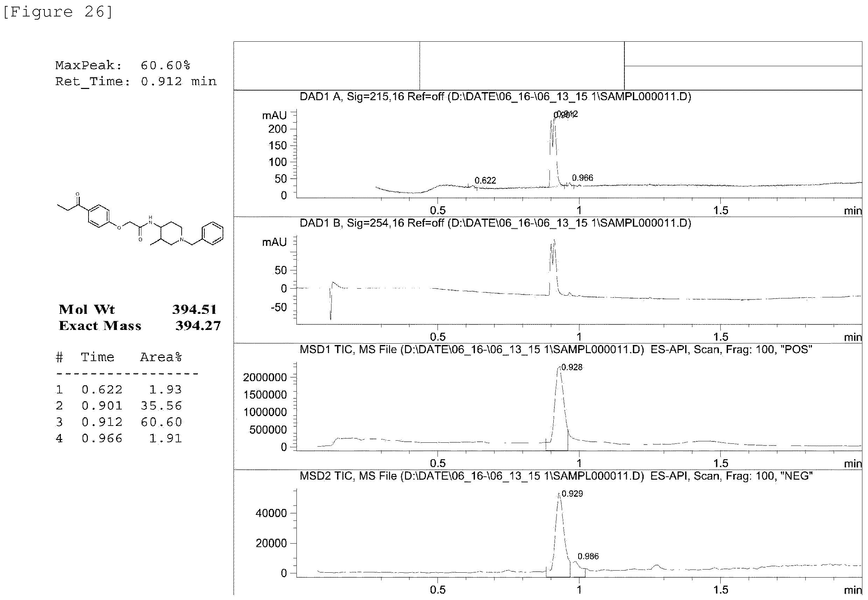

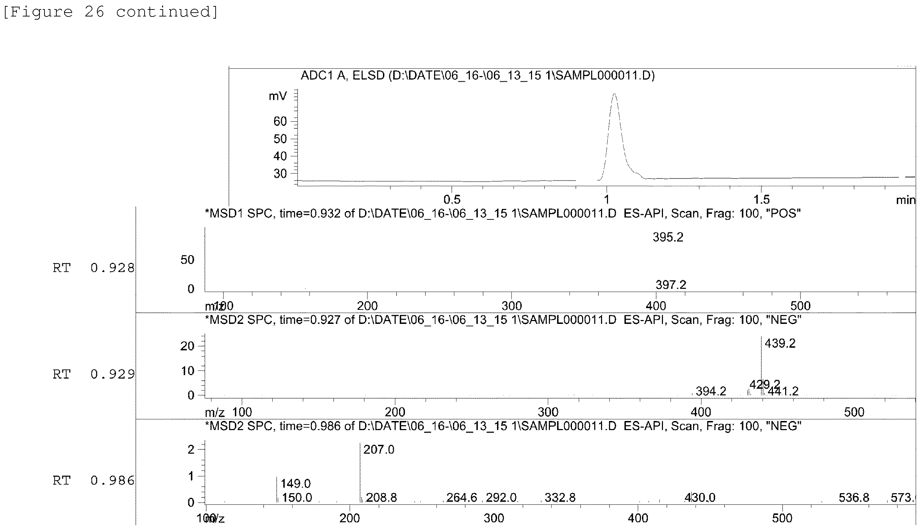

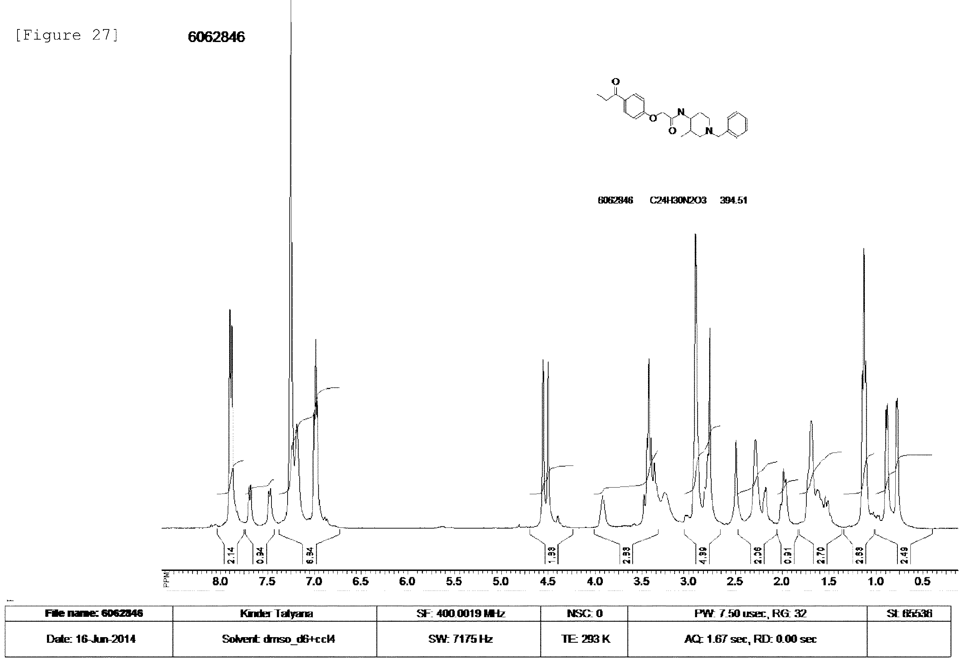

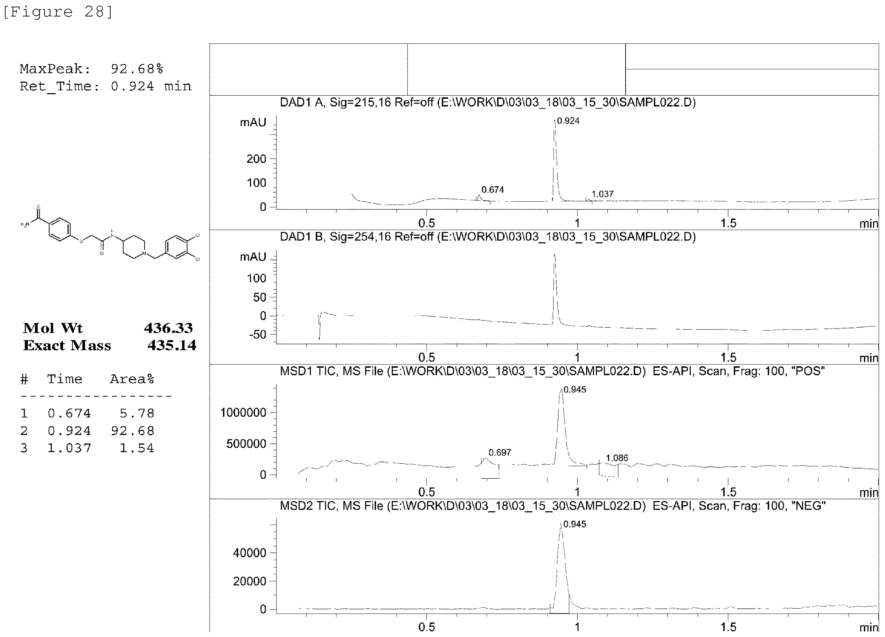

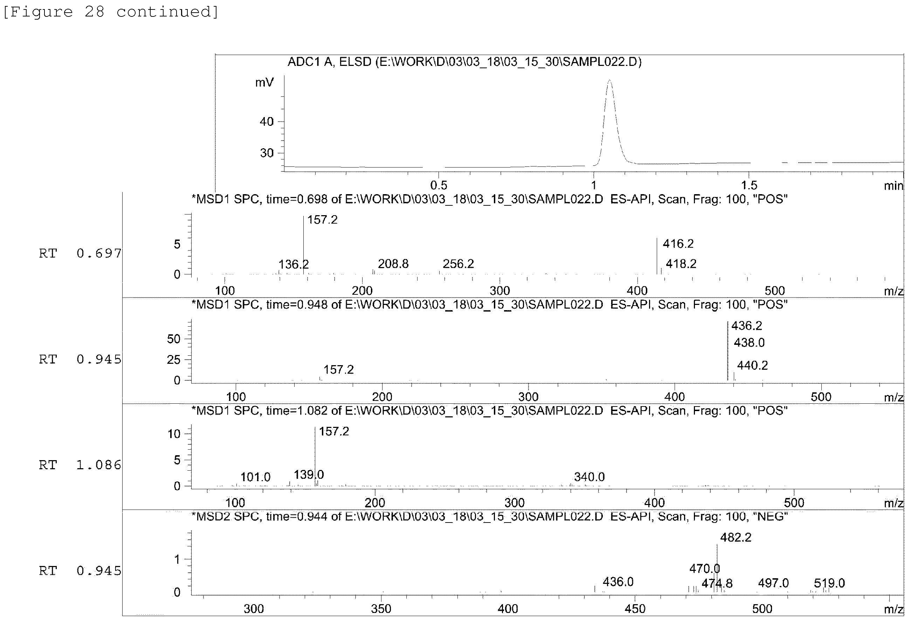

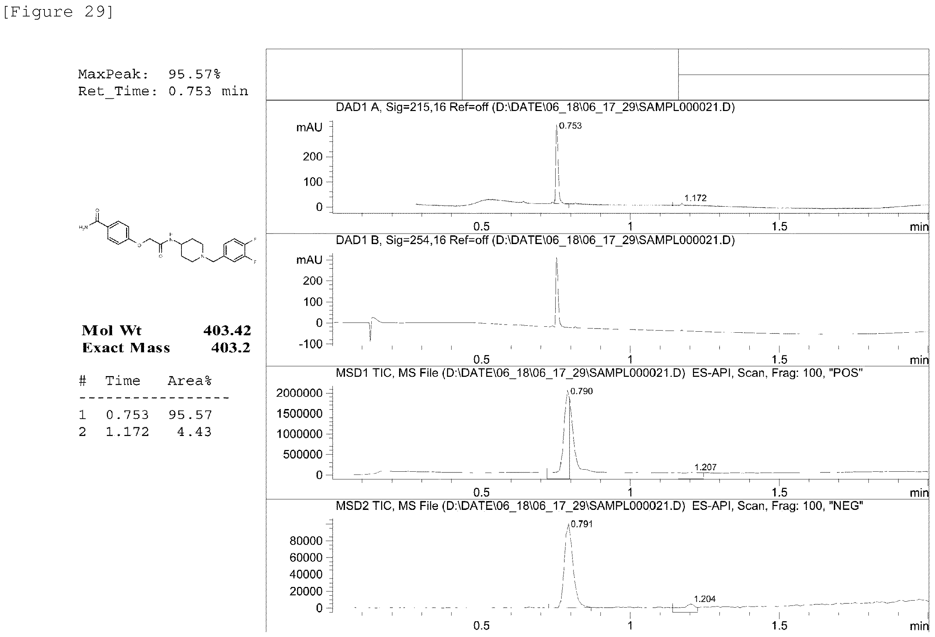

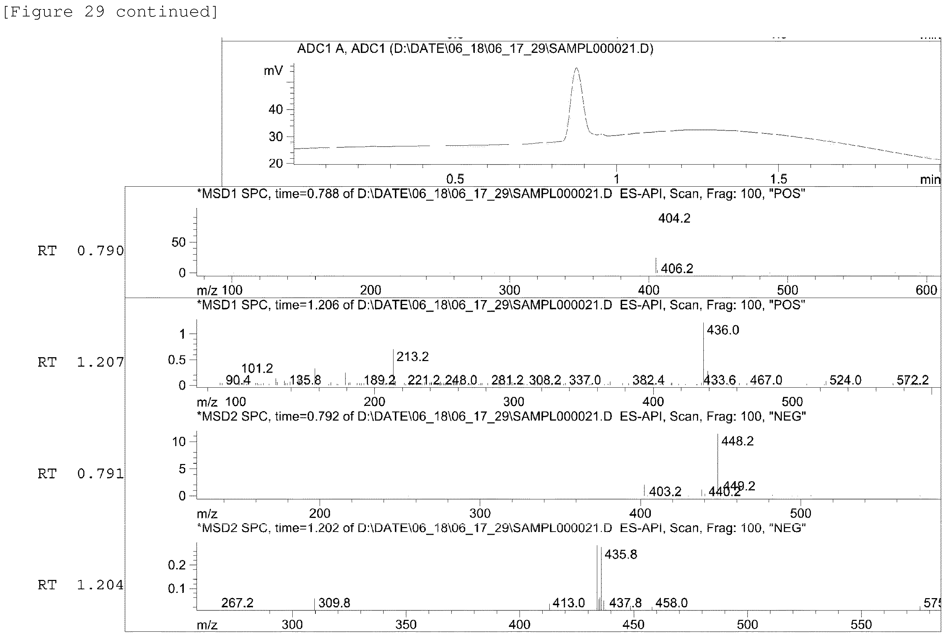

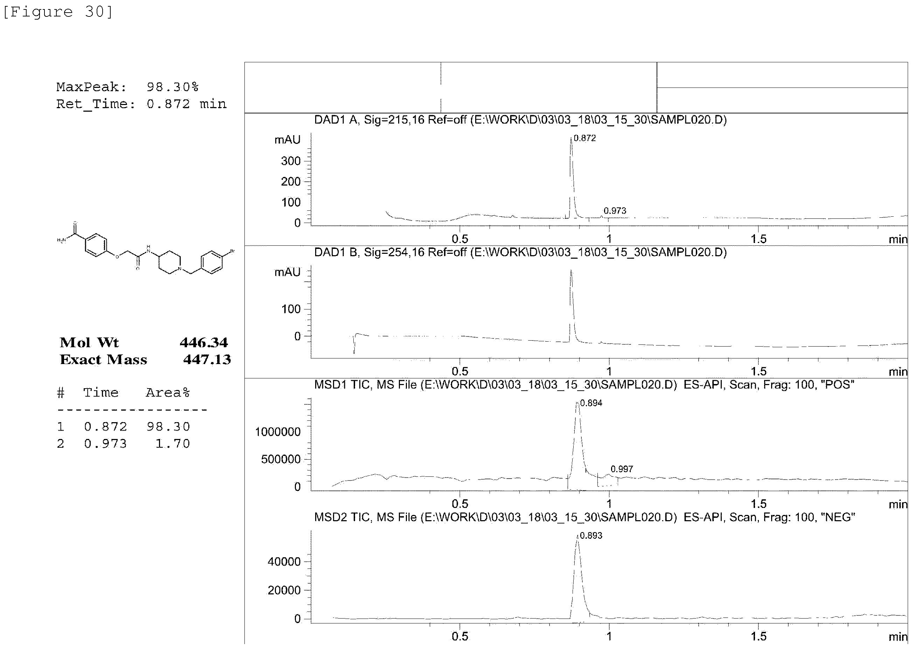

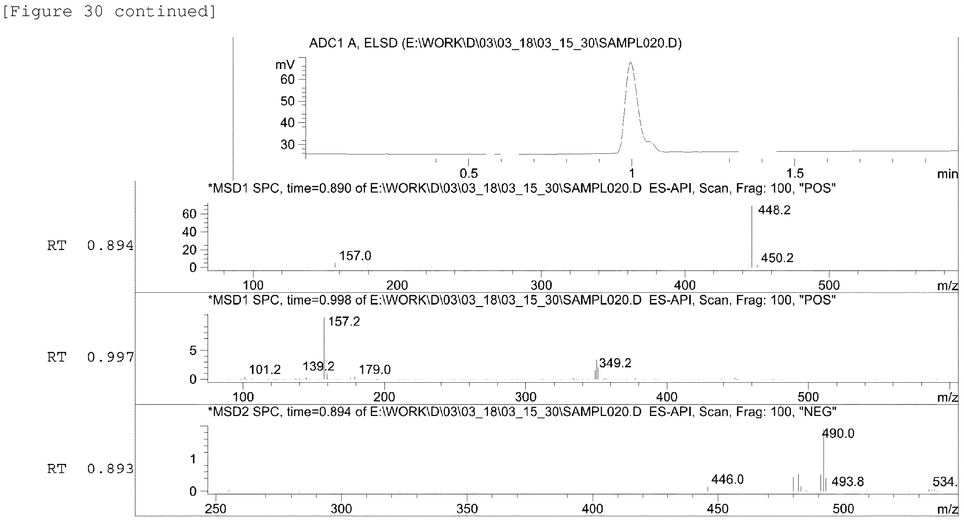

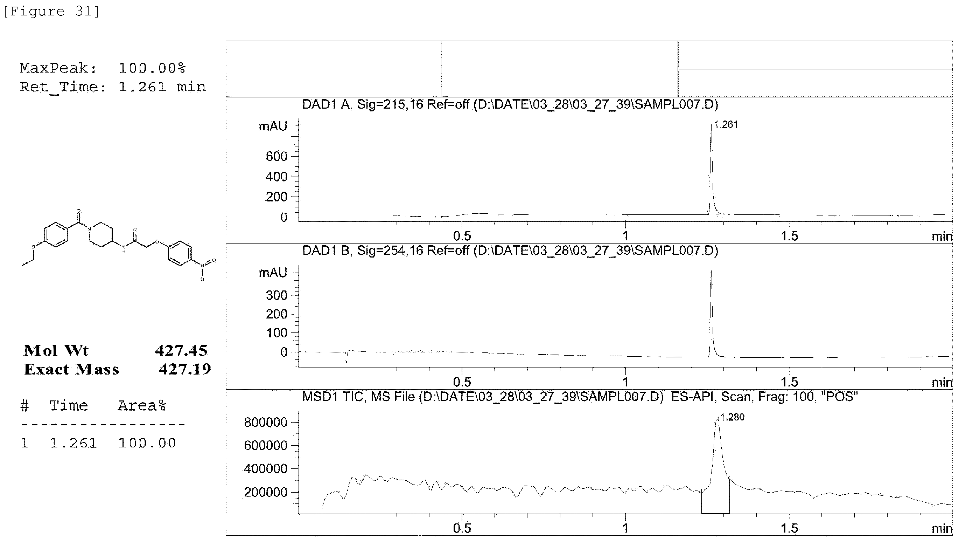

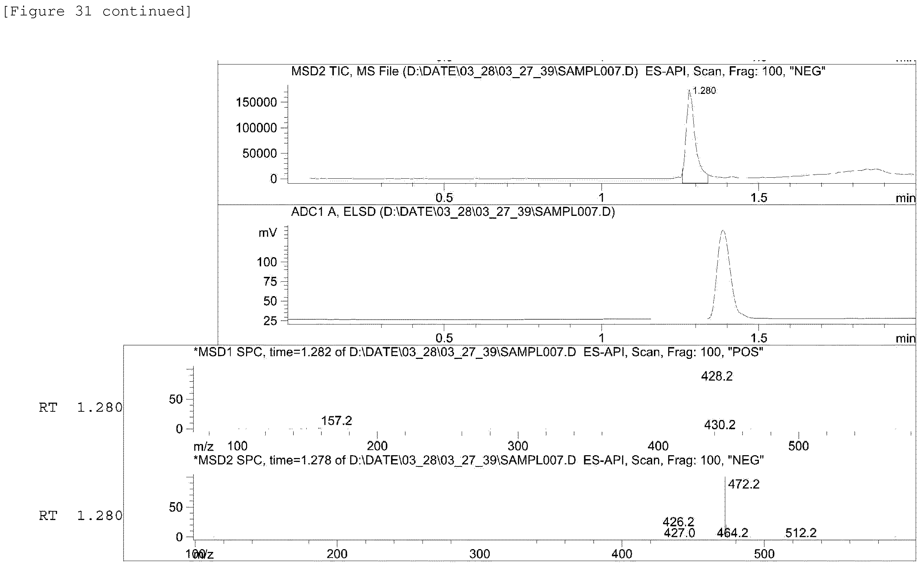

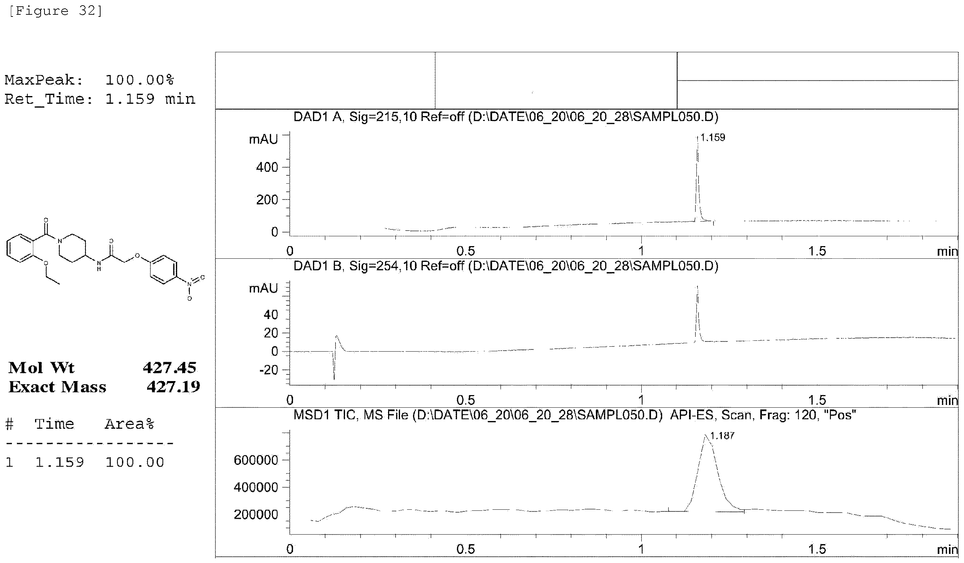

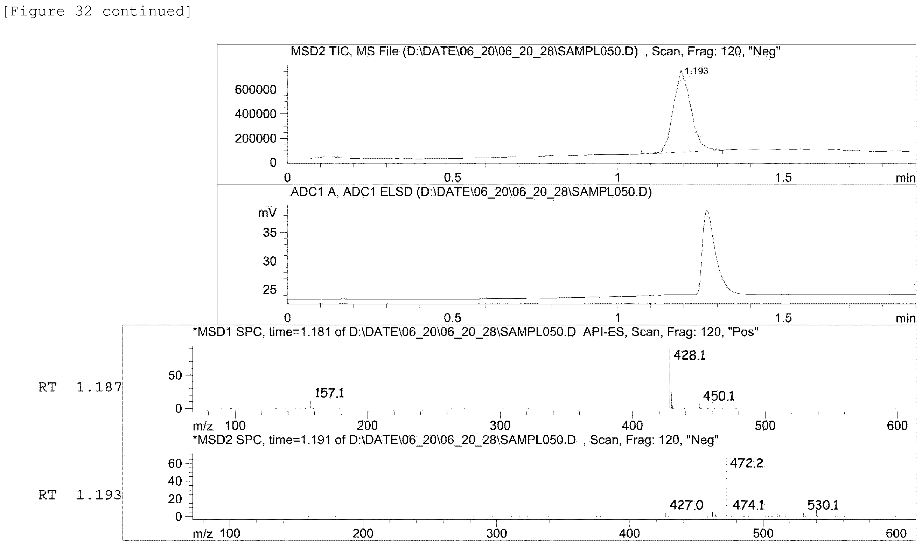

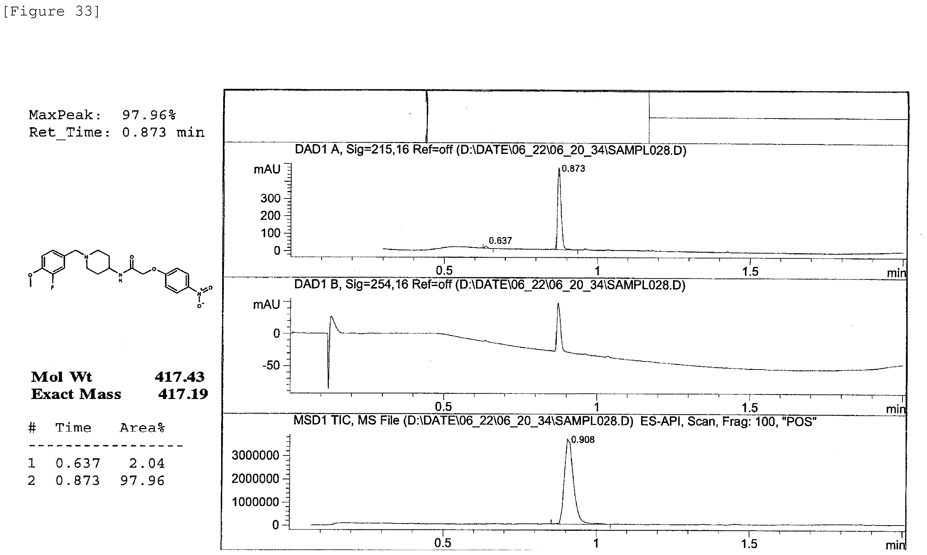

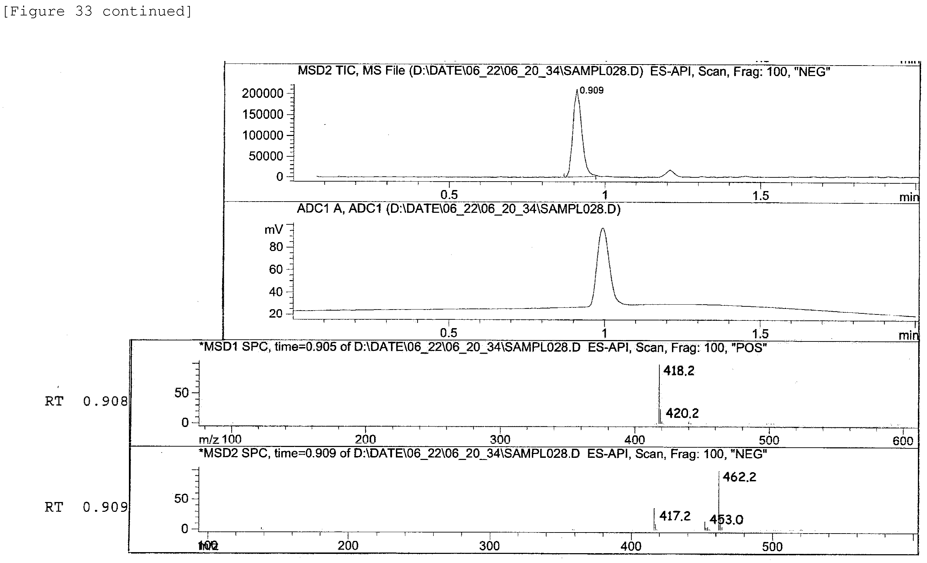

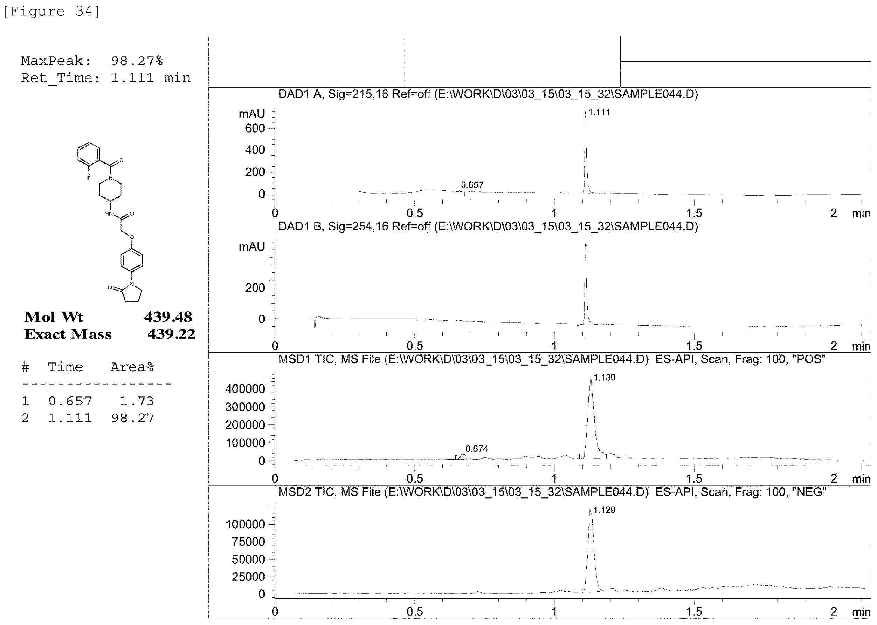

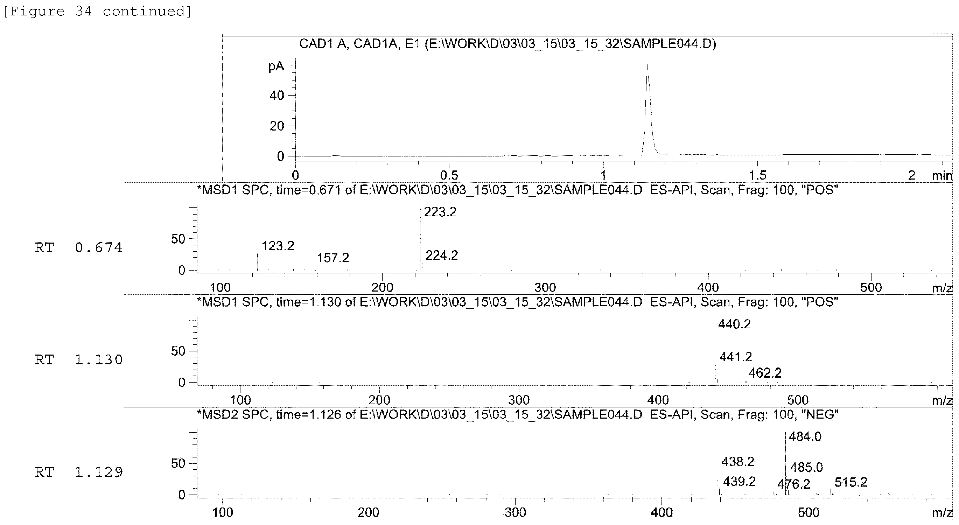

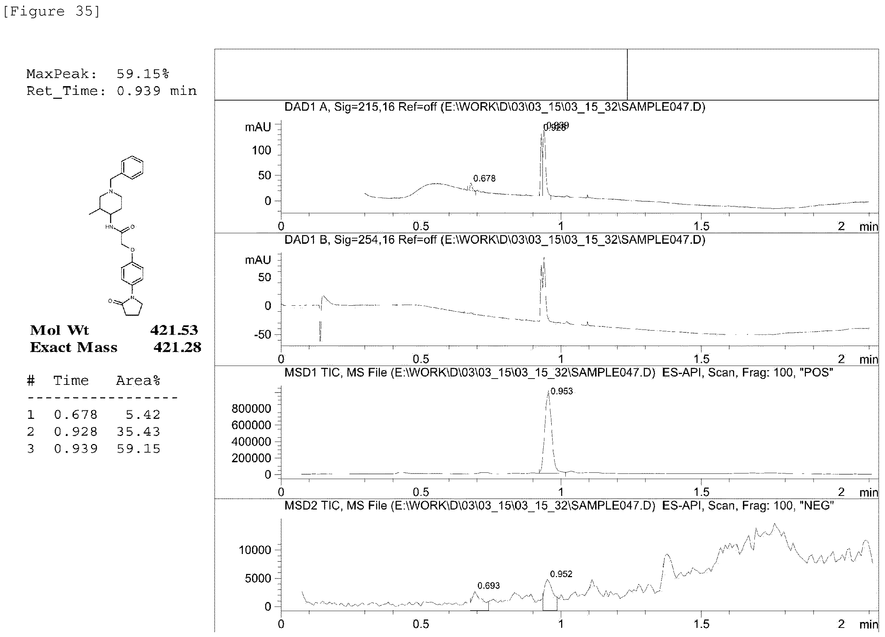

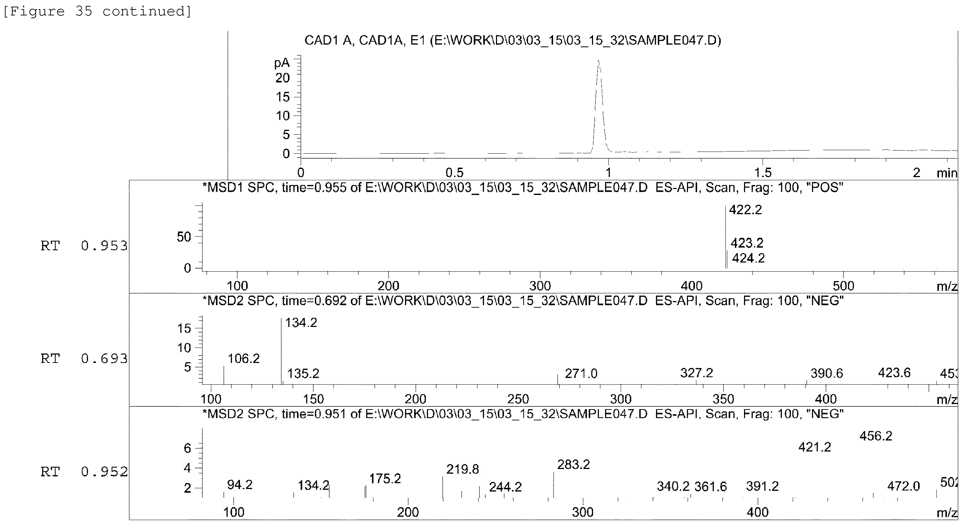

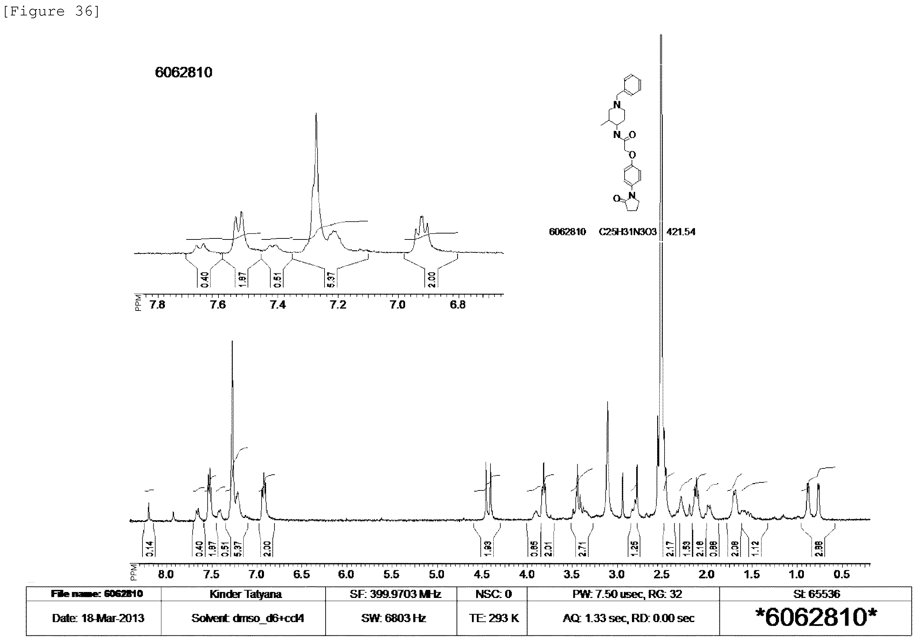

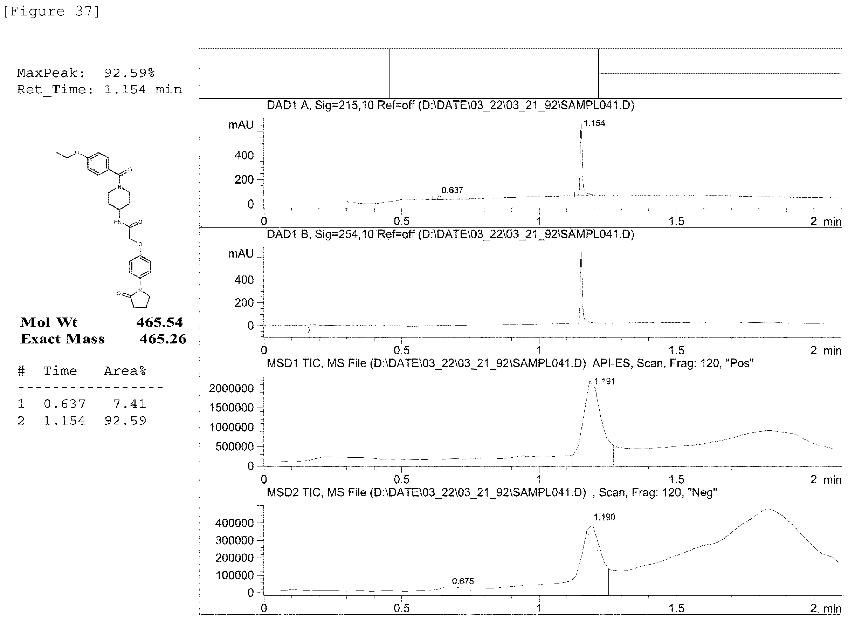

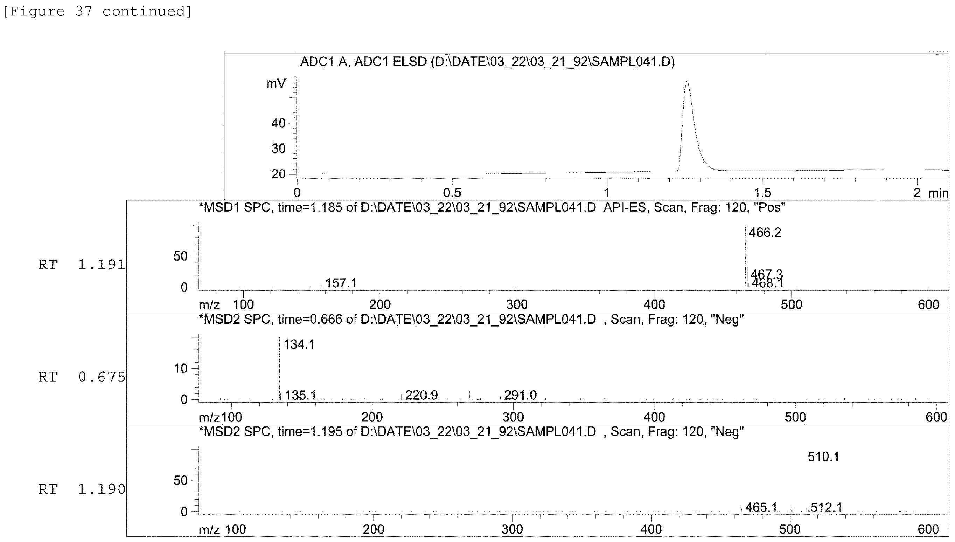

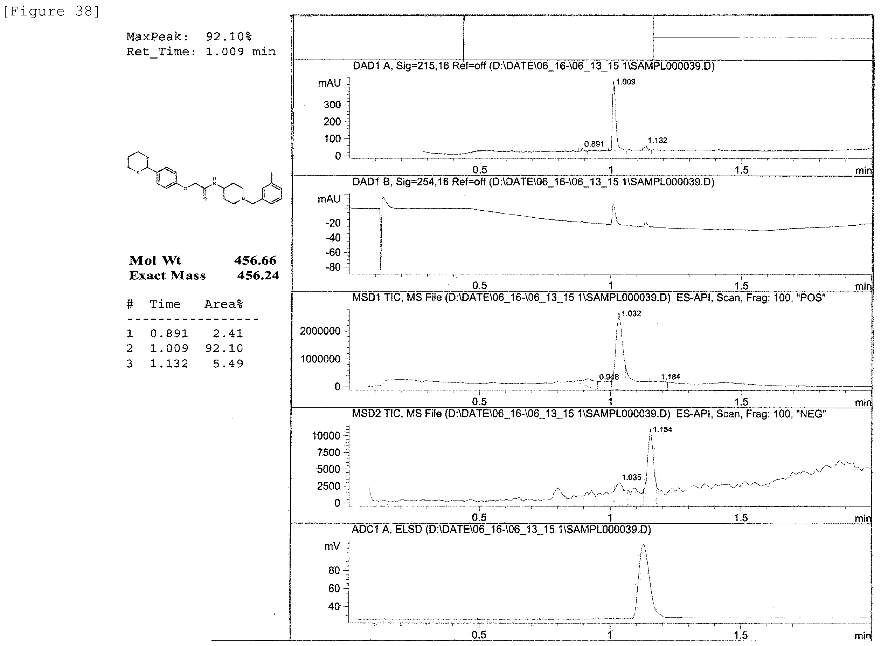

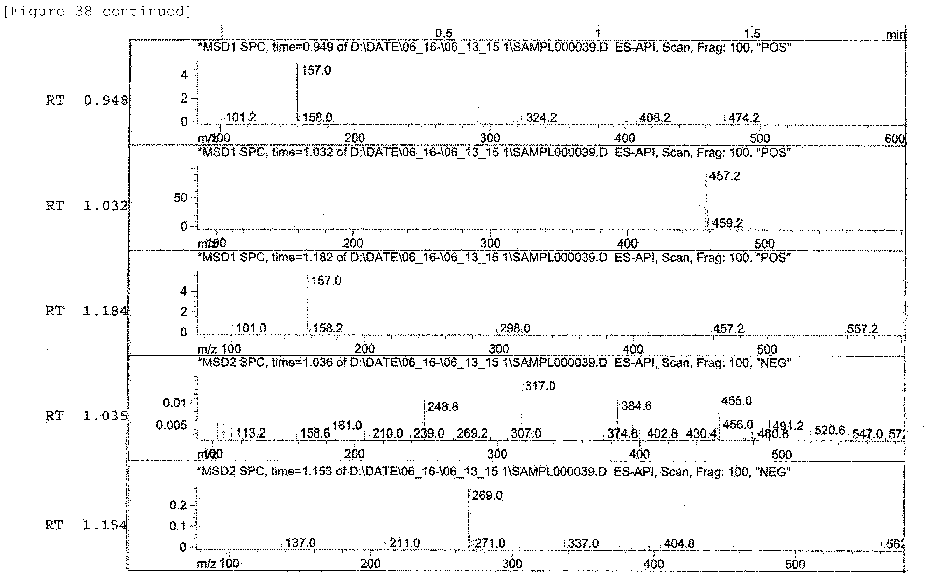

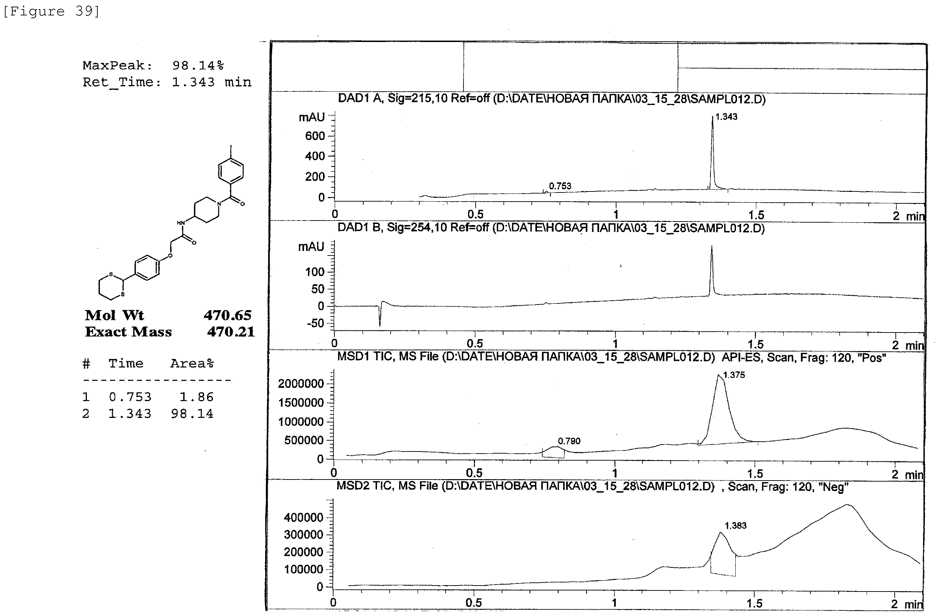

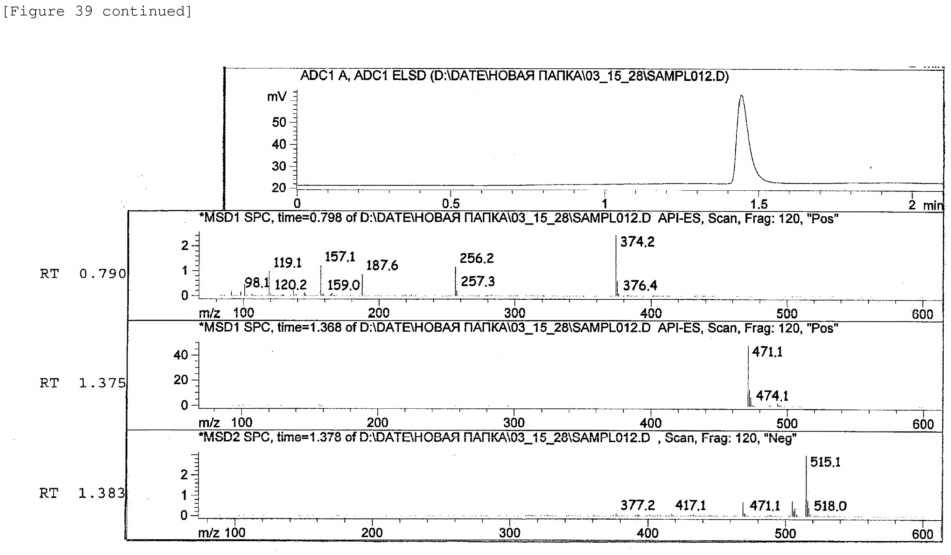

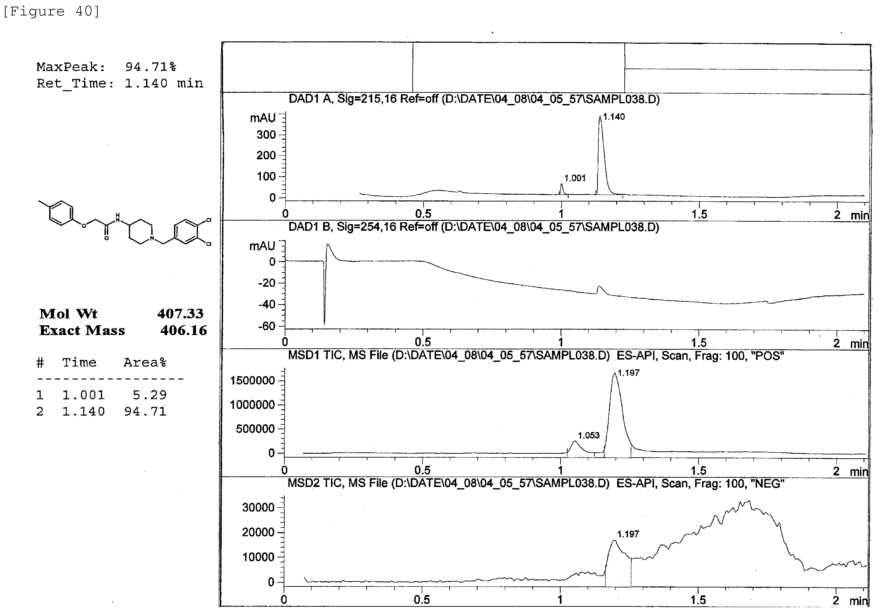

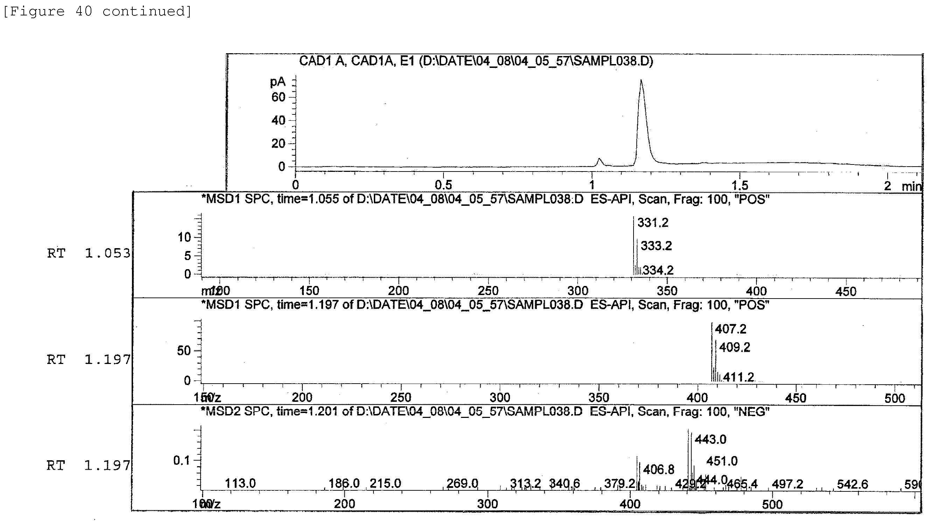

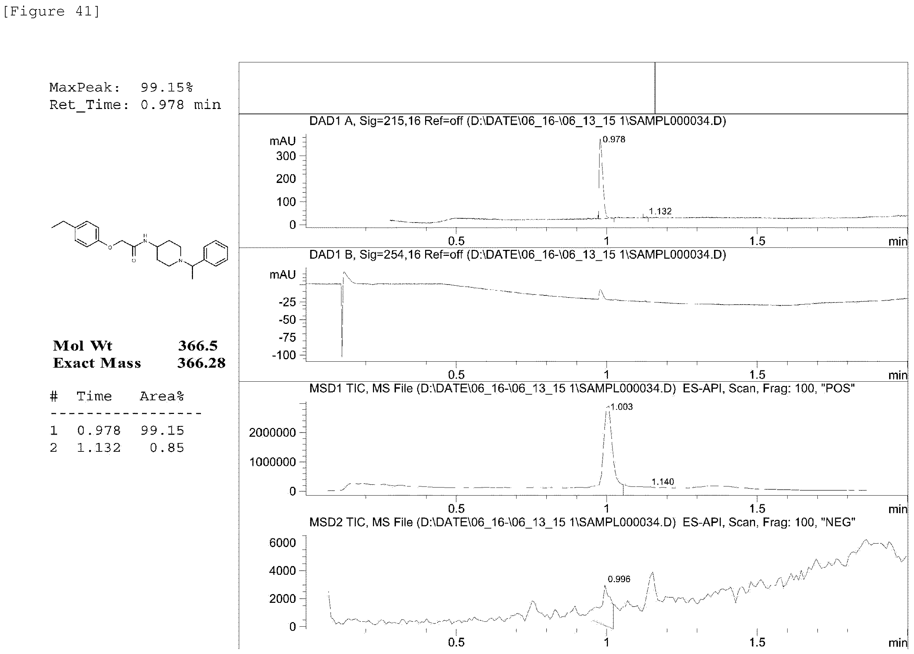

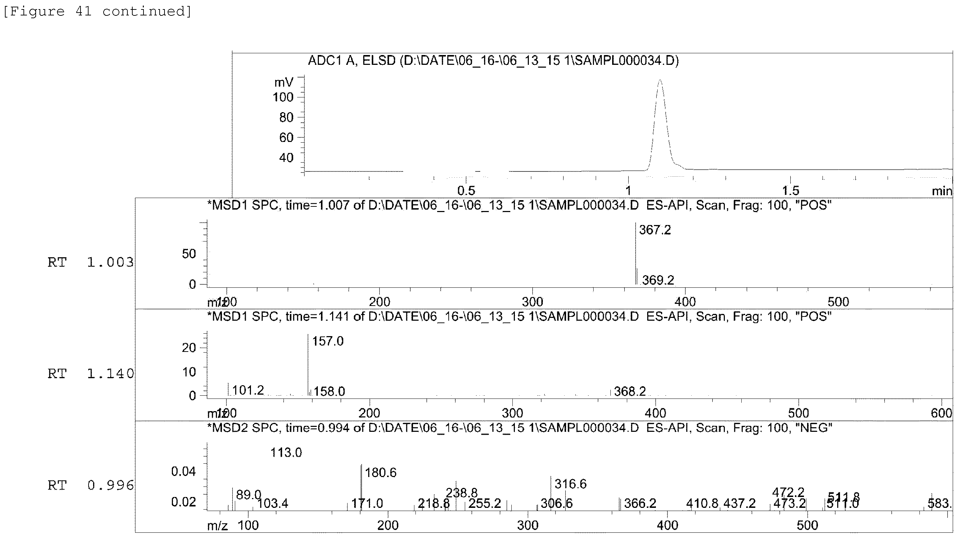

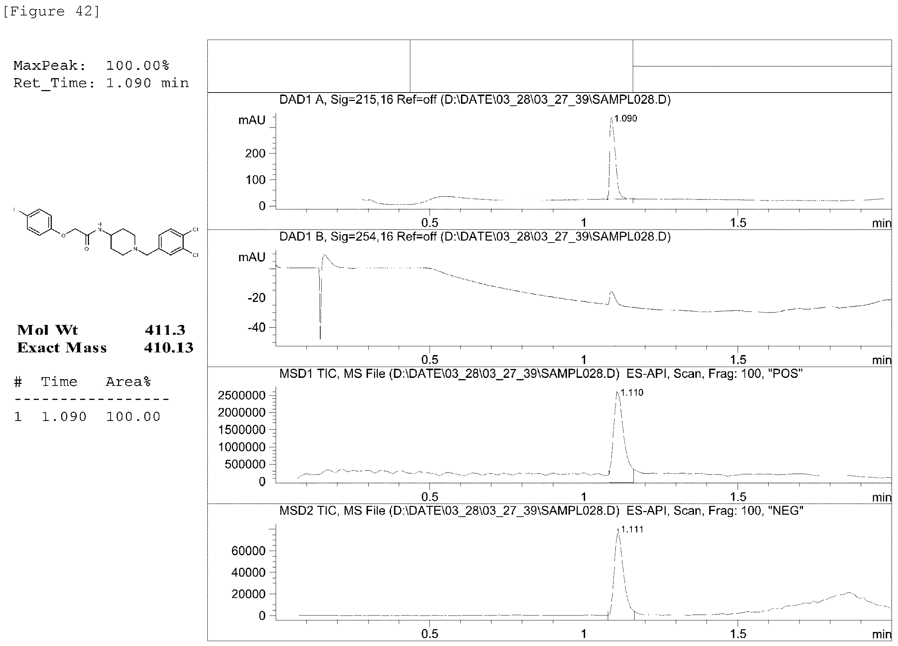

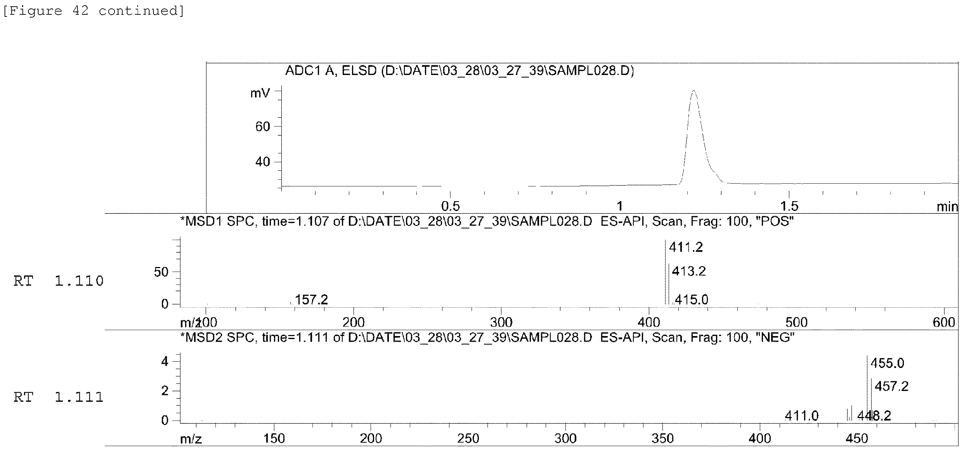

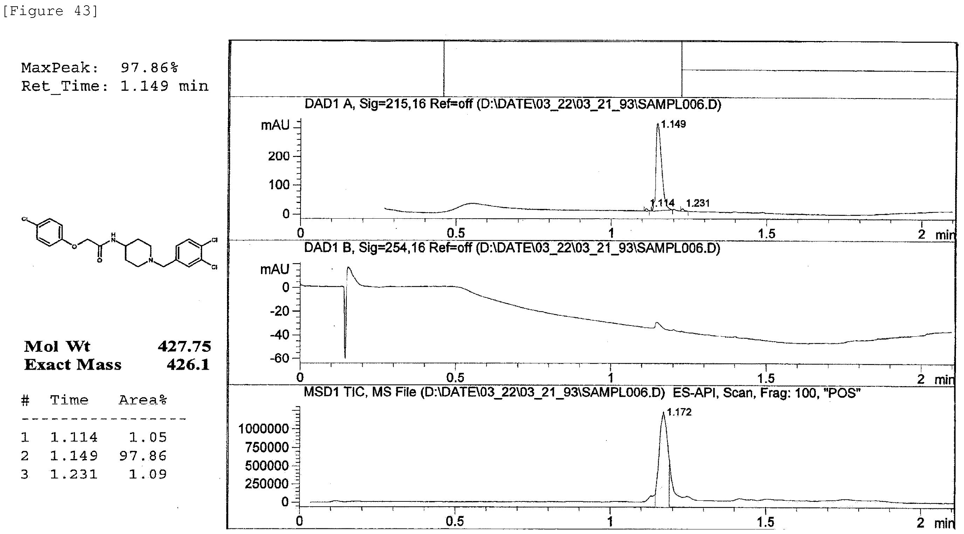

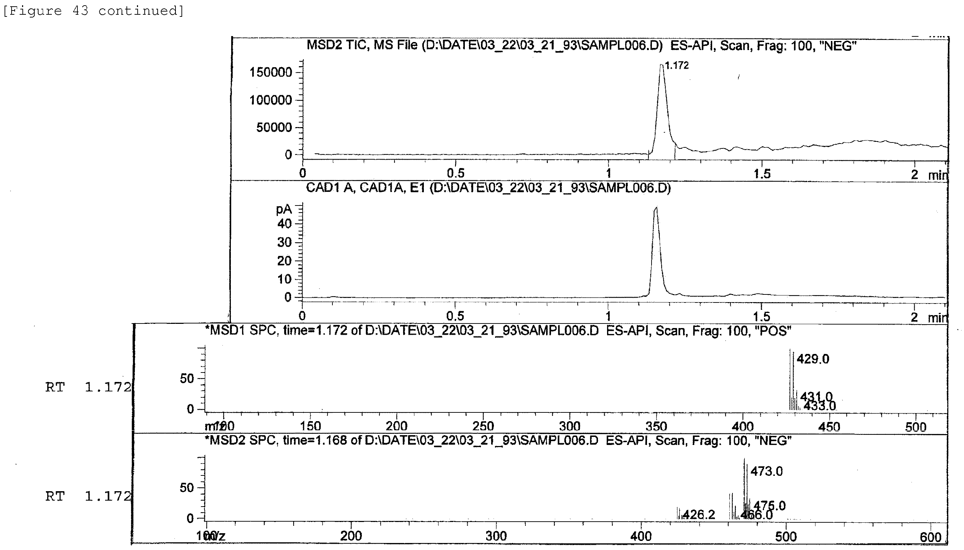

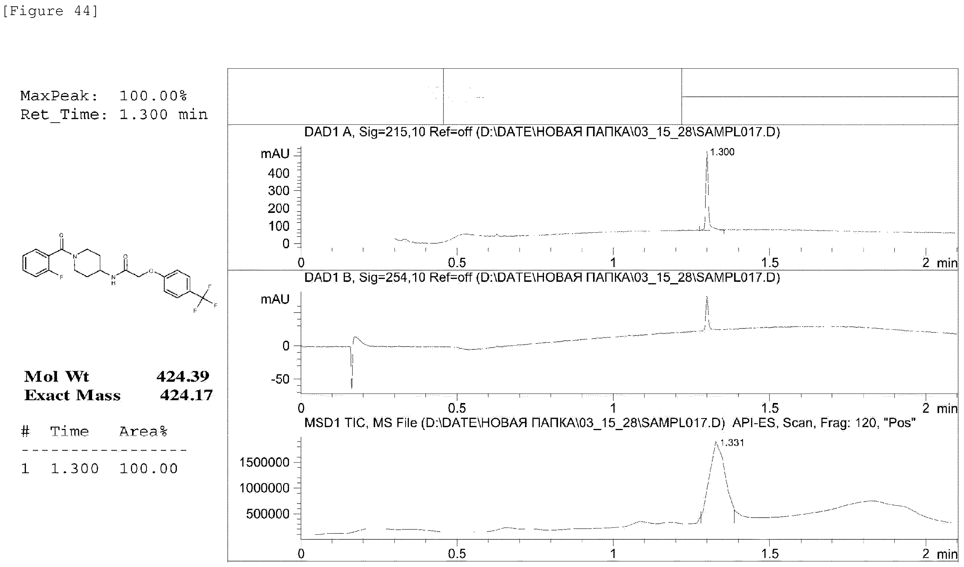

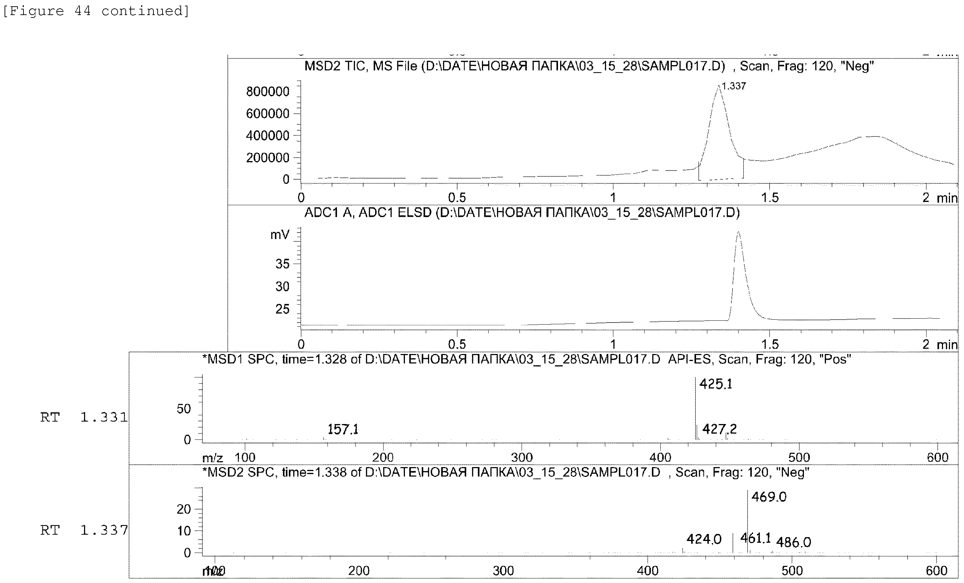

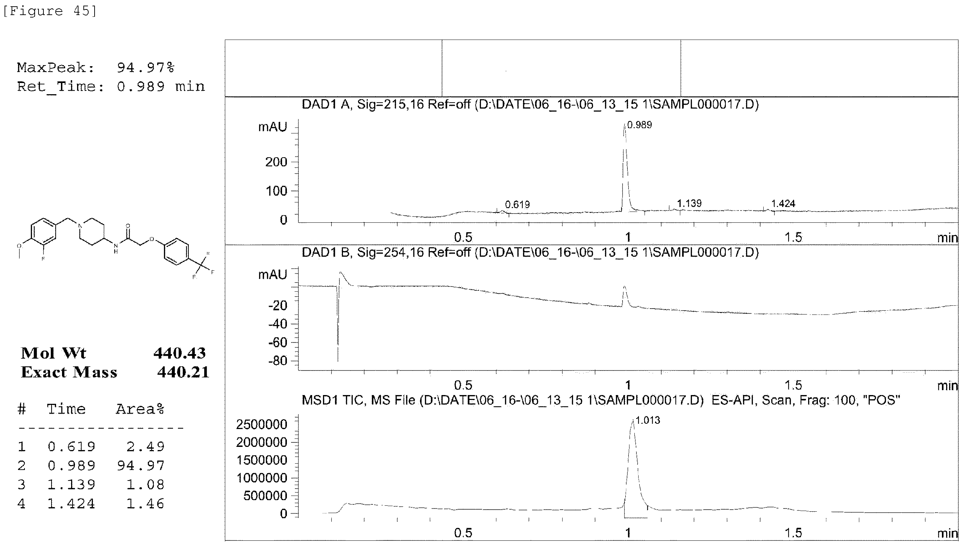

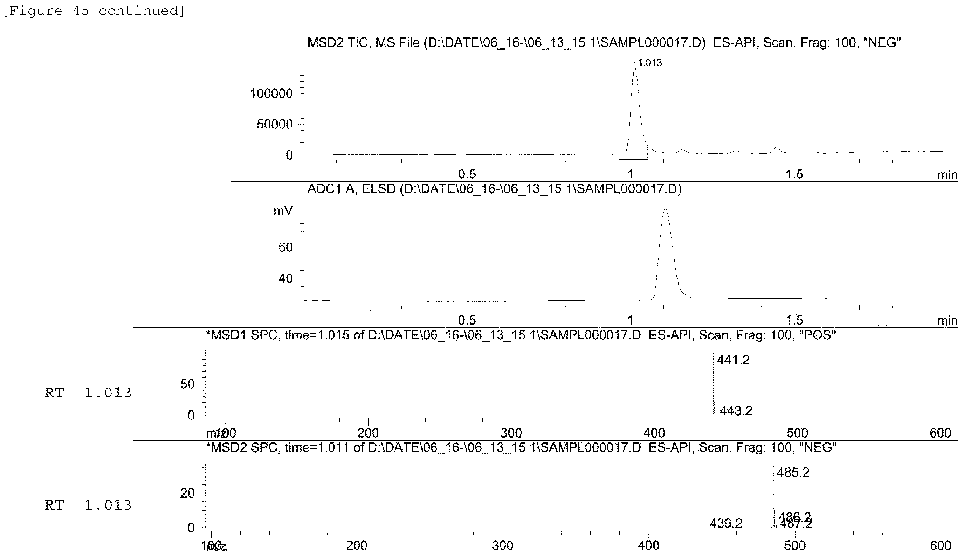

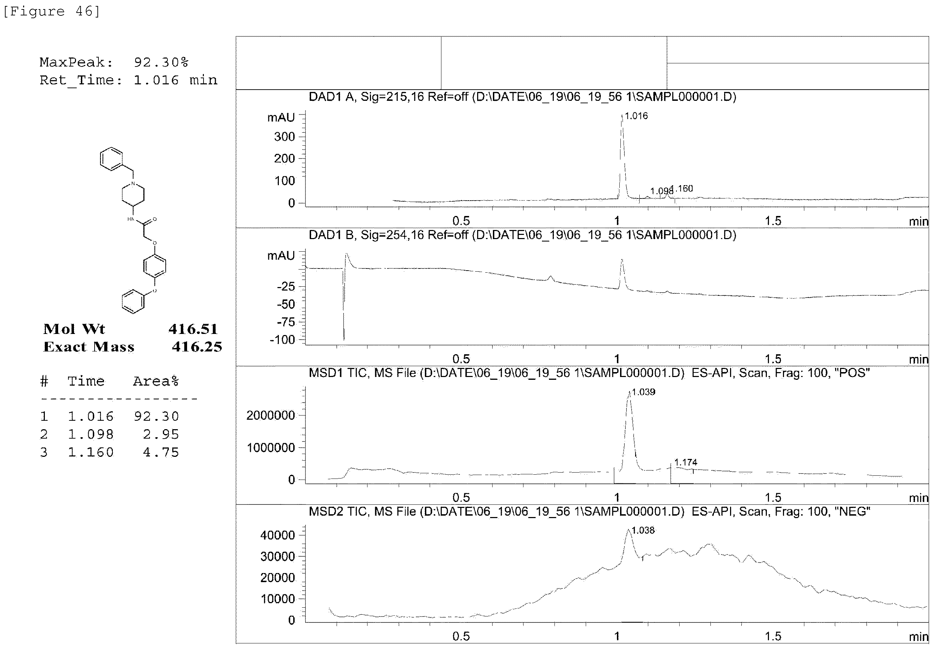

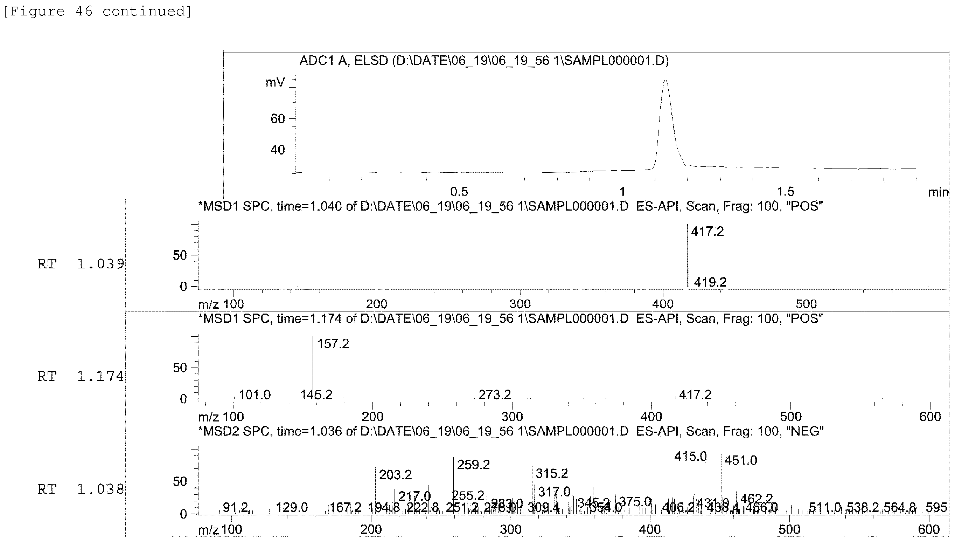

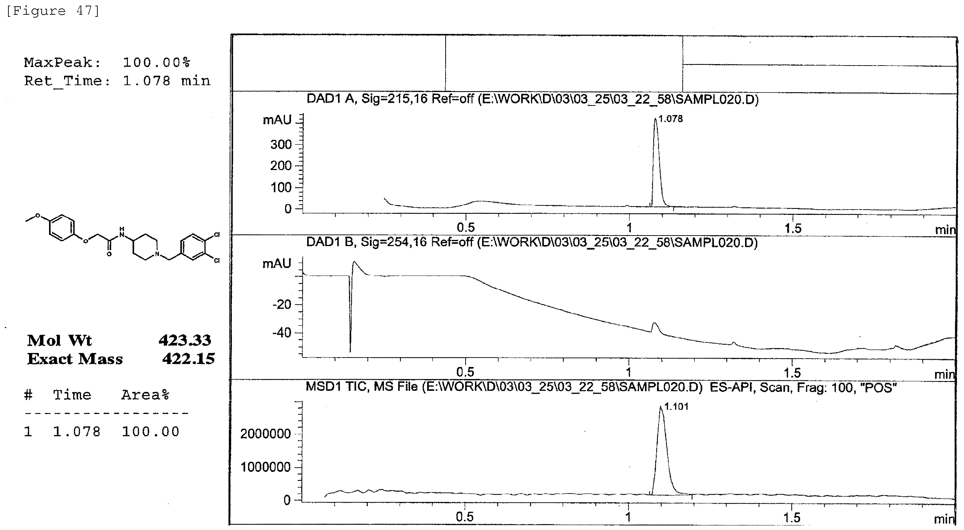

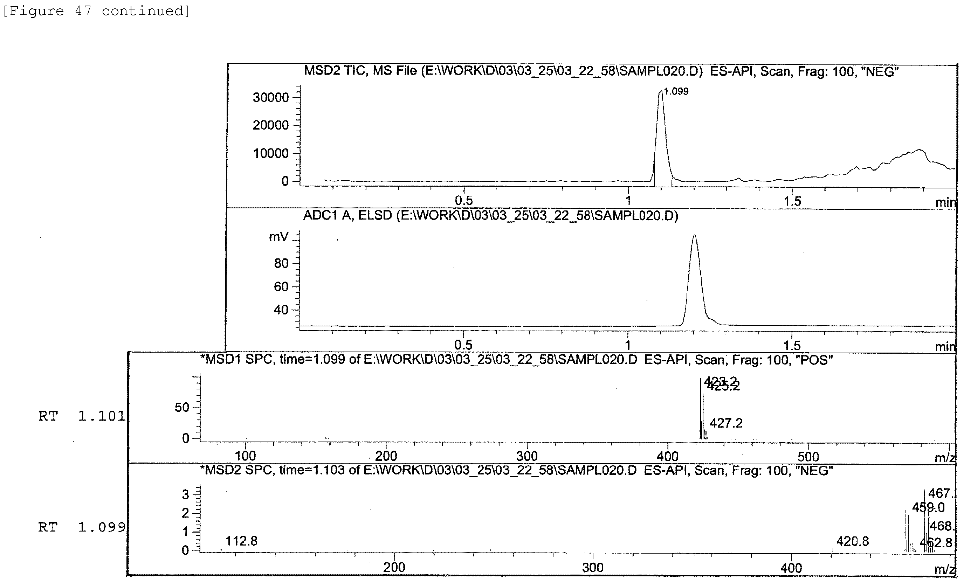

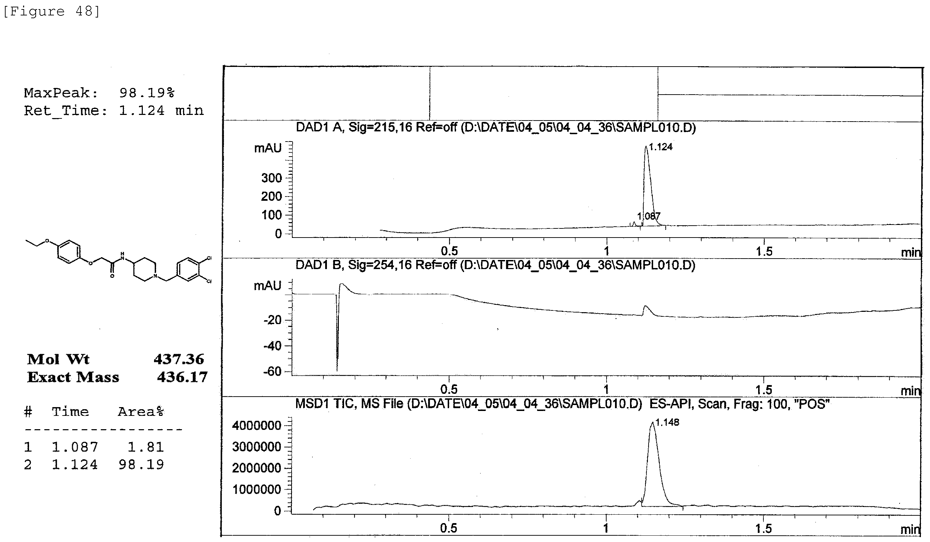

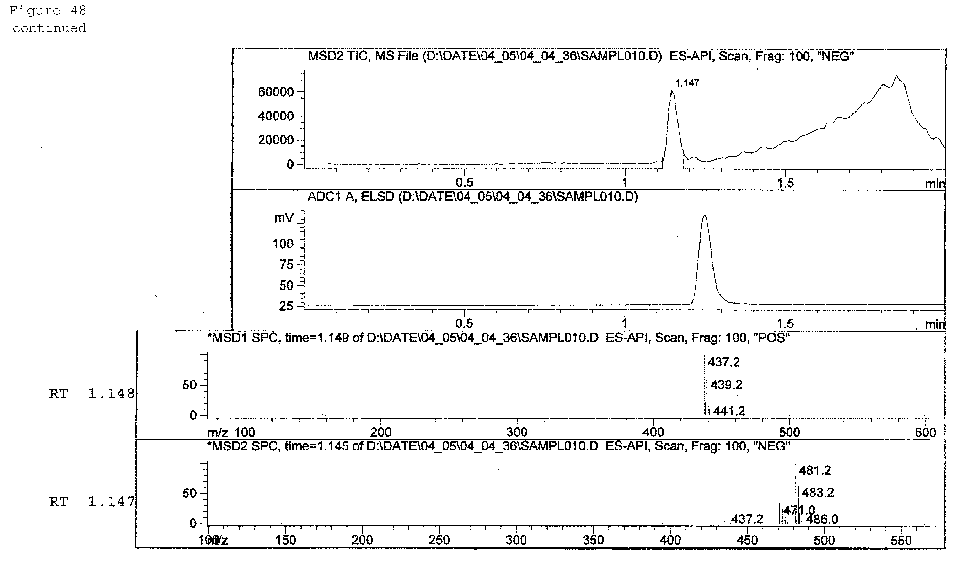

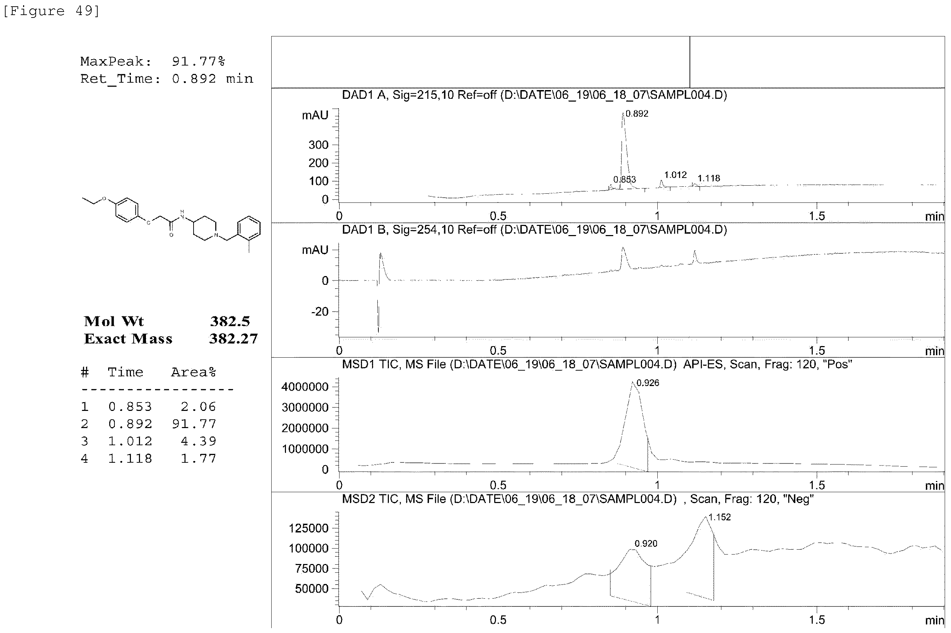

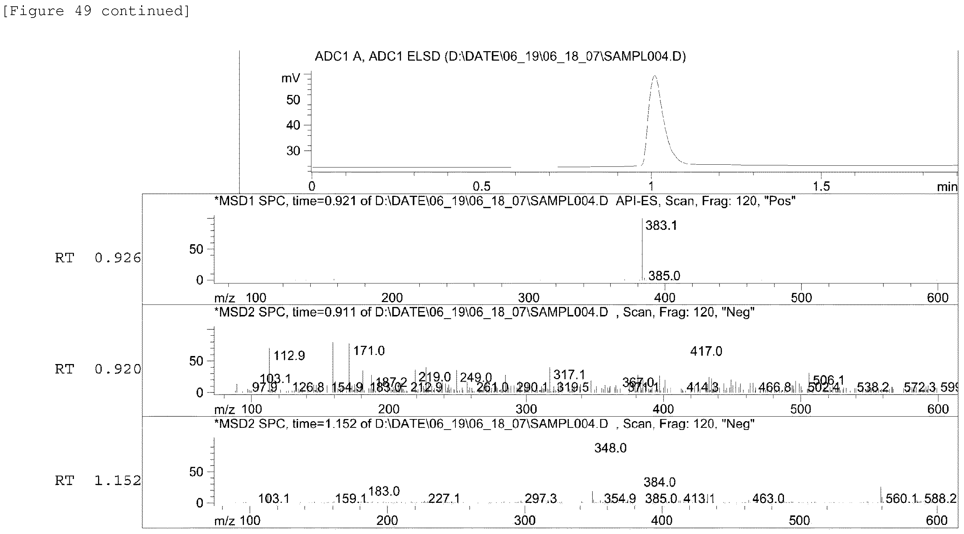

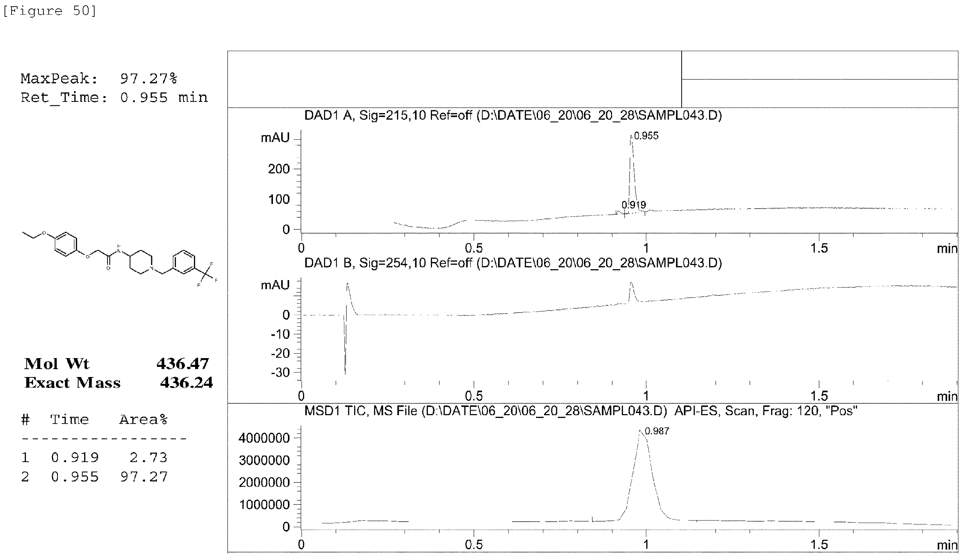

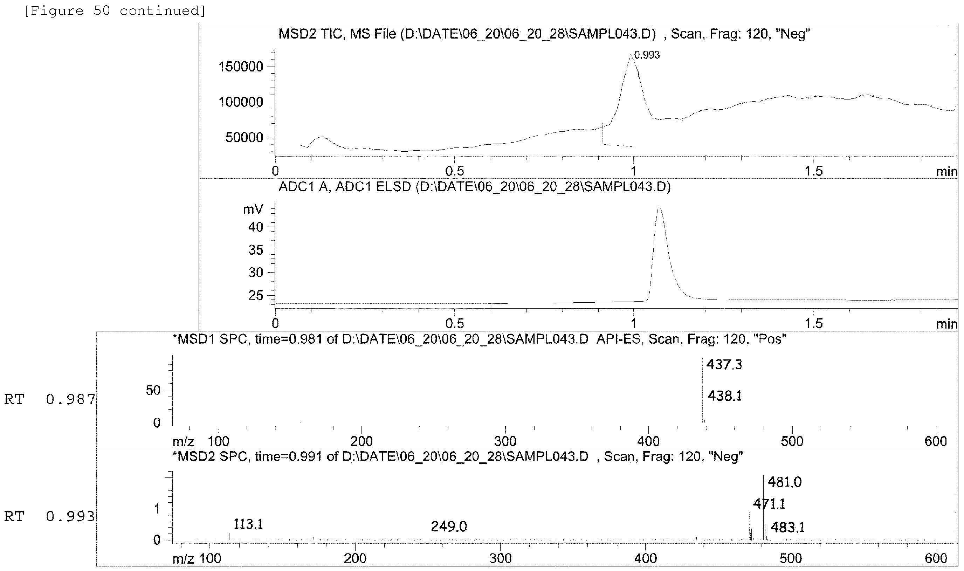

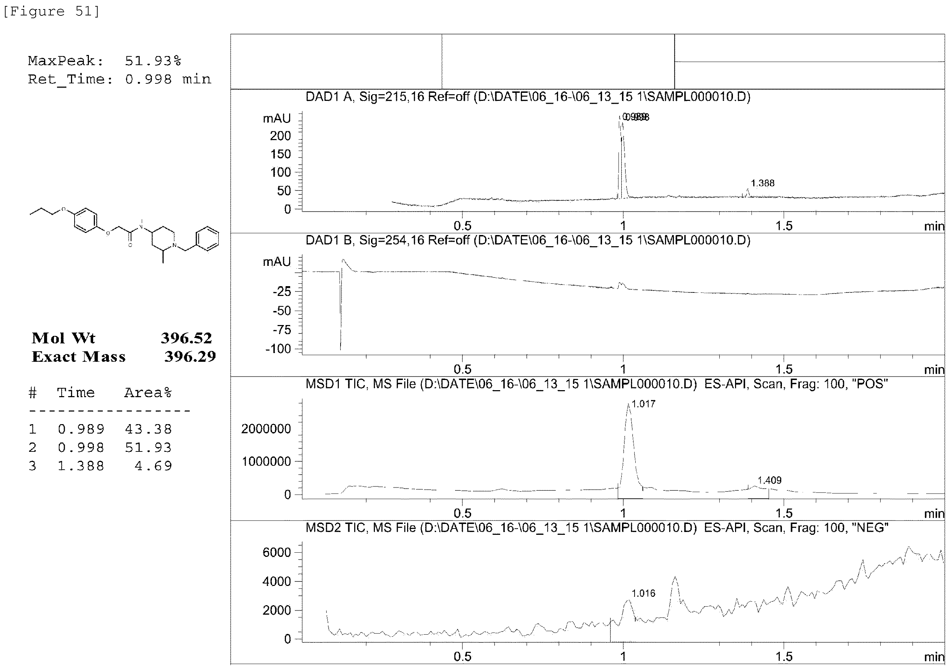

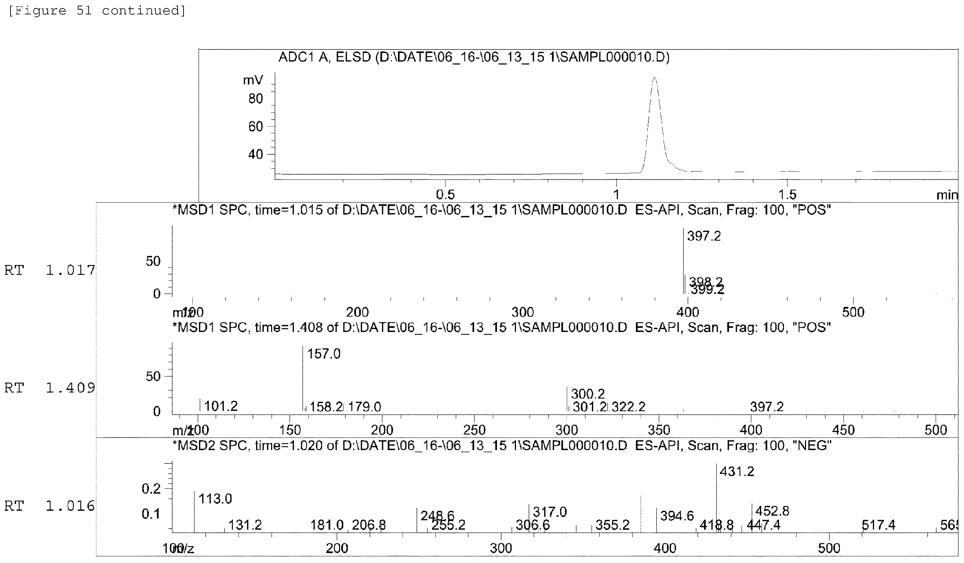

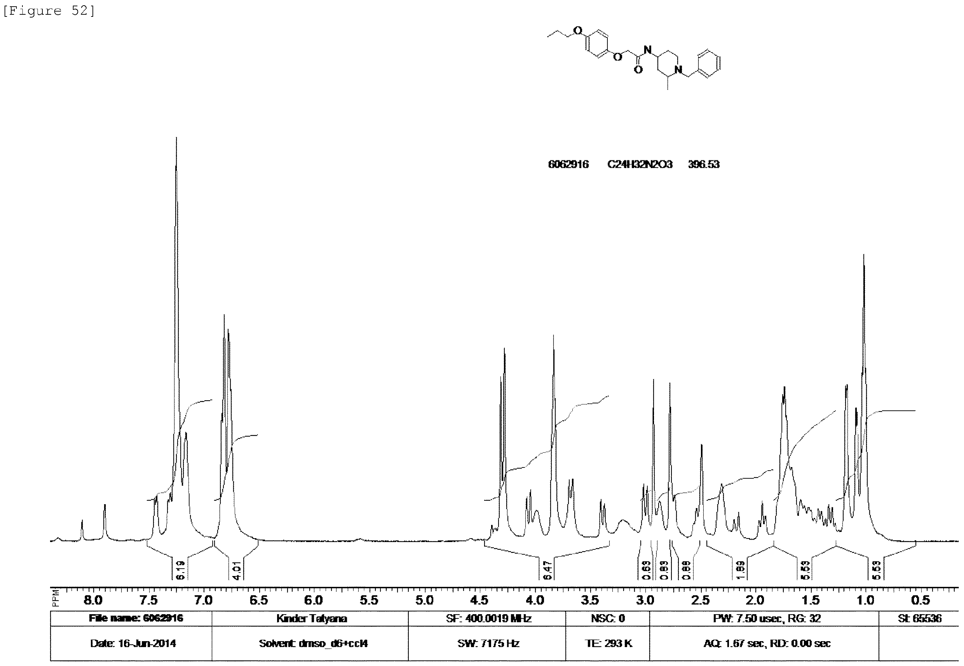

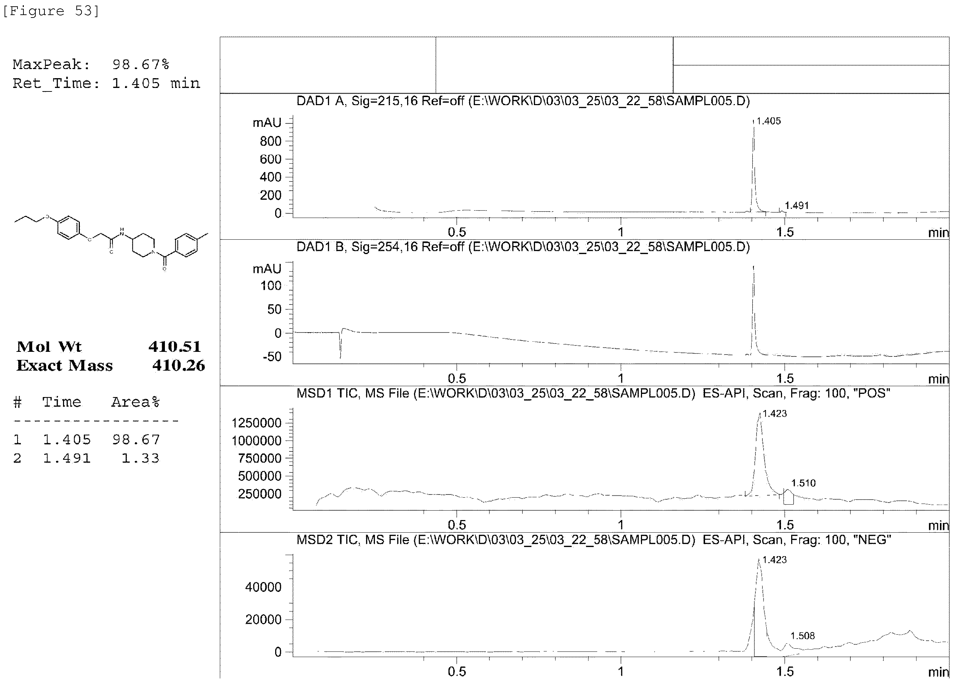

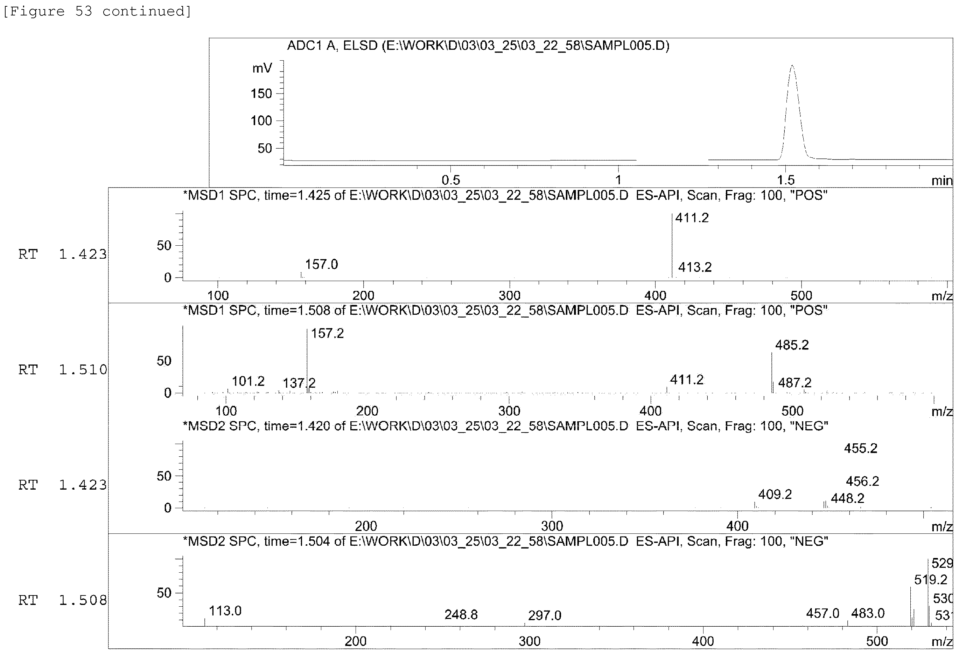

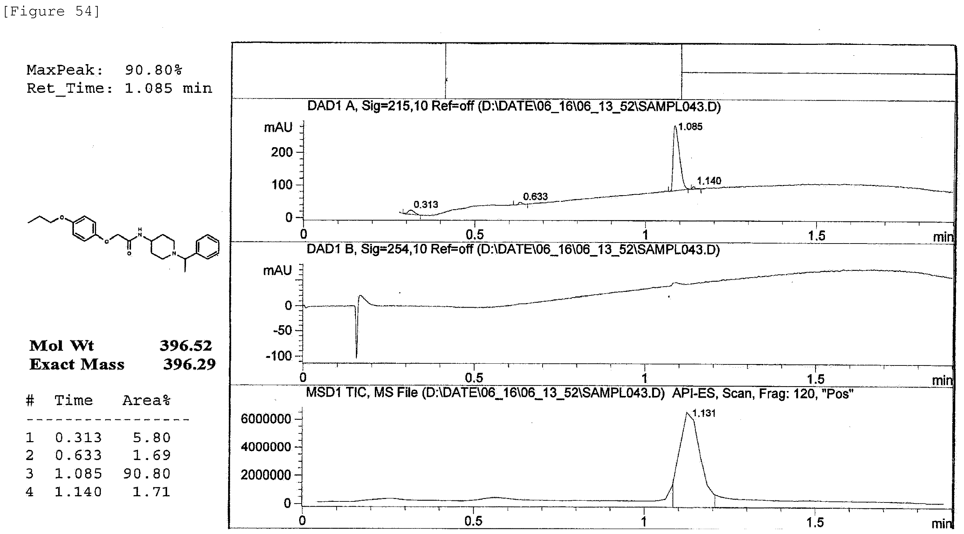

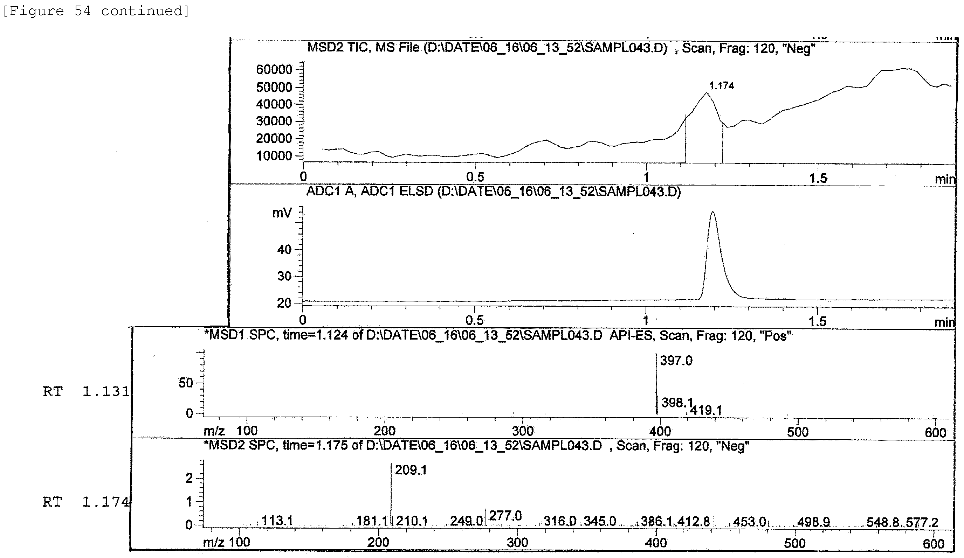

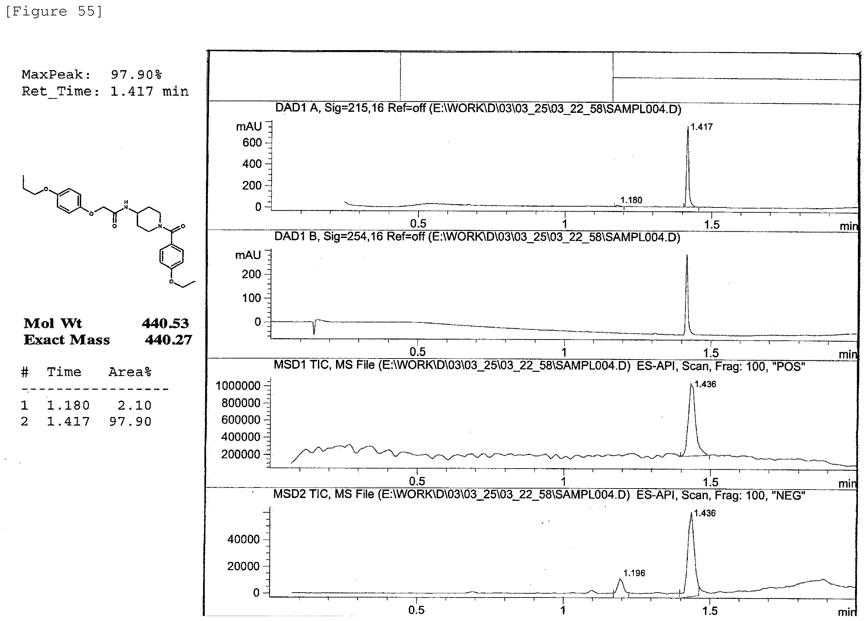

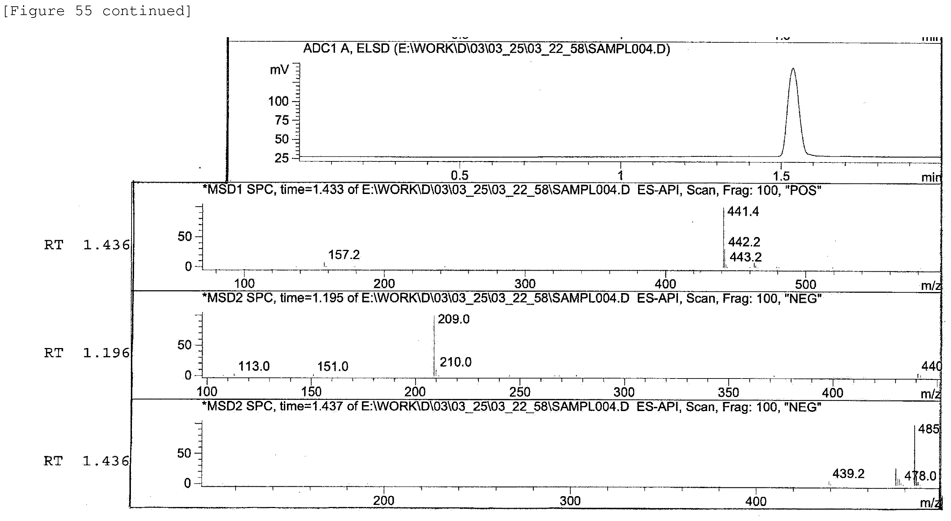

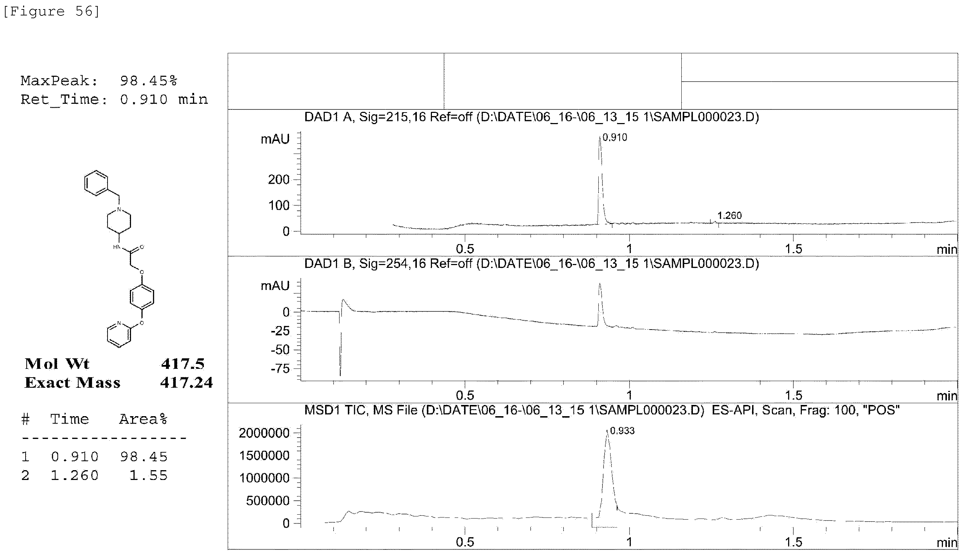

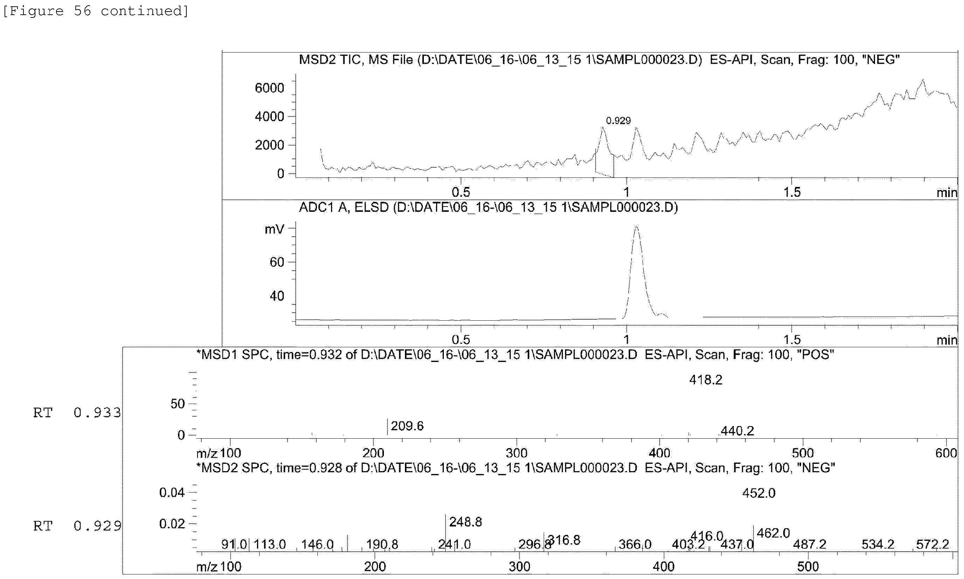

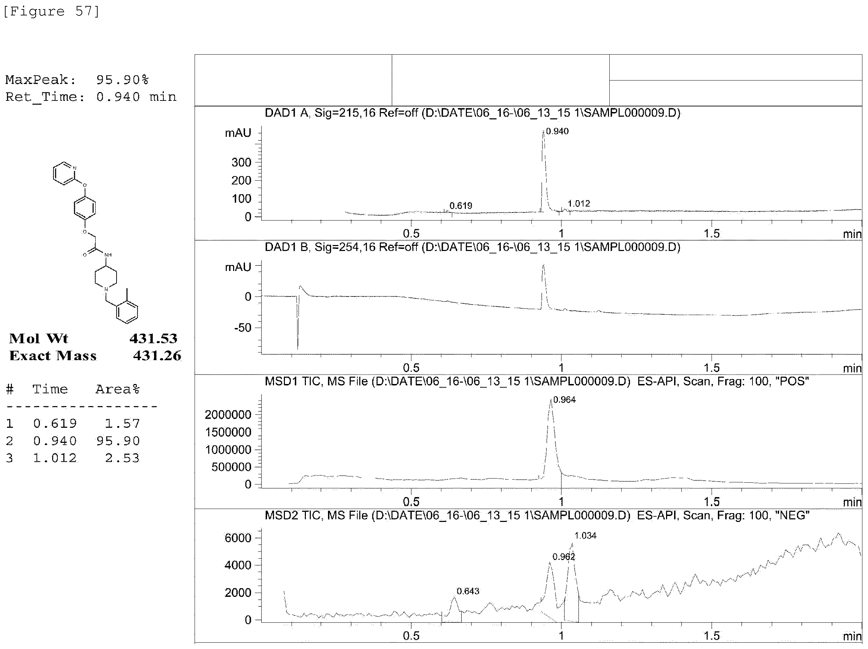

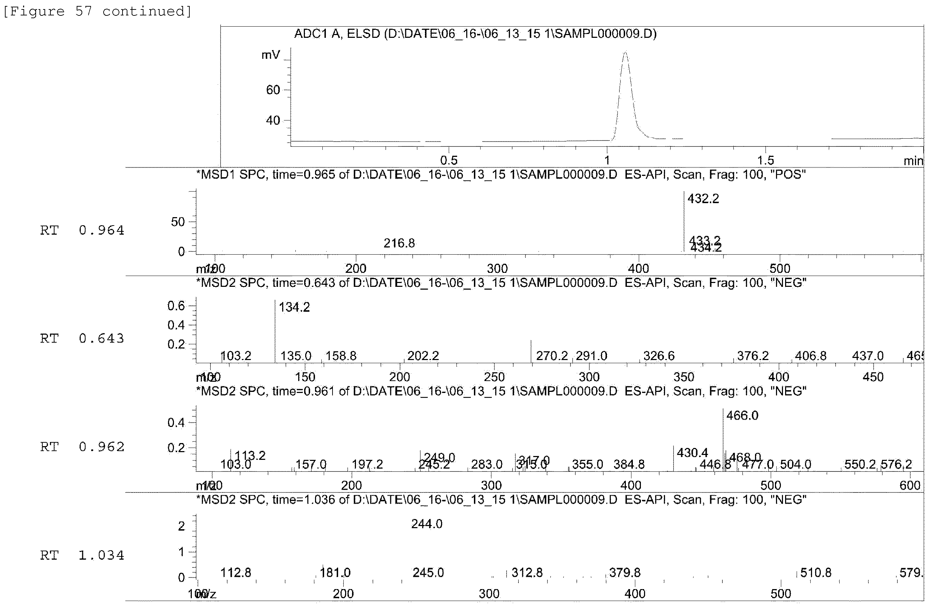

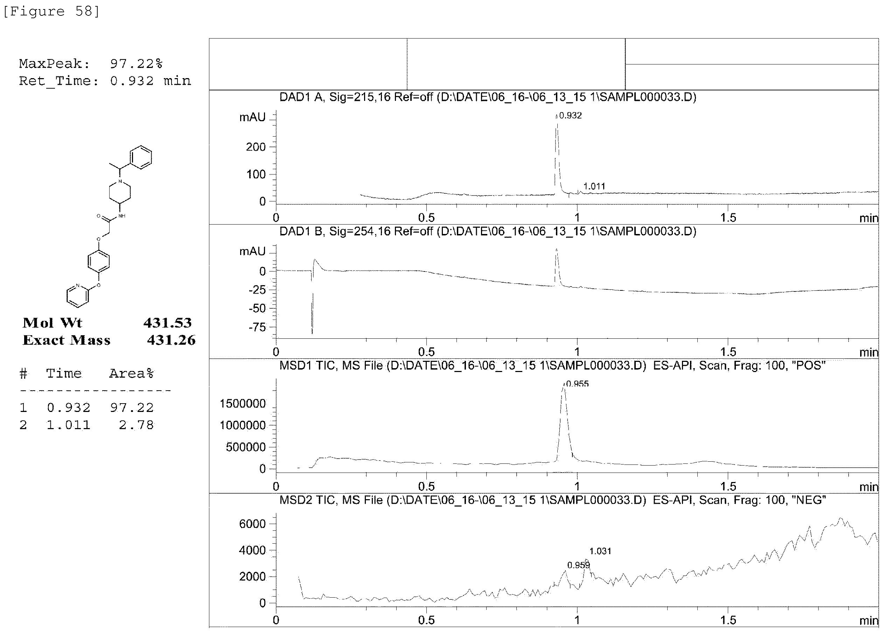

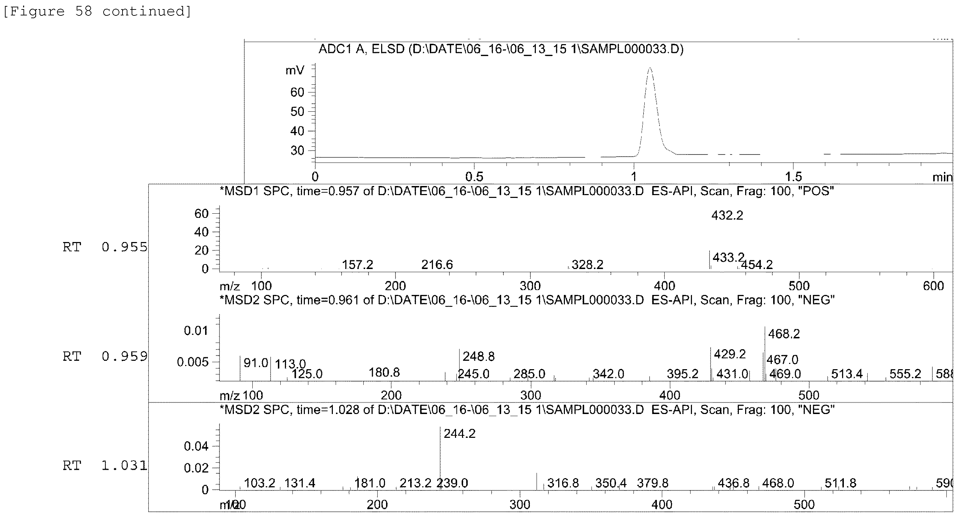

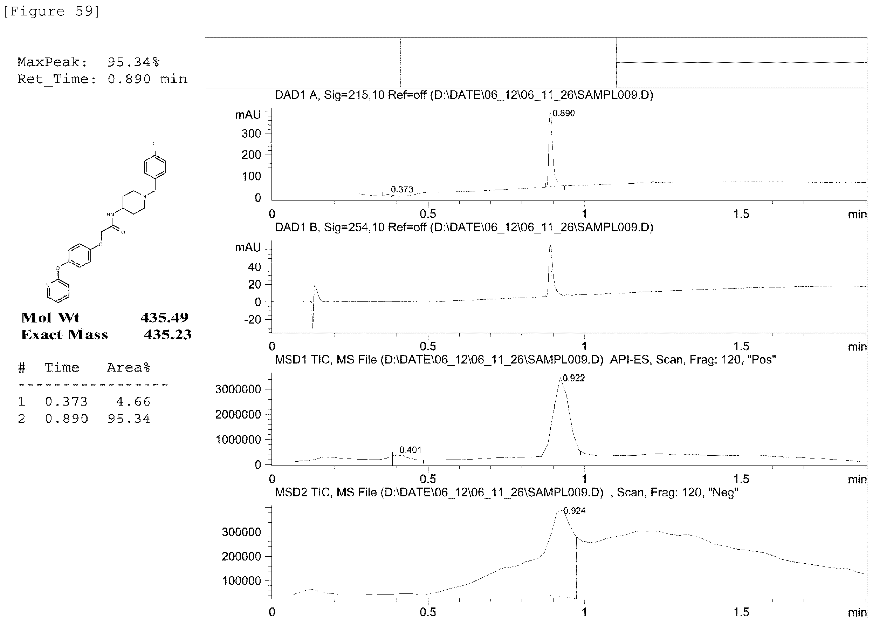

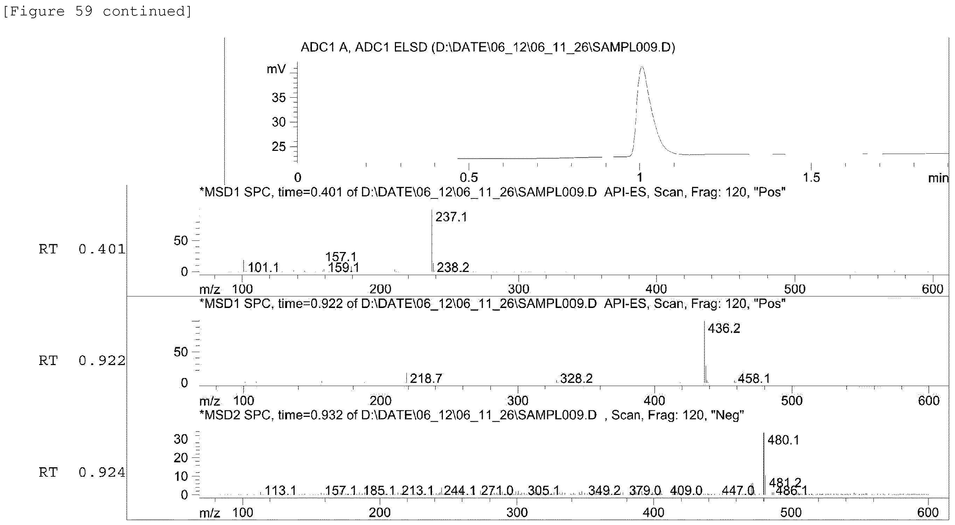

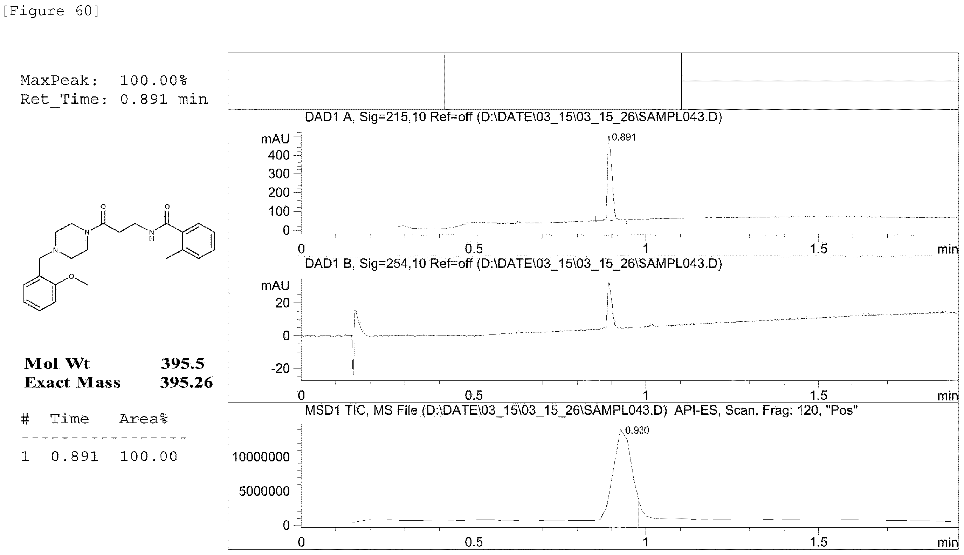

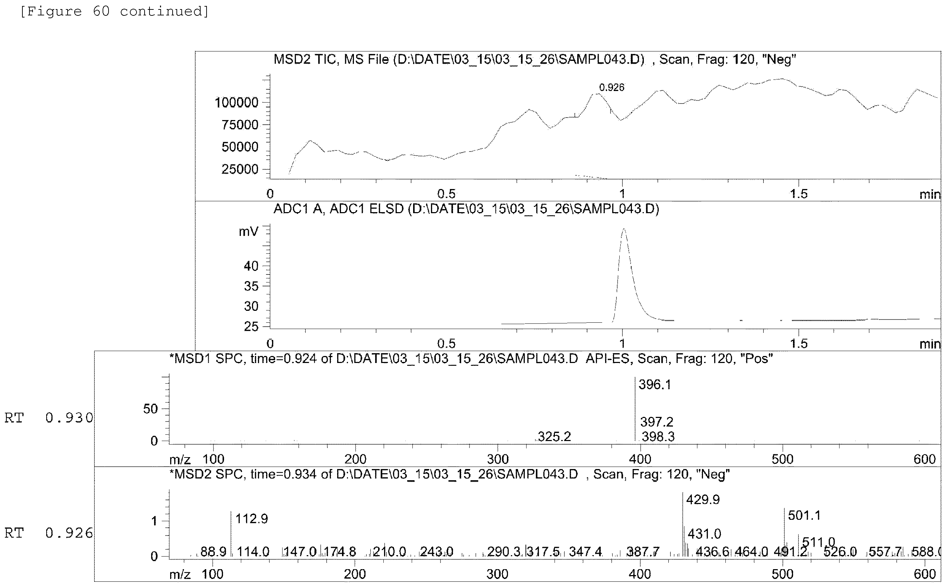

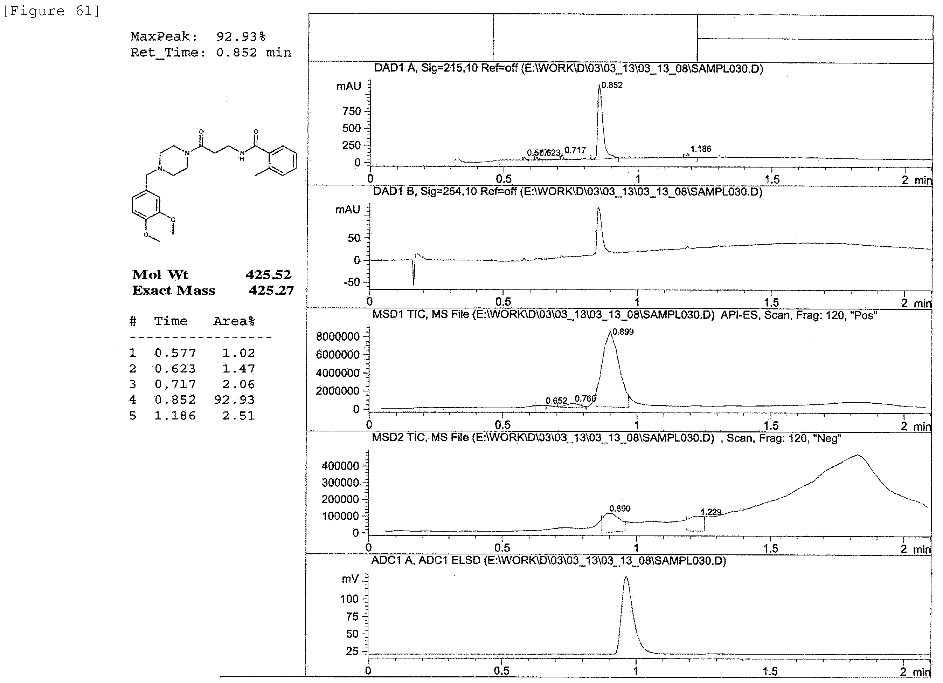

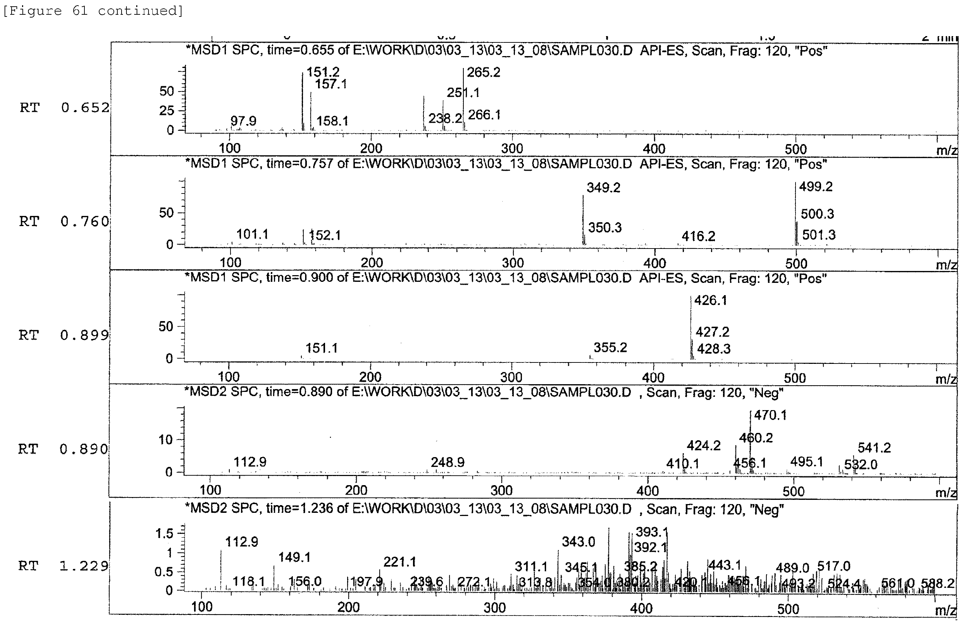

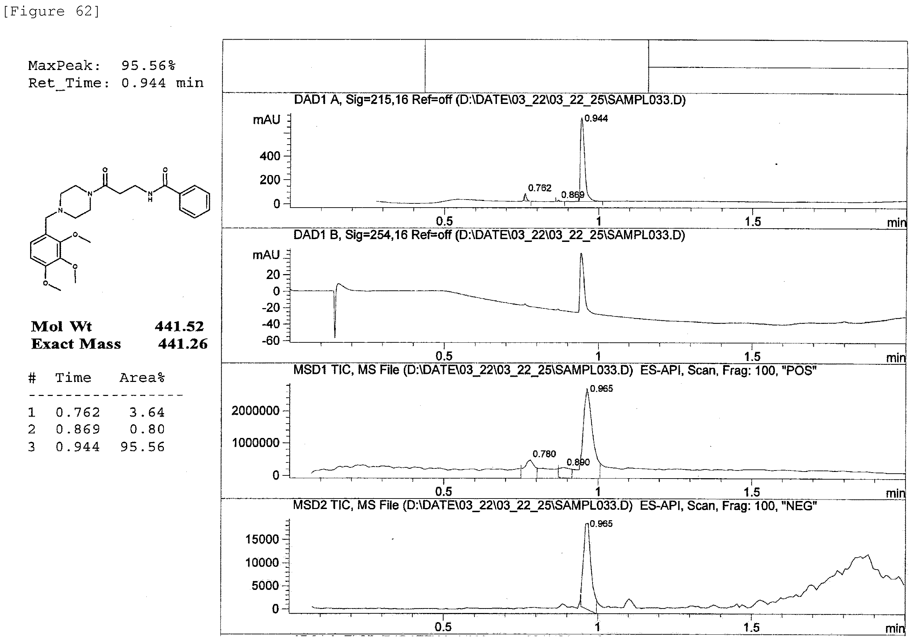

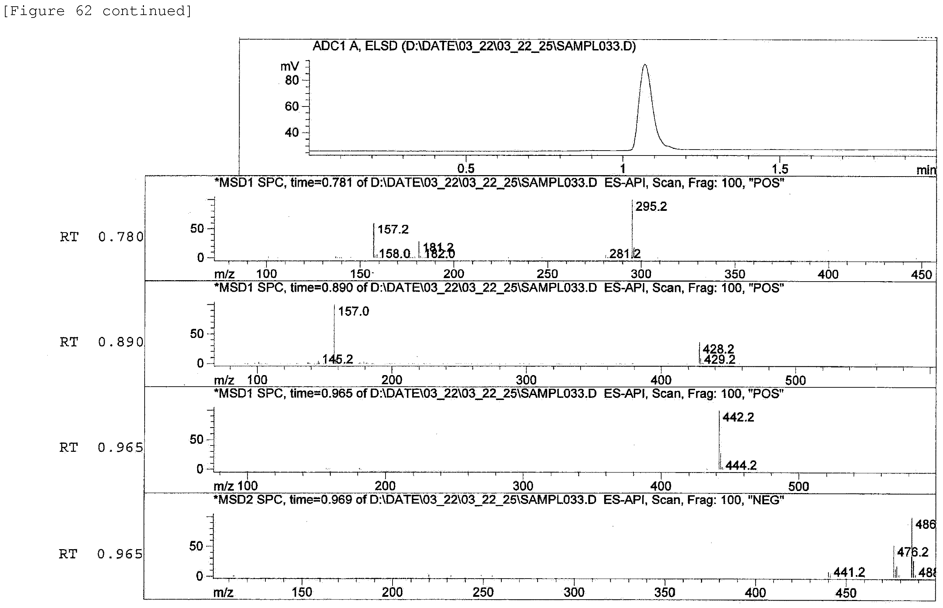

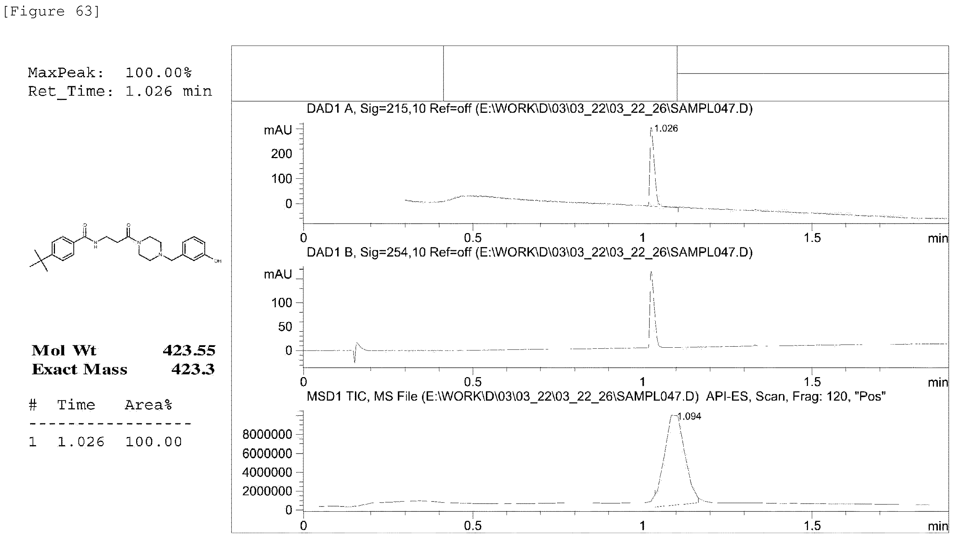

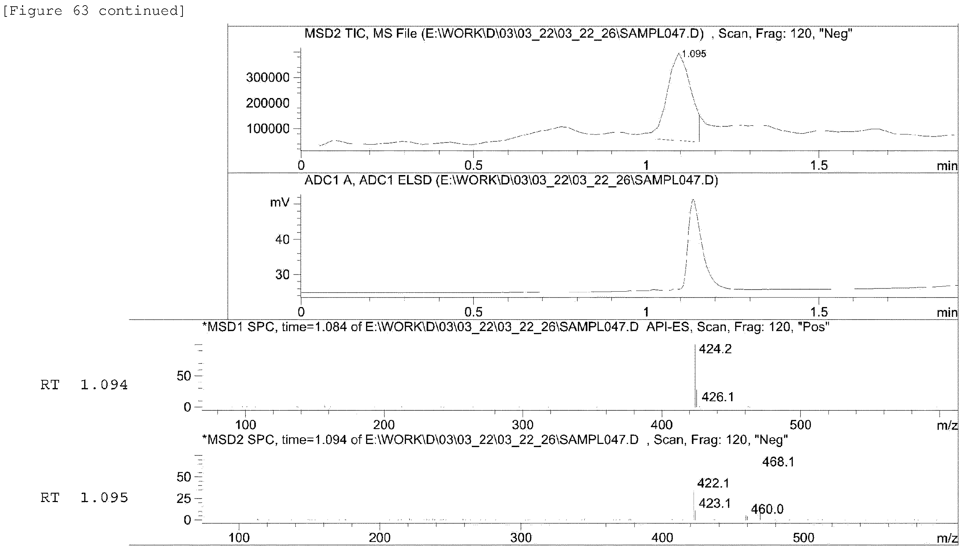

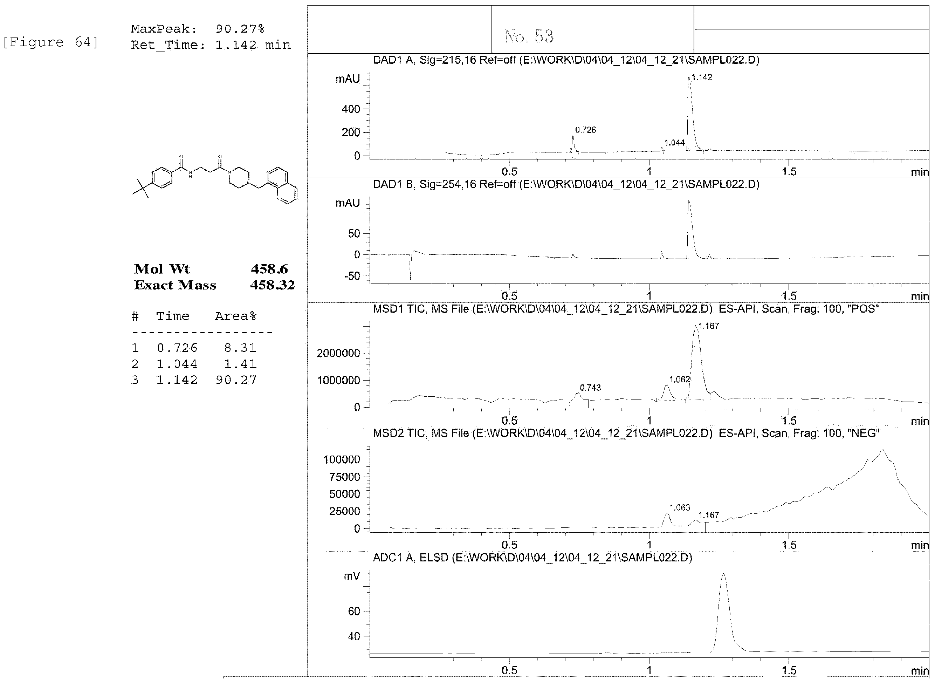

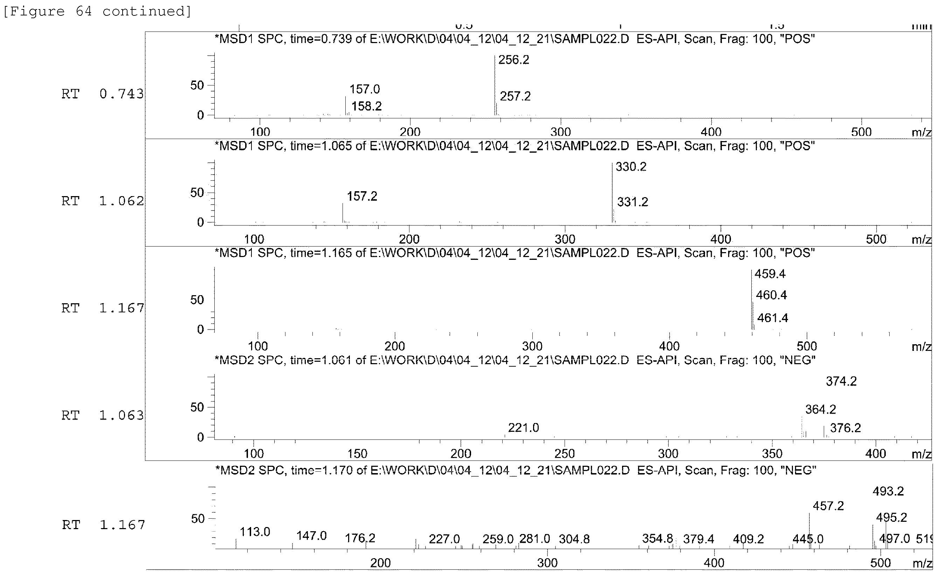

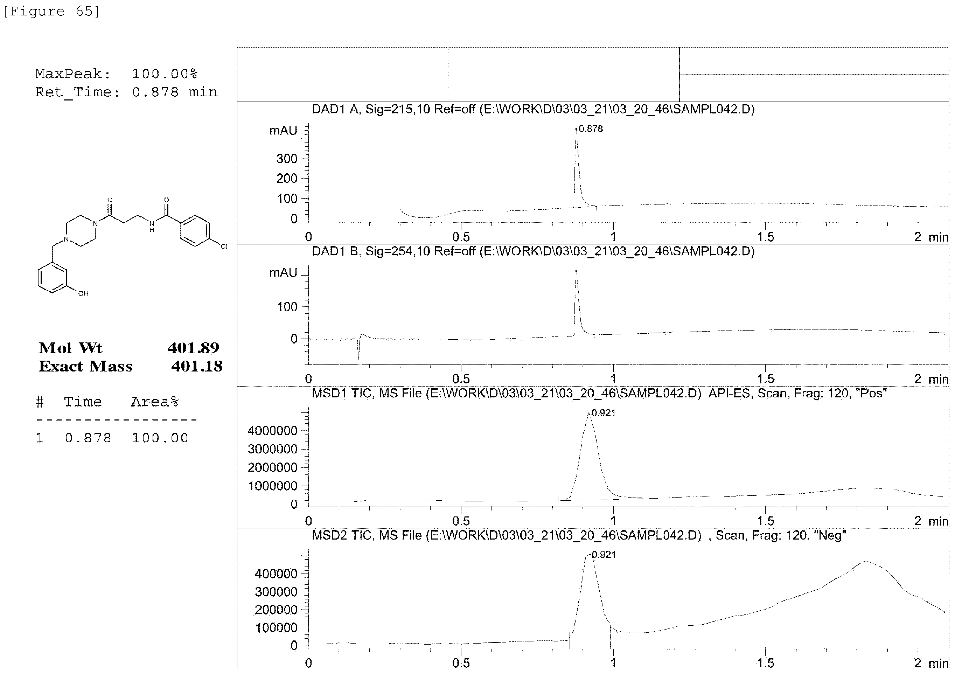

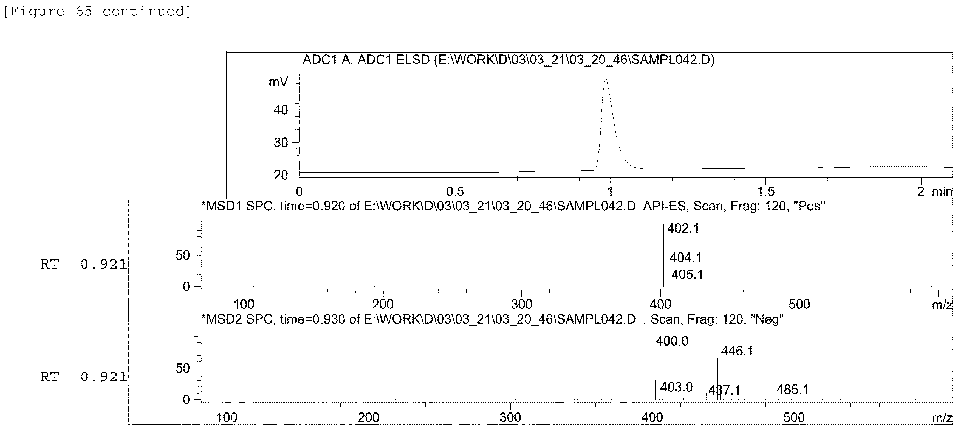

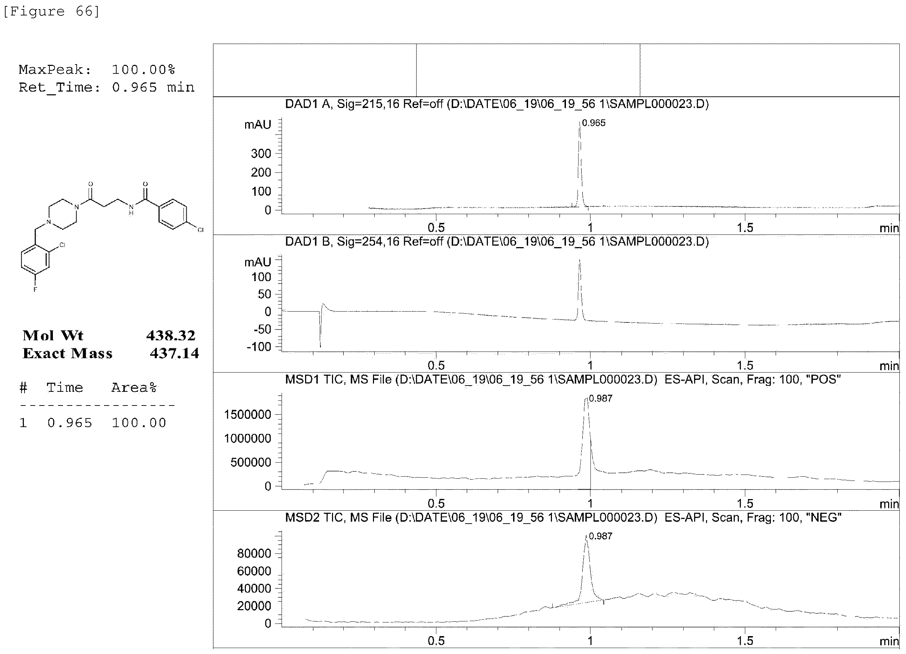

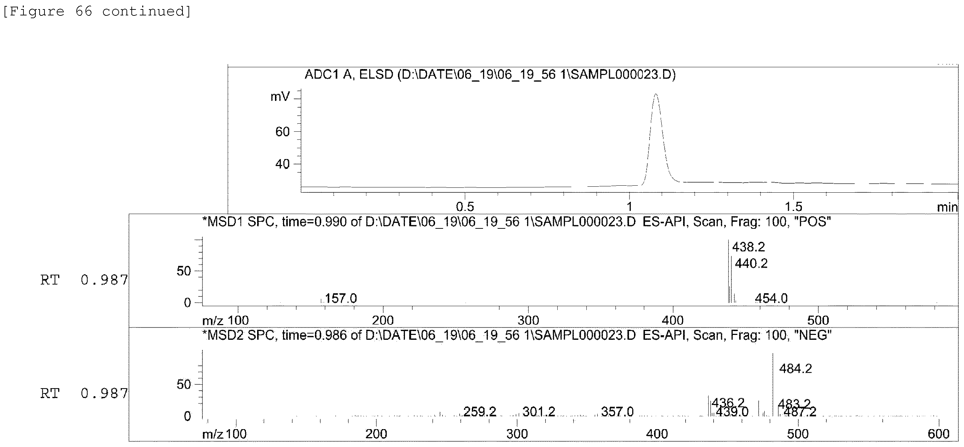

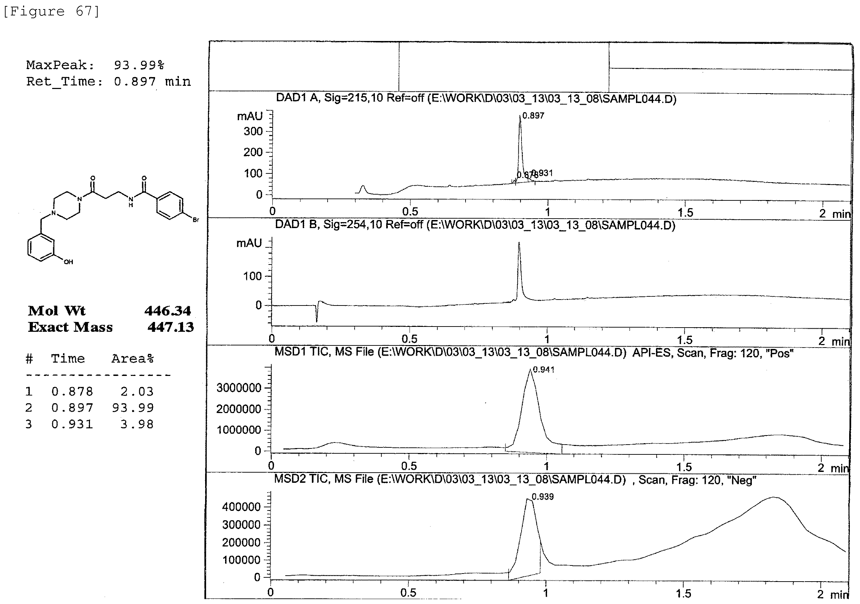

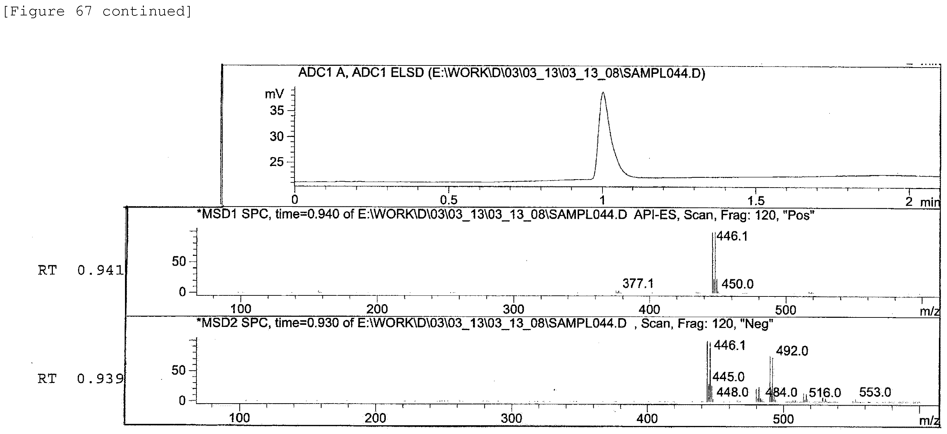

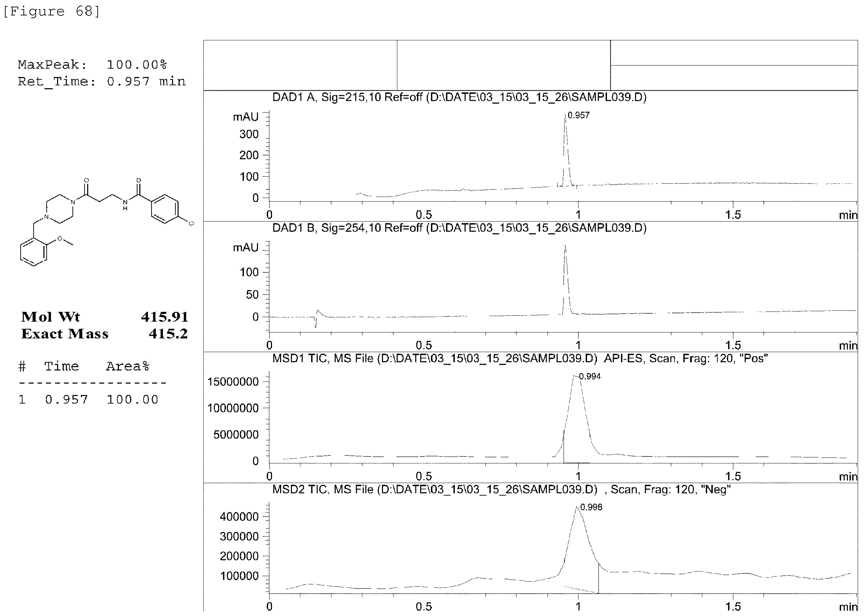

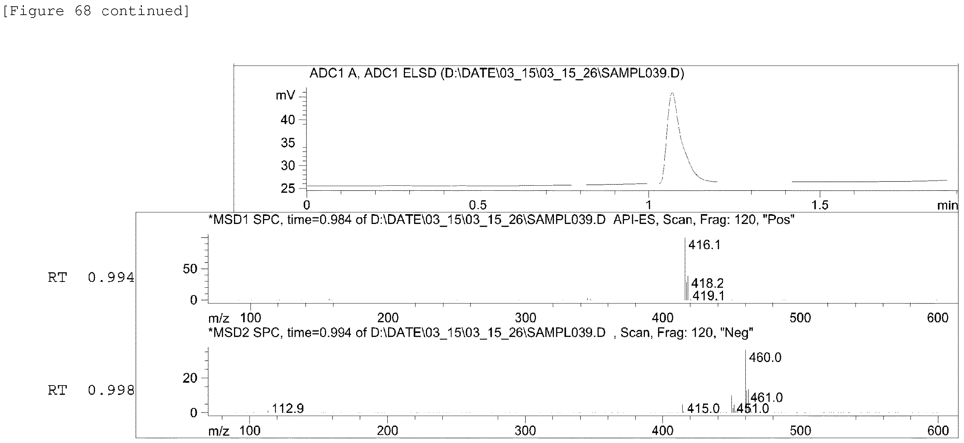

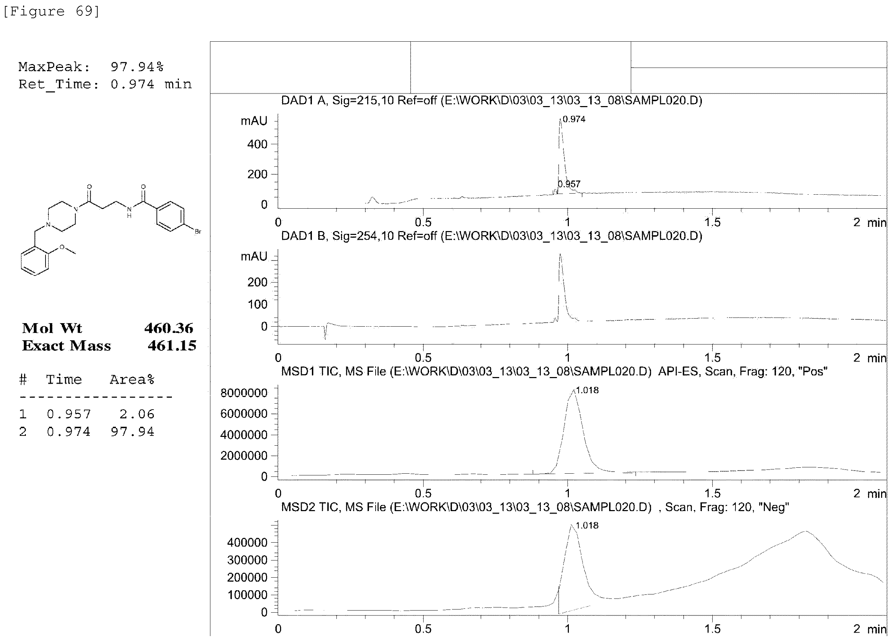

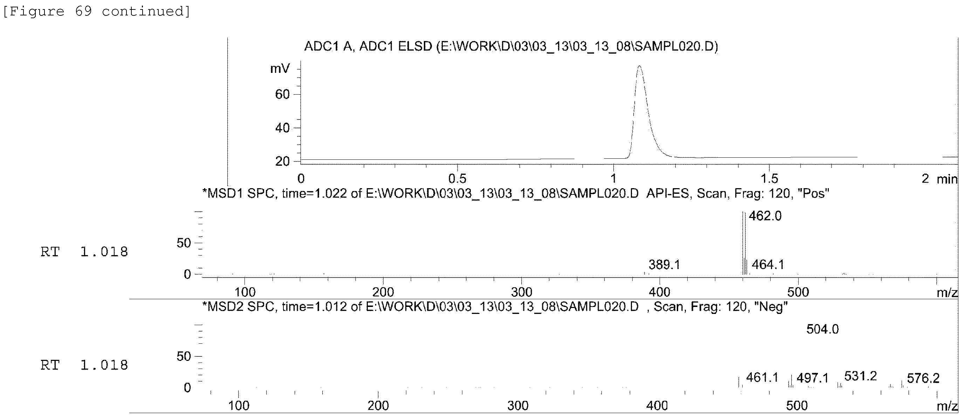

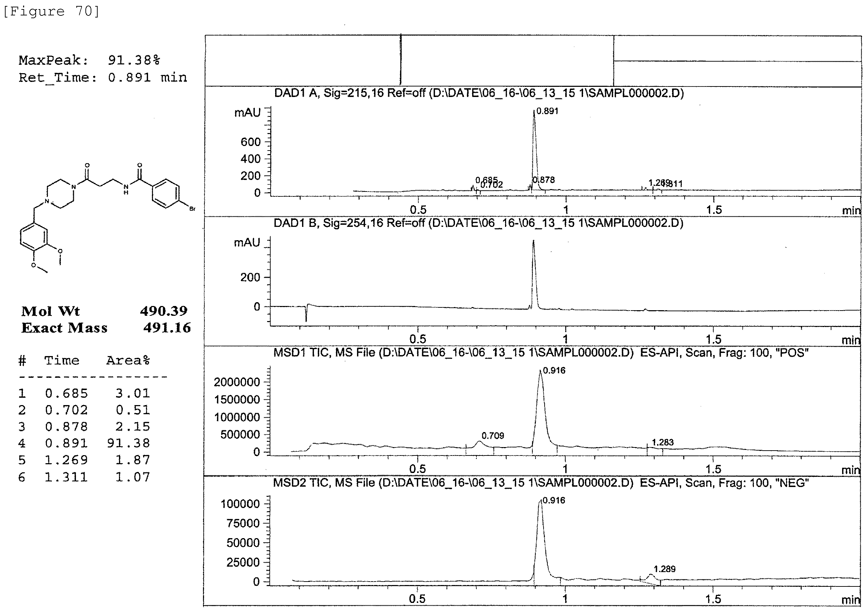

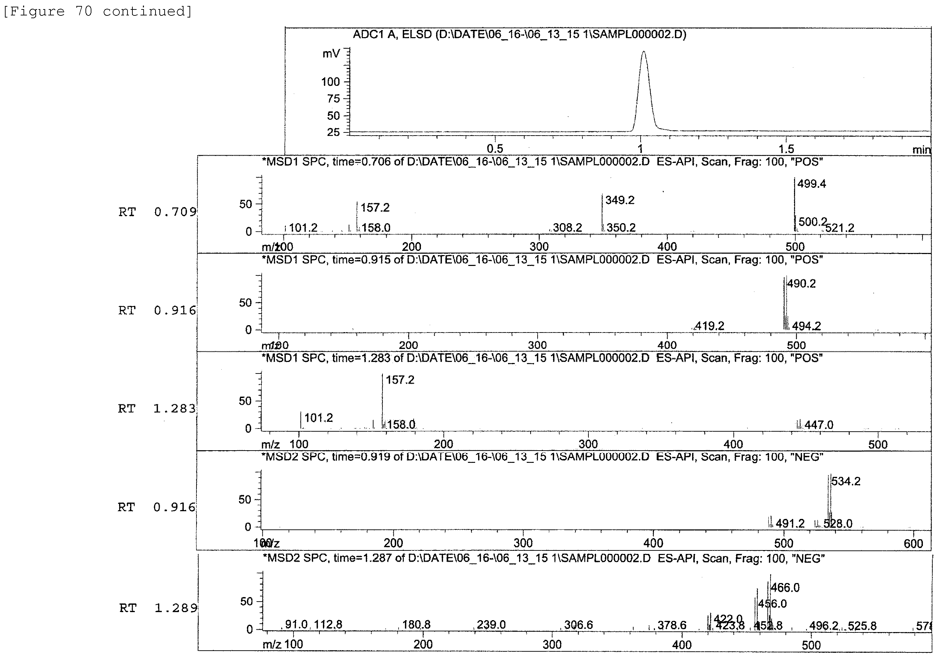

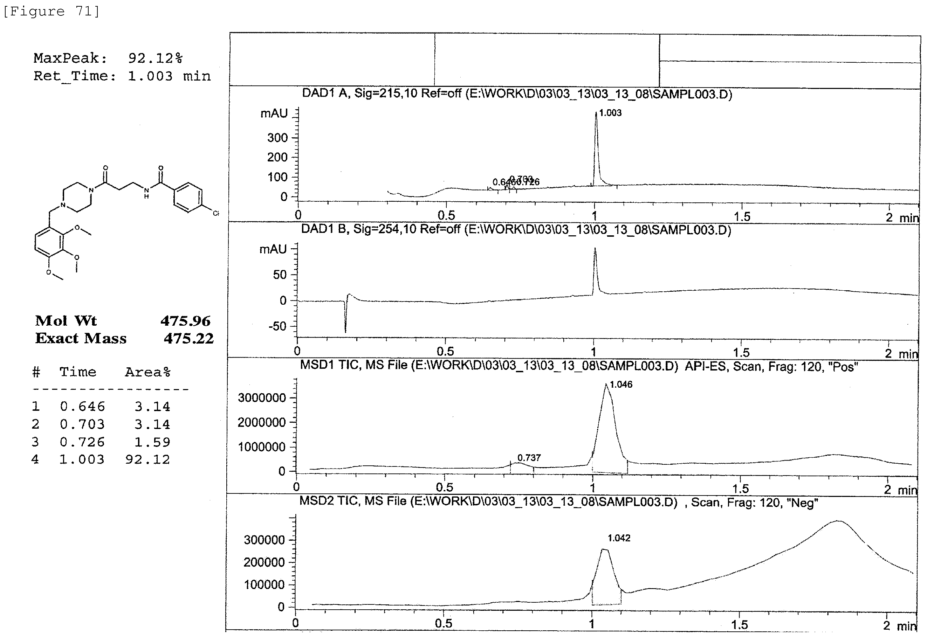

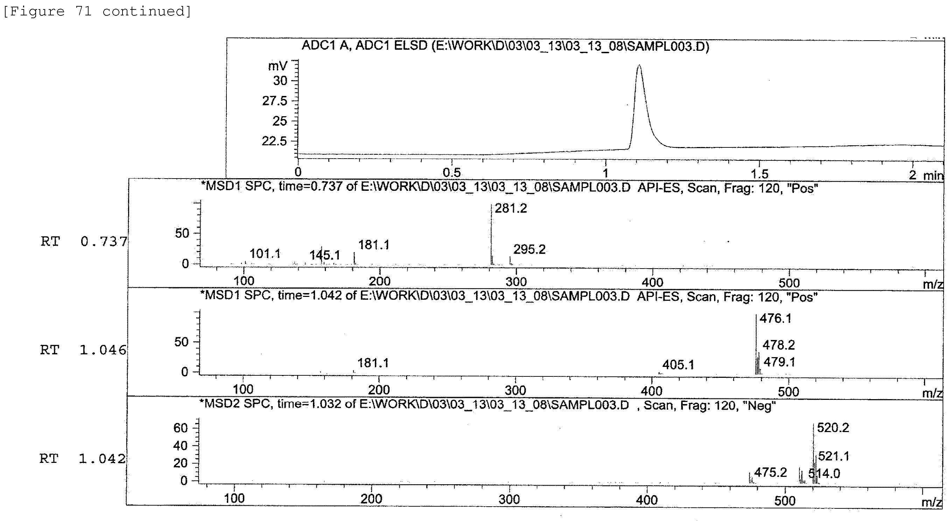

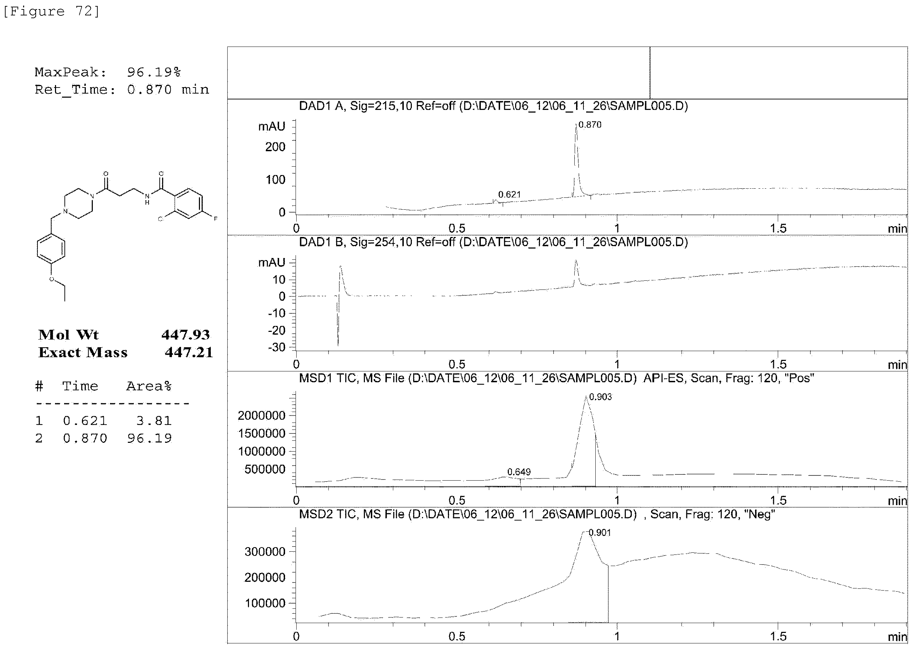

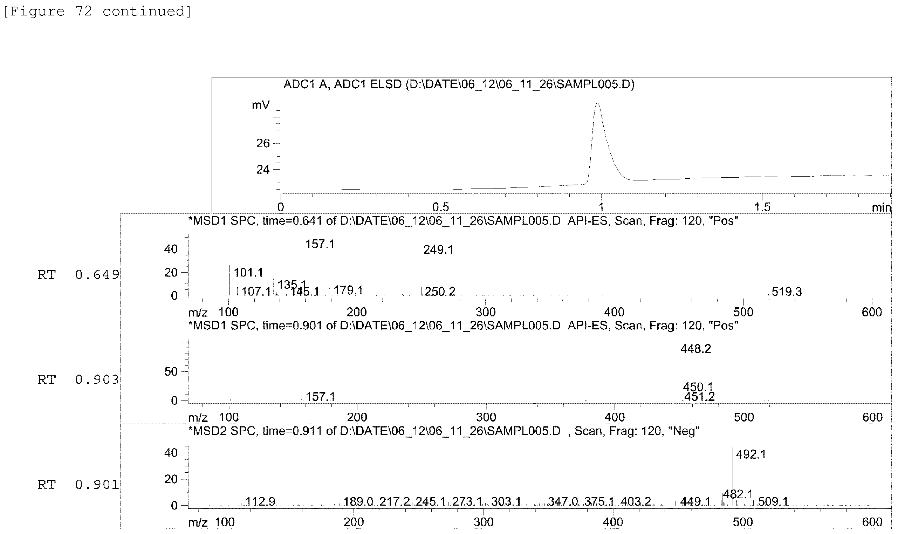

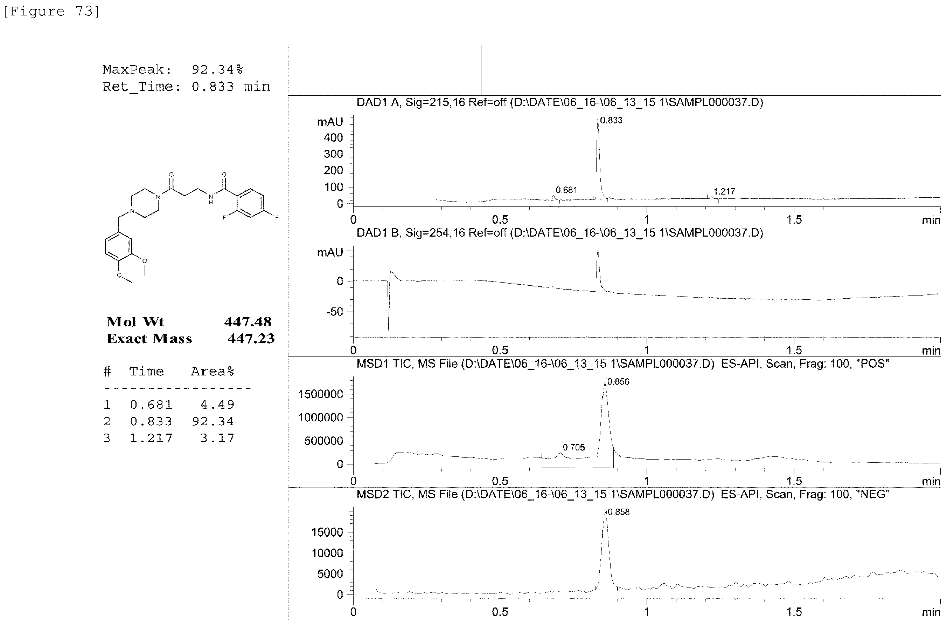

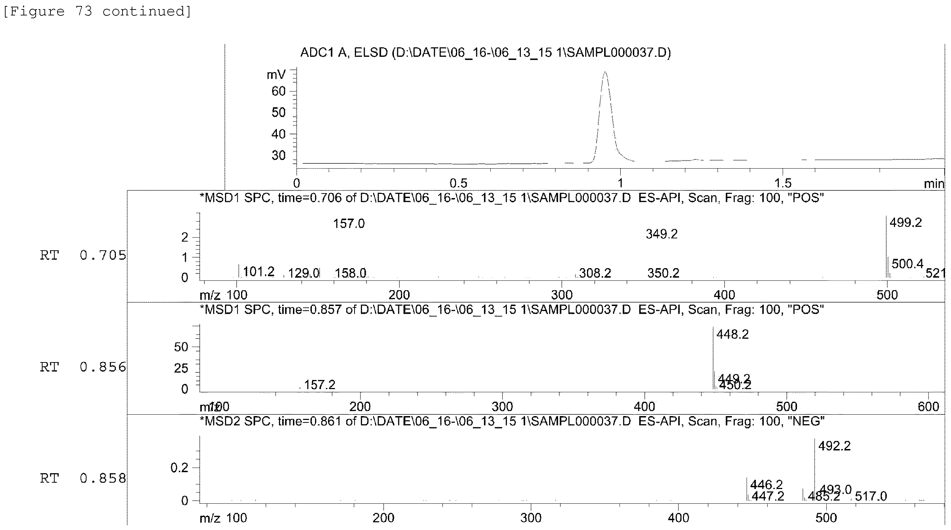

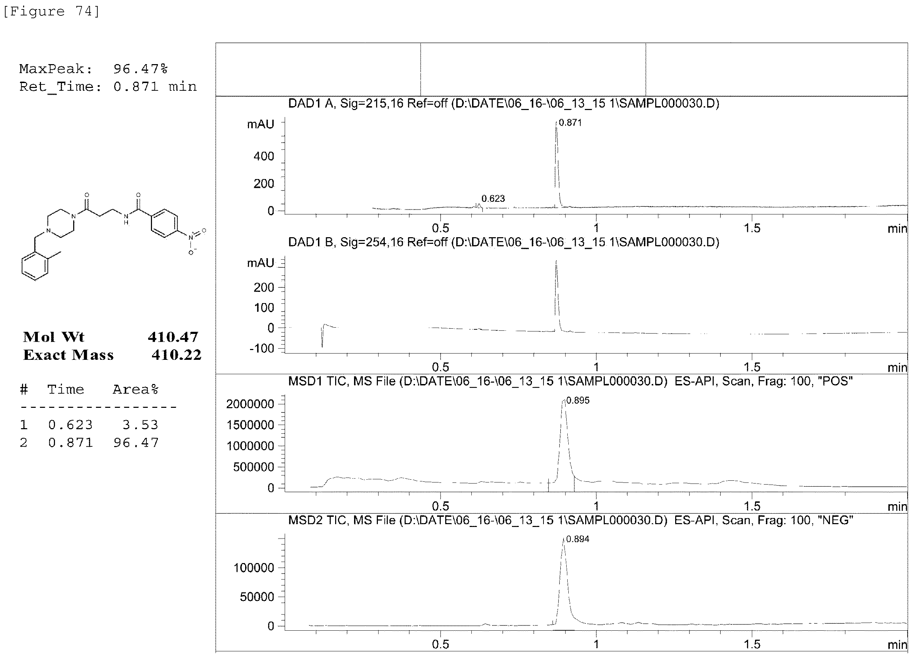

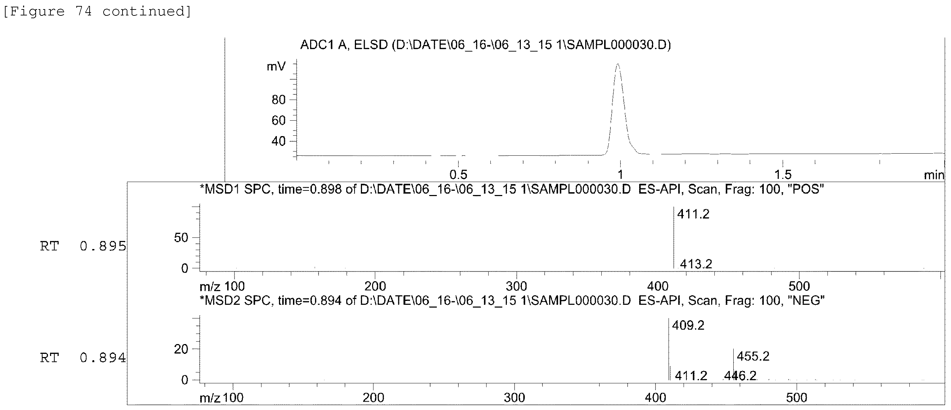

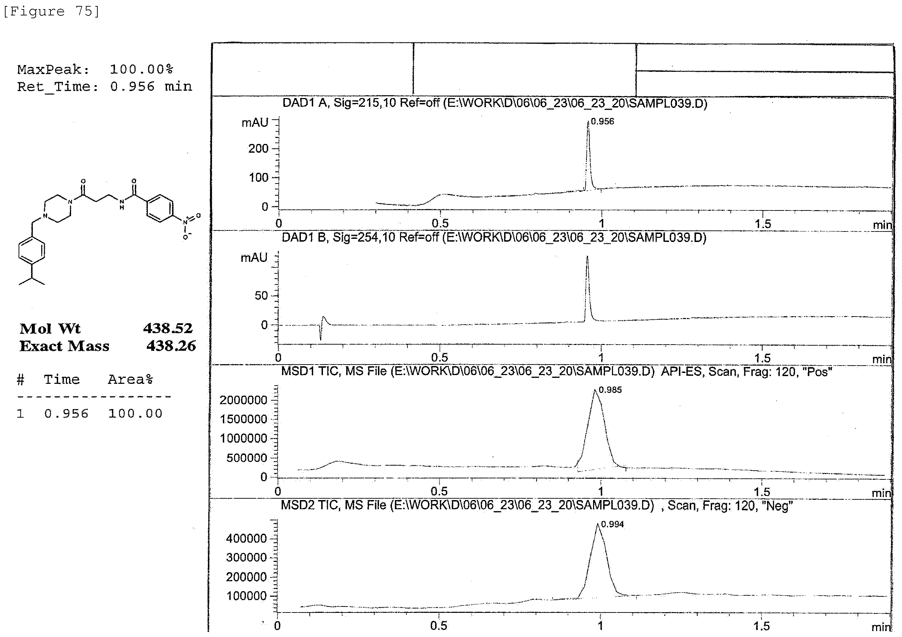

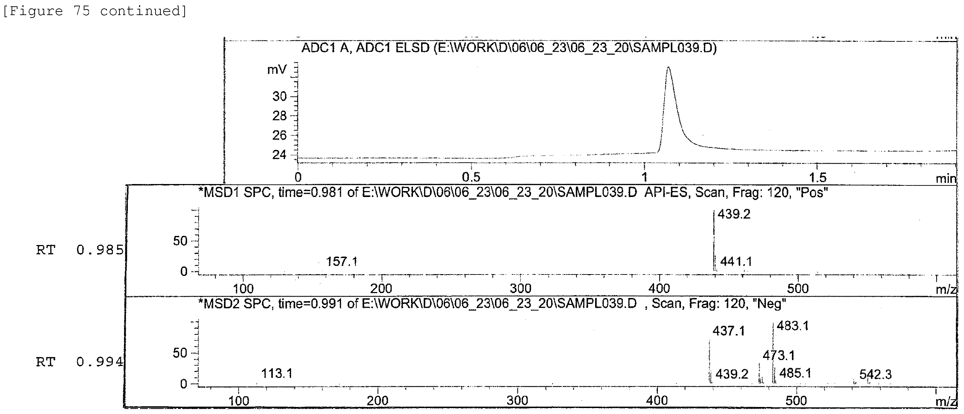

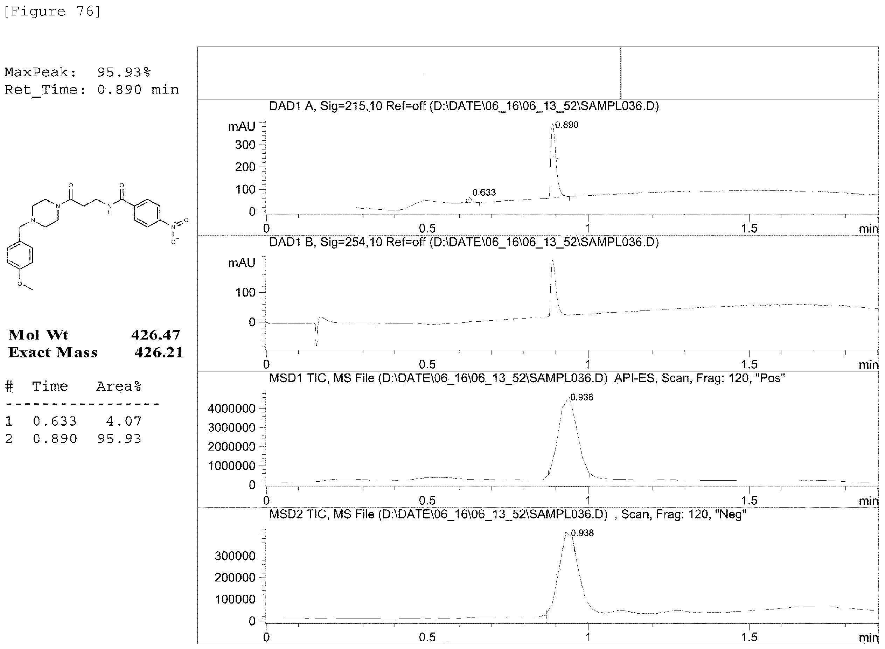

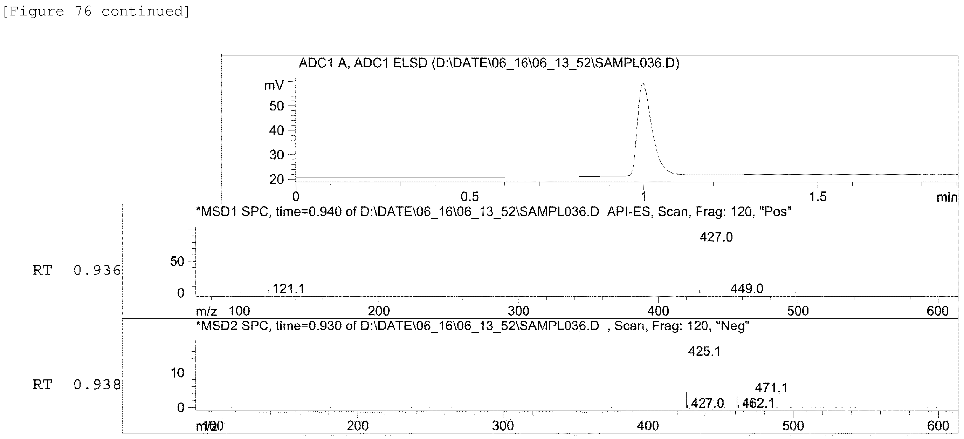

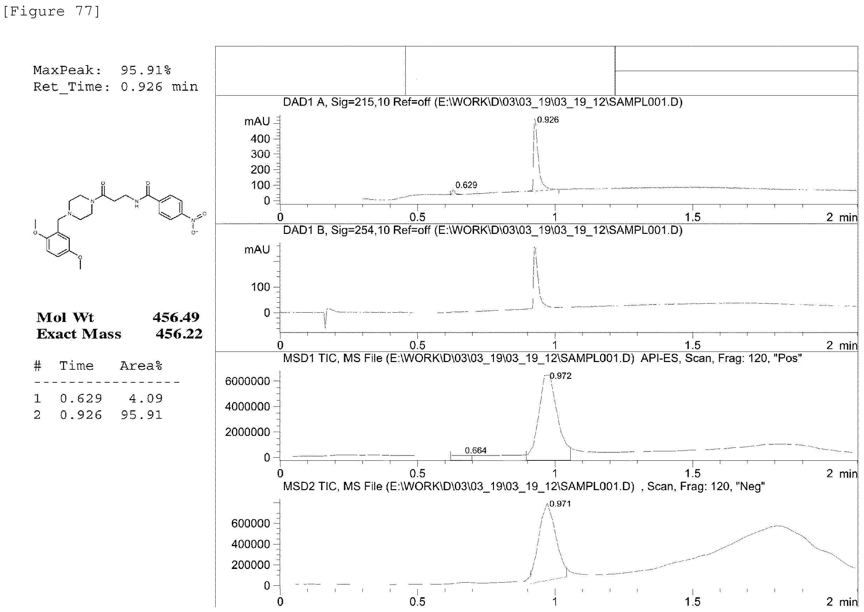

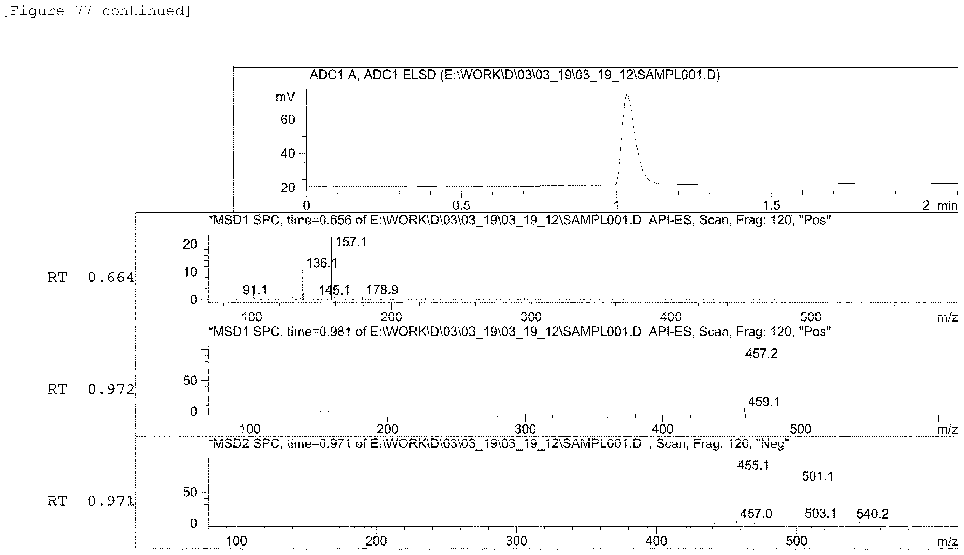

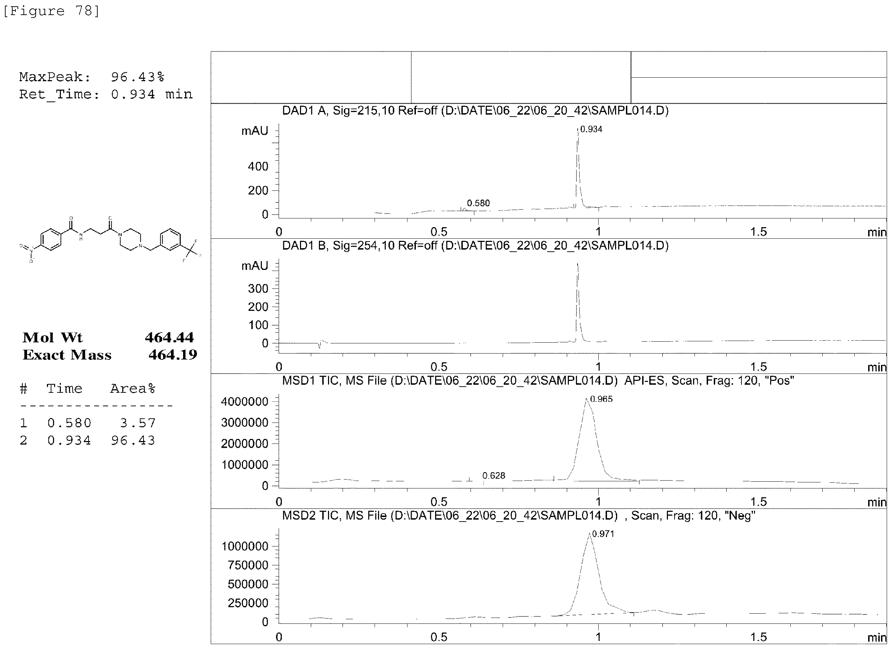

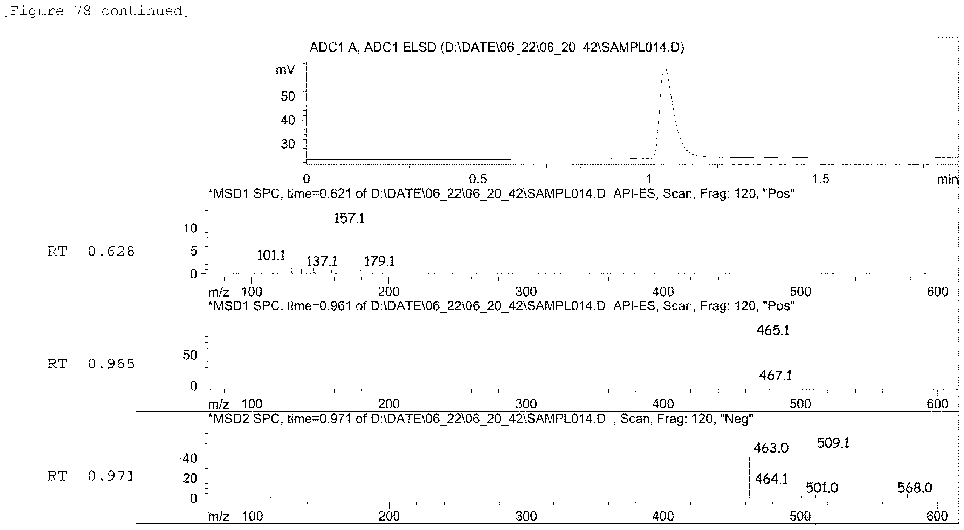

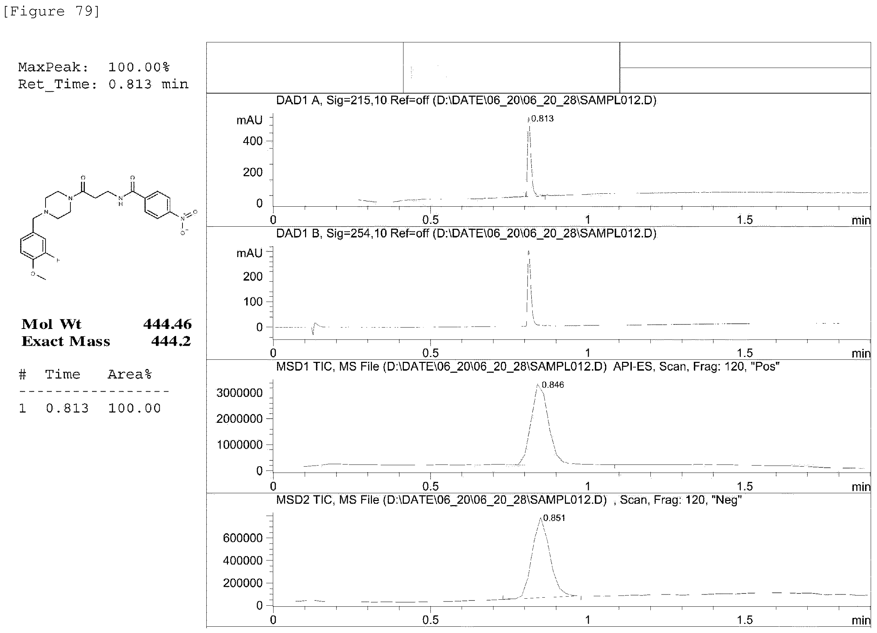

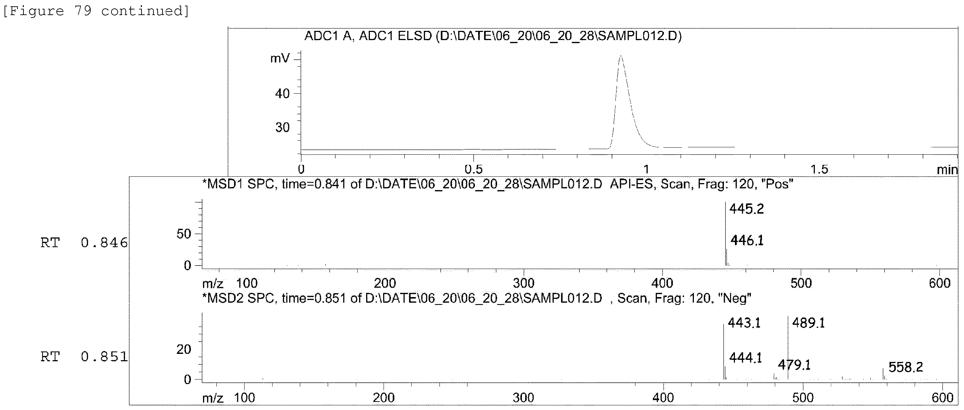

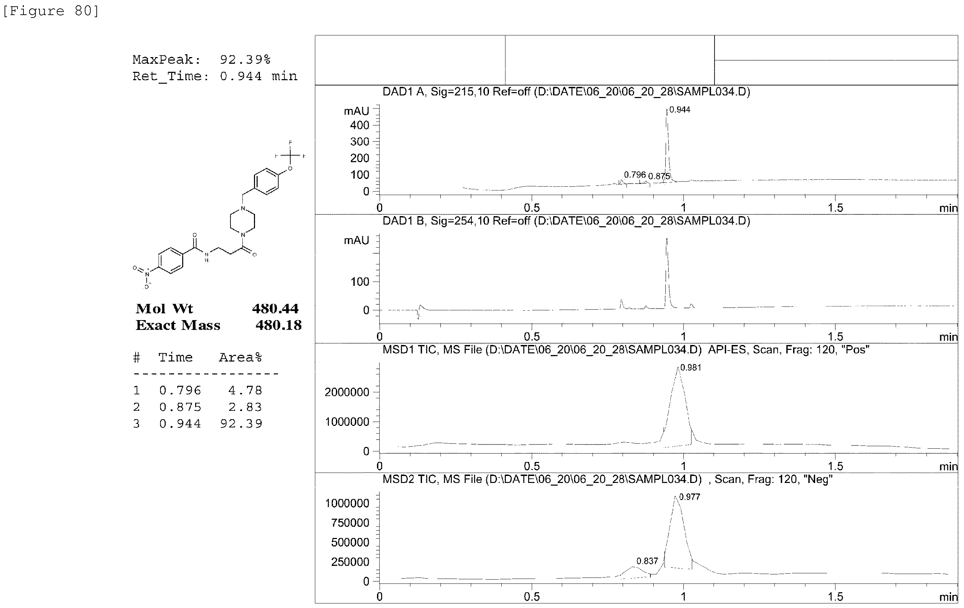

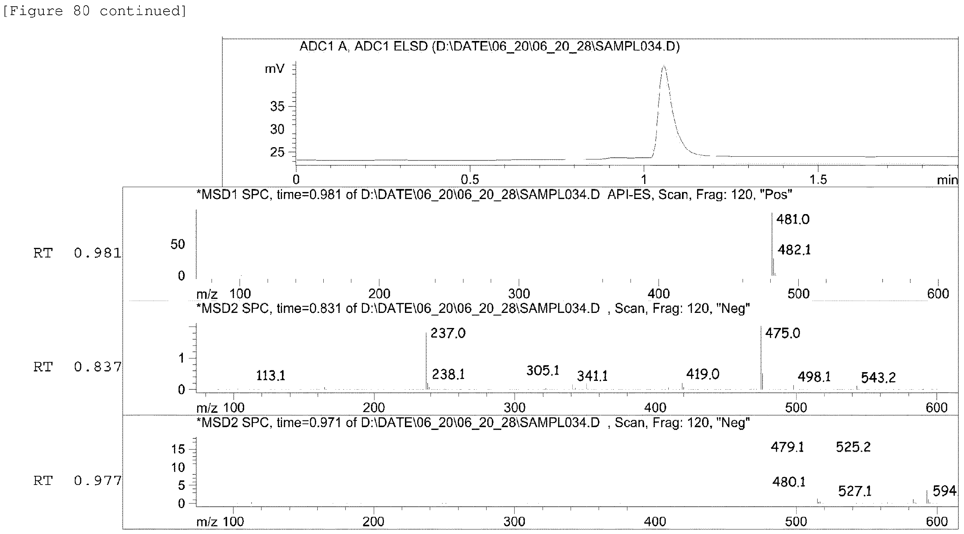

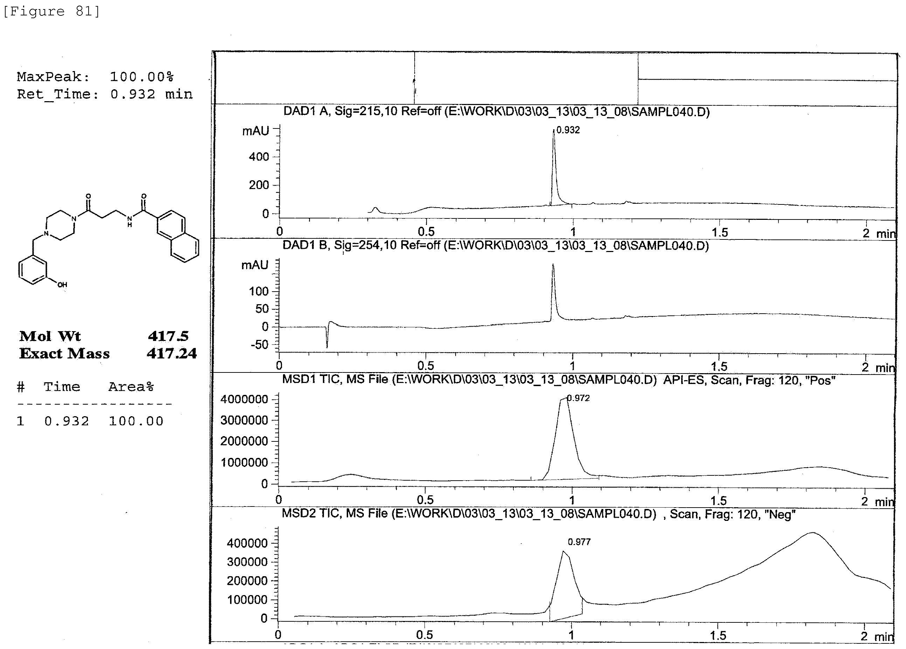

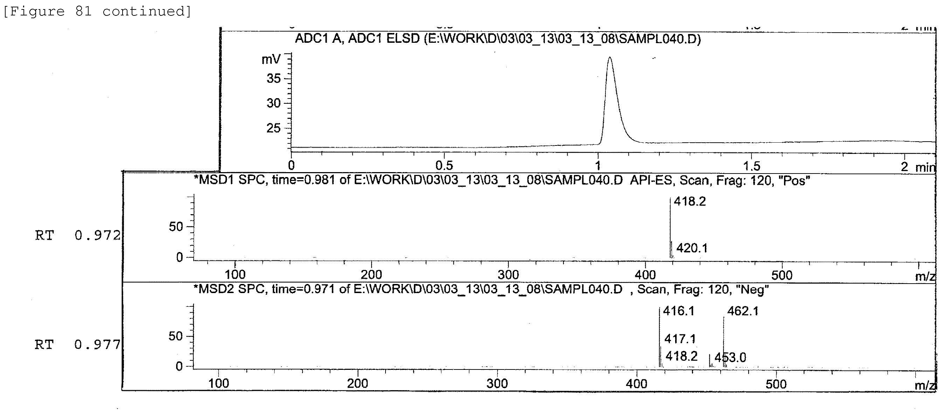

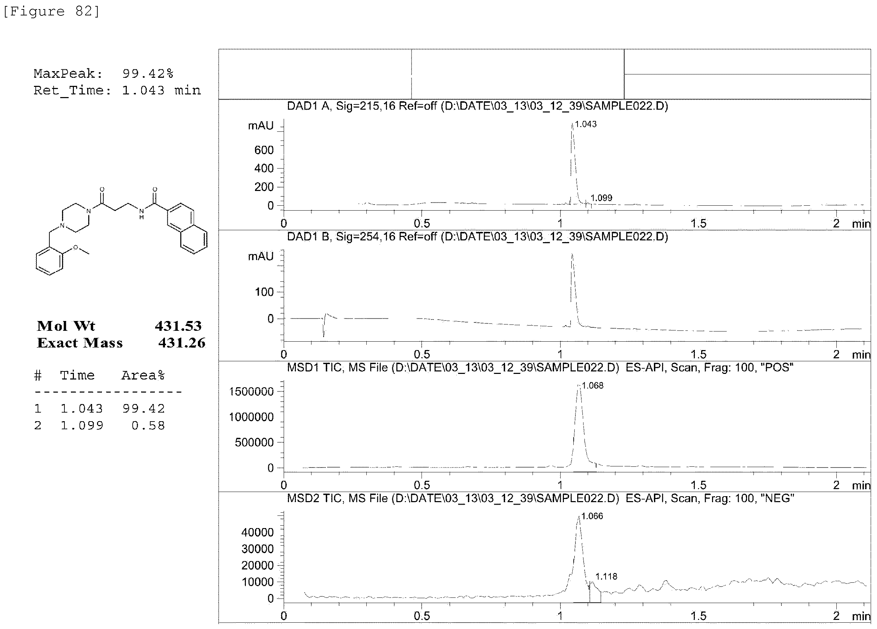

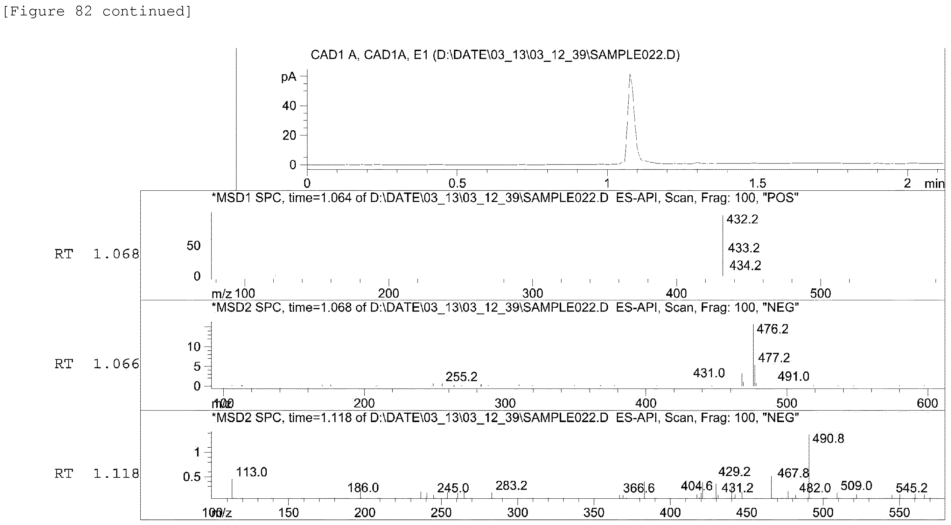

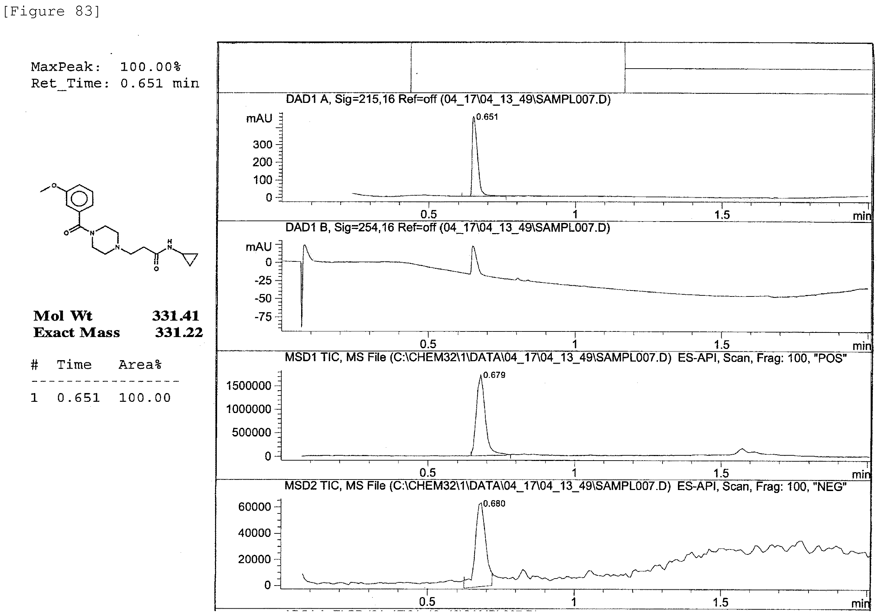

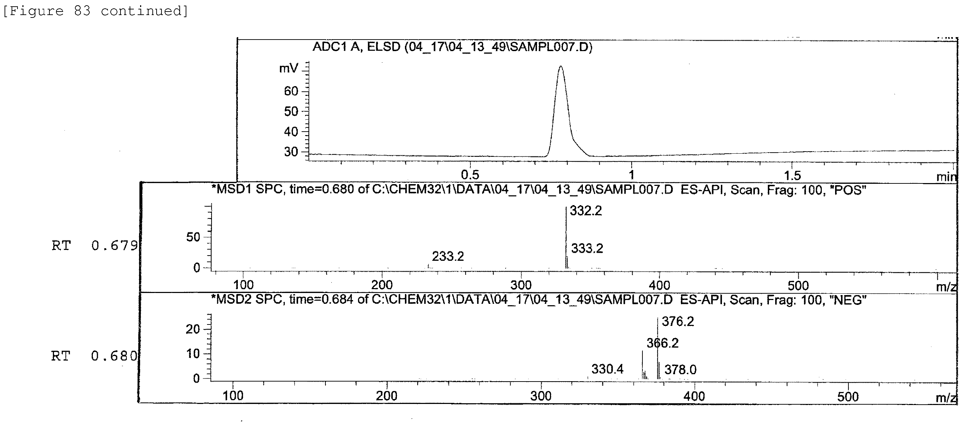

LCMS or NMR chart of compounds obtained in the preparation examples 1 to 72 is depicted in Fig. 8 to 83.

### Preparation Example 73

### Synthesis of Compound 73<AdipoRon:

### 2-(4-benzoyl-phenoxy)-N-(1-benzylpiperidin-4-yl)acetamide>

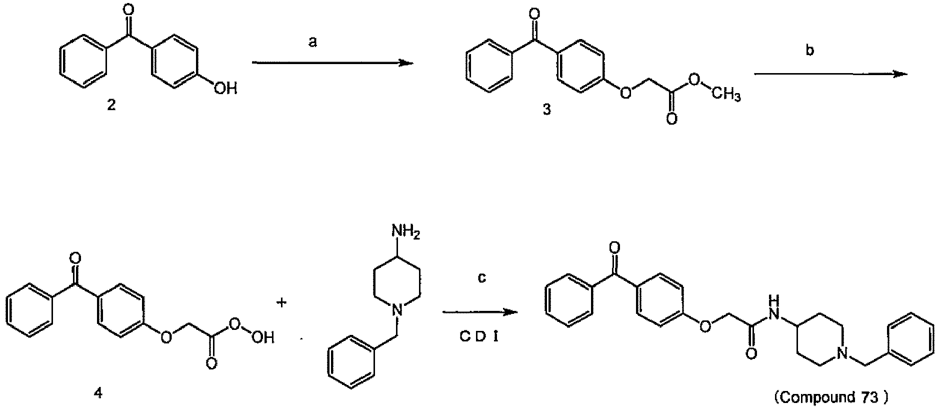

Hydroxybenzophenone 2, methyl chloroacetate and K₂CO₃ were mixed in acetone, refluxed and stirred to afford ester 3 (step a) . After completion of the reaction, an aqueous NaOH solution was added thereto, followed by acidification with HCl to obtain carboxylic acid 4 (step b). Then, 4-amino-1-benzylpiperidine was added to the carboxylic acid 4, followed by conducting the reaction in the same manner as in the preparation example 12, using carbonyl diimidazole (CDI) as a condensing agent, to obtain Compound 73.
¹H-NMR (600MHz, DMSO-d₆): *δ* 8.04 p.p.m. (d, j = 8.0 Hz, 1H), 7.74 (d, J = 9.1 Hz, 2H), 7.68 (d, J = 7.1 Hz, 2H), 7.65 (t, j = 7.6 Hz, 1H), 7.55 (t, j =5.8 Hz, 2H), 7.27-7.32 (m, 4H), 7.23 (t, J = 7.1 Hz, 1H), 7.09 (d, J = 9.0 Hz, 2H), 4.58 (s, 2H), 3.63 (m, 1H), 3.44 (s, 2H), 2.74 (d, J = 11.7 Hz, 2H), 1.99 (t, J = 11.5 Hz, 2H), 1.70 (d, J = 9.8 Hz, 2H), 1.50 p.p.m. (dq, J₁ = 3.0 Hz, J₂ = 11.9 Hz, 2H);
¹³C-NMR (150 MHz, DMSO-d₆) : δ 194.4, 166.2, 161.5, 138.6, 137.7, 132.2, 132.0, 129.8, 129.3, 128.7, 128.5, 128.1, 126.8, 114.6, 66.9, 62.1, 51.9, 46.1, 31.4

### Preparation Example 74

### Synthesis of Compound 74

### (4-tert-butyl-N-(3-{4-[(4-methoxyphenyl)methyl]piperazin-1-yl} -3-oxopropyl) benzamide <No. 112254>)

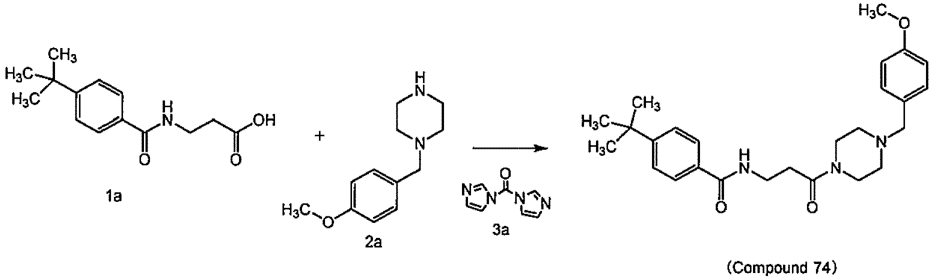

Acid 1a was loaded into a 10 ml flask (0.285 g, 1.0 equivalent), carbodiimidazole 3a (0.213g, 1.15 equivalent) was added and 1ml of DMFA was added. The reaction mixture was heated to 70°C for 1 hr till its dissolving. Amine 2a (0.236g, 1.0 equivalent) was added to the reaction mixture then and it was stored at 100°C for another 2 hr. The reaction mixture was cooled, 8 ml of water was added. The precipitate was triturated by a spatula, filtered and washed with 50% water/ isopropanol. After drying (without additional purification), 0.33 g of Compound 74 was obtained.

### Test Example 1: Measurement of AMPK phosphorylation ability in human AdipoRl-expressing cell line

The AMPK phosphorylation ability of the respective compounds according to the present invention was measured by using differentiated C2C12 cells or HEK 293T cells.

### 1. Measurement of AMPK phosphorylation ability (Assay 1)

### (1) Cell culture

### i) Evaluation by using HEK 293T cells

HEK 293T cells (ATCC; CRL-3216, medium; DMEM, Fetal Bovine Serum (FBS)) were cultured in a 12- or 24-well plate until they reached 100% confluency (37°C, 5% CO₂).

After starvation, a test compound listed in Tables 10 and 11 (concentration: 25 µM, 50 µM) was added thereto, and after 10 minutes, the medium was discarded and the plate was frozen with liquid nitrogen.

### ii) Evaluation by using C2C12 cells

C2C12 cells (ATCC; CRL-1772, medium; DMEM, 10% FBS) were cultured in a 12- or 24-well plate until they reached 100% confluency (37°C, 5% CO₂).

The medium was changed to 2.5% Horse Serum (HS) and differentiation was started. The medium (DMEM, 2.5% HS) was replaced every three days.

After starvation, a test compound listed in Table 12 (concentration: 10 µM) was added thereto, and after 10 minutes, the medium was discarded and the plate was frozen with liquid nitrogen.

### (2)Western blotting

### i) Preparation of sample

To the 12- or 24-well cell plate which had been frozen was added 100 or 150 µL of Cell lysis buffer pH 7.4 (50 mM HEPES (pH 7.4), 2 mM sodium orthovanadate, 10 mM sodium pyrophosphate, 10 mM NaF, 2 mM EDTA, 2 mM EGTA, 0.1% (v/v) NP-40, 0.2 mM PMSF), and the cells were scraped off with a cell scraper, suspended and transferred into a micro centrifugal tube. And then, the cells were ultrasonically crushed (1 second × 3 times) with a sonicator. Centrifugation was performed at 4°C at 15,000 rpm for 15 minutes, and the supernatant was recovered into a new tube.

To 100 µL of the supernatant was added 25 µL of 5 × sample buffer (280 mM Tris-HCl (pH 6.9), 40% glycerol, 10% β-mercaptoethanol, 13% SDS, a small amount of bromophenol blue) and mixed together. The rest of the supernatant was used for protein quantification, and the protein concentrations were uniformed by adding 1 × sample buffer depending on the concentration of each sample.

### ii) Polyacrylamide gel electrophoresis

An electrophoresis chamber was filled with 1 × SDS buffer (25 mM Tris-HCl, 250 mM glycine, 0.1% SDS), and the sample was applied to a well of polyacrylamide gel. Electrophoresis was performed until the bromophenol blue in the sample reached to the bottom of the gel.

### iii) Blotting

A PVDF membrane (GE; Hybond-P) was soaked in methanol for several minutes, and thereafter soaked in 1 × Transfer buffer.
1 × Transfer buffer; 10% 10 × Transfer buffer, 20% Me-OH, 70% distilled water
10 × Transfer buffer; 479 mM Tris-HCl, 386 mM glycine, 0.37% SDS

A filter paper was soaked in 1 × Transfer buffer sufficiently, above which the PVDF membrane and the gel after electrophoresis were stacked in this order, and thereabove a filter paper which had been soaked in 1 × Transfer buffer sufficiently was further stacked, from which an air was removed and the resultant was set in semi-dry type transfer apparatus and transferred (7V, 35 minutes).

### iv) Blocking

An unnecessary portion of the blotted membrane was cut off, and the rest was immediately soaked in blocking buffer (5% skimmed milk in Rinse buffer), and shaken at room temperature at 40 rpm for 3 hours.
Rinse buffer; 10 mM Tris-HCl (pH 7.5), 0.1 mM EDTA, 10% Triton X-100, 150 mM NaCl

### v) Antibody reaction

After blocking, the membrane was transferred into Rinse buffer, and washed at room temperature for 10 minutes while shaking. The membrane was soaked in a primary antibody solution (AMPK alpha Antibody (Cell signaling; #2532) 1/1,000 in Rinse buffer, Phospho-AMPK alpha (Thr172) (40H9) antibody (Cell signaling; #2535) 1/1,000 in Rinse buffer), and shaken at 4°C at 40 rpm overnight.

The membrane was transferred into Rinse buffer, and washed at room temperature for 45 minutes while shaking (the Rinse buffer was replaced every 3 to 5 minutes).

The membrane was soaked in a secondary antibody solution (goat anti-rabbit IgG-HRP Antibody (Santa Cruz; sc-2030) 1/2,000 in Rinse buffer), and shaken at room temperature at 40 rpm for 1 hour.

### vi) Detection of signals

After the membrane was washed for 45 minutes in the same way as after the primary antibody reaction, the membrane was soaked in a chemiluminescent reaction solution (ECL Western Blotting Detection Reagents, GE Healthcare) for about 1 minute. An excess of the reaction solution was removed, and then the membranes were arranged on a transparent film and sandwiched with it, with which an X-ray film was exposed in a darkroom (for about several seconds to 60 seconds) to develop the film.

### vii) Quantification

The X-ray film was scanned to take the image, for which the band was measured by using the image analysis software "Image J" to determine the respective amounts of pAMPK and AMPK.

### (3) Results

The compounds according to the present invention promoted phosphorylation of AMPK and activated AMPK (Tables 10 to 12).

### 2. Measurement of AMPK phosphorylation ability (Assay 2)

### (1) Cell culture

### i) Evaluation by using HEK 293T cells

HEK 293T cells (medium; DMEM, 10% FBS) were cultured in a 24-well plate until they reached 60% confluency (37°C, 5% CO₂).

First, 1 µL of Lipofectamine RNAiMAX and 50 µL of OPTI-MEM were mixed, and then 5 µM siRNA (sense siRNA sequence: GAGACUGGCAACAUCUGGACAtt (SEQ ID NO: 1)) and 50 µL of OPTI-MEM were mixed and the resultants were left to stand at room temperature for 5 minutes. And then, the 1 µL Lipofectamine RNAiMAX/ 50 µL OPTI-MEM and the 5 µM siRNA/ 50 µL OPTI-MEM were mixed, and the resultant was left to stand at room temperature for 10 minutes, which was added to the cells.

After 5 minutes, the medium (DMEM, 10% FBS) was replaced, and after starvation on the next day, a test compound listed in Tables 10 and 11 was added thereto. After 10 minutes, the medium was discarded and the plate was frozen with liquid nitrogen.

### ii) Evaluation by using C2C12 cells

C2C12 cells (ATCC; CRL-1772, medium; DMEM, 10% Fetal Bovine Serum (FBS)) were cultured in a 12- or 24-well plate until they reached 100% confluency (37°C, 5% CO₂). The medium was changed to DMEM, 2.5% Horse Serum (HS) and differentiation was started. The medium (DMEM, 2.5% HS) was replaced two days after the start of differentiation. The medium (DMEM, 2.5% HS) was further replaced two days after that.

On the next day following the replacement of the medium, first, 1 µL of Lipofectamine RNAiMAX and 50 µL of OPTI-MEM were mixed, and then 5 µM siRNA (sense siRNA sequence: GAGACUGGCAACAUCUGGACAtt (SEQ ID NO: 1)) and 50 µL of OPTI-MEM were mixed and the resultants were left to stand at room temperature for 5 minutes. And then, the 1 µL Lipofectamine RNAiMAX/ 50 µL OPTI-MEM and the 5 µM siRNA/ 50 µL OPTI-MEM were mixed, and the resultant was left to stand at room temperature for 10 minutes, which was added to the cells.

After 5 minutes, the medium (DMEM, 2.5% HS) was replaced, and after starvation on the next day, a test compound listed in Table 12 was added thereto. After 10 minutes, the medium was discarded and the plate was frozen with liquid nitrogen.

### (2) Western blotting

The respective amounts of pAMPK and AMPK were determined in the same way as in the above Assay 1.

### (3) Results

The promotion of AMPK phosphorylation by the compounds according to the present invention was significantly reduced by adding siRNA which acts specifically on AdipoR1 (Tables 10 to 12). Accordingly, this indicates that the compounds according to the present invention increased AMPK phosphorylation via AdipoR1.

**[Table 10]**

| Compound Number | Assay 1 | | Assay 2 | |
|---|---|---|---|---|
| | pAMPK/AMPK (%) | | pAMPK/AMPK (ratio) siRNA | |
| | 25 µM | 50 µM | unrelated siRNA | AdipoR1 siRNA |
| Compound 1 | 70.10 | 63.92 | | |
| Compound 2 | 77.78 | 90.47 | | |
| Compound 3 | 39.02 | 44.32 | | |
| Compound 4 | 47.44 | 45.44 | | |
| Compound 5 | 69.72 | 62.88 | | |
| Compound 6 | 24.57 | 60.56 | | |
| Compound 7 | 79.86 | 94.13 | | |
| Compound 8 | 77.49 | 72.23 | | |
| Compound 9 | 23.69 | 62.46 | | |
| Compound 10 | 83.62 | 72.56 | | |
| Compound 11 | 83.12 | 102.94 | 2.58 | 3.75 |
| Compound 12 | 106.36 | 82.93 | 2.51 | 2.47 |
| Compound 13 | 40.65 | 39.95 | | |
| Compound 14 | 63.62 | 51.87 | | |
| Compound 15 | 88.71 | 88.18 | 3.27 | 3.49 |
| Compound 16 | 55.49 | 46.30 | | |
| Compound 17 | 88.72 | 75.82 | | |
| Compound 18 | 59.58 | 72.81 | | |
| Compound 19 | 83.61 | 95.02 | 3.88 | 3.75 |
| Compound 20 | 47.30 | 60.30 | | |
| Compound 21 | 81.11 | 83.12 | 2.34 | 1.92 |
| Compound 22 | 61.45 | 67.26 | | |
| Compound 23 | 51.41 | 49.81 | | |
| Compound 24 | 23.68 | 60.46 | | |
| Compound 25 | 40.07 | 42.87 | | |
| Compound 26 | 55.06 | 56.65 | | |
| Compound 27 | 45.54 | 37.30 | | |
| Compound 28 | 71.21 | 78.89 | | |
| Compound 29 | 72.45 | 74.42 | | |
| Compound 30 | 122.70 | 89.75 | 2.49 | 2.35 |
| Compound 31 | 80.60 | 80.60 | 3.17 | 2.61 |
| Compound 32 | 88.09 | 91.40 | 3.38 | 4.14 |
| Compound 33 | 106.31 | 84.10 | 2.88 | 2.63 |
| Compound 34 | 80.64 | 80.50 | 2.32 | 2.01 |
| Compound 35 | 80.77 | 83.74 | 3.38 | 2.01 |

**[Table 11]**

| Compound Number | Assay 1 | | Assay 2 | |
|---|---|---|---|---|
| | pAMPK/AMPK (%) | | pAMPK/AMPK (ratio) siRNA | |
| | 25 µM | 50 µM | unrelated siRNA | AdipoR1 siRNA |
| Compound 36 | 80.42 | 84.19 | 2.52 | 2.56 |
| Compound 37 | 83.83 | 80.12 | 3.11 | 2.69 |
| Compound 38 | 112.70 | 87.54 | 2.67 | 2.43 |
| Compound 39 | 64.98 | 61.18 | | |
| Compound 40 | 80.20 | 91.33 | 2.25 | 2.25 |
| Compound 41 | 62.03 | 69.38 | | |
| Compound 42 | 69.97 | 89.62 | | |
| Compound 43 | 77.13 | 82.95 | | |
| Compound 44 | 72.14 | 78.77 | | |
| Compound 45 | 67.44 | 82.01 | | |
| Compound 46 | 80.93 | 80.11 | 2.58 | 2.74 |
| Compound 47 | 80.31 | 80.39 | 2.78 | 2.44 |
| Compound 48 | 80.04 | 85.28 | 3.42 | 3.16 |
| Compound 49 | 42.40 | 34.07 | | |
| Compound 50 | 39.00 | 71.13 | | |
| Compound 51 | 33.33 | 55.14 | | |
| Compound 52 | 66.63 | 85.17 | | |
| Compound 53 | 91.44 | 81.80 | 2.26 | 2.04 |
| Compound 54 | 57.51 | 33.78 | | |
| Compound 55 | 61.64 | 105.20 | | |
| Compound 56 | 49.23 | 40.38 | | |
| Compound 57 | 30.26 | 45.31 | | |
| Compound 58 | 41.20 | 62.58 | | |
| Compound 59 | 74.09 | 78.14 | | |
| Compound 60 | 34.44 | 76.81 | | |
| Compound 61 | 78.20 | 76.51 | | |
| Compound 62 | 72.75 | 80.92 | | |
| Compound 63 | 65.40 | 68.22 | | |
| Compound 64 | 80.29 | 83.01 | 4.86 | 3.57 |
| Compound 65 | 62.79 | 69.84 | | |
| Compound 66 | 43.00 | 55.80 | | |
| Compound 67 | 70.98 | 70.70 | | |
| Compound 68 | 53.92 | 45.94 | | |
| Compound 69 | 80.04 | 100.79 | 5.28 | 4.03 |
| Compound 70 | 84.71 | 88.96 | 2.43 | 2.46 |
| Compound 71 | 78.18 | 65.40 | | |
| Compound 72 | 42.05 | 46.92 | | |

**[Table 12]**

| Compound number | Assay 1 | Assay 2 | |
|---|---|---|---|
| | pAMPK/AMPK (%) | pAMPK/AMPK (ratio) siRNA | |
| | 10 µM | unrelated siRNA | AdipoR1 siRNA |
| 73 | 84.33 | 4.75 | 2.68 |
| 74 | 86.43 | 3.70 | 2.07 |
| No.103694 | 81.05 | 4.67 | 3.34 |
| No.168198 | 84.85 | 4.53 | 2.97 |
| No.189474 | 88.87 | 2.00 | 1.62 |
| No.189640 | 82.40 | 1.85 | 1.82 |
| No.191294 | 89.56 | 3.25 | 3.54 |
| No.183665 | 99.03 | 2.88 | 2.83 |
| No.195831 | 99.10 | 2.88 | 2.98 |
| No.209705 | 81.28 | 1.52 | 1.81 |
| No.274971 | 81.64 | 2.38 | 2.51 |
| No.196462 | 82.72 | 2.06 | 2.58 |
| No.197248 | 89.63 | 2.09 | 2.55 |
| No.211156 | 93.79 | 2.36 | 2.59 |
| No.197372 | 85.37 | 1.78 | 1.96 |
| No.198637 | 87.39 | 2.24 | 2.51 |
| No.214617 | 91.97 | 2.21 | 2.78 |
| No.200737 | 83.71 | 2.13 | 2.68 |
| No.206685 | 90.15 | 1.76 | 2.39 |
| No.251327 | 83.18 | 2.85 | 3.15 |
| No.260544 | 81.90 | 3.79 | 4.12 |
| No.264785 | 80.62 | 3.70 | 3.58 |
| No.473771 | 85.03 | 1.67 | 2.39 |
| No.268508 | 80.12 | 2.58 | 2.87 |
| No.268949 | 86.36 | 3.10 | 2.82 |
| No.484140 | 81.88 | 2.34 | 2.30 |
| No.272299 | 91.22 | 2.83 | 2.60 |
| No.272350 | 84.20 | 2.03 | 2.54 |
| No.492284 | 90.14 | 2.05 | 1.88 |
| No.466151 | 89.26 | 2.22 | 1.94 |
| No.550212 | 80.04 | 1.91 | 2.14 |

### Test Example 2: Affinity for AdipoR1 and AdipoR2

As described below, surface plasmon resonance (SPR) was performed to measure the affinity of Compound 73 (hereinafter referred to as "AdipoRon"),for both AdipoR1 and AdipoR2.

### i) Method

Surface plasmon resonance measurements were performed by a BIAcore X100 system (GE Healthcare) and sensor chip SA (GE Healthcare). Human AdipoR1 and AdipoR2 were expressed with the baculovirus system, and purified to homogeneity. The AdipoR1 and AdipoR2 samples were then reconstituted into egg-phosphatidylcholine liposomes containing biotinyl phosphatidylethanolamine (Avanti), as previously reported^{a}. Mouse full-length adiponectin was generated as previously described^{b,c,d,e}. AdipoR1 and AdipoR2 were immobilized onto a sensor chip SA to levels of 2, 500-3,000 response units (RU) using standard immobilization protocols (GE Healthcare). We used a rhodopsin receptor as control, and observed that AdipoRon indeed does not react a rhodopsin receptor at all.

Experiments were performed at 25ºC using AdipoRon (0.49-31.25 *µ*M) and adiponectin (1.5 ng-3.75 *µ*g) and using electrophoresis buffer (20 mM Hepes (pH 7.4), 200 mM NaCl, 10% glycerol, 0.05% (v/v) surfactant P20). Biacore X100 Evaluation Software was used to determine the equilibrium dissociation constant (K_{D}) of the compound or proteins.

### <Reference>

a) Hino, T., et al., G-protein-coupled receptor inactivation by an allosteric inverse-agonist antibody. Nature 482, 237-240 (2012).
b) Yamauchi, T., et al., Globular adiponectin protected ob/ob mice from diabetes and apoE deficient mice from atherosclerosis. J. Biol. Chem. 278, 2461-2468 (2003).
c) Iwabu, M., et al., Adiponectin and AdipoR1 regulate PGC-1alpha and mitochondria by Ca (2+) and AMPK/SIRT1. Nature 464, 1313-1319 (2010).
d) Yamauchi, T., et al., The fat-derived hormone adiponectin reverses insulin resistance associated with both lipoatrophy and obesity. Nature Med. 7, 941-946 (2001).
e) Yamauchi, T., et al., Adiponectin stimulates glucose utilization and fatty-acid oxidation by activating AMP-activated protein kinase. Nature Med. 8, 1288-1295 (2002).

### ii) Results

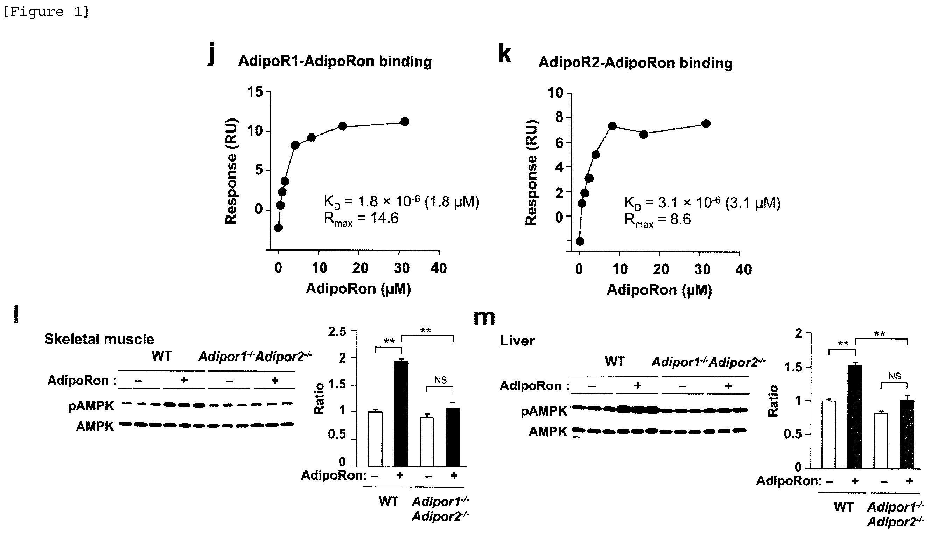

AdipoRon bound to both AdipoR1 and AdipoR2 with affinities (KD 1.8 and 3.1 *µ*M; Rmax 14.6 and 8.6 RU, respectively) (Fig. 1j, k).

### Test Example 3: Various kinds of pharmacological action in compounds of present invention

The results of various pharmacological tests performed on various kinds of pharmacological activities in Compound 73 (AdipoRon) and Compound 74 (No.112254) are summarized below.

### 1. Materials and methods

### (1) Mice

The original Adipor1^{+/-}/Adipor2^{+/-}mice (C57BL/6 and 129/Sv mixed background)^{a} were backcrossed with C57Bl/6 mice more than seven times. All experiments in this study were conducted on male littermates.

Mice were 6-10 week of age at the time of the experiment. They were housed in cages and maintained on a 12 hr light-dark cycle. For the experiment depicted in Figure 4&5, we used diets, standard chow (CE-2, CLEA Japan Inc.) with the following composition: 25.6% (wt/wt) protein, 3.8% fiber, 6.9% ash, 50.5% carbohydrates, 4% fat and 9.2% water. For all other experiments, high-fat diet 32 consisting of 25.5% (wt/wt) protein, 2.9% fiber, 4.0% ash, 29.4% carbohydrates, 32% fat and 6.2% water (CLEA Japan Inc.).

Male Lepr^{-/-} (aged 6-10 week) were purchased from Jackson Laboratory. When the mice in the same genotype received different treatments, they were randomly assigned to treatment groups.
a) Yamauchi, T., et al., Targeted disruption of AdipoR1 and AdipoR2 causes abrogation of adiponectin binding and metabolic actions. Nature Med. 13, 332-339 (2007).

### (2) C2C12 Cells

The mouse C2C12 myoblasts (ATCC, CRL-1772) were grown in 90% Dulbecco's modified high glucose Eagle's medium containing 10% (v/v) fetal bovine serum. When the cells were 80% confluent, the myoblasts were induced to differentiate into myotubes by replacing the medium with a low serum differentiation medium (98% Dulbecco's modified high glucose Eagle's medium, 2.5% (v/v) horse serum), which was changed daily.

### (3) Measurement of plasma concentrations of AdipoRon

We measured the plasma concentrations of AdipoRon by HPLC and TSQ Quantum Ultra (ThermoFisher) in C57BL/6 mice orally administered with 50 mg of AdipoRon per kg bodyweight. For quantitative analyses, 20 *µ*l of plasma was mixed with acetonitrile. The mixture was mixed vigorously for 30 sec and then centrifuged at 10,000 rpm for 3 min at 4°C. An aliquot of the supernatant was injected into the quantitative LC/MS system. Quantitative analyses were performed using an Accela HPLC system (ThermoFisher). The liquid chromatographic separations were carried out by using syncronis C18, 150 x 2.1 mm, 3 *µ*m. Column temperature was held at 40°C. The flow rate was 0.2 ml/min. Elution solvent A was 0.1% formic acid in water and solvent B was 0.1% formic acid in methanol. The chromatographic conditions were as follows: 0-5 min 45% A/55% B; 5-6.5 min 5% A/95% B, 6.5-10 min 45% A/55% B. A typical injection volume was 3 *µ*l. Quantitative analyses were performed using a TSQ Quantum Ultra triple-stage quadrupole mass spectrometer. Electro-spray ionization in positive ion mode (ESI+) was used for ionization and selective reaction monitoring (SRM) mode was chosen for detection. The optimized precursor ions pairs were m/z 429.251 →91.057 for AdipoRon. The optimized MS parameters were as follows: Ion Spray: 4,000 V, vaporizer temperature: 530°C, Sheath gas pressure: 50 psi, Auxiliary gas pressure: 5 psi, and Capillary temperature: 270°C. Collision pressure: 1.5 mTorr. Peak areas were automatically integrated using LCquan Version 4.5.6 (Thermo Fisher).

### (4) Primary hepatocytes

Hepatocytes were isolated from 8-week old male mice by the collagenase perfusion method^{a,b} with slight modifications. Cells were plated at 3.75 × 10⁵ cells/ml on collagen I-coated plates in Williams medium E supplemented with 10% (v/v) fetal bovine serum, 10 nM dexamethasone, and 10 nM insulin.
a) Yamauchi, T., et al., Targeted disruption of AdipoR1 and AdipoR2 causes abrogation of adiponectin binding and metabolic actions. Nature Med. 13, 332-339 (2007).
b) Tsuchida, A., et al., Insulin/Foxo1 pathway regulates expression levels of adiponectin receptors and adiponectin sensitivity. J. Biol. Chem. 279, 30817-30822 (2004).

### (5) Isolated muscles.

For analyses for insulin signaling and phosphorylation of AMPK, isolated gastrocnemius muscles were preincubated for 50 min at 37°C in Krebs-Ringer-Bicarbonate (KRB) buffer (117 mM NaCl, 2.5 mM CaCl₂, 1.2 mM KH₂PO₄, 1.2 mM MgSO₄, 24.6 mM NaHCO₃) containing 2 mM Pyruvate and were stimulated for 10 min with or without 100 nM insulin^{a}.
a) Bruning, J.C., et al., A muscle-specific insulin receptor knockout exhibits features of the metabolic syndrome of NIDDM without altering glucose tolerance. Mol. Cell 2, 559-569 (1998).

### (6) Respiratory Chain Complex I activity.

Respiratory chain complex I assays were carried out as described previously^{a-c}, with slight modifications. For complex I assay, mitochondria (0.5 mg/ml) was incubated in a solution of 1 mM EGTA and 20 mM Tris-HCl, pH 7.2, in the presence of 500 *µ*M NADH and 5 mM KCN. Complex I activity was assessed by the oxidation rate of NADH (measuring absorbance at 340 nm in a spectrophotometer) after addition of 100*µ*M decylubiquinone as an electron acceptor.
a) Brunmair, B., et al., Thiazolidinediones, like metformin, inhibit respiratory complex I: a common mechanism contributing to their antidiabetic actions? Diabetes 53, 1052-1059 (2004).
b) El-Mir, M.Y., et al., Dimethylbiguanide inhibits cell respiration via an indirect effect targeted on the respiratory chain complex I. J. Biol. Chem. 275, 223-228 (2000).
c) Hinke, S.A., et al., Methyl succinate antagonises biguanide-induced AMPK-activation and death of pancreatic beta-cells through restoration of mitochondrial electron transfer. Br. J. Pharmacol. 150, 1031-1043 (2007).

### (7) Mitochondrial content assay.

Mitochondrial content assays were carried out as described previously^{a,b}, with slight modifications. For quantification of mitochondrial content, we used mtDNAas previously described^{c}.
a) Iwabu, M., et al., Adiponectin and AdipoR1 regulate PGC-1alpha and mitochondria by Ca(2+) and AMPK/SIRT1. Nature 464, 1313-1319 (2010).
b) Civitarese, A.E. et al., Role of adiponectin in human skeletal muscle bioenergetics. Cell Metab. 4, 75-87 (2006).
c) Heilbronn, L.K., et al., Glucose tolerance and skeletal muscle gene expression in response to alternate day fasting. Obes. Res. 13, 574-581 (2005).

### (8) AMPK phosphorylation in vivo.

To study AMPK phosphorylation in vivo, we injected 50 mg of AdipoRon per kg bodyweight intravenously into mice through an inferior vena cava catheter^{a}.
a) Yamauchi, T., et al., Adiponectin stimulates glucose utilization and fatty-acid oxidation by activating AMP-activated protein kinase. Nature Med. 8, 1288-1295 (2002).

### (9) Real-time PCR, immunoprecipitation and immunoblotting

Real-time PCR was carried out according to the method described previously^{a,b,c}. Total RNA was prepared from cells or tissues with Trizol (Invitrogen) according to the manufacturer's instructions. We used a real-time PCR method to quantify the mRNAs¹⁹, with slight modifications.

Immunoblotting was carried out according to the method described previously by which the livers, muscles or white adipose tissues were freeze-clamped in liquid nitrogen in situ^{b,d,e}.

The procedures used for immunoprecipitation and immunoblotting have been described previously^{a,f}. Muscle, liver or cell lysates were incubated with 60 µl of protein G sepharose linked to antibodies against phosphotyrosine (4G10) (Merck Millipore). Beads were then washed, the resultant was boiled for 5 min in a sample buffer and the immunoprecipitates were subjected to immunoblotting. Phosphorylation and/or the protein levels of AMPKα (Cell signaling #2535, #2532), IRβ (Upstate 05-321, Santa Cruz sc-711), AKT (Cell signaling #9271, #9272), GSK3 (Cell signaling #5558, #9315) and tubulin (NeoMarkers RB-9281-PO) were determined as described^{a,g, h}.

Representative data from one of 2-5-independent experiments are shown.
a) Yamauchi, T., et al., Targeted disruption of AdipoR1 and AdipoR2 causes abrogation of adiponectin binding and metabolic actions. Nature Med. 13, 332-339 (2007).
b) Kubota, N., et al., Pioglitazone ameliorates insulin resistance and diabetes by both adiponectin-dependent and -independent pathways. J. Biol. Chem. 281, 8748-8755 (2006).
c) Yamauchi, T., et al., Cloning of adiponectin receptors that mediate antidiabetic metabolic effects. Nature 423, 762-769 (2003) .
d) Kamei, N., et al., Overexpression of MCP-1 in adipose tissues causes macrophage recruitment and insulin resistance. J. Biol. Chem. 281, 26602-26614 (2006).
e) Yamauchi, T., et al., The fat-derived hormone adiponectin reverses insulin resistance associated with both lipoatrophy and obesity. Nature Med. 7, 941-946 (2001).
f) Kubota, N., et al., Dynamic functional relay between insulin receptor substrate 1 and 2 in hepatic insulin signaling during fasting and feeding. Cell Metab. 8, 49-64 (2008).
g) Iwabu, M., et al., Adiponectin and AdipoR1 regulate PGC-1alpha and mitochondria by Ca (2+) and AMPK/SIRT1. Nature 464, 1313-1319 (2010).
h) Cross, D.A., et al., Inhibition of glycogen synthase kinase-3 by insulin mediated by protein kinase B. Nature 378, 785-789 (1995).

### (10) Lipid metabolism, lipid peroxidation and other materials

Skeletal muscle homogenates were extracted, and tissue triglyceride content was determined as described previously^{a-c}, with some modifications. To investigate whether oxidative stress was increased, we measured lipid peroxidation, a marker of oxidative injury, as represented by plasma thiobarbituric acid reactive substance (TBARS) as described previously⁴. Briefly, tissue samples were homogenized in a buffer solution containing 50 mM Tris-HCl (pH 7.4) and 1.15% KCl, and then centrifuged. The supernatant was used for the assay. The levels of lipid peroxidation in tissue homogenate and plasma were measured in terms of the amount of TBARS using the LPO test (Wako Pure Chemical Industries).

The measurements of [¹⁴C]CO₂ production from [1-¹⁴C] palmitic acid were performed using livers and skeletal muscles, as described previously^{d,e}.

Plasma glucose levels were determined by using a glucose B-test (Wako Pure Chemical Industries). Plasma insulin was measured with an insulin immunoassay (Shibayagi). Plasma NEFAs (Wako Pure Chemical Industries) were assayed by enzymatic methods.

All measurements were performed in blinded fashion.
a) Wu, H., et al., MEF2 responds to multiple calcium-regulated signals in the control of skeletal muscle fiber type. EMBO J. 19, 1963-1973 (2000).
b) Westfall, M.V. & Sayeed, M.M., Effect of Ca2(+)-channel agonists and antagonists on skeletal muscle sugar transport. Am. J. Physiol. 258, R462-468 (1990).
c) Merrill, G.F., Kurth, E.J., Hardie, D.G., & Winder, W.W., AICA riboside increases AMP-activated protein kinase, fatty acid oxidation, and glucose uptake in rat muscle. Am. J. Physiol. 273, E1107-1112 (1997).
d) Yamauchi, T., et al., Targeted disruption of AdipoR1 and AdipoR2 causes abrogation of adiponectin binding and metabolic actions. Nature Med. 13, 332-339 (2007).
e) Iwabu, M., et al., Adiponectin and AdipoR1 regulate PGC-1alpha and mitochondria by Ca (2+) and AMPK/SIRT1. Nature 464, 1313-1319 (2010).

### (11) Insulin resistance index.

The oral glucose tolerance test (OGTT) and insulin tolerance test (ITT) were conducted as previously described^{a,b} with slight modifications. The areas of the glucose and insulin curves were calculated by multiplying the cumulative mean height of the glucose values (1 mg/ml = 1 cm) and insulin values (lng/ml = 1 cm), respectively, by time (60 min = 1 cm)^{c,a}. The insulin resistance index was calculated from the product of the areas of glucose and insulin × 10⁻² in the glucose tolerance test. The results are expressed as the percentage of the value respect to control littermates.
a) Yamauchi, T., et al., The fat-derived hormone adiponectin reverses insulin resistance associated with both lipoatrophy and obesity. Nature Med. 7, 941-946 (2001).
b) Yamauchi, T., et al., Adiponectin stimulates glucose utilization and fatty-acid oxidation by activating AMP-activated protein kinase. Nature Med. 8, 1288-1295 (2002).
c) Yamauchi, T., et al., Targeted disruption of AdipoR1 and AdipoR2 causes abrogation of adiponectin binding and metabolic actions. Nature Med. 13, 332-339 (2007).

### (12) Hyperinsulinemic euglycemic clamp study

Clamp studies were carried out as described previously^{a,b}, with slight modifications. In brief, 2-3 days before the study, an infusion catheter was inserted into the right jugular vein under general anesthesia with sodium pentobarbital. Studies were performed on mice under conscious and unstressed conditions after a 6 h fast. A primed continuous infusion of insulin (Humulin R, Lilly) was given (10.0 milliunits/kg/min),and the blood glucose concentration, monitored every 5 min, was maintained at 120 mg/dl by administration of glucose (5 g of glucose per 10 ml enriched to 20% with [6,6-²H2] glucose (Sigma)) for 120 min. Blood was sampled via tail tip bleeds at 90, 105, and 120 min for determination of the rate of glucose disappearance (Rd). Rd was calculated according to nonsteady-state equations, and endogenous glucose production (EGP) was calculated as the difference between Rd and exogenous glucose infusion rates^{a,b,c}.
a) Kubota, N., et al., Pioglitazone ameliorates insulin resistance and diabetes by both adiponectin-dependent and -independent pathways. J. Biol. Chem. 281, 8748-8755 (2006).
b) Kamei, N., et al., Overexpression of MCP-1 in adipose tissues causes macrophage recruitment and insulin resistance. J. Biol. Chem. 281, 26602-26614 (2006)
c) Iwabu, M., et al., Adiponectin and AdipoR1 regulate PGC-lalpha and mitochondria by Ca (2+) and AMPK/SIRT1. Nature 464, 1313-1319 (2010).

### (13) Measurement of exercise capacity in mice

The treadmill exercise test regimen was 15 m/min for 20 min. Exercise endurance was assessed by dividing 20 min by the number of times a mouse was unable to avoid electrical shocks.

### (14) Statistical analysis

Results are expressed as mean ± s.e.m. Differences between two groups were assessed using unpaired two-tailed t-tests. Data involving three or more groups were assessed by analysis of variance (ANOVA).

### 2. Results

### (1) Phosphorylation induction of AMPK in mice skeletal muscle and liver

Intravenous injection of AdipoRon(50mg/kg body weight) significantly induced phosphorylation of AMPK in skeletal muscle and liver of wild-type(WT) mice but not in Adipor1/r2 double-knockout mice (Fig.1l, m), indicating that AdipoRon could activate AMPK in skeletal muscle and liver via AdipoR1/R2.

### (2) Amelioration of glucose intolerance and insulin resistance

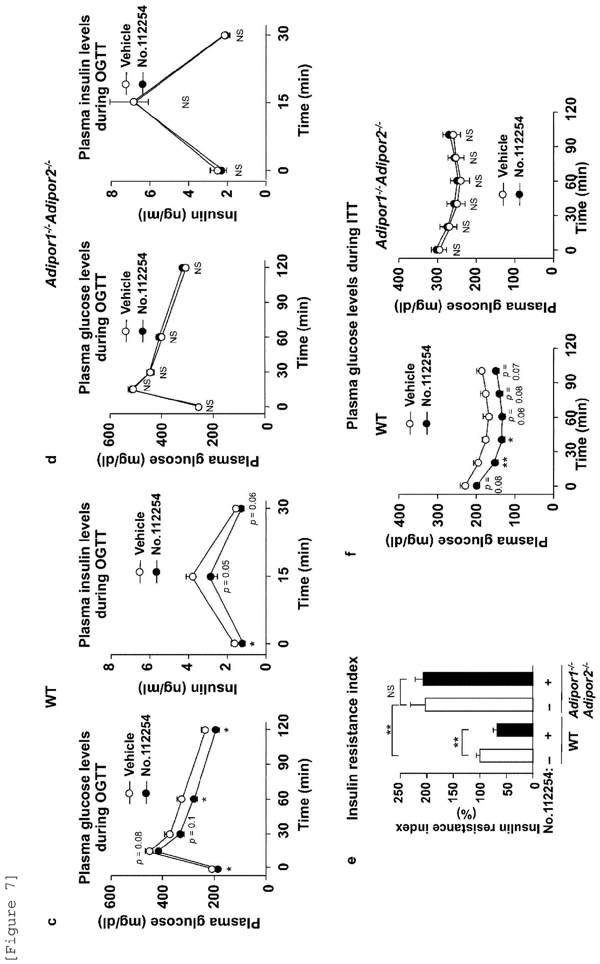

i) We measured plasma concentrations of AdipoRon in C57B6 WT mice after oral administration of 50 mg/kg of AdipoRon, and found that the maximal concentration (Cₘₐₓ) of AdipoRon was 11.8 µM (Fig. 2a).
ii) Insulin tolerance was determined by using HF diet-induced obese mice orally administered with AdipoRon for 10 days (50 mg/kg body weight) . Additionally, intake of HF diet did not significantly affect body weight (Fig. 2b) or food intake (Fig. 2c) in mice.
   The results showed significant decrease in fasting plasma glucose and insulin levels as well as glucose and insulin responses during oral glucose tolerance tests (OGTT) in WT mice administered with AdipoRon (Fig. 2d). The decrease in glucose levels in the face of reduced plasma insulin levels indicates improved insulin sensitivity (Fig. 2d, f).
   In the same test except for using Adipor1/r2-double knockout mice, HF diet-induced hyperglycemia and hyperinsulinemia were not ameliorated (Fig. 2e, f).
   In addition, it was confirmed that Compound 74 (No. 112254), like AdipoRon, could improve glucose intolerance as well as insulin resistance(Fig. 7c-f).
iii) Hyperinsulinemic euglycemic clamps were further conducted in mice on a HF diet after 10 days of administration of the compounds. The glucose infusion rate (GIR) was significantly increased (Fig. 2h, left), the endogenous glucose production (EGP) was significantly suppressed (Fig. 2h, middle), and the glucose disposal rate (Rd) was significantly increased (Fig. 2h, right) in AdipoRon-administered WT mice. None of these parameters were improved by AdipoRon in Adiporl/r2 double-knockout mice (Fig. 2i).

### (3) Effects on lipid metabolism

Oral administration of AdipoRon for 10 days (50 mg/kg body weight) reduced plasma concentrations of triglycerides (TG) and free fatty acid (FFA) in WT mice fed a HF diet (Fig. 2j, k), which were not observed in Adipor1/r2 double-knockout mice (Fig. 2j, k).

### (4) Activation on AdipoR1-AMPK-PGC-1α pathways in skeletal muscle

In skeletal muscle of WT mice orally administered with AdipoRon, expressions of genes involved in mitochondrial biogenesis such as Ppargc1a and estrogen-related receptor α (Esrra), mitochondrial DNA replication/translation such as mitochondrial transcription factor A (Tfam), and oxidative phosphorylation (OXPHOS) such as cytochrome c oxidase subunit II (mt-Co2) were increased (Fig. 3a) . AdipoRon also increased mitochondrial DNA content in the skeletal muscle of WT mice (Fig. 3b). These effects were totally obliterated in Adipor1/r2 double-knockout mice (Fig. 3a, b).

Additionally, AdipoRon increased the levels of type I fiber marker Troponin I (slow) (Tnni1) but not in Adipor1/r2 double-knockout mice (Fig. 3a).

Mice fed with a HF diet were challenged with involuntary physical exercise by treadmill running and then muscle endurance was assessed. AdipoRon administration significantly increased exercise endurance in WT mice, but not in Adipor1/r2 double-knockout mice (Fig. 3c) fed with a HF diet.
ii) AdipoRon significantly increased genes involved in fatty acid oxidation such as medium-chain acyl-CoA dehydrogenase (Acadm) (Fig. 3a). This result was associated with decreased TG content in the skeletal muscle of WT mice but not of Adipor1/r2 double-knockout mice fed with a HF diet.
iii) AdipoRon significantly increased expression levels for oxidative stress detoxifying genes such as manganese superoxide dismutase (Sod2) (Fig. 3a), and decreased oxidative stress markers such as thiobarbituric acid reactive substance (TBARS) (Fig. 3h) in the skeletal muscle of WT mice. But such effect was not observed in Adipor1/r2 double-knockout mice fed with a HF diet.

### (5) Activation of AdipoR1-AMPK pathway and AdipoR2-PPARα pathway in the liver

i) The activation of AdipoR1/AMPK pathway in the liver has been reported to reduce the expression of genes involved in hepatic gluconeogenesis such as Ppargc1a, phosphoenolpyruvate carboxykinase 1 (Pck1), and glucose-6-phosphatase(G6pc).
   AdipoRon significantly decreased the expressions of Ppargc1a, and G6pc in the liver of WT mice (Fig. 3d) but such effect was not observed in Adipor1/r2 double-knockout mice (Fig. 3d) fed with a HF diet.
ii) Activation of AdipoR2 can increase PPARα levels and activate PPARα pathways, leading to increased fatty acid oxidation and reduction of oxidative stress.

AdipoRon increased the expression levels of the gene encoding PPARα itself (Pparα) and its target genes, including genes involved in fatty-acid combustion such as acyl-CoA oxidase (Acox1), genes involved in energy dissipation such as uncoupling protein 2 (Ucp2), and genes encoding oxidative stress detoxifying enzymes such as catalase (Cat) in the liver of WT mice (Fig. 3d) but not in Adipor1/r2 double-knockout mice (Fig. 3d) fed a HF diet. In fact, AdipoRon significantly reduced TG content (Fig. 3e) and oxidative stress, as measured by TBARS (Fig. 3f) in the liver of WT mice but contrarily such effect was not observed in Adipor1/r2 double-knockout mice fed with a HF diet (Fig. 3e, f).

### (6) Reduced expression level of pro-inflammatory cytokines

AdipoRon reduced the expression level of the genes encoding pro-inflammatory cytokines such as TNFα (Tnf) and MCP-1 (Ccl2) in the liver of WT mice (Fig. 3d) but contrarily such effect was not observed in Adiporl/r2 double-knockout mice fed with a HF diet (Fig. 3d).

### (7) Reduced inflammation in white adipose tissue (WAT)

AdipoRon reduced the expression levels of the genes encoding pro-inflammatory cytokines such as Tnf, IL-6 (Il6), and Ccl2 in the WAT (white adipose tissue) of WT mice but contrarily such effect was not observed in Adipor1/r2 double-knockout mice fed with a HF diet (Fig. 3g).

Interestingly, AdipoRon reduced the levels of macrophage markers such as TBARS (Fig. 3h) and F4/80 (Emr1), and especially the levels of markers for classically activated M1 macrophages such as CD11c (Itgax) but not affect the levels of markers for the alternatively activated M2 macrophages such as CD206 (Mrcl) in the WAT of WT mice fed with a HF diet (Fig. 3g), whereas these changes were not observed in Adiporl/r2 double-knockout mice (Fig. 3g, h).

### (8) Anti-diabetic effects

2-week oral administration of AdipoRon (50 mg/kg body weight) to genetically obese rodent model, Lepr^{-/-} (also known as db/db) mice exhibited decreased plasma adiponectin concentration and decreased plasma glucose level in the same manner as in the case of intraperitoneal(IP) injection of adiponectin into db/db mice (Fig. 4a, left, right). Also, without affecting body weight, food intake, liver weight and WAT weight (Fig.4b-e), orally administered AdipoRon for 2 weeks significantly ameliorated glucose intolerance, insulin resistance and dyslipidemia (Fig. 4f-i).

### (9) Effects on TG content, oxidative stress, and inflammation in insulin target tissues in db/db mice

In the skeletal muscles of db/db mice orally administered with AdipoRon, significantly increased were the levels of genes involved in mitochondrial biogenesis functions and DNA content (Fig. 5a, b), and also the levels of Acadm t and Sod2 (Fig. 5a), which were associated with decreased TG content and TBARS (Fig. 5c, b), respectively.

In the liver, AdipoRon significantly decreased expressions of Ppargc1a, Pck1 and G6pc (Fig. 5e), but increased the expression of Pparα and its target genes (Fig. 5e), which were associated with significantly reduced TG content (Fig. 5f) and oxidative stress (Fig. 5g), and reduced expression levels of the genes encoding pro-inflammatory cytokines.

In the WAT, AdipoRon reduced the expression levels of the genes encoding pro-inflammatory cytokines, macrophage markers, especially the levels of markers for classically activated M1 macrophages, but not the levels of markers for the alternatively activated M2 macrophages. (Fig. 5h).

### (10) Prolonged lifespan of db/db mice on a HF diet

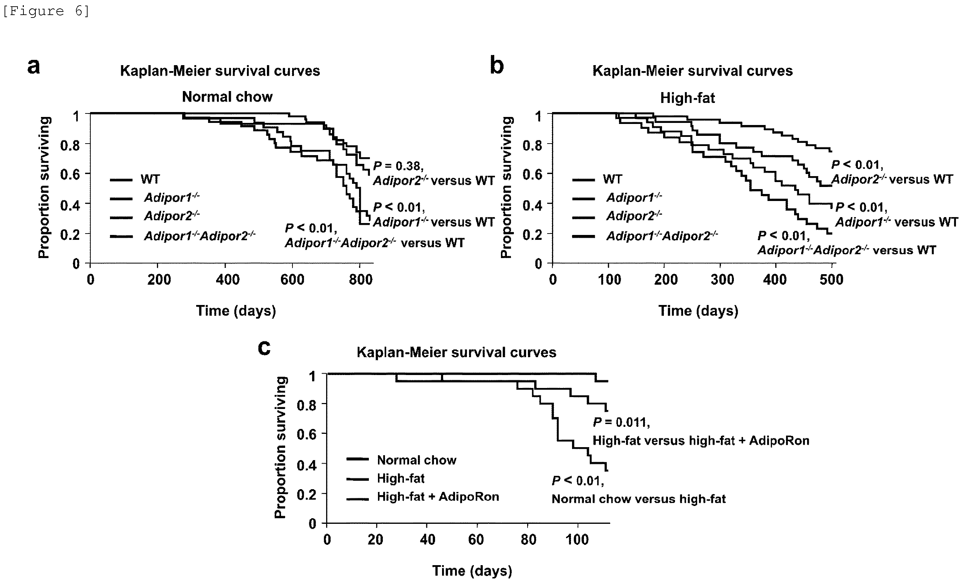

Wild type, Adipor1^{-/-}, Adipor2^{-/-}, and Adipor1^{-/-}Adipor2^{-/-} -knockout mice fed with (a) a normal chow diet (n = 50, 32, 29 and 35, respectively), or (b) a high-fat diet (n = 47, 33, 35 and 31, respectively) were used. The survival rate was recorded daily, and survival curves were plotted using the Kaplan-Meier method. As a result, Adipor1/r2 double-knockout mice showed shortened lifespan as compared with WT mice under both of the normal chow diet and the high-fat diet (Fig. 6a and b).

Because the HF diet has been reported to shorten the lifespan, we examined whether orally administered AdipoRon could prolong the shortened lifespan due to a HF diet or not.

That is, the db/db mice were randomly divided into 3 groups: a normal chow group (Normal chow, n = 20), high-fat group (High-fat, n = 20) and high-fat and additionally AdipoRon group (High-fat + AdipoRon, n = 20), which were treated with AdipoRon at a daily dose of 30 mg kg⁻¹ body weight. The survival rate was recorded daily.

As a result, lifespan of db/db mice on a HF diet was markedly shortened as compared with that on a normal chow. Surprisingly, AdipoRon significantly rescued the shortened lifespan of db/db mice on a HF diet (Fig. 6c).

## Claims

1. A compound of the following formula (1b) or a salt thereof: 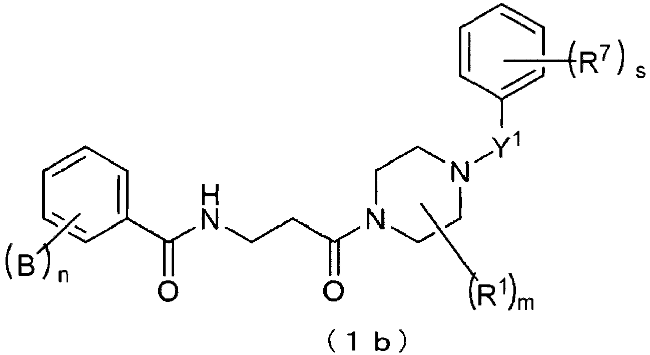 wherein
Y¹ represents - (CHR²)ₐ-, wherein R² represents H or a C₁₋₃ alkyl group, and a represents 1, or R² represents H or a C₁₋₇ alkyl group, and a represents 2, or -CO-;
R¹ represents a C₁₋₇ alkyl group, and a plurality of R¹s may be the same or different from each other;
m represents an integer of 0 to 4
B represents
-CO-R⁴; -O-R⁴, wherein R⁴ represents a C₁₋₇ alkyl group, a substituted or unsubstituted phenyl group, or a substituted or unsubstituted pyridyl group;
-CONR⁵R⁶, wherein R⁵ represents H, a C₃₋₇ cycloalkyl group, a C₁₋₄ alkoxy C₁₋₄ alkyl group, a norbornyl C₁₋₄ alkyl group, or a Ar²-C₁₋₄ alkyl group in which Ar² is a substituted or unsubstituted aryl group or a heteroaryl group, and R⁶ represents H, a C₁₋₇ alkyl group, a C₂₋₄ alkenyl group or a C₂₋₄ alkynyl group;
a C₁₋₇ alkyl group;
a C₃₋₇ cycloalkyl group;
a halo C₁₋₇ alkyl group;
a phenyl group; a halogen atom; -NO₂; or a group having a formula of 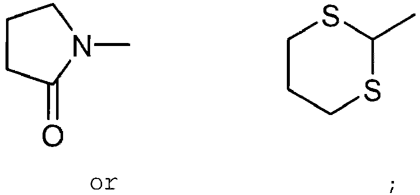
n represents an integer of 0 to 3, in which if n is 2 or more, Bs may be the same or different from each other;
R⁷ represents a C₁₋₄ alkyl group, a halo C₁₋₄ alkyl group, a halogen atom, a C₁₋₄ alkoxy group, or a hydroxyl group; and
s represents an integer of 0 to 3 (if it is 2 or more, then a plurality of R⁷ may be the same or different from each other).

2. The compound or salt thereof according to claim 1,
wherein Y¹ represents -CH₂-, and
wherein B is a C₁₋₇ alkyl group; a C₃₋₇ cycloalkyl group; a halogen atom or -NO₂.

3. A compound of the following formula or a salt thereof:

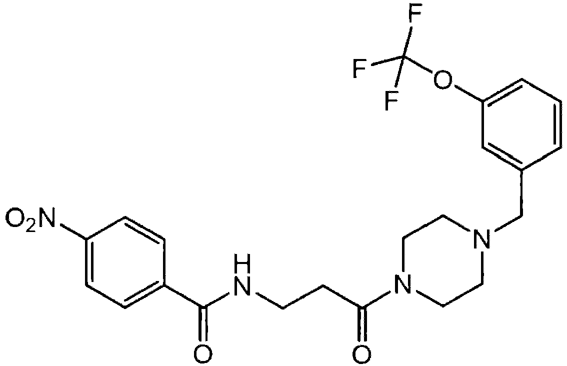

4. A medicament comprising the compound or a salt thereof according to any one of claims 1 to 3, and a pharmaceutically acceptable carrier.

5. The medicament according to claim 4 for use in preventing, treating or ameliorating a symptom or disease due to decreased production of adiponectin or decreased activation of adiponectin receptor.

6. The medicament for use according to claim 5, wherein the symptom or disease due to decreased production of adiponectin or deceased activation of adiponectin receptor is type II diabetes, hypertension, lipid metabolism disorder, mitochondrial dysfunction, lifestyle-related disease, malignant tumor due to lifestyle-related disease or obesity.

7. An activator of adiponectin receptor comprising the compound or a salt thereof according to any one of claims 1 to 3 as an active ingredient.

8. The activator of adiponectin receptor according to claim 7, wherein the adiponectin receptor is AdipoR1 or AdipoR2.

9. The compound or a salt thereof according to any one of claims 1 to 3 for use in prevention, amelioration, or treatment of a symptom or disease caused by decreased production of adiponectin or decreased activation of adiponectin receptor.

10. The compound or a salt thereof for use according to claim 9, wherein the symptom or, disease caused by decreased production of adiponectin or decreased activation of adiponectin receptor is type II diabetes, hypertension, lipid metabolism disorder, mitochondrial dysfunction, lifestyle-related disease, malignant tumor due to lifestyle-related disease, or obesity.

11. The compound or a salt thereof according to any one of claims 1 to 3 for use in activation of an adiponectin receptor.

## Patentansprüche

1. Verbindung der folgenden Formel (1b) oder ein Salz derselben: 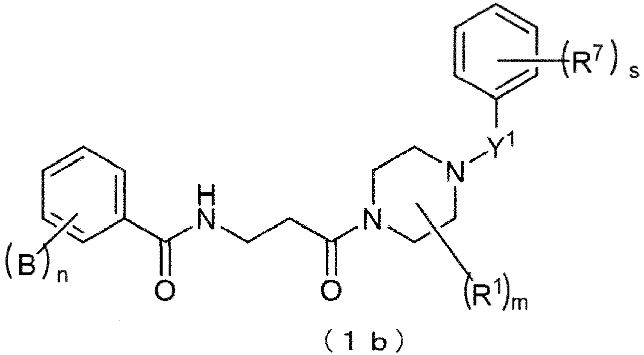
wobei Y¹ für -(CHR²)ₐ-, wobei R² für H oder eine C₁₋₃ Alkylgruppe steht und a für 1 steht oder R² für H oder eine C₁₋₇ Alkylgruppe steht und a für 2, oder für -CO- steht;
R¹ für eine C₁₋₇ Alkylgruppe steht und eine Vielzahl von R¹ gleich oder voneinander verschieden sein kann;
m für eine ganze Zahl von 0 bis 4 steht;
B für -CO-R⁴; -O-R⁴, wobei R⁴ für eine C₁₋₇ Alkylgruppe, eine substituierte oder unsubstituierte Phenylgruppe oder eine substituierte oder unsubstituierte Pyridylgruppe steht;
-CONR⁵R⁶, wobei R⁵ für H, eine C₃₋₇ Cycloalkylgruppe, eine C₁₋₄ Alkoxy-C₁₋₄ Alkylgruppe, eine Norbornyl-C₁₋₄ Alkylgruppe oder eine Ar²-C₁₋₄ Alkylgruppe steht, bei der Ar² für eine substituierte oder unsubstituierte Arylgruppe oder eine Heteroarylgruppe steht, und R⁶ für H, eine C₁₋₇ Alkylgruppe, eine C₂₋₄ Alkenylgruppe oder eine C₂₋₄ Alkinylgruppe steht;
eine C₁₋₇ Alkylgruppe; eine C₃₋₇ Cycloalkylgruppe; eine Halo-C₁₋₇ Alkylgruppe; eine Phenylgruppe; ein Halogenatom; -NO₂ oder eine Gruppe mit einer Formel von 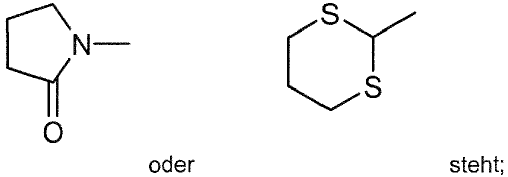 steht;
n für eine ganze Zahl von 0 bis 3 steht, bei der, wenn n 2 oder mehr ist, B gleich oder voneinander verschieden sein kann;
R⁷ für eine C₁₋₄ Alkylgruppe, eine Halo-C₁₋₄ Alkylgruppe, ein Halogenatom, eine C₁₋₄ Alkoxygruppe oder eine Hydroxylgruppe steht; und
s für eine ganze Zahl von 0 bis 3 steht (wenn s 2 oder mehr ist, dann kann eine Vielzahl von R⁷ gleich oder voneinander verschieden sein).

2. Verbindung oder ein Salz derselben nach Anspruch 1, wobei Y¹ für -CH₂- steht und wobei B für eine C₁₋₇ Alkylgruppe, eine C₃₋₇ Cycloalkylgruppe, ein Halogenatom oder-NO₂ steht.

3. Verbindung der folgenden Formel oder ein Salz derselben:

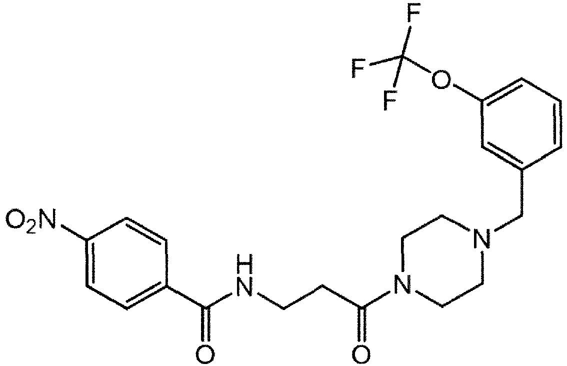

4. Arzneimittel, umfassend die Verbindung oder ein Salz derselben nach einem der Ansprüche 1 bis 3 und einen pharmazeutisch verträglichen Träger.

5. Arzneimittel nach Anspruch 4, zur Verwendung bei der Vorbeugung, Behandlung oder der Linderung eines Symptoms oder einer Erkrankung aufgrund einer verringerten Erzeugung von Adiponectin oder einer verringerten Aktivierung des Adiponectin-Rezeptors.

6. Arzneimittel zur Verwendung nach Anspruch 5, wobei das Symptom oder die Erkrankung aufgrund der verringerten Erzeugung von Adiponectin oder der verringerten Aktivierung des Adiponectin-Rezeptors Typ-II-Diabetes, Bluthochdruck, eine Fettstoffwechsel-Erkrankung, mitochondriale Dysfunktion, eine Lebensstil-bedingte Erkrankung, ein maligner Tumor aufgrund einer Lebensstil-bedingten Erkrankung oder Adipositas ist.

7. Aktivator des Adiponectin-Rezeptors, der die Verbindung oder ein Salz derselben nach einem der Ansprüche 1 bis 3 als Wirkstoff umfasst.

8. Aktivator des Adiponectin-Rezeptors nach Anspruch 7, wobei der Adiponectin-Rezeptor AdipoR1 oder AdipoR2 ist.

9. Verbindung oder ein Salz derselben nach einem der Ansprüche 1 bis 3, zur Verwendung bei der Vorbeugung, Linderung oder der Behandlung eines Symptoms oder einer Erkrankung, die durch eine verringerte Erzeugung von Adiponectin oder eine verringerte Aktivierung des Adiponectin-Rezeptors hervorgerufen wird.

10. Verbindung oder ein Salz derselben zur Verwendung nach Anspruch 9, wobei das Symptom oder die Erkrankung, die durch eine verringerte Erzeugung von Adiponectin oder eine verringerte Aktivierung des Adiponectin-Rezeptors hervorgerufen wird, Typ-II-Diabetes, Bluthochdruck, eine Fettstoffwechsel-Erkrankung, mitochondriale Dysfunktion, eine Lebensstil-bedingte Erkrankung, ein maligner Tumor aufgrund einer Lebensstil-bedingten Erkrankung oder Adipositas ist.

11. Verbindung oder ein Salz derselben nach einem der Ansprüche 1 bis 3, zur Verwendung bei der Aktivierung eines Adiponectin-Rezeptors.

## Revendications

1. Composé de formule (1b) suivante ou un sel de celui-ci : 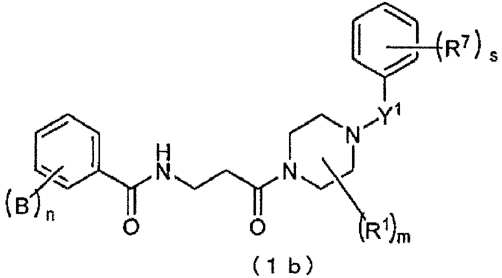 dans lequel
Y¹ représente -(CHR²)ₐ- où R² représente H ou un groupe alkyle en C₁ à C₃, et a représente 1, ou R² représente H ou un groupe alkyle en C₁ à C₇, et a représente 2, ou -CO- ;
R¹ représente un groupe alkyle en C₁ à C₇, et plusieurs R¹ peuvent être identiques ou différents ;
m représente un entier de 0 à 4
B représente -CO-R⁴ ; -O-R⁴, où R⁴ représente un groupe alkyle en C₁ à C₇, un groupe phényle substitué ou non substitué, ou un groupe pyridyle substitué ou non substitué ;
-CONR⁵R⁶ où R⁵ représente H, un groupe cycloalkyle en C₃ à C₇, un groupe (alcoxy en C₁ à C₄) alkyle en C₁ à C₄, un groupe norbornyl-alkyle en C₁ à C₄, ou un groupe Ar²-alkyle en C₁ à C₄ dans lequel Ar² est un groupe aryle substitué ou non substitué ou un groupe hétéroaryle, et R⁶ représente H, un groupe alkyle en C₁ à C₇, un groupe alcényle en C₂ à C₄ ou un groupe alcynyle en C₂ à C₄ ;
un groupe alkyle en C₁ à C₇ ;
un groupe cycloalkyle en C₃ à C₇ ;
un groupe halogénoalkyle en C₁ à C₇ ;
un groupe phényle ; un atome d'halogène ; -NO₂ ; ou un groupe de formule 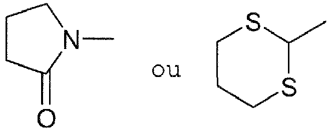
n représente un entier de 0 à 3 et, si n vaut 2 ou plus, les B peuvent être identiques ou différents ;
R⁷ représente un groupe alkyle en C₁ à C₄, un groupe halogénoalkyle en C₁ à C₄, un atome d'halogène, un groupe alcoxy en C₁ à C₄, ou un groupe hydroxyle ; et
s représente un entier de 0 à 3 (s'il vaut 2 ou plus, alors plusieurs R⁷ peuvent être identiques ou différents) .

2. Composé ou sel de celui-ci selon la revendication 1,
dans lequel Y¹ représente -CH₂-, et
dans lequel B est un groupe alkyle en C₁ à C₇ ; un groupe cycloalkyle en C₃ à C₇ ; un atome d'halogène ou -NO₂.

3. Composé de formule suivante ou un sel de celui-ci :

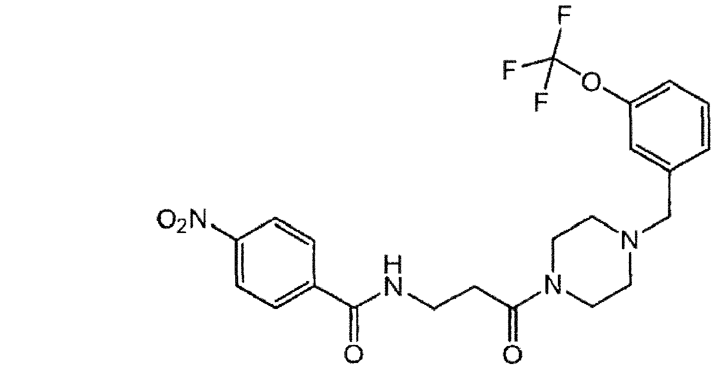

4. Médicament comprenant le composé ou un sel de celui-ci selon l'une quelconque des revendications 1 à 3, et un véhicule pharmaceutiquement acceptable.

5. Médicament selon la revendication 4, pour une utilisation dans la prévention, le traitement ou l'amélioration d'une maladie ou d'un symptôme dû à une production réduite d'adiponectine ou à une activation réduite de récepteur d'adiponectine.

6. Médicament pour une utilisation selon la revendication 5, dans lequel la maladie ou le symptôme dû à une production réduite d'adiponectine ou à une activation réduite de récepteur d'adiponectine est un diabète de type II, une hypertension, un trouble du métabolisme des lipides, un dysfonctionnement mitochondrial, une maladie liée au style de vie, une tumeur maligne due à une maladie liée au style de vie, ou l'obésité.

7. Activateur de récepteur d'adiponectine comprenant le composé ou un sel de celui-ci selon l'une quelconque des revendications 1 à 3 en tant que principe actif.

8. Activateur de récepteur d'adiponectine selon la revendication 7, dans lequel le récepteur d'adiponectine est AdipoR1 ou AdipoR2.

9. Composé ou un sel de celui-ci selon l'une quelconque des revendications 1 à 3 pour une utilisation dans la prévention, le traitement ou l'amélioration d'une maladie ou d'un symptôme dû à une production réduite d'adiponectine ou à une activation réduite de récepteur d'adiponectine.

10. Composé ou un sel de celui-ci pour une utilisation selon la revendication 9, dans lequel la maladie ou le symptôme dû à une production réduite d'adiponectine ou à une activation réduite de récepteur d'adiponectine est un diabète de type II, une hypertension, un trouble du métabolisme des lipides, un dysfonctionnement mitochondrial, une maladie liée au style de vie, une tumeur maligne due à une maladie liée au style de vie, ou l'obésité.

11. Composé ou un sel de celui-ci selon l'une quelconque des revendications 1 à 3, pour une utilisation dans l'activation d'un récepteur d'adiponectine.
